(19) Europäisches Patentamt
European Patent Office
Office européen des brevets

(11) EP 1 668 025 B1

(12) **EUROPEAN PATENT SPECIFICATION**

(45) Date of publication and mention of the grant of the patent:
**25.06.2014 Bulletin 2014/26**

(21) Application number: **04789044.7**

(22) Date of filing: **23.09.2004**

(51) Int Cl.:
*C07H 21/04* (2006.01) *C07H 21/02* (2006.01)
*C12N 15/85* (2006.01) *C12N 15/86* (2006.01)
*C07K 16/00* (2006.01) *C12Q 1/70* (2006.01)
*C12Q 1/68* (2006.01) *A61K 35/76* (2006.01)
*C07K 14/005* (2006.01) *C07K 14/085* (2006.01)
*C12N 7/00* (2006.01) *C12N 15/11* (2006.01)

(86) International application number:
**PCT/US2004/031504**

(87) International publication number:
**WO 2005/030139 (07.04.2005 Gazette 2005/14)**

(54) **SENECA VALLEY VIRUS BASED COMPOSITIONS AND METHODS FOR TREATING DISEASE**

ZUSAMMENSETZUNGEN AUF BASIS DES SENECA VALLEY VIRUS UND VERFAHREN ZUR BEHANDLUNG VON KRANKHEITEN

COMPOSITIONS A BASE DU VIRUS DE "SENECA VALLEY" ET METHODES DE TRAITEMENT DE LA MALADIE

(84) Designated Contracting States:
**AT BE BG CH CY CZ DE DK EE ES FI FR GB GR HU IE IT LI LU MC NL PL PT RO SE SI SK TR**

(30) Priority: **26.09.2003 US 506182 P**

(43) Date of publication of application:
**14.06.2006 Bulletin 2006/24**

(73) Proprietor: **Novartis AG**
**4056 Basel (CH)**

(72) Inventors:
- **HALLENBECK, Paul, L.**
  **Malvern, PA 19355 (US)**
- **HAY, Carl, M.**
  **Damascus, MD 20872 (US)**
- **GANESH, Shanthi**
  **San Francisco, CA 94107 (US)**
- **POLICE, Seshidar, Reddy**
  **Chester Springs, PA 19425 (US)**
- **XU, Ling**
  **Boyds, MD 20841 (US)**
- **YANG, Jingping**
  **North Potomac, MD 20878 (US)**
- **CHENG, Cheng**
  **Rockville, MD 20852 (US)**

(74) Representative: **Lawrence, John et al**
**Barker Brettell LLP**
**100 Hagley Road**
**Edgbaston**
**Birmingham**
**B16 8QQ (GB)**

(56) References cited:
**WO-A1-99/08692**

- **DATABASE UniProt [Online] 1 October 2002 (2002-10-01), "Polyprotein." XP002427168 retrieved from EBI accession no. UNIPROT: Q8JW41 Database accession no. Q8JW41**
- **DATABASE UniProt [Online] 1 May 2000 (2000-05-01), "Polyprotein." XP002427169 retrieved from EBI accession no. UNIPROT: Q9QCE4 Database accession no. Q9QCE4**
- **SMYTH M ET AL: "Bovine enterovirus as an oncolytic virus: Foetal calf serum facilitates its infection of human cells" INTERNATIONAL JOURNAL OF MOLECULAR MEDICINE, vol. 10, no. 1, July 2002 (2002-07), pages 49-53, XP002427153 ISSN: 1107-3756**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ADACHI MASAZUMI ET AL: "Destruction of human retinoblastoma after treatment by the E variant of encephalomyocarditis virus." JOURNAL OF NEURO-ONCOLOGY MAY 2006, vol. 77, no. 3, May 2006 (2006-05), pages 233-240, XP002427154 ISSN: 0167-594X
- RUSSELL S.J.ET AL.: 'RNA viruses as Virotherapy agents.' CANCER GENE THERAPY, [Online] vol. 9, November 2002, pages 961 - 966, XP002988849
- HUANG T. ET AL.: 'Oncolysis of hepatic Metastasis of Colorectal Cancer by Recomb' MOLECULAR THERAPY, [Online] vol. 8, no. 3, September 2003, pages 434 - 440, XP002988850
- J.T. MULLEN ET AL.: 'Viral Oncolysis' THE ONCOLOGIST. vol. 7, April 2002, pages 106 - 119, XP002988851
- GROMEIER M ET AL.: 'Intergenic poliovirus recombinants for the treatment of malignant glioma' PNAS vol. 97, no. 12, June 2000, pages 6803 - 6808, XP002259551
- SPYROU V. ET AL.: 'Transmission and Pathogenicity of encephalomyocarditis virus (EMCV) AMONGST RATS.' VET. RES. vol. 35, January 2004, pages 113 - 122, XP002988852
- BAKONYI T. ET AL.: 'Complete genome analysis and molecar characteriyation of usutu virus that emerged in Austria in 2001 Comparison with South African Strain SAAR-1776 and other flavivirus.' VIROLOGY vol. 328, October 2004, pages 301 - 310, XP004581383

## Description

This disclosure contains material that is subject to copyright protection. The copyright owner has no objection to the facsimile reproduction by anyone of the patent document or the patent disclosure, as it appears in the U.S. Patent and Trademark Office patent file or records, but otherwise reserves any and all copyright rights.

## BACKGROUND OF THE INVENTION

Virotherapy holds great promise for treating cancer. Oncolytic viruses, which aim to specifically bind and kill cancer cells, whether native and/or engineered, may be more efficacious and less toxic than alternative treatments, such as chemotherapy and radiation. In addition, oncolytic virus therapy is the only therapy known that can amplify the therapeutic at the pharmacologically desired site.

A key aspect of cancer therapy is to achieve a high rate of killing of cancer cells versus normal cells. Accomplishing this goal has been difficult for many reasons, including the wide array of cell types involved, the systemic dissemination of cancer cells due to metastases, and the narrow biological differences between normal and cancer cells. While progress has been made, much still needs to be done to improve upon current cancer therapies.

In the past, surgeons have tried to remove tumors surgically without substantially harming the patient. Even complete removal of a primary tumor does not ensure survival since earlier metastases to unknown sites in the body are left undetected. There is also some research suggesting that surgical intervention may enhance the growth of distant metastases due to removal of tumor cells producing angiogenesis inhibitors. Finally, in many cases, the tumor grows back at the original site after surgical removal. Radiation aims to selectively destroy the most rapidly proliferating cells at the expense of the others. However, tumor cells can escape radiation therapy either by becoming resistant or by being in a non-dividing state during treatment. In addition, radiation is not always selective in that many normal cells are actively dividing and killed by the treatment (gastrointestinal cells, hair follicles, etc.).

Like radiation, chemotherapy is not completely selective and thus destroys many normal cells, and does not kill all tumor cells due to drug resistance and/or division state of the cell. Thus, chemotherapy and radiation therapies exploit a small differential sensitivity that exists between normal and cancer cells, giving them a narrow therapeutic index. A small therapeutic index is clearly an undesirable property of any modality to treat cancer. Therefore, novel cancer therapeutic approaches overcoming these limitations are desired.

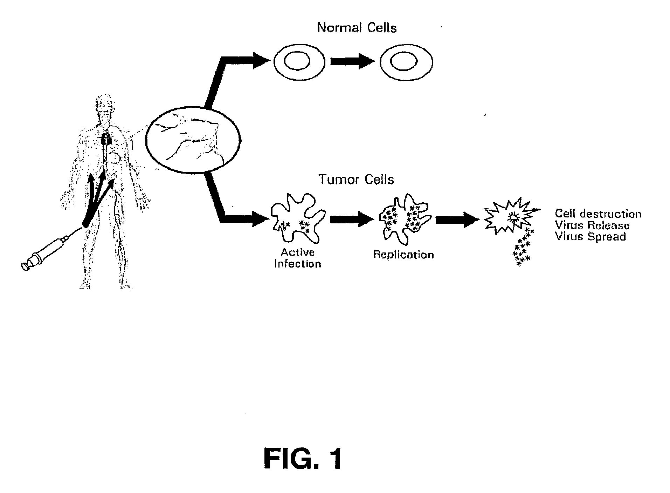

One such novel approach is oncolytic virus therapy. Initially, replication-defective viruses carrying cytotoxic transgenes were utilized in attempts to treat cancer. However, they were found to be inefficient in transduction of tumors and not adequately selective toward cancers. To overcome this limitation, viruses were either modified to replicate selectively in tumor cells or viruses were discovered to have natural tumor-selective properties. These oncolytic viruses thus had the properties to replicate, spread, and kill tumor cells selectively through a tumor mass by locally injecting the virus or by systemically delivering the virus (Figure 1).

Despite the early promise of this newly defined class of anti-cancer therapeutics, several limitations remain that may limit their use as a cancer therapeutic. Therefore, there is an ongoing need for novel oncolytic viruses that can be utilized for cancer therapy.

## SUMMARY OF THE INVENTION

A novel RNA picornavirus has been discovered (hereafter referred to as Seneca Valley virus ("SVV")) whose native properties include the ability to selectively kill some types of tumors. As demonstrated below in the examples, SVV selectively kills tumor lines with neurotropic properties, in most cases with a greater than 10,000 fold difference in the amount of virus necessary to kill 50% of tumor cells versus normal cells *(i.e.,* the $EC_{50}$ value). This result also translates *in vivo*, where tumor explants in mice are selectively eliminated. Further, *in vivo* results indicate that SVV is not toxic to normal cells, in that up to $1x10^{14}$ vp/kg (vector or virus particles per kilogram) systemically administered causes no mortality and no visible clinical symptoms in immune deficient or immune competent mice.

SW elicits efficacy at doses as low as $1x10^8$ vp/kg; therefore, a very high therapeutic index of >100,000 is achieved. Efficacy is very robust in that 100% of large pre-established tumors in mice can be completely eradicated *(see* Example 11). This efficacy may be mediated with a single systemic injection of SVV without any adjunct therapy. Furthermore, SW injected mice show neither clinical symptoms nor recurrence of tumors for at least 200 days following injection. SW can also be purified to high titer and can be produced at >200,000 virus particles per cell in permissive cell lines. SVV-based viral therapy therefore shows considerable promise as a safe, effective and new line of treatment for selected types of cancers. Further, SVV has a small and easily manipulatable genome, simple and fast lifecycle, and a well-understood capsid, and thus is amenable to modification. These properties, at least in part, allow for methods that generate modified SVVs that have new cell or tissue specific tropisms, such that SVV-based therapy can be directed to new tumor types resistant to infection by the original SVV isolate.

Accordingly, the present invention provides an isolated nucleic acid comprising a nucleic acid sequence having at least 95% or 99% sequence identity to SEQ ID NOs: 1, 3, 5, 7, 9, 11, 13, 15, 17, 19, 21, or a contiguous portion of any one of these sequences that is at least 50 nucleotides in length.

The present invention also provides an isolated polypeptide encoded by a nucleic acid having at least 95% or 99% sequence identity to a nucleic acid sequence comprising SEQ ID NOs: 1, 3, 5, 7, 9, 11, 13, 15, 17, 19, 21, or to a contiguous portion of any one of these sequences that is at least 50 nucleotides in length.

In one aspect, the invention provides an isolated polypeptide comprising an amino acid sequence having at least 95% or 99% sequence identity to SEQ ID NOs: 2,4, 6, 8, 10, 12, 14, 16, 18, 20, 22.

In another aspect, the invention provides an isolated antibody which specifically binds a polypeptide comprising an amino acid sequence having at least 95% or 99% sequence identity to SEQ ID NOs: 2, 4, 6, 8, 10, 12, 14, 16, 18, 20, 22. The isolated antibody can be generated such that it binds to any protein epitope or antigen of SEQ ID NO:2. Further, the antibody can be a polyclonal antibody, a monoclonal antibody or a chimeric antibody.

In one aspect, the invention provides an isolated SW or derivative or relative thereof, having a genomic sequence comprising a sequence that is at least 80%, 85%, 90%, 95% or 99% identical to SEQ ID NO: 1.

In another asepct, the invention provides an isolated virus having all the identifying characteristics and nucleic acid sequence of American Type Culture Collection (ATCC) Patent Deposit number PTA-5343. Some of the viruses of the present invention are directed to the PTA-5343 isolate, variants, homologues, relatives, derivatives and mutants of the PTA-5343 isolate, and variants, homologues, derivatives and mutants of other viruses that are modified in respect to sequences of SW (both wild-type and mutant) that are determined to be responsible for its oncolytic properties.

The present invention further provides an isolated SW comprising the following characteristics: a single stranded RNA genome (positive (+) sense strand) of ~7.5 kilobases (kb); a diameter of ~27 nanometers (nm); a capsid comprising at least 3 proteins that have approximate molecular weights of about 31 kDa, 36 kDa and 27 kDa; a buoyant density of approximately 1.34 g/mL on cesium chloride (CsCl) gradients; and replication competence in tumor cells wherein the 31 kDa capsid protein (VP1) can comprise an amino acid sequence that is at least 80%, 85%, 90%, 95% or 99% identical to SEQ ID NO:8; the 36 kDa capsid protein (VP2) can comprise an amino acid sequence that is at least 80%, 85%, 90%, 95% or 99% identical to SEQ ID NO:4; and the 27 kDa capsid protein (VP3) can comprise an amino acid sequence that is at least 80%, 85%, 90%, 95% or 99% identical to SEQ ID NO:6.

The isolated SW derivative or relative may comprise the following characteristics: replication competence in tumor cells, tumor-cell tropism, and lack of cytolysis in normal cells. In another aspect, the virus is replication competent in tumor cell types having neuroendocrine properties.

In other aspects, the present invention provides: a pharmaceutical composition comprising an effective amount of a virus of the invention and a pharmaceutically acceptable carrier.

In yet another aspect, the invention provides a method of purifying a virus of the invention, comprising: infecting a cell with the virus; harvesting cell lysate; subjecting cell lysate to at least one round of gradient centrifugation; and isolating the virus from the gradient.

In another aspect, the invention provides a virus or derivative thereof for use to treat cancer wherein the virus has a genomic sequence that comprises a sequence that is at least 95% or 99% identical to SEQ ID NOs: 1, 3, 5, 7, 9, 11, 13, 15, 17, 19, 21 or to a portion of SEQ ID NO: 1.

In the methods directed to cancer disclosed herein, the virus can be a picornavirus. The picornavirus can be a cardiovirus, erbovirus, aphthovirus, kobuvirus, hepatovirus, parechovirus, teschovirus, enterovirus or rhinovirus. The cardiovirus can be selected from the group consisting of: vilyuisk human encephalomyelitis virus, Theiler's murine encephalomyelitis virus, encephalomyocarditis virus and SW. The encephalomyocarditis virus can be selected from the group of isolates consisting of: CA-131395, LA-97-1278, IL-92-48963, IA-89-47752, NJ-90-10324, MN-88-36695, and NC-88-23626. The SW can be a virus having the ATCC deposit number PTA-5343 or a virus comprising a nucleic acid sequence that is at least 95% or 99% identical to SEQ ID NO:1.

The present invention also provides a virus of the invention for use in a treatment comprising killing an abnormally proliferative cell. In one aspect, the abnormally proliferative cell is a tumor cell. In various aspects of this use, the tumor cell is selected from the group consisting of: human small cell lung cancer, human retinoblastoma, human neuroblastoma, human medulloblastoma, mouse neuroblastoma, Wilms' tumor, and human non-small cell lung cancer.

The present invention also provides a method for making an oncolytic cardiovirus, the method comprising: (a) comparing a SW genomic sequence with a test virus genomic sequence, wherein the SVV genomic sequence comprises a sequence that is at least 95% identical to SEQ ID NO: 1; (b) identifying at least a first amino acid difference between a polypeptide encoded by the SW genomic sequence and a polypeptide encoded by the test virus genomic sequence; (c) mutating the test virus genomic sequence such that the polypeptide encoded by the test virus genomic sequence has at least one less amino acid difference to the polypeptide encoded by the SW genomic sequence; (d) transfecting the mutated test virus genomic sequence into a tumor cell; and (e) determining whether the tumor cell is lytically infected by the mutated test virus genomic sequence. In one aspect, the amino acid(s) mutated in the test virus are amino acids in a structural region, such as in the capsid encoding region. In another aspect, the amino acids mutated in the test virus are amino acids in a non-structural region.

In one aspect of the method for making an oncolytic virus, the SVV genomic sequence is obtained from the isolated SVV having the ATCC deposit number PTA-5343 or from a virus comprising a sequence that is at least 95% or 99% identical to SEQ ID NO: 1 In another aspect of this method, the step of mutating the test virus genomic sequence comprises mutating a cDNA having the test virus genomic sequence. In another aspect of this method, the step of transfecting the mutated test virus genomic sequence comprises transfecting RNA, wherein the RNA is generated from the cDNA having the mutated test virus genomic sequence.

In another aspect of the method for making an oncolytic cardiovirus, the test virus is a picornavirus. The test picornavirus can be a teschovirus, enterovirus, rhinovirus, cardiovirus, erbovirus, apthovirus, kobuvirus, hepatovirus, parechovirus or teschovirus. In another aspect, the test virus is a cardiovirus. In another aspect, the amino acid differences identified in the methods for making an oncolytic virus are between a SVV capsid protein and a test virus capsid protein sequence. In another aspect for making an oncolytic virus, the test virus genomic sequence is selected from the group consisting of: Vilyuisk human encephalomyelitis virus, Theiler's murine encephalomyelitis virus, and encephalomyocarditis virus. In another aspect, the test virus genomic sequence is selected from an encephalomyocarditis virus. The encephalomyocarditis virus can be selected from the group of isolates consisting of: CA-131395, LA-97-1278, IL-92-48963, IA-89-47752, NJ-90-10324, MN-88-36695, and NC-88-23626. In yet another aspect, the encephalomyocarditis virus or any other test virus can be selected from an isolate having a nucleic acid sequence comprising at least 95% or 99% sequence identity to SVV of ATCC deposit number PTA-5343 or SEQ ID NO: 1.

In another aspect of the method for making an oncolytic cardiovirus, the amino acid difference between the test virus and SVV is in a capsid protein region of SW, wherein the amino acid difference is aligned within SVV SEQ ID NO:4, 6 or 8.

The present invention also provides a method for making a mutant virus having an altered cell-type tropism, the method comprising: (a) creating a library of viral mutants comprising a plurality of nucleic acid sequences wherein the library of viral mutants is created from a parental sequence comprising a sequence that is at least 95% identical to SEQ ID NO: 1; (b) transfecting the library of viral mutants into a permissive cell, such that a plurality of mutant viruses is produced; (c) isolating the plurality of mutant viruses; (d) incubating a non-permissive cell with the isolated plurality of mutant viruses; and (e) recovering a mutant virus that was produced in the non-permissive cell, thereby making a mutant virus having an altered tropism. In one aspect, this method further comprises the steps of: (f) incubating the recovered mutant virus in the non-permissive cell; and (g) recovering a mutant virus that that was produced in the non-permissive cell. In another aspect, the method further comprises iteratively repeating steps (f) and (g).

In one aspect of the method for making a mutant virus having an altered cell-type tropism, the incubating is conducted in a multi-well high-throughput platform wherein the platform comprises a different non-permissive cell-type in each well. In this aspect, the method can further comprise screening the platform to identify which wells contain a mutant virus that kills the cells. In another aspect, the screening is conducted by analyzing light absorbance in each well.

In another aspect of the method for making a mutant virus having an altered cell-type tropism, the non-permissive cell is a tumor cell.

In another aspect of the method for making a mutant virus having an altered cell-type tropism, the step of creating the library of viral mutants comprises: (i) providing a polynucleotide having a sequence identical to a portion of a genomic sequence of a virus; (ii) mutating the polynucleotide in order to generate a plurality of different mutant polynucleotide sequences; and (iii) ligating the plurality of mutated polynucleotides into a vector having the genomic sequence of the virus except for the portion of the genomic sequence of the virus that the polynucleotide in step (i) contains, thereby creating the library of viral mutants. In one aspect, the genomic sequence of a virus is from a picornavirus. In another aspect, the genomic sequence of a virus comprises a sequence that is at least 95% or 99% identical to SEQ ID NO: 1.

In another aspect, in the step of creating the library of viral mutants, the mutating of step (ii) is conducted by random insertion of nucleotides into the polynucleotide. In one aspect, the random insertion of nucleotides is conducted by trinucleotide-mutagenesis (TRIM). In another asepct, at least a portion of the nucleotides inserted into the polynucleotide encodes an epitope tag. In another aspect, in the step of creating the library of viral mutants, the mutating of step (ii) is conducted in a capsid encoding region of the polynucleotide.

For all the methods of the present disclosure, the virus can be a picornavirus. The picornavirus can be a cardiovirus, erbovirus, aphthovirus, kobuvirus, hepatovirus, parechovirus, teschovirus, entrovirus, or rhinovirus. The cardiovirus can be SW. The SW can be a SVV having the ATCC Patent Deposit No. PTA-5343 or a SW comprising a sequence that is at least 95% or 99% identical to SEQ ID NO: 1.

Further, the cardiovirus can be selected from the group consisting of: vilyuisk human encephalomyelitis virus, Theiler's murine encephalomyelitis virus, and encephalomyocarditis virus. In one aspect, the encephalomyocarditis virus is selected from the group of isolates consisting of: CA-131395, LA-97-1278, IL-92-48963, IA-89-47752, NJ-90-10324, MN-88-36695, and NC-88-23626.

The viruses of the present invention, and the compositions thereof, can be used in the manufacture of a medicament for treating the diseases mentioned herein. Further, the viruses and composition thereof of the invention can be used for the treatment of the diseases mentioned herein. Thus, in one aspect of the present invention, the present invention provides the use of SVV (or
compositions thereof) for the treatment of cancer or in the manufacture of a medicament for treating cancer.

**Deposit Information**

The following material has been deposited with the American Type Culture Collection (ATCC), 10801 University Blvd., Manassas, Virginia, 20110-2209, U.S.A., under the terms of the Budapest Treaty on the International Recognition of the Deposit of Microorganisms for the Purposes of Patent Procedure. All restrictions on the availability of the deposited material will be irrevocably removed upon the granting of a patent. Material: Seneca Valley Virus (SVV). ATCC Patent Deposit Number: PTA-5343. Date of Deposit: July 25,2003.

## BRIEF DESCRIPTION OF THE DRAWINGS

**Figure 1** shows a schematic of virotherapy using oncolytic viruses. Oncolytic viruses have the properties to replicate, spread and kill tumor cells selectively through a tumor mass by locally injecting the virus or by systemically delivering the virus.

**Figure 2** shows purified SVV stained with uranyl acetate and examined by transmission electron microscopy. Spherical virus particles are about 27 nm in diameter.

**Figure 3** is an electron micrograph of an SVV-infected PER.C6 cell that has a large crystalline inclusion and large vesicular bodies.

**Figure 4A** shows an analysis of SVV RNA. SVV genomic RNA is extracted using guanidium thiocyanate and a phenol extraction method using Trizol (Invitrogen Corp., Carlsbad, CA). RNA is resolved through a 1.25% denaturing agarose gel. The band is visualized by ethidium bromide (EtBr) staining and photographed. In lane 2, a predominant band of SVV genomic RNA is observed, indicating that the size of the full-length SW genome is about 7.5 kilobases.

**Figure 4B** is a schematic showing the genome structure and protein products generated from polyprotein processing for picornaviruses, including SW.

**Figures 5A**-**5E** presents the nucleotide sequence of SVV (SEQ ID NO:1) and the encoded amino acid sequence (SEQ ID NO:2). The stop codon is depicted by a "*" at positions 5671-3. As a general note, in sequence disclosures that include positions where the exact nucleotide is being confirmed, these positions are represented by an "n". Therefore, in codons that possess an "n", the relevant amino acid is depicted by a "x".

**Figures 6A-6D** presents the nucleotide sequence (SEQ ID NO: 1) of the majority of the full-length genome of SVV. The nucleotide sequence was derived from the SW isolate having the ATCC Patent Deposit Number: PTA-5343. Date of Deposit: July 25, 2003.

**Figures 7A-7B** presents the amino acid sequence (SEQ ID NO:2) encoded by SEQ ID NO: 1.

**Figure 8** presents the nucleotide sequence (SEQ ID NO:3) of the partial 1B or VP2 encoding region of SW. This sequence is identical to nucleotides 4-429 of SEQ ID NO:1.

**Figure 9** presents the amino acid sequence (SEQ ID NO:4) of the partial SW VP2 protein that is encoded by SEQ ID NO:3. The sequence listed in SEQ ID NO:4 is identical to amino acids 2-143 of SEQ ID NO:2.

**Figure 10** presents the nucleotide sequence (SEQ ID NO:5) of the 1C or VP3 encoding region of SW. This sequence is identical to nucleotides 430-1146 of SEQ ID NO:1.

**Figure 11** presents the amino acid sequence (SEQ ID NO:6) of the SVV VP3 protein that is encoded by SEQ ID NO:5. The sequence listed in SEQ ID NO:6 is identical to amino acids 144-382 of SEQ ID NO:2.

**Figure 12** presents the nucleotide sequence (SEQ ID NO:7) of the ID or VP1 encoding region of SW. This sequence is identical to nucleotides 1147-1923 of SEQ ID NO: 1.

**Figure 13** presents the amino acid sequence (SEQ ID NO:8) of the SVV VP1 protein that is encoded by SEQ ID NO:7. The sequence listed in SEQ ID NO:8 is identical to amino acids 383-641 of SEQ ID NO:2.

**Figure 14** presents the nucleotide sequence (SEQ ID NO:9) of the 2A encoding region of SW. This sequence is identical to nucleotides 1924-1965 of SEQ ID NO: 1.

**Figure 15** presents the amino acid sequence (SEQ ID NO: 10) of the SW 2A protein that is encoded by SEQ ID NO:9. The sequence listed in SEQ ID NO:10 is identical to amino acids 642-655 of SEQ ID NO:2.

**Figure 16** presents the nucleotide sequence (SEQ ID NO: 11) of the 2B encoding region of SVV. This sequence is identical to nucleotides 1966-2349 of SEQ ID NO:1.

**Figure 17** presents the amino acid sequence (SEQ ID NO: 12) of the SVV 2B protein that is encoded by SEQ ID NO: 11. The sequence listed in SEQ ID NO: 12 is identical to amino acids 656-783 of SEQ ID NO:2.

**Figure 18** presents the nucleotide sequence (SEQ ID NO:13) of the 2C encoding region of SVV. This sequence is identical to nucleotides 2350-3315 of SEQ ID NO: 1.

**Figure 19** presents the amino acid sequence (SEQ ID NO: 14) of the SW 2C protein that is encoded by SEQ ID NO:13. The sequence listed in SEQ ID NO:14 is identical to amino acids 784-1105 of SEQ ID NO:2.

**Figure 20** presents the nucleotide sequence (SEQ ID NO: 15) of the 3A encoding region of SVV. This sequence is identical to nucleotides 3316-3585 of SEQ ID NO:1.

**Figure 21** presents the amino acid sequence (SEQ ID NO: 16) of the SW 3A protein that is encoded by SEQ ID NO:15. The sequence listed in SEQ ID NO: 16 is identical to amino acids 1106-1195 of SEQ ID NO:2.

**Figure 22** presents the nucleotide sequence (SEQ ID NO:17) of the 3B encoding region of SVV. This sequence is identical to nucleotides 3586-3651 of SEQ ID NO: 1.

**Figure 23** presents the amino acid sequence (SEQ ID NO:18) of the SW 3B protein that is encoded by SEQ ID NO:17. The sequence listed in SEQ ID NO:18 is identical to amino acids 1196-1217 of SEQ ID NO:2.

**Figure 24** presents the nucleotide sequence (SEQ ID NO: 19) of the 3C encoding region of SVV. This sequence is identical to nucleotides 3652-4284 of SEQ ID NO:1.

**Figure 25** presents the amino acid sequence (SEQ ID NO:20) of the SW 3C protein that is encoded by SEQ ID NO:19. The sequence listed in SEQ ID NO:20 is identical to amino acids 1218-1428 of SEQ ID NO:2.

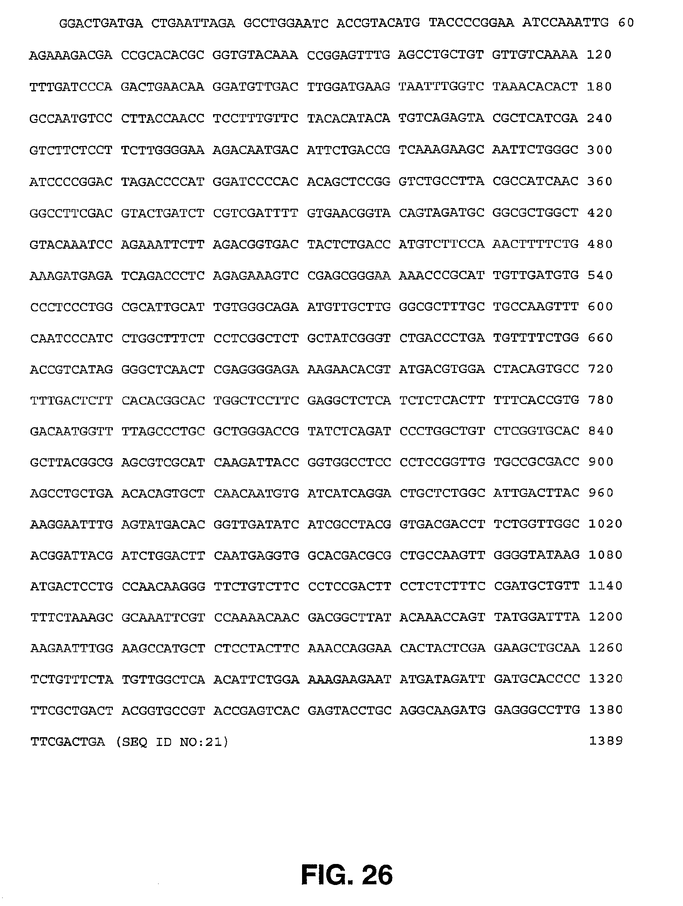

**Figure 26** presents the nucleotide sequence (SEQ ID NO:21) of the 3D encoding region of SW. This sequence is identical to nucleotides 4285-5673 of SEQ ID NO:1.

**Figure 27** presents the amino acid sequence (SEQ ID NO:22) of the SW 3D protein that is encoded by SEQ ID NO:21. The sequence listed in SEQ ID NO:22 is identical to amino acids 1429-1890 of SEQ ID NO:2.

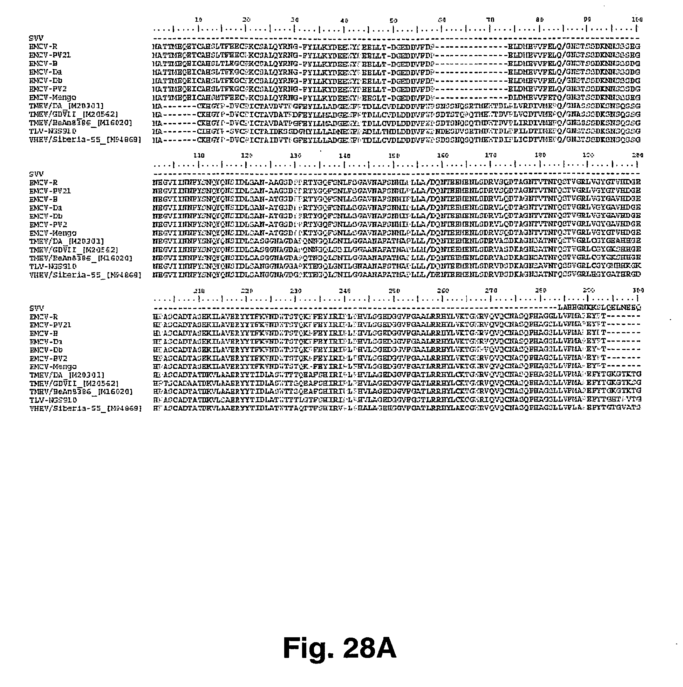

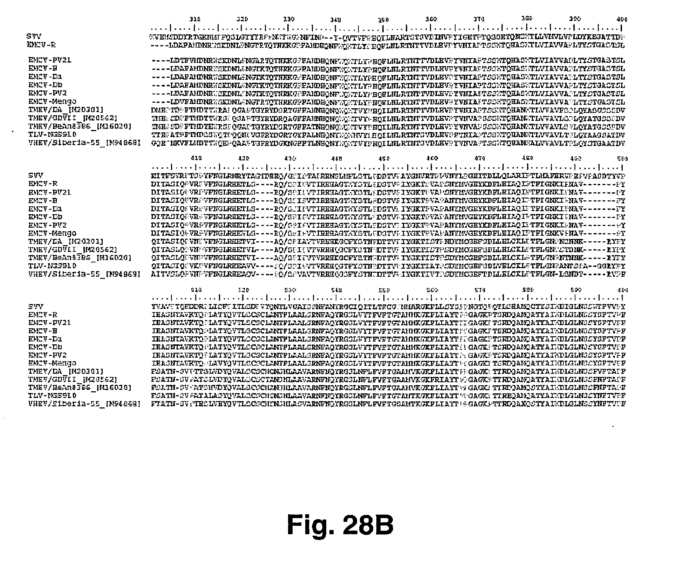

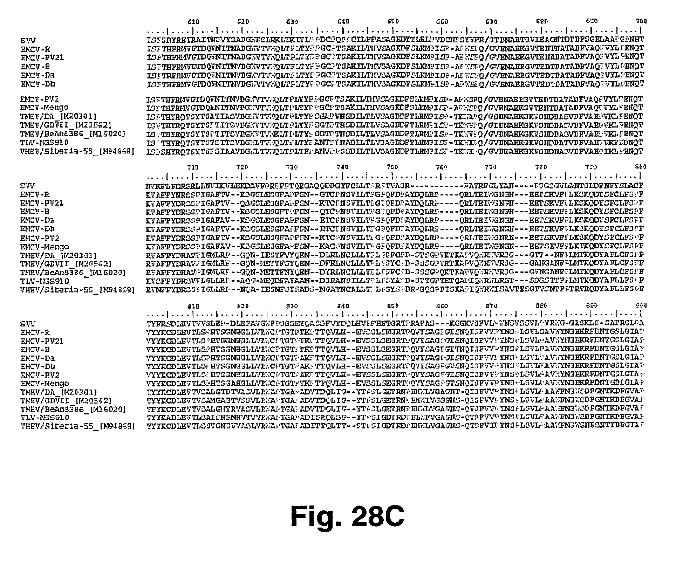

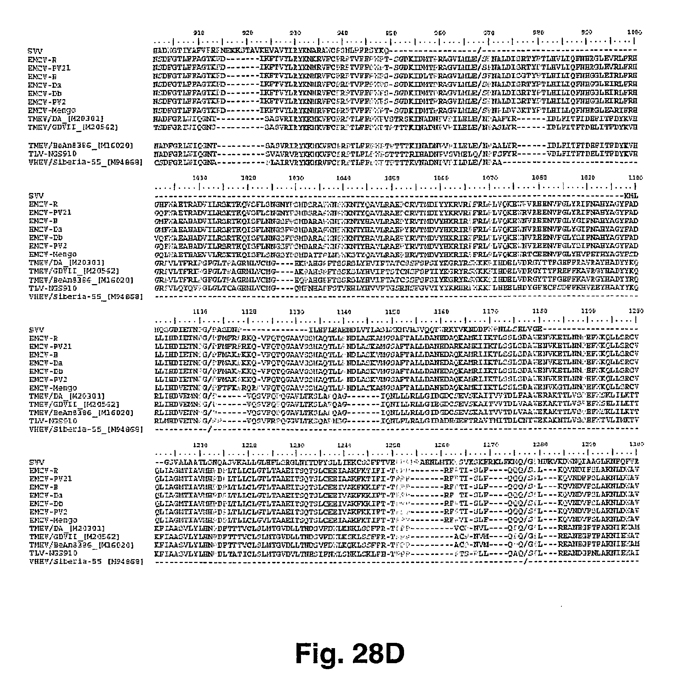

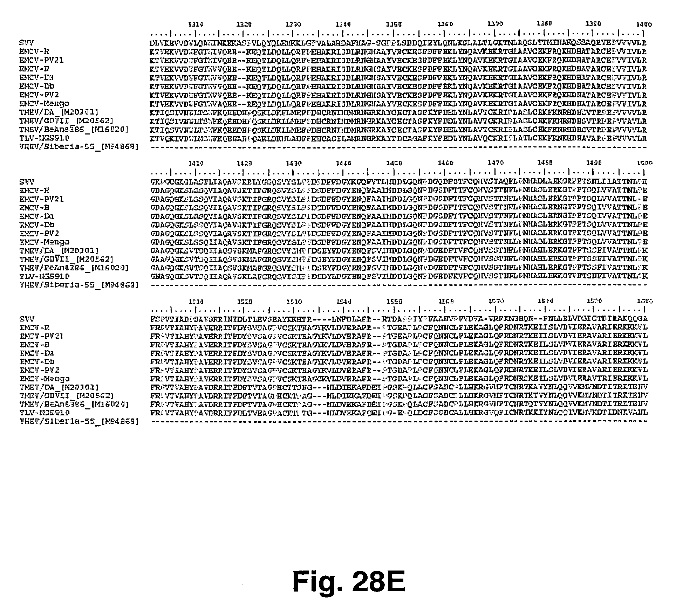

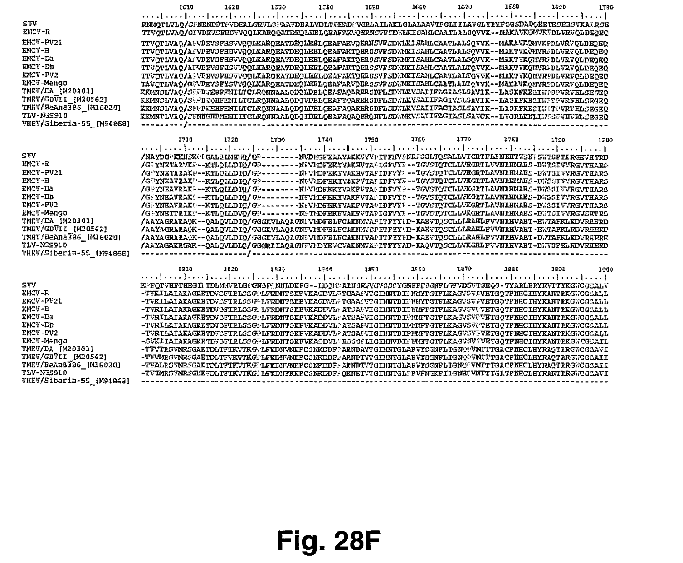

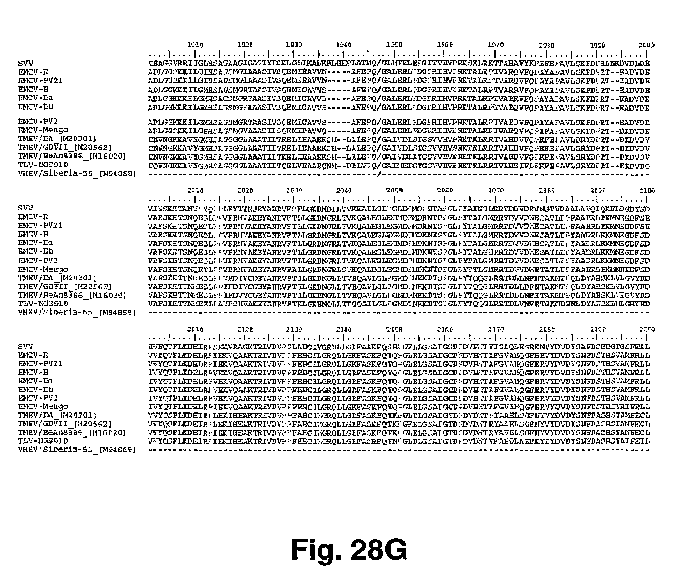

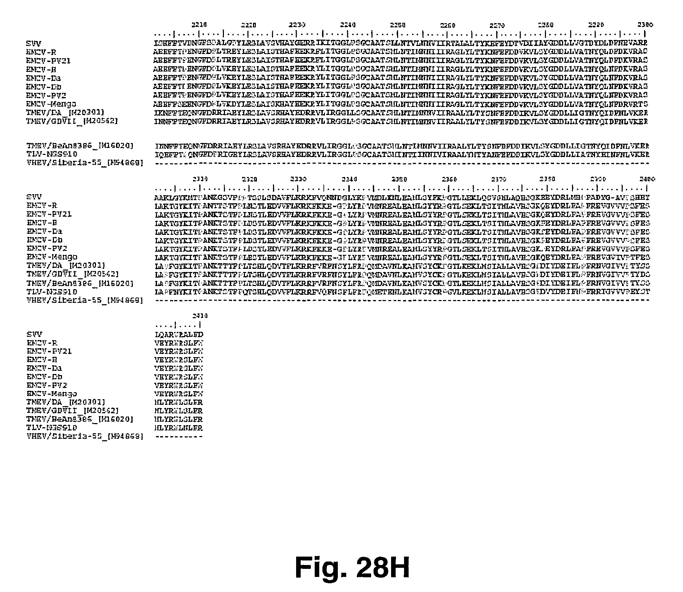

**Figures 28A-28H** present an amino acid sequence alignment between SW SEQ ID NO:2 and various members of the *Cardiovirus* genus, such as Encephalomyocarditis virus (EMCV; species *Encephalomyocarditis virus),* Theiler's murine encephalomyocarditis virus (TMEV; species *Theilovirus),* a rat TMEV-like agent (TLV; species *Theilovirus),* and Vilyuisk human encephalomyelitis virus (VHEV; species *Theilovirus).* The specific sequences of the various Cardioviruses are presented in: SEQ ID NOs: 23 (EMCV-R), 24 (EMCV-PV21), 25 (EMCV-B), 26 (EMCV-Da), 27 (EMCV-Db), 28 (EMCV-PV2), 29 (EMCV-Mengo), 30 (TMEV/DA), 31 (TMEV/GDVII), 32 (TMEV/BeAn8386), 33 (TLV-NGS910) and 34 (VHEV/Siberia-55).
Number positions in Figure 28 do not correspond to the numbering of the sequence listings. The "/" symbol indicates cleavage sites where the polyprotein is cleaved into its final functional products. For example, the alignment between positions 1 and 157 is in the 1A (VP4) region. The alignment between positions: 159 and 428 is in the 1B (VP2) region; 430 and 668 is in the 1C (VP3) region; 670 and 967 is in the 1D (VP1) region; 969 and 1111 is in the 2A region; 1112 and 1276 is in the 2B region; 1278 and 1609 is in the 2C region; 1611 and 1700 is in the 3A region; 1702 and 1723 is in the 3B region; 1725 and 1946 is in the 3C region; 1948 and 2410 is in the 3D region. The alignment also shows regions of potential conservation or similarity between the viral sequences as can be determined by standard sequence analysis programs. The alignments were generated using BioEdit 5.0.9 and Clustal W 1.81.

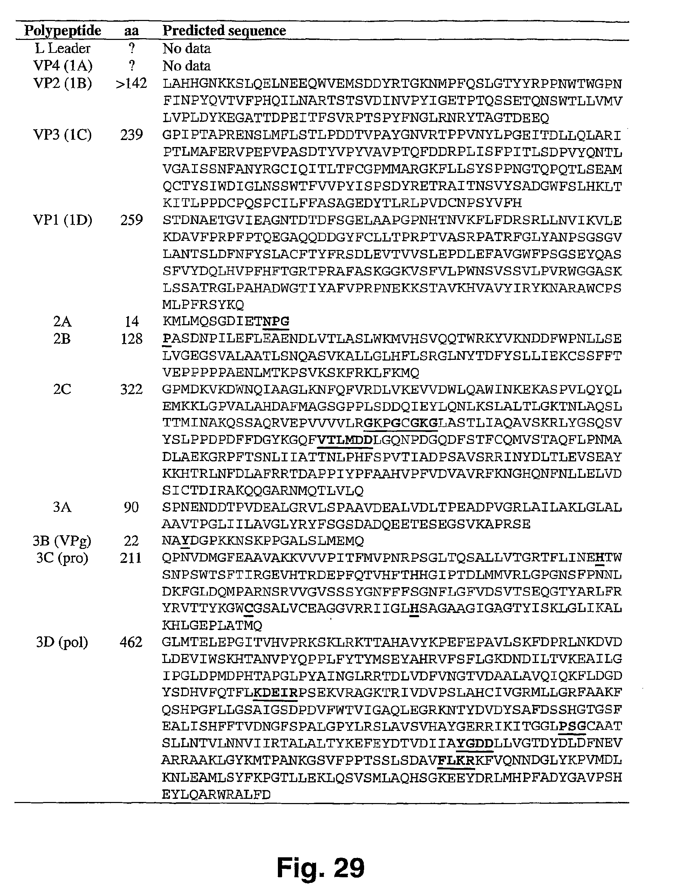

**Figure 29** lists the final polypeptide products of SVV (SEQ ID NOS: 35-44, respectively, in order of appearance). Some conserved motifs are bolded and underlined: 2A/2B "cleavage" (NPGP SEQ ID NO: 111); 2C ATP-binding (GxxGxGKS/T SEQ ID NO: 112 and hyhyhyxxD); 3B (VPg)/RNA attachment residue (Y); 3C (pro) active site residues (H, C, H); 3D (pol) motifs (KDEL/IR SEQ ID NO: 113, PSG, YGDD SEQ ID NO: 114, FLKR SEQ ID NO: 115).

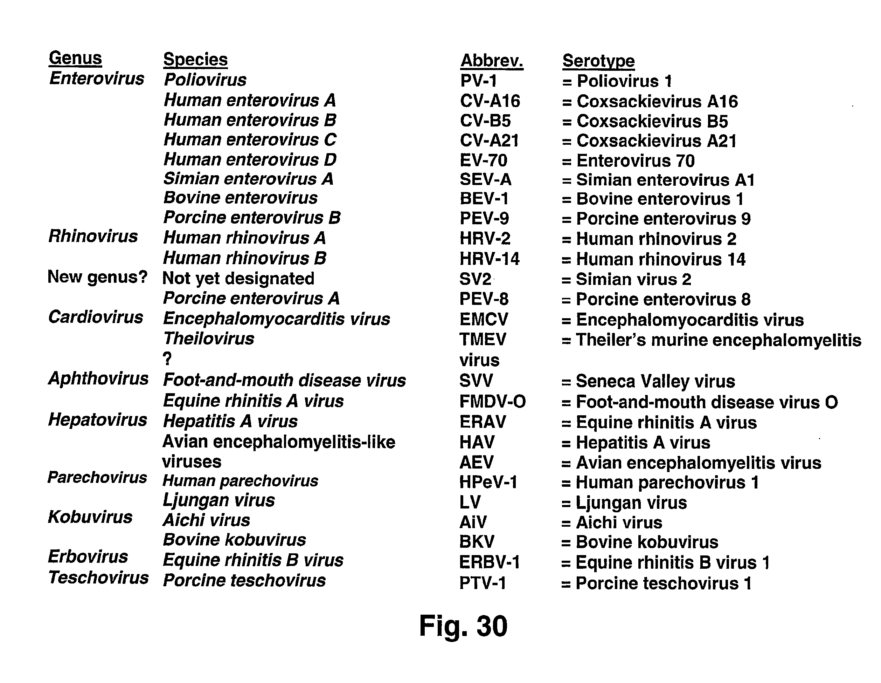

**Figure 30** lists the picornavirus species that were used in sequence analyses to determine the phylogenetic relationship between SVV and these picornaviruses (see Example 4).

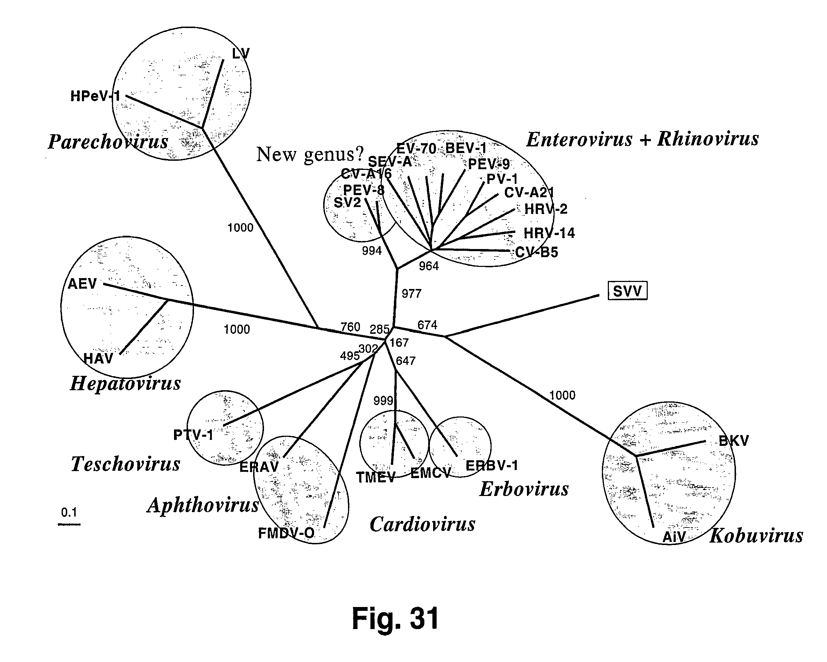

**Figure 31** shows the phylogenetic relationship between SVV (SEQ ID NO:4) and other picornaviruses in view of VP2 sequence analyses. The figure shows a bootstrapped neighbor-joining tree (see Example 4).

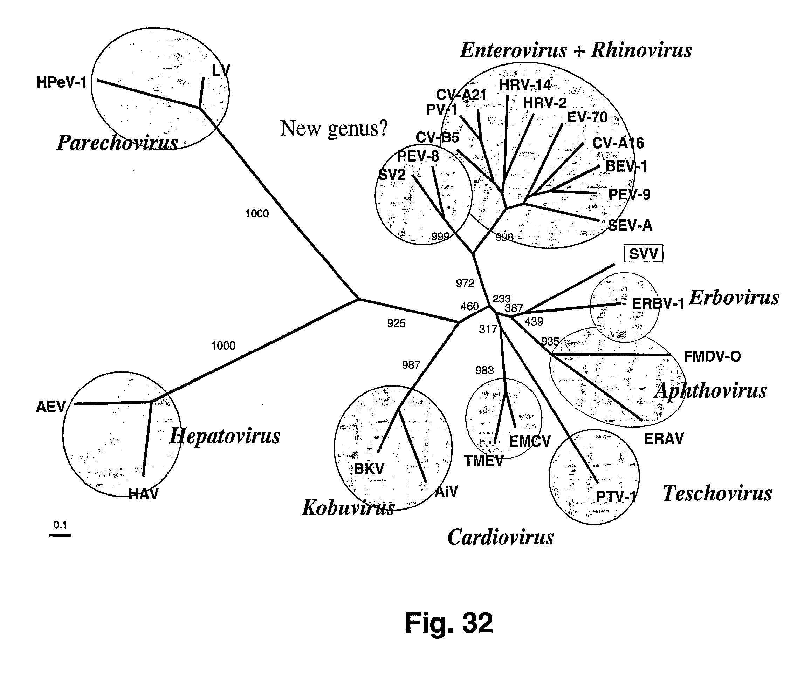

**Figure 32** shows a bootstrapped neighbor-joinining tree for VP3 between SVV (SEQ ID NO:6) and other picornaviruses (see Example 4).

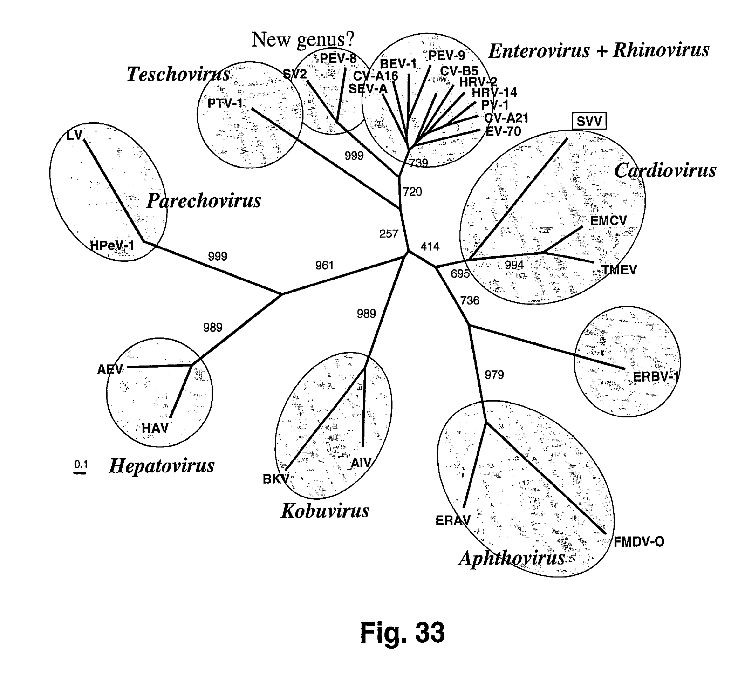

**Figure 33** shows a bootstrapped neighbor-joinining tree for VP1 between SVV (SEQ ID NO:8) and other picornaviruses (see Example 4).

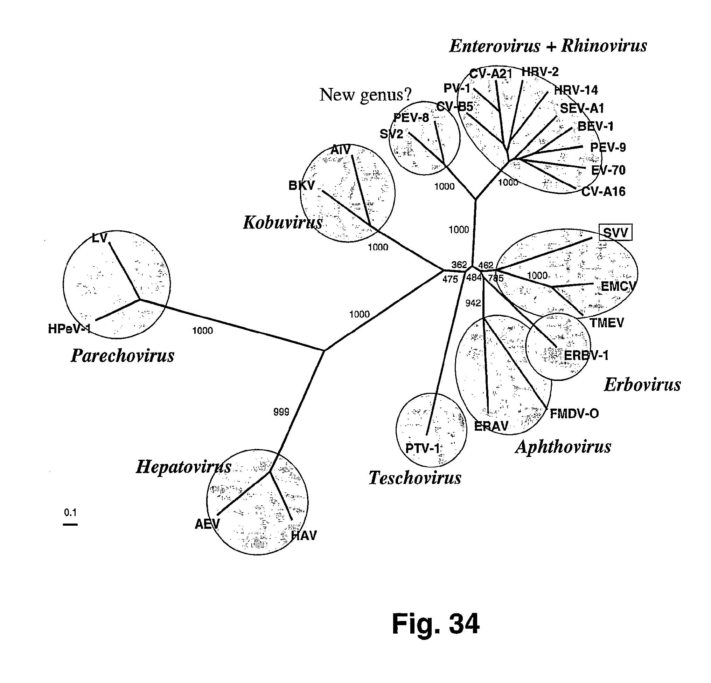

**Figure 34** shows a bootstrapped neighbor-joinining tree for P1 (*i.e.,* 1A, 1B, 1C and 1D) between SVV *(i.e.,* partial P1 - amino acids 2-641 of SEQ ID NO:2) and other picornaviruses (see Example 4).

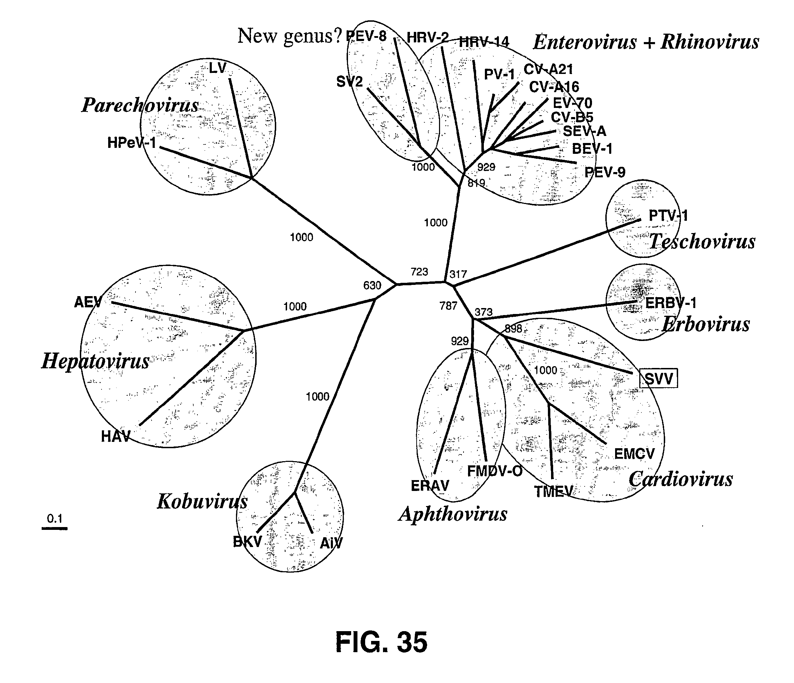

**Figure 35** shows a bootstrapped neighbor-joinining tree for 2C between SVV (SEQ ID NO: 14) and other picornaviruses (see Example 4).

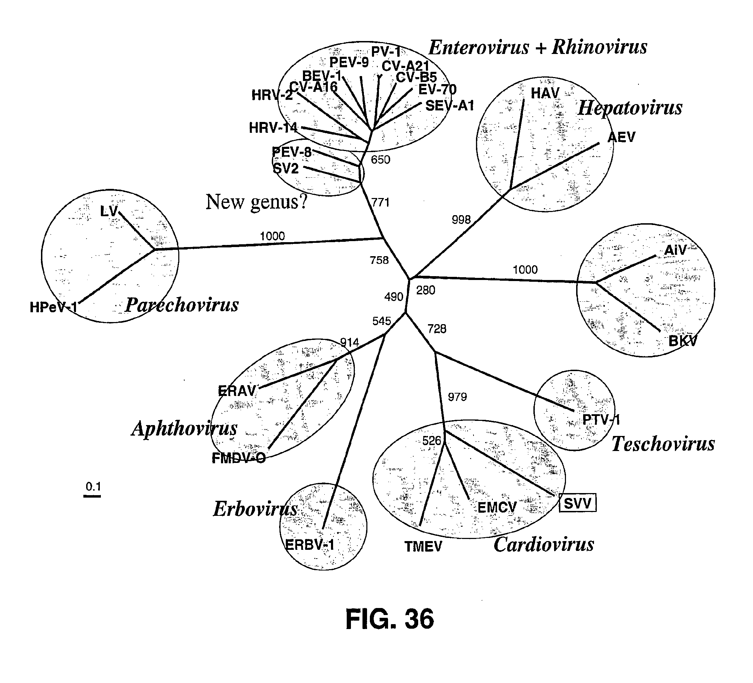

**Figure 36** shows a bootstrapped neighbor-joinining tree for 3C (pro) between SVV (SEQ ID NO:20) and other picornaviruses (see Example 4).

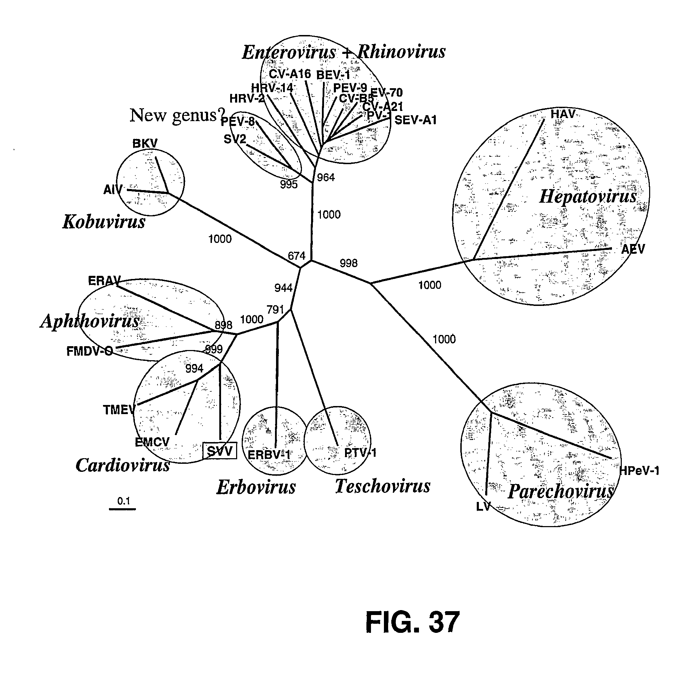

**Figure 37** shows a bootstrapped neighbor-joinining tree for 3D (pol) between SVV (SEQ ID NO:22) and other picornaviruses (see Example 4).

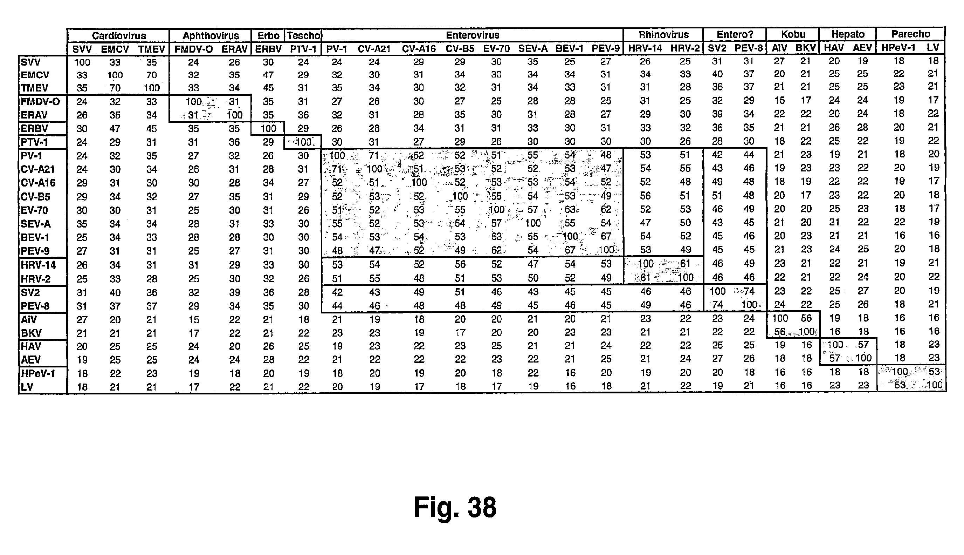

**Figure 38** presents the actual amino acid percent identities of VP2 between SVV (SEQ ID NO:4) and other picornaviruses (see Example 4).

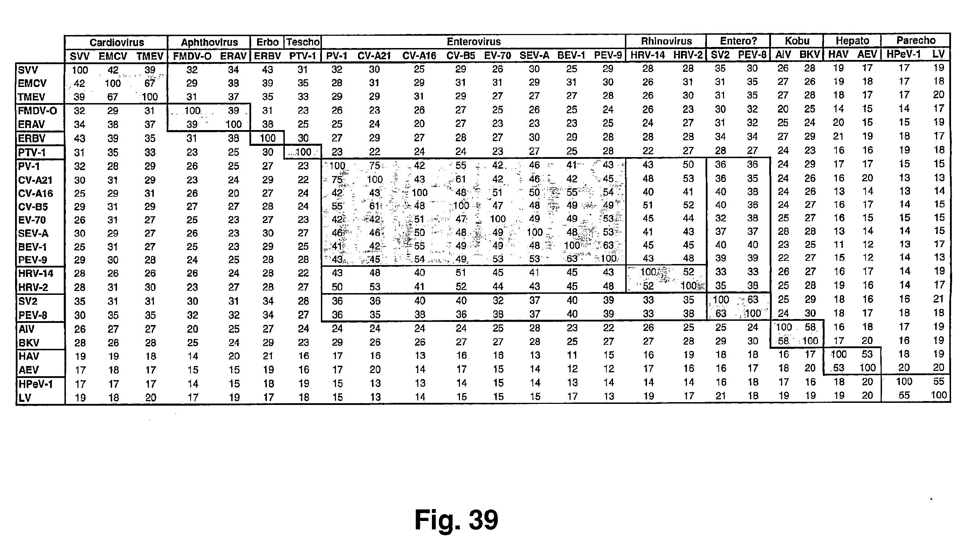

**Figure 39** presents the actual amino acid percent identities of VP3 between SVV (SEQ ID NO:6) and other picornaviruses (see Example 4).

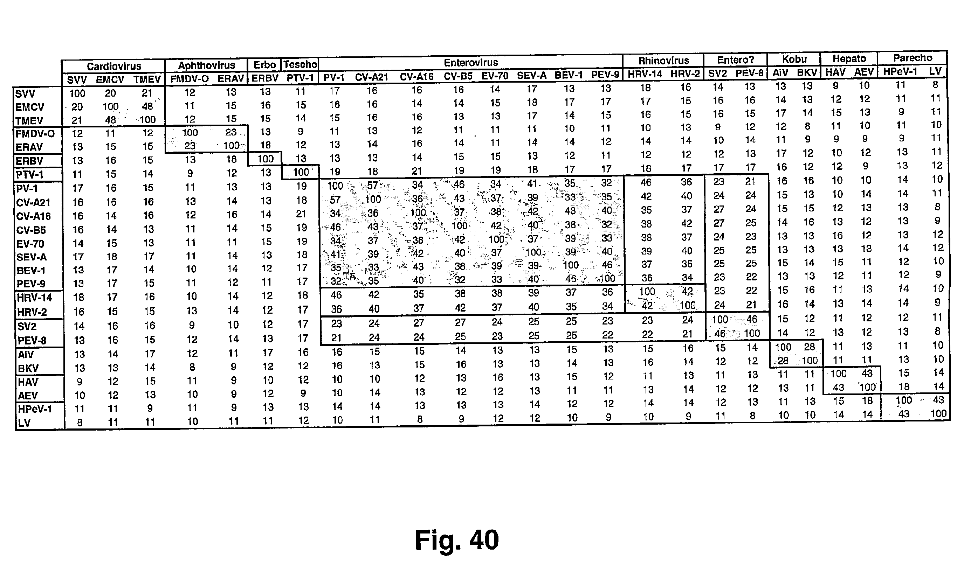

**Figure 40** presents the actual amino acid percent identities of VP1 between SVV (SEQ ID NO:8) and other picornaviruses (see Example 4).

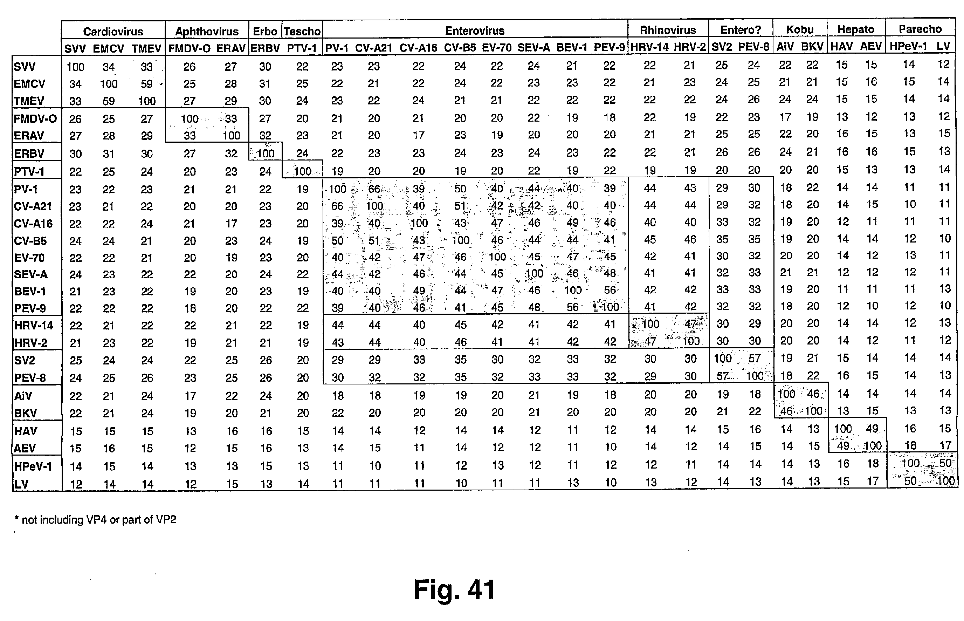

**Figure 41** presents the actual amino acid percent identities of P1 between SVV (partial capsid or P1 - amino acids 2-641 of SEQ ID NO:2) and other picornaviruses (see Example 4).

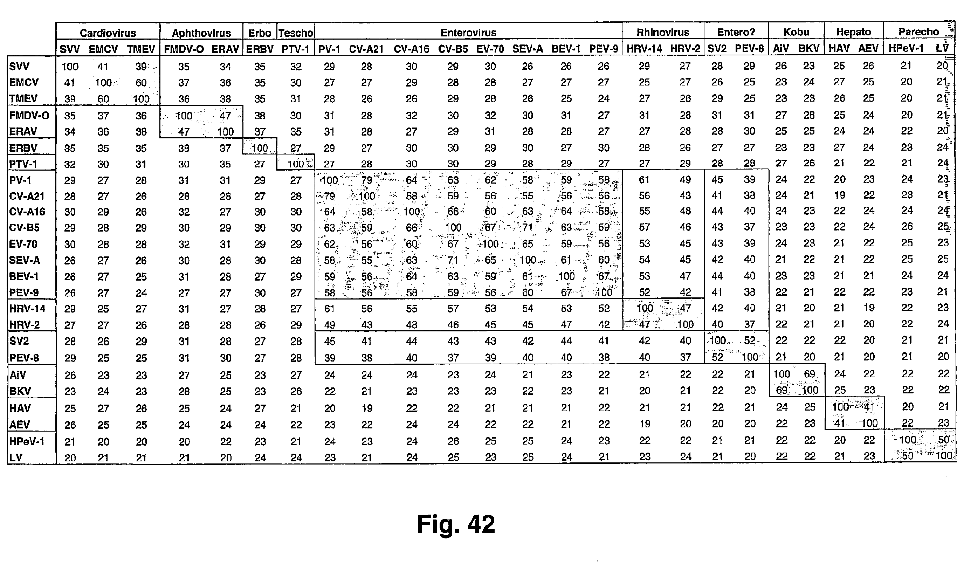

**Figure 42** presents the actual amino acid percent identities of 2C between SW (SEQ ID NO:14) and other picornaviruses (see Example 4).

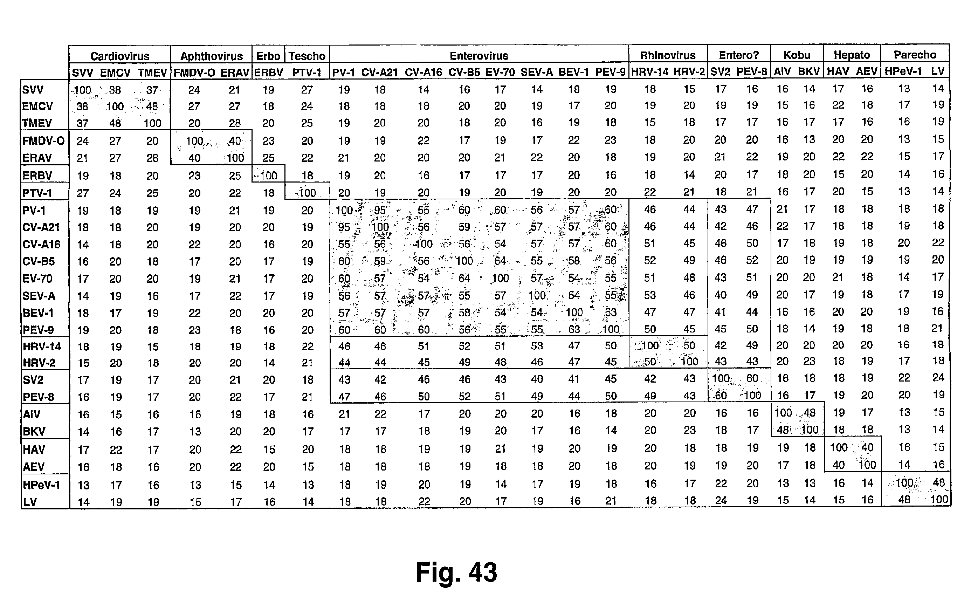

**Figure 43** presents the actual amino acid percent identities of 3C (pro) between SW (SEQ ID NO:20) and other picornaviruses (see Example 4).

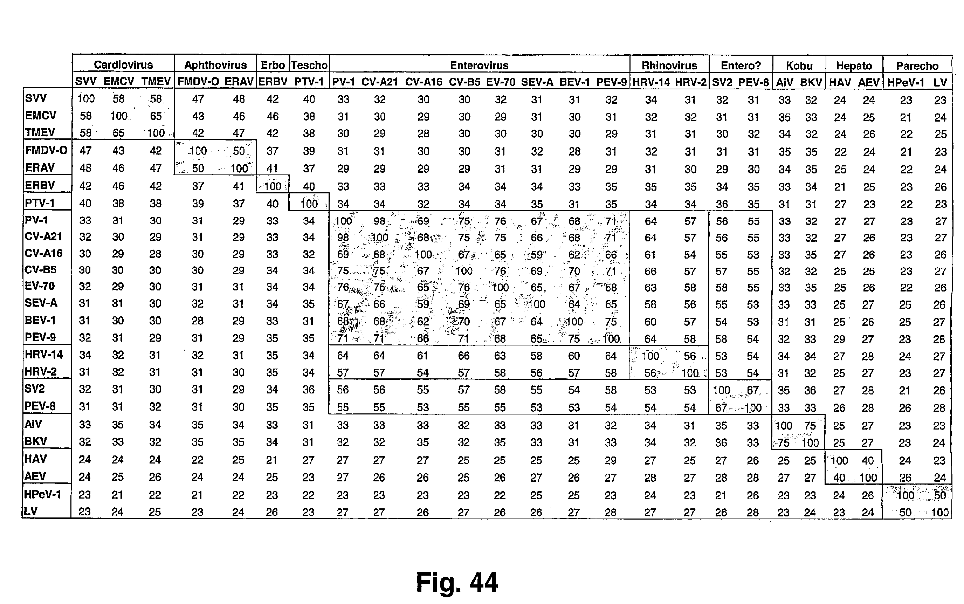

**Figure 44** presents the actual amino acid percent identities of 3D (pol) between SVV (SEQ ID NO:22) and other picornaviruses (see Example 4).

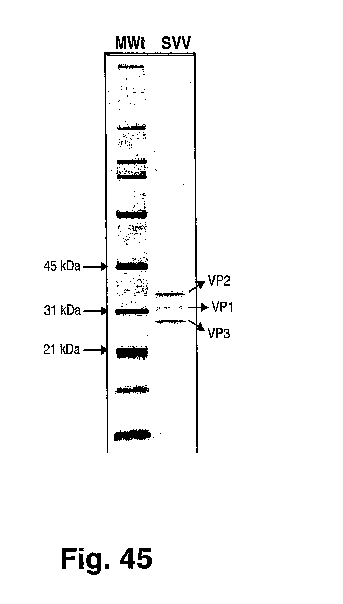

**Figure 45** shows the VP2 (-36 kDa), VP1 (~31 kDa) and VP3 (~27 kDa) proteins of SVV as analyzed by SDS-PAGE.

Purified SVV was subjected to SDS-PAGE and proteins were visualized by silver stain. Lane "MWt" is molecular weight markers; lane "SVV" contains structural proteins of SVV. The sizes of three molecular weight markers and the names of viral proteins are also given.

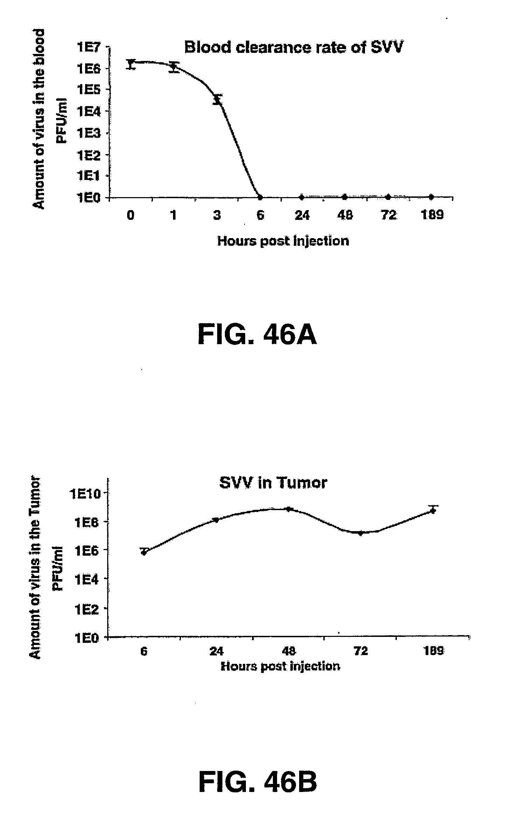

**Figures 46A-46B** show the amounts of SW in blood and tumor following systemic administration (Example 7). H446 tumor bearing nude mice were treated with SVV at a dose of $1x10^{12}$ vp/kg by tail vein injection. The mice were bled at 0, 1, 3, 6, 24,48, 72 hours and at 7 days post-injection, and the plasma was separated from the blood immediately after collection, diluted in infection medium, and used to infect PER.C6 cells. The tumors were harvested at 6, 24, 48, 72 hours and at 7 days post-injection. The tumors were cut into small sections and suspended in one mL of medium and CVL was made.

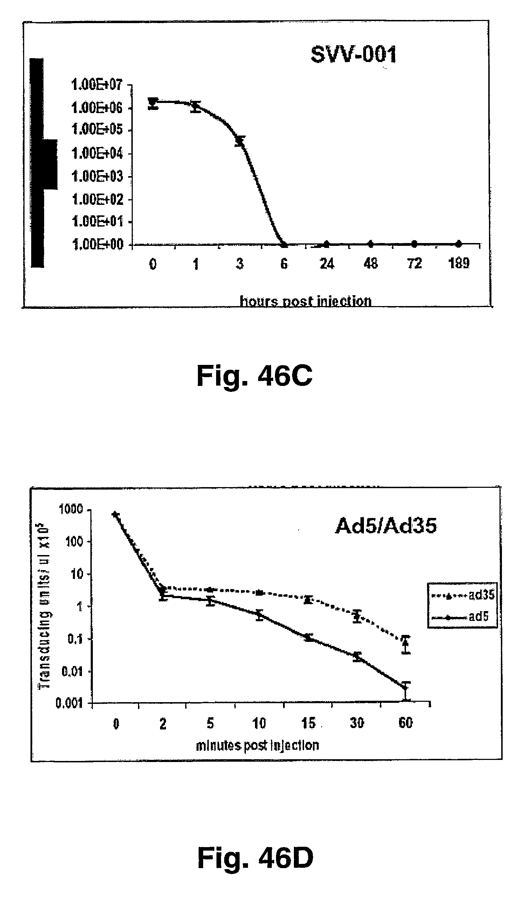

**Figures 46C-46D** presents data relating to SVV *clearance in vivo.* The figures show that SVV exhibits a substantially longer resident time in the blood compared to similar doses of i.v. adenovirus (Example 7), and therefore SVV has a slower clearance rate than adenovirus *in vivo.* Following a single intravenous (i.v.) dose, SVV remains present in the blood for up to 6 hours (Figure 46C; Figure 46C is a duplication of Figure 46A for comparison purposes to Figure 46D), whereas adenovirus is cleared or depleted from the blood in about an hour (Figure 46D).

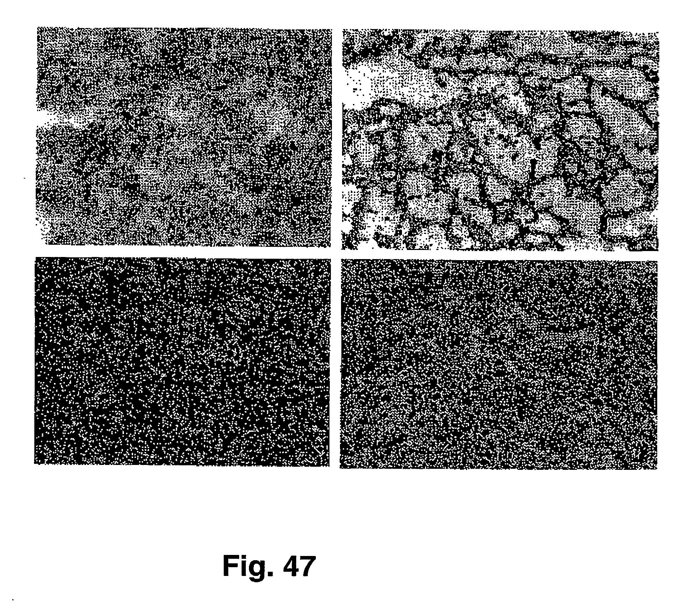

**Figure 47** shows immunohistochemistry and hematoxylin and eosin (H&E) staining of H446 xenograft sections (Example 7). H446 tumor bearing nude mice were treated with Hank's balanced salt solution (HBSS) or SVV at a dose of $1x10^{12}$ vp/kg by tail vein injection. The mice were sacrified at 3 days post-injection and the tumors were collected. The virus proteins in the tumor cells are visualized by immunohistochemistry using SVV-specific mouse antibodies (upper panels). The general morphology of H446 tumor cells collected from HBSS or SVV treated mice were stained by H&E stain (lower panels).

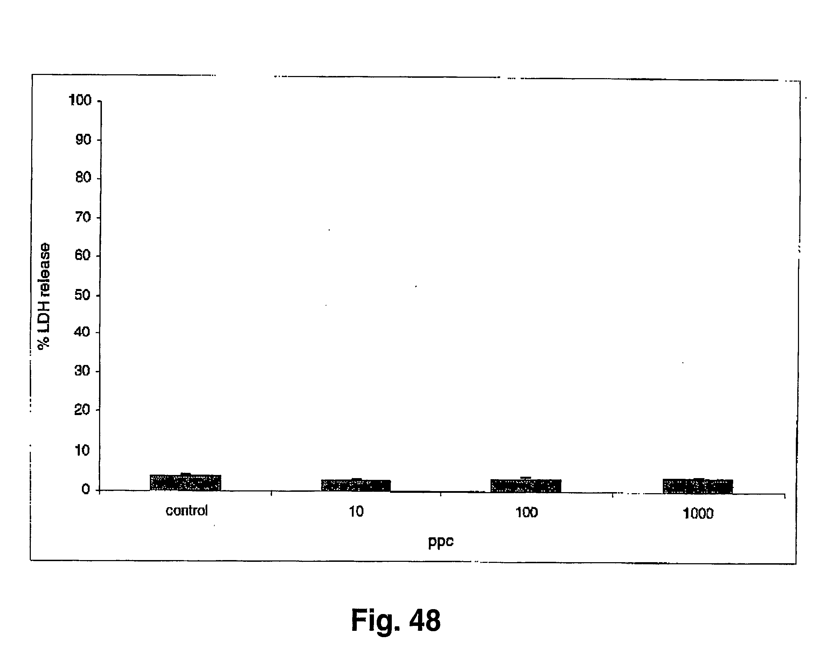

**Figure 48** shows SVV mediated cytotoxicity in primary human hepatocytes (Example 9). Primary human hepatocytes plated in collagen coated 12-well plates were infected with SVV at 1, 10 and 100 and 1000 particles per cell (ppc). Three days after infection, the cell associated lactate dehydrogenase (LDH) and LDH in the culture supernatant were measured separately. Percent cytotoxicity was determined as a ration of LDH units in supernatant over maximal cellular LDH plus supernatant LDH.

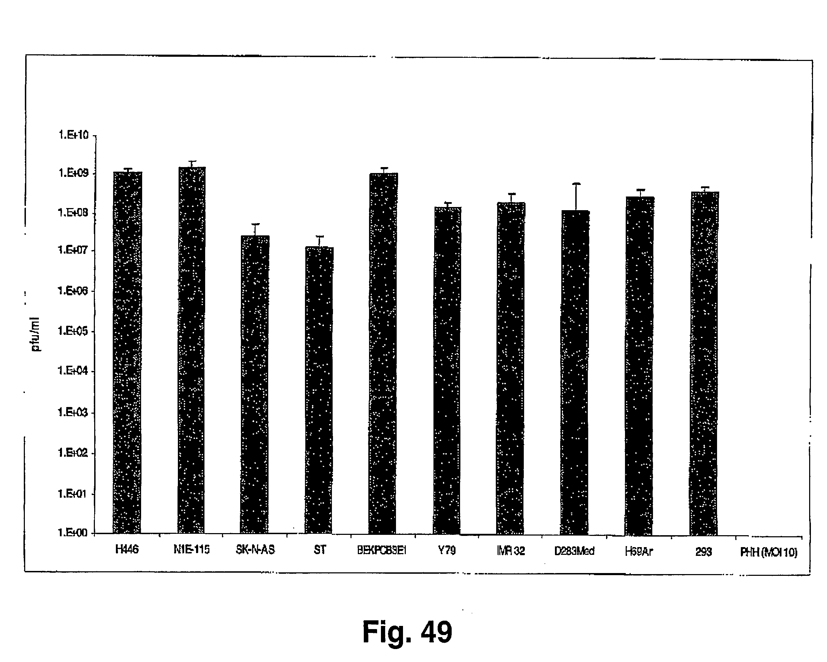

**Figure 49** shows virus production by SVV in selected cell lines. To assess the replicative abilities of SW, selected normal cells and tumor cells were infected with SW at one virus particle per cell (ppc) (Example 9). After 72 hours, cells were harvested and CVL was assayed for titer on PER.C6 cells. For each cell line, the efficiency of SVV replication was expressed as plaque forming units per milliliter (pfu/ml).

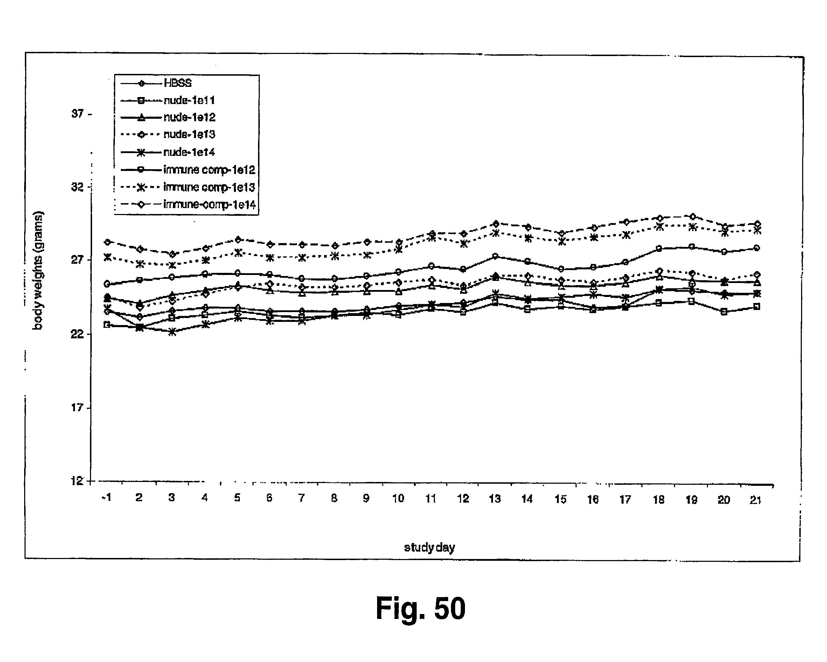

**Figure 50** shows toxicity in nude and CD1 mice according to body weights (Example 10). The mean body weight of mice in each treatment group were measured different days post virus administration. Mice were injected with a single dose of SVV or PBS by tail vein on day 1.

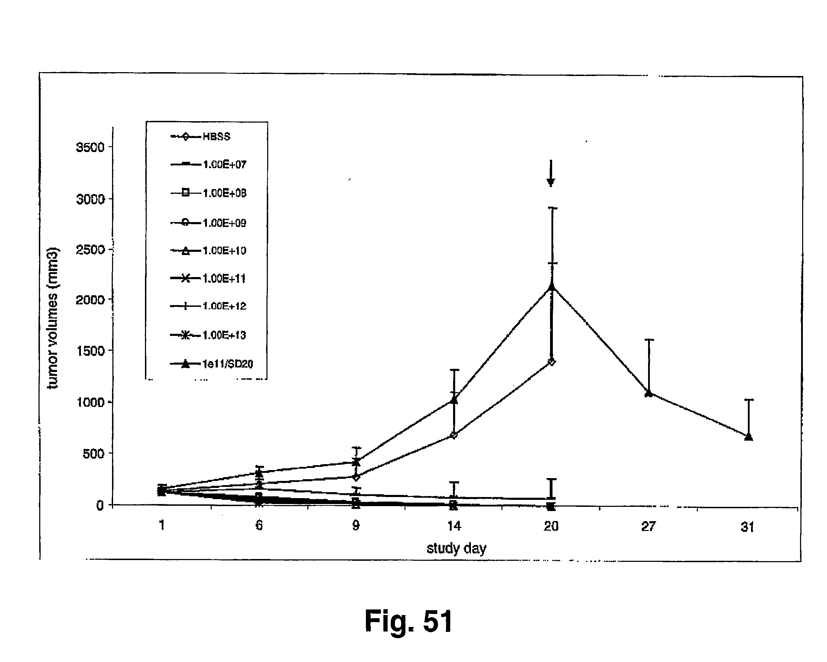

**Figure 51** shows efficacy in a H446 xenograft model. H446 tumors are established in nude mice and the mice are sorted into groups (n=10) and treated via tail vein injection with either HBSS or six different doses of SVV (Example 11). On study day 20, five mice from the HBSS group that bear large tumors (mean tumor volume = 2000 $mm^3$) were injected with $1x10^{11}$ vp/kg (indicated by an arrow). Data is expressed as mean tumor volume + standard deviation (SD).

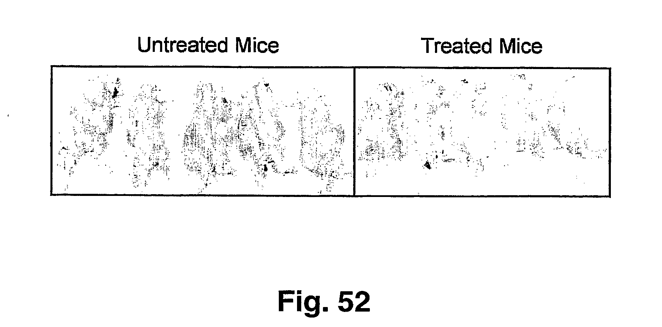

**Figure 52** shows a picture of H446 xenograft nude mice that have been untreated or treated with SW (Example 11). The efficacy of SVV is very robust in that 100% of large pre-established tumors were completely eradicated. SVV-treated mice show neither clinical symptoms nor recurrence of tumors for at least 200 days following injection.

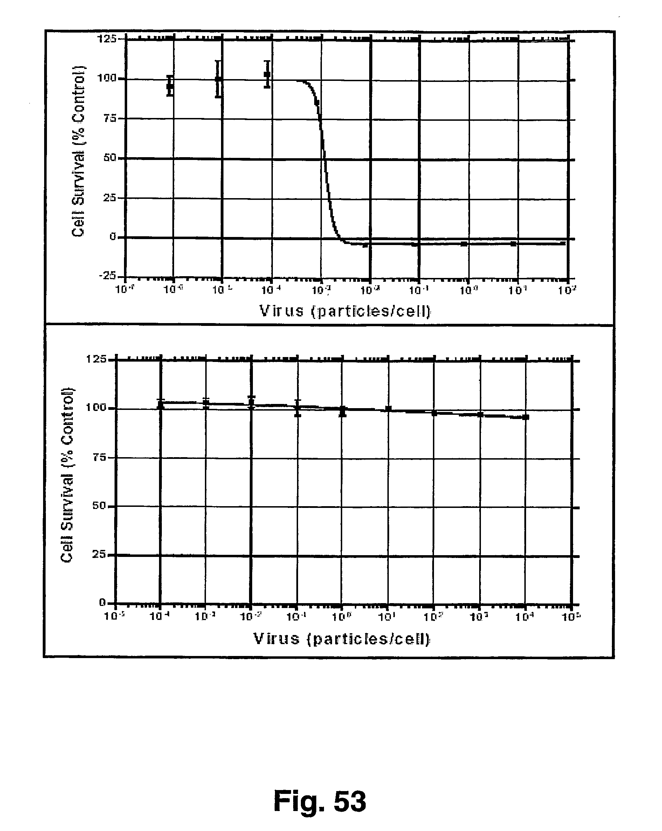

**Figure 53** presents data relating to SVV tumor specificity and efficacy *in vitro* (Example 11). The graphs show cell survival following incubation of either H446 human small cell lung carcinoma (SCLC) tumor cells (top graph) or normal human H460 cells (bottom graph). SVV specifically killed the tumor cells with an $EC_{50}$ of approximately $10^{-3}$ particles per cell. In contrast, normal human cells were not killed at any concentration of SVV.

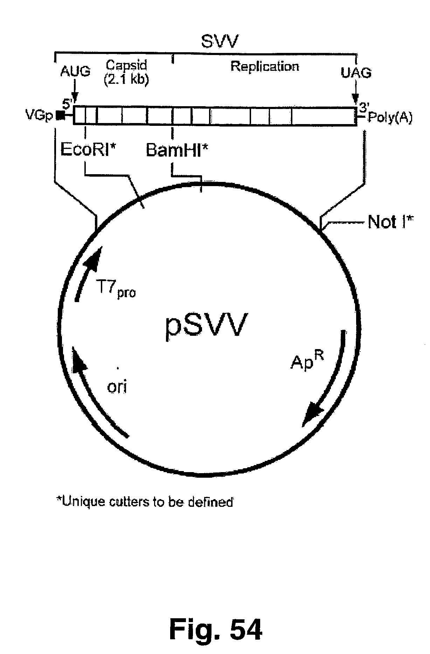

**Figure 54** depicts a representative plasmid containing the complete genome of SW (Example 15). The presence of the T7 promoter on the vector upstream of the SVV sequence allows for the *in vitro* transcription of the SVV sequence such that SVV RNA molecules can be generated.

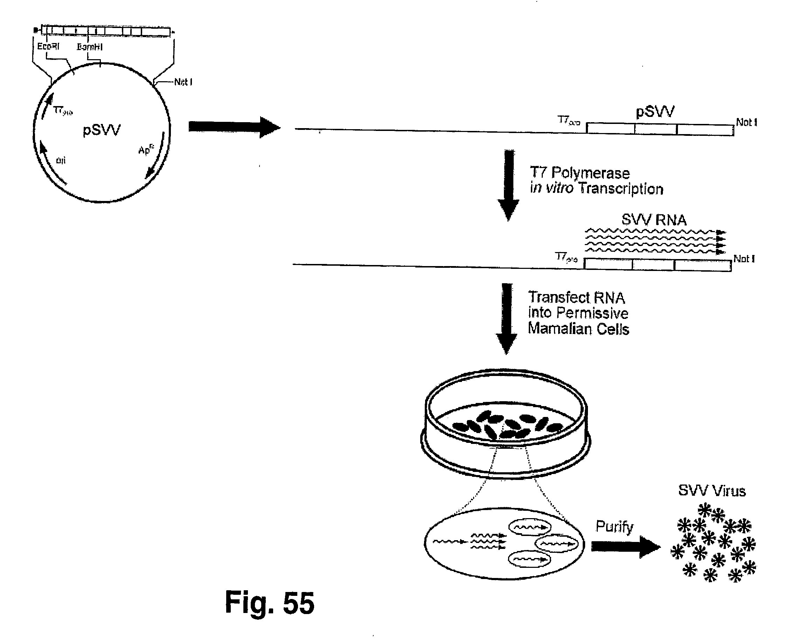

**Figure 55** depicts a schematic for the construction of a full-length and functional genomic SVV plasmid and subsequent SVV virus production (Example 16). To obtain a functional genomic SVV clone, the complete genome of a SVV can be cloned into a vector with a T7 promoter. This can be accomplished by making cDNA clones of the virus, sequencing them and cloning contiguous pieces into one plasmid, resulting in the plasmid depicted "pSVV". The plasmid with the full genome of SVV can then be reverse-transcribed to generate SVV RNA. The SW RNA is then transfected into permissive mammalian cells and SVV virus particles can then be recovered and purified.

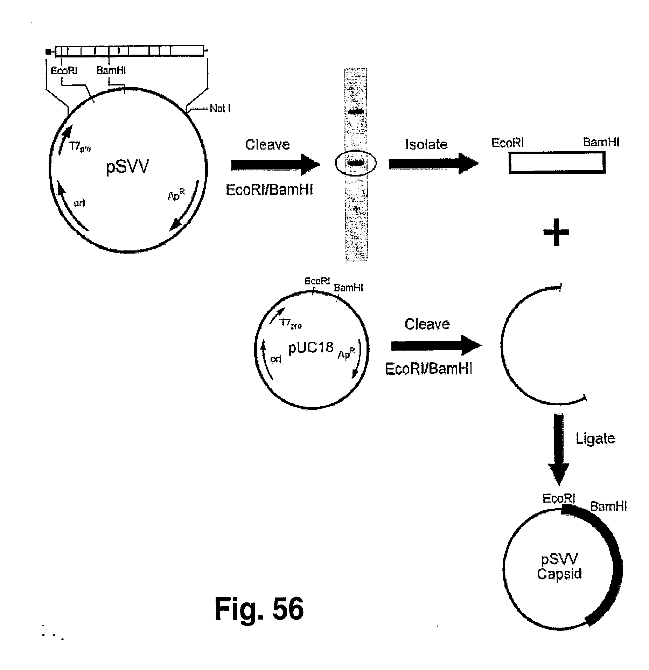

**Figure 56** depicts a schematic for the construction of a vector ("pSVV capsid") containing the coding sequence (*i.e.,* coding regions for 1A-1D) for the SVV capsid (Example 16). The pSVV capsid can then be used to generate a library of SVV capsid mutants.

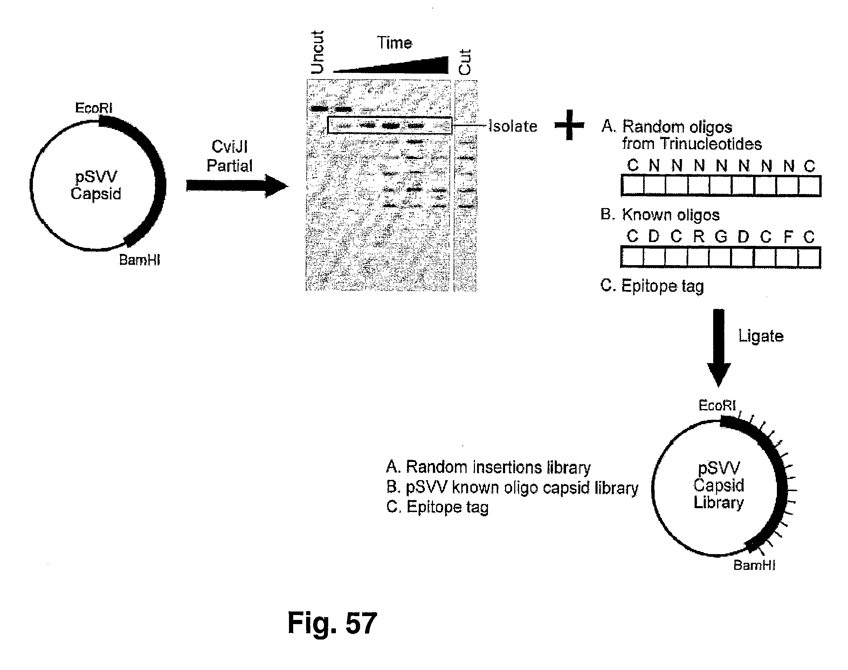

**Figure 57** shows one method of mutating the SVV capsid for the generation of a library of SW capsid mutants (Example 16). The figure illustrates the insertion of an oligonucleotide sequence at random sites of the plasmid. The oligonucleotides can be random oligonucleotides, oligonucleotides with known sequences, or an oligonucleotide encoding an epitope tag. In the figure, the restriction enzyme CviJI randomly cleaves the pSVV capsid DNA. Linearized pSVV capsid DNA that has been cut at a single site is isolated and purified from a gel, and ligated with oligonucleotides.

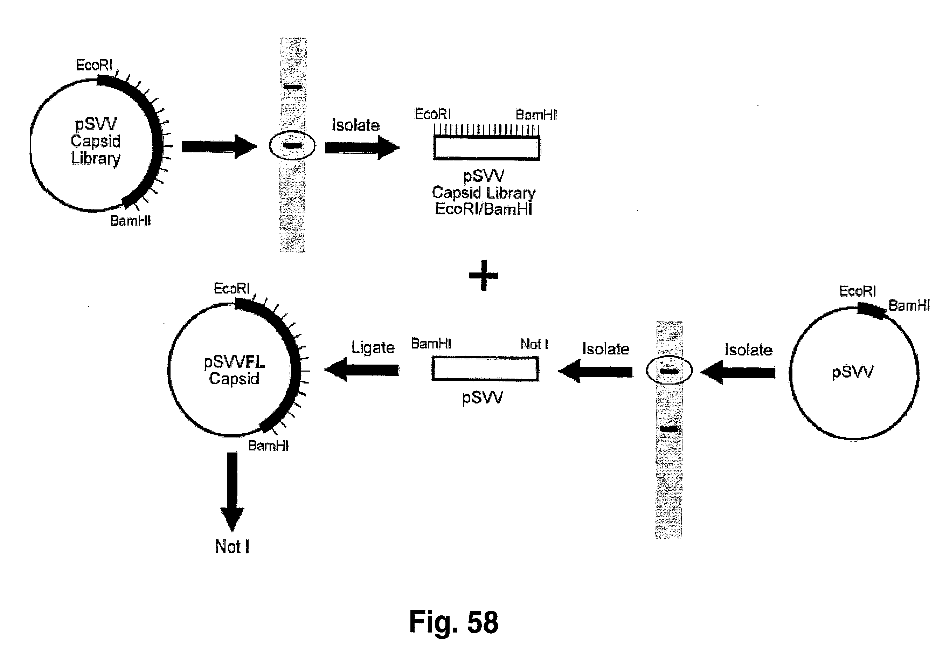

**Figure 58** presents a scheme to generate a library of full-length SVV mutants comprising sequence mutations in the capsid encoding region (Example 16). For example, the capsid encoding region from a pSVV capsid mutant library (generated according to the scheme depicted in Figure 57, for example) is isolated by restriction digestion and gel purification. The vector containing the full-length SVV sequence is also digested such that the capsid encoding region is cut out. The capsid encoding region from the pSVV capsid mutant library is then ligated to the pSVV vector that is missing its wild-type capsid sequence, thereby generating a library of full-length SVV mutants (the "pSVVFL" vector) having a plurality of mutations in the capsid encoding region.

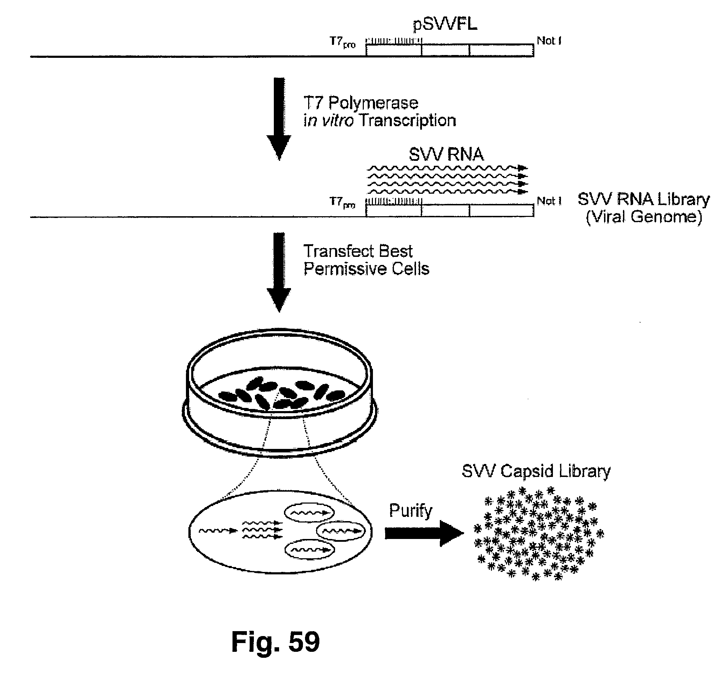

**Figure 59** presents a general method for producing the SVV virus particles comprising a library of capsid mutations (Example 16). The pSVVFL vector is reverse-transcribed to generate SVV RNA. The SVV RNA is transfected into permissive cells, wherein SVV mutant virus particles are produced. The virus particles lyse the cells and a population of SVV virus particles comprising a plurality of capsid variants, "SVV capsid library," are isolated.

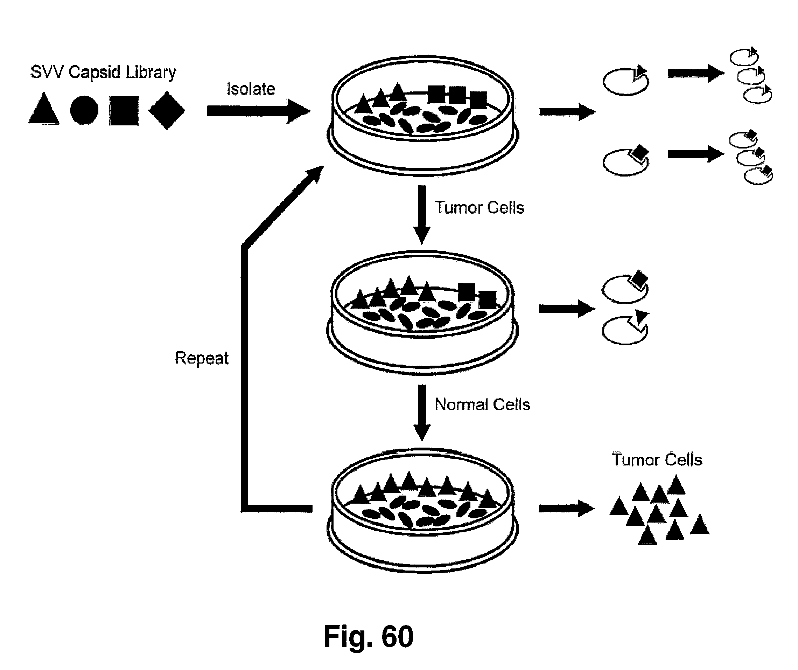

**Figure 60** shows a general method for screening SW capsid mutants that can specifically infect tumor cells while being unable to infect non-tumor cells. The SVV capsid library is incubated with a tumor cell line or tissue of interest. After an initial incubation period, the cells are washed such that SVV virus particles that were unable to gain entry into the cells are eliminated. The cells are then maintained in culture until viral lysis is observed. Culture supernatant is then collected to isolate SW capsid mutants that were able to lytically infect the tumor cell. These viruses can then be grown-up by infecting a known permissive cell-line prior to a counter-screen. A counter-screen is performed by incubating the SW capsid mutant viruses that were able to infect the tumor cell with normal cells. Only those viruses that remain unbound in the supernatant are collected, thereby isolating mutant SW viruses that have tumor-specificity. This process can be repeated to further refine the isolation of SW tumor-specific viruses.

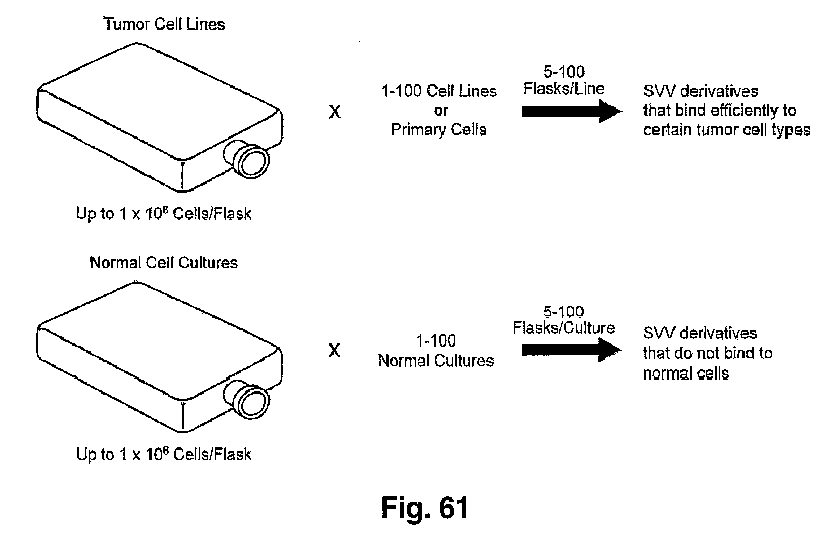

**Figure 61** shows a traditional method for testing whether virus mutants can bind and/or infect cell lines. Traditional methods require what are often inefficient methods for growing cell-lines, *i.e.* flasks, such that a mass-screen of a library of virus mutants in relation to a number of different cell-lines becomes burdensome.

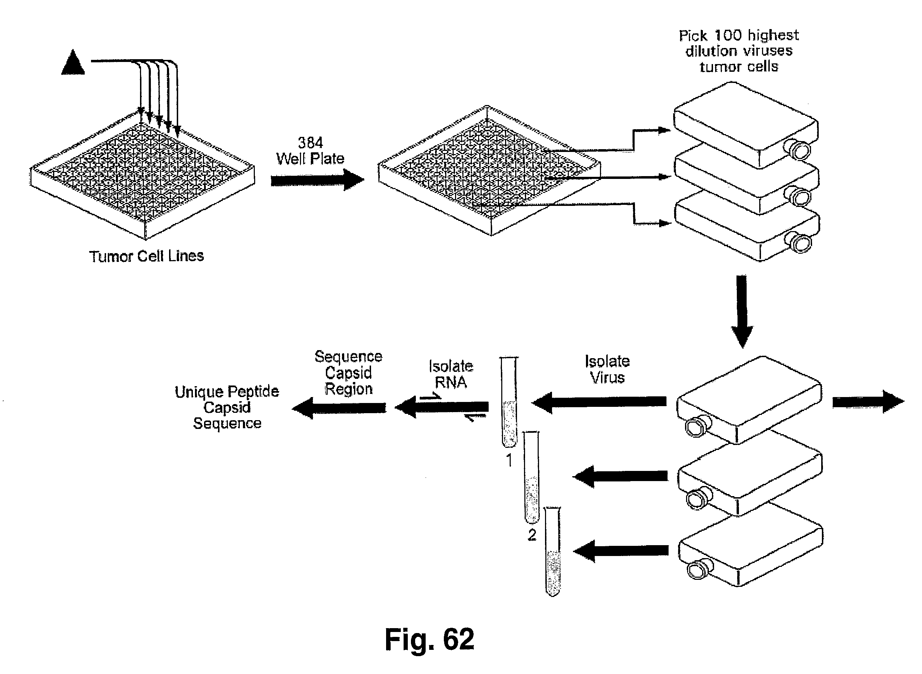

**Figure 62** shows a high-throughput method of the invention for screening virus mutants that have the ability to specifically infect different cell-lines (Example 16). In this figure, a number of different tumor cell-lines are grown in a 384 well-plate. To each well, a sample of a virus is added (for example, a sample of a SVV capsid library). From those wells which show cytopathic effects, the media is collected such that any viruses in the media can be amplified by infecting permissive cell lines (for example, for SVV, H446 or PER.C6) in flasks or large tissue culture plates. The viruses are grown such that the RNA can be isolated and the sequence analyzed to determine the encoded peptide sequence inserted by the oligonucleotide-insertion mutagenesis of the capsid. The peptide itself can then be tested to determine whether it can bind to a tumor cell-type specifically.

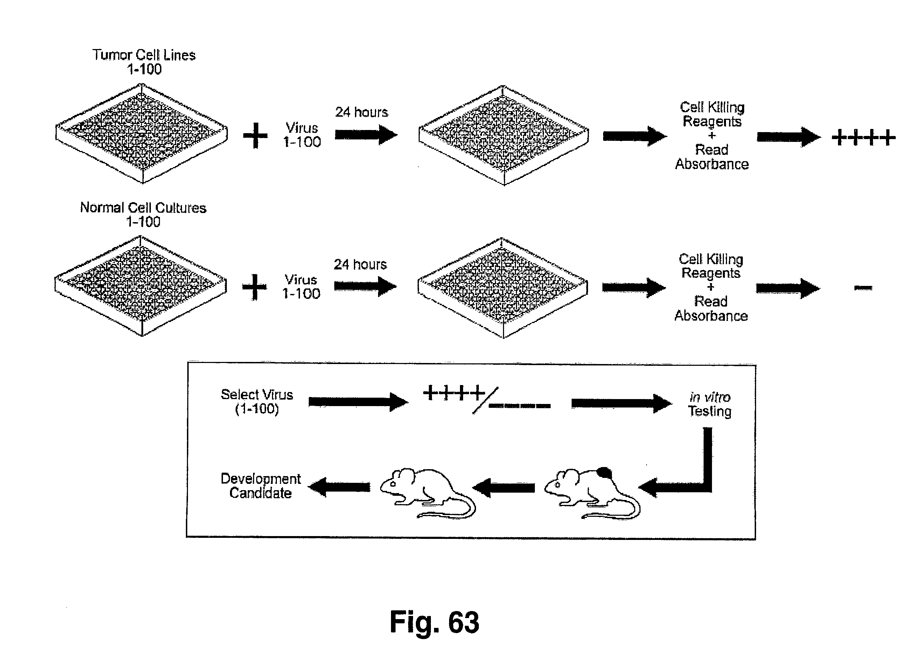

**Figure 63** shows another high-throughput screening schematic (Example 16). Tumor and normal cell lines are grown in multi-well plates. Viruses are added to each well to test whether the cells are killed by virus-mediated lysis. Cytopathic effects can be quickly assayed by reading the light-absorbance in each well. Viruses from the wells showing cytopathic effects are grown up and tested in further *in vitro* (re-testing of tumor and normal cell lines) and *in vivo* models (testing whether the virus can kill explanted tumors in mice).

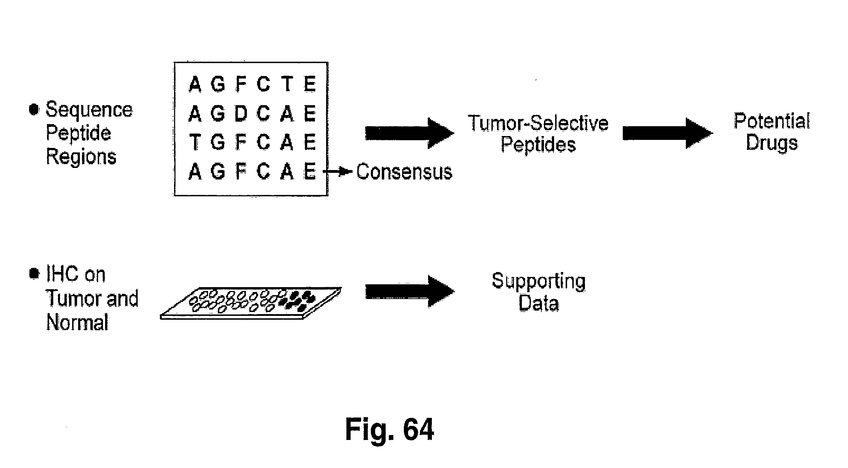

**Figure 64** shows that SVV capsid mutants (SEQ ID NOS: 45-48, respectively, in order of appearance) having new tumor-specific tropisms can be analyzed to generate tumor-selective peptides. Those SVV capsid mutants that enable the specific infection of a tumor cell line are sequenced to determine the peptide encoded by the oligonucleotide insertion. An amino acid consensus sequence can thereby be determined from the successful capsid mutants. Peptides having the consensus sequence can then be tested to determine whether they can bind specifically to the tumor cell-type in question. Tumor-selective peptides can then be attached to toxins or drugs in order to serve as tumor-specific targeting vehicles.

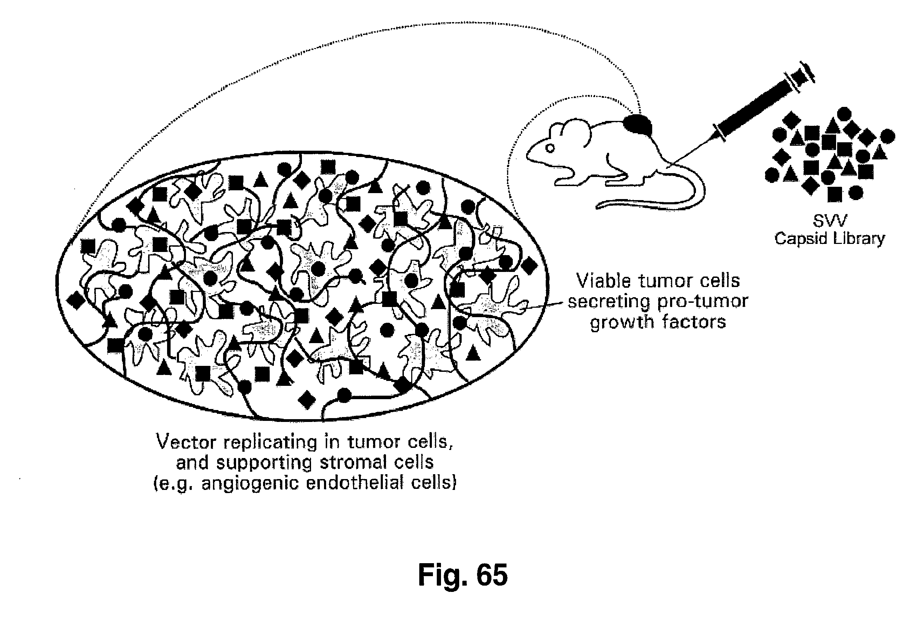

**Figure 65** illustrates that an SVV capsid library can be first *tested in vivo.* Mice (including normal, athymic, nude, CD-1 transgenics, etc.) can be explanted with a specific tumor. These mice are then injected with a SW derivative library, such as a SW capsid library. At certain time points, tumor cells are recovered from the mice, such that in those mice that display the elimination of a tumor, viruses will be isolated from initial tumor samples and grown-up in permissive cell lines.

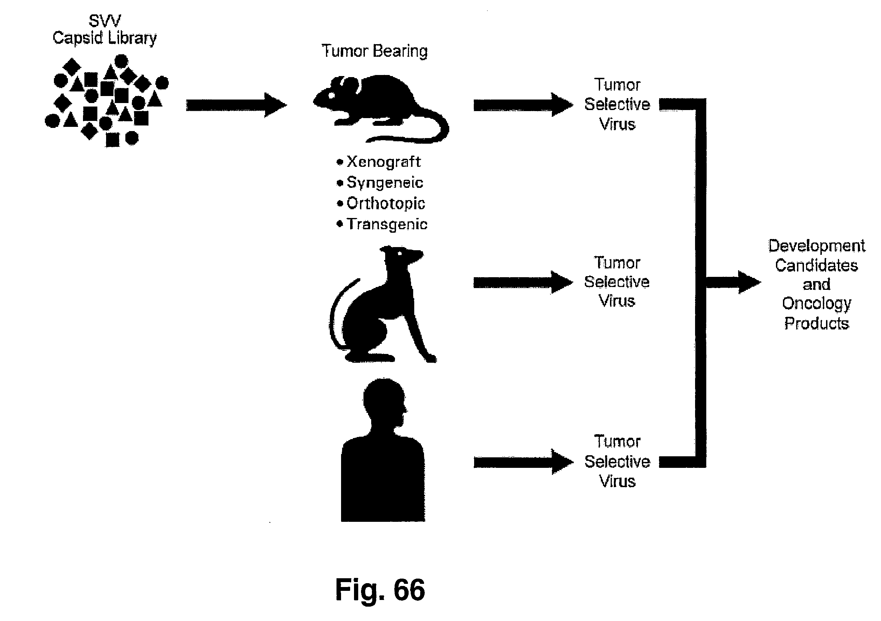

**Figure 66** shows a clinical testing program for the SVV derivatives of the present invention.

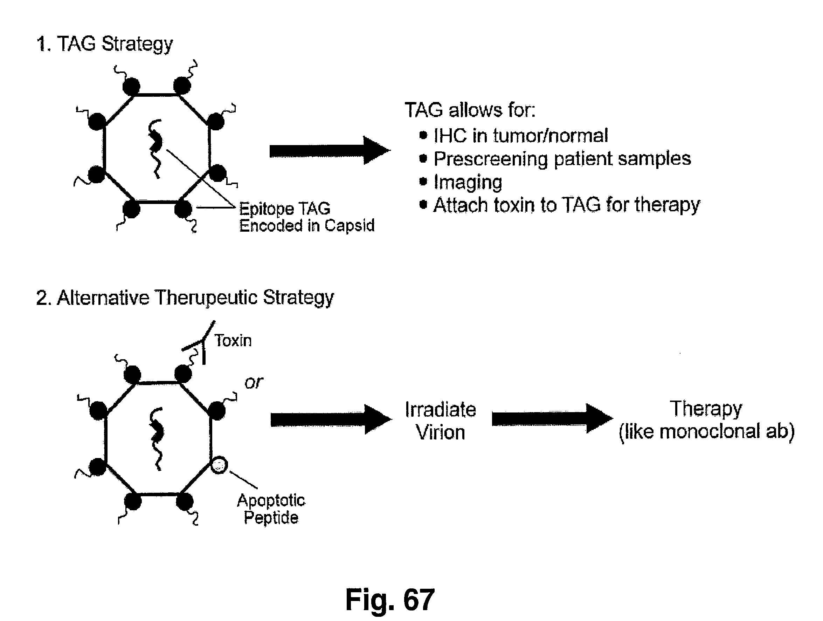

**Figure 67** illustrates that SW derivatives (with new tumor tropisms) encoding epitope tags in their capsid can be used for a variety of purposes. They can be used as a screening reagent to detect whether a specific tumor cell is present in tissue samples by assaying for the presence of the epitope. Alternatively, toxins or other therapeutics can be attached to the epitope tag, and the virus then administered to patients. Further, wild-type or derivative SVV can be irradiated or inactivated such that the virus particle itself is used as a therapeutic device. Either the virus particle induces cellular apoptosis due to the presence of apoptosis-inducing peptides, or the particle can have a toxin or some other therapeutic attached such that the virus is used a specific-targeting delivery device.

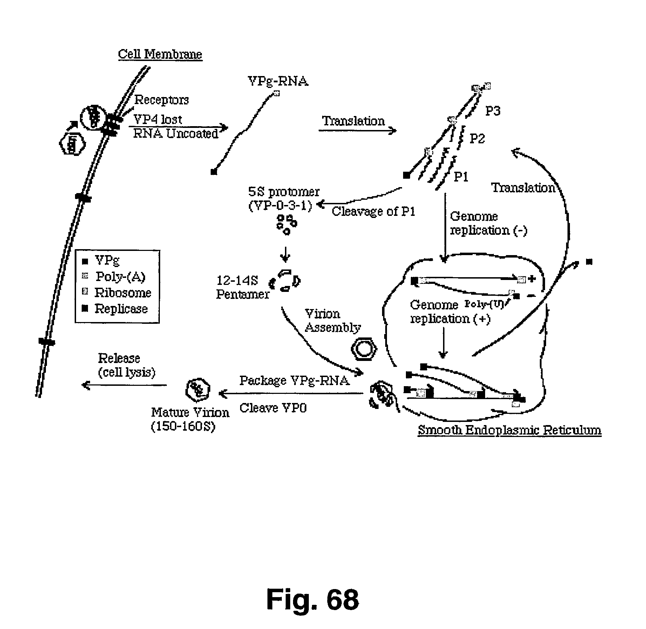

**Figure 68** shows the basic life-cycle of the picornavirus.

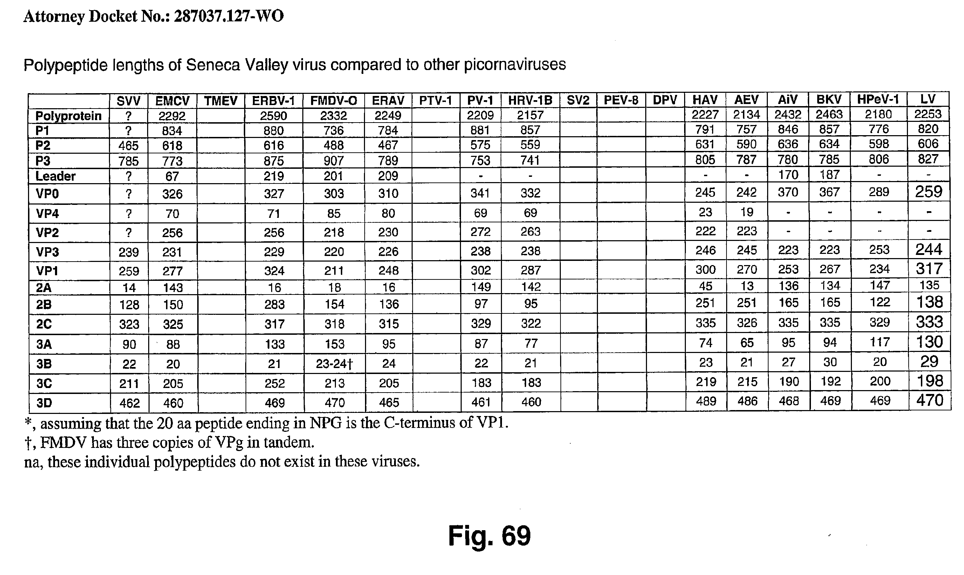

**Figure 69** shows a comparison of the polypeptide lengths of SVV compared to other picornaviruses.

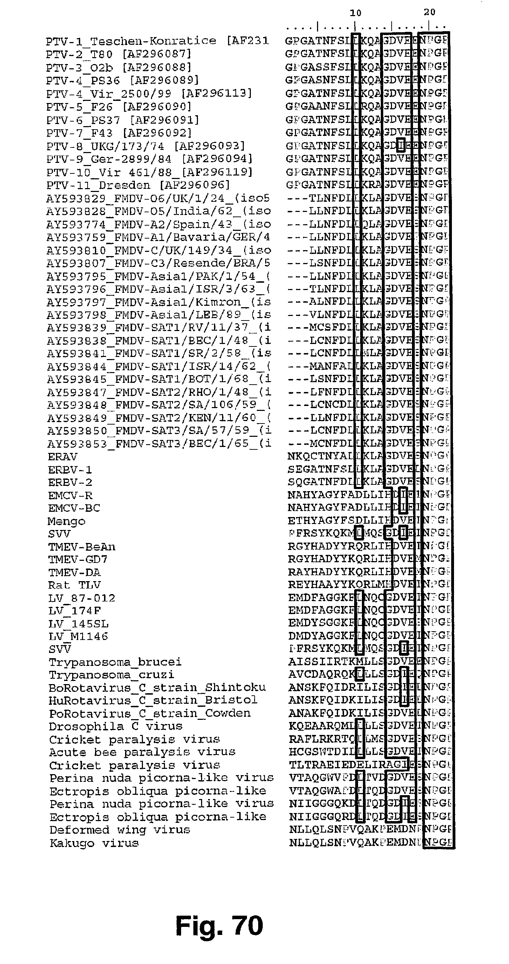

**Figure 70** lists an amino acid comparison of the picornavirus 2A-like NPG/P proteins (SEQ ID NOS: 49-110, respectively, in order of appearance). The sequence for SVV is listed at residues 635-656 of SEQ ID NO: 2.

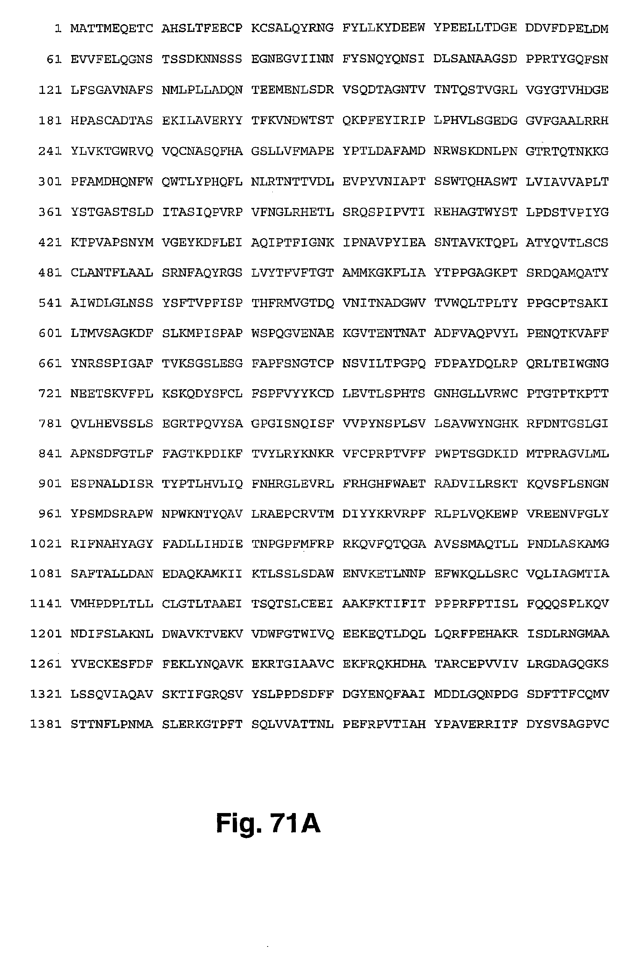

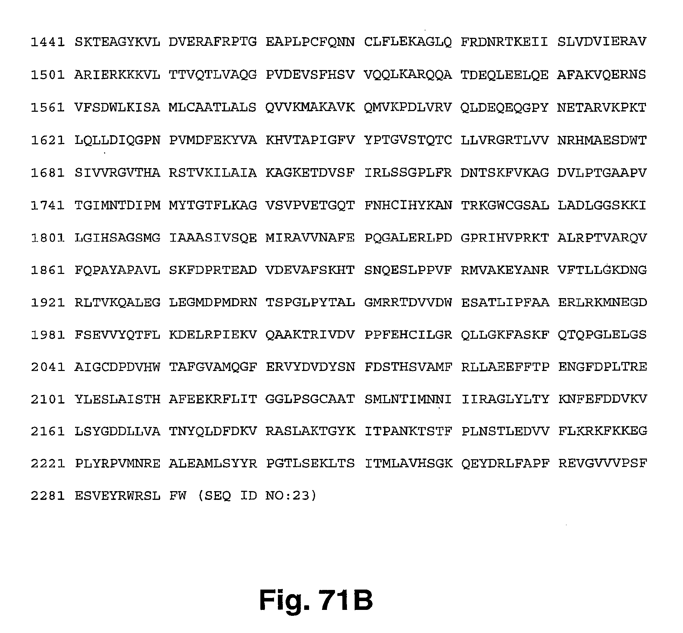

**Figure 71** lists the amino acid sequence (SEQ ID NO:23) for EMCV-R.

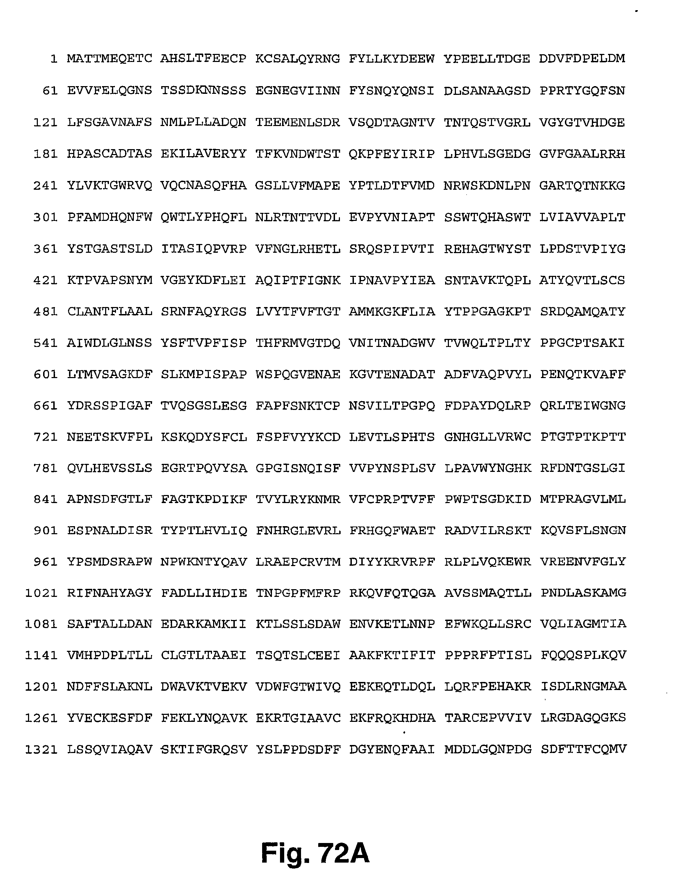

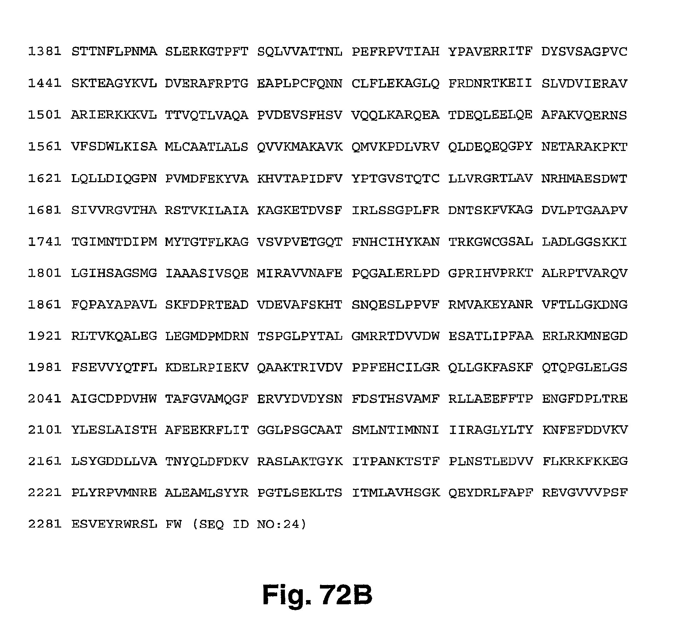

**Figure 72** lists the amino acid sequence (SEQ ID NO:24) for EMCV-PV21 (Accession CAA52361).

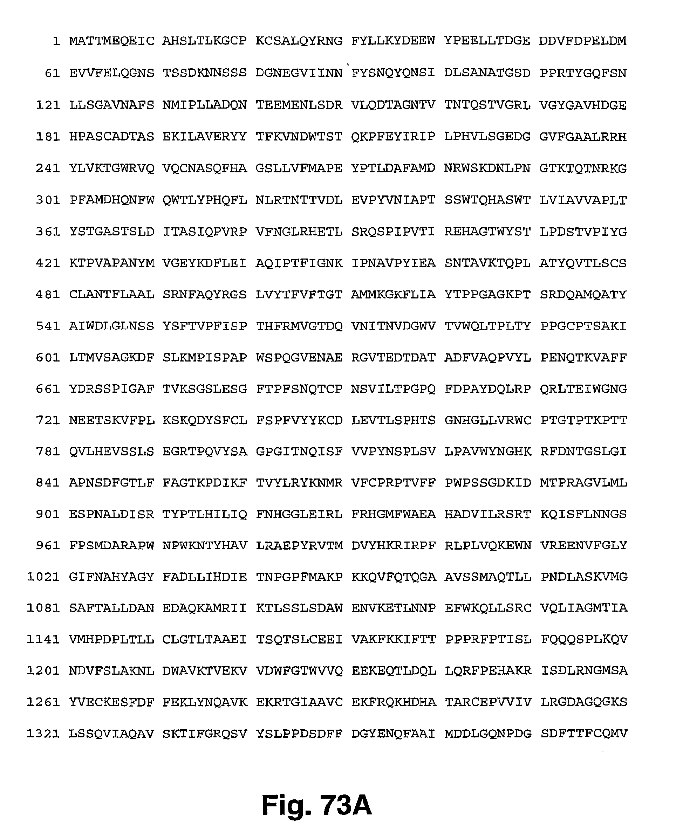

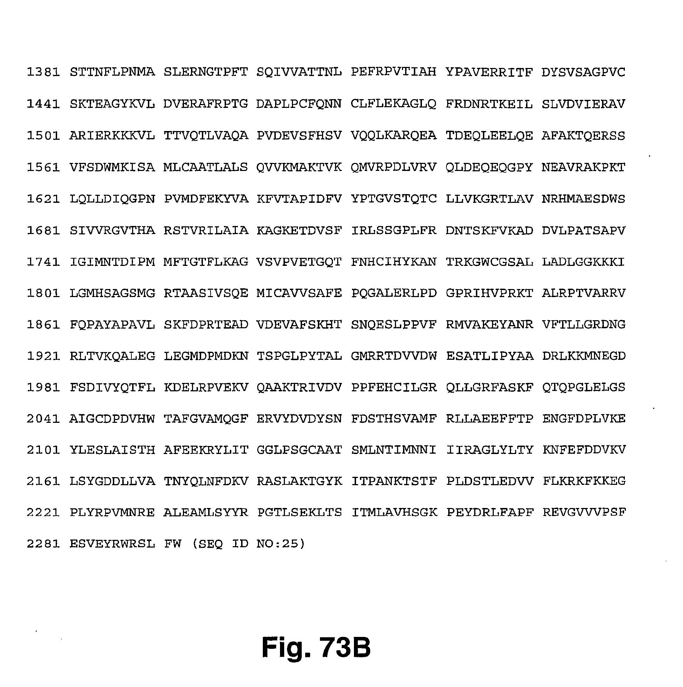

**Figure 73** lists the amino acid sequence (SEQ ID NO:25) for EMCV-B (Accession P17593).

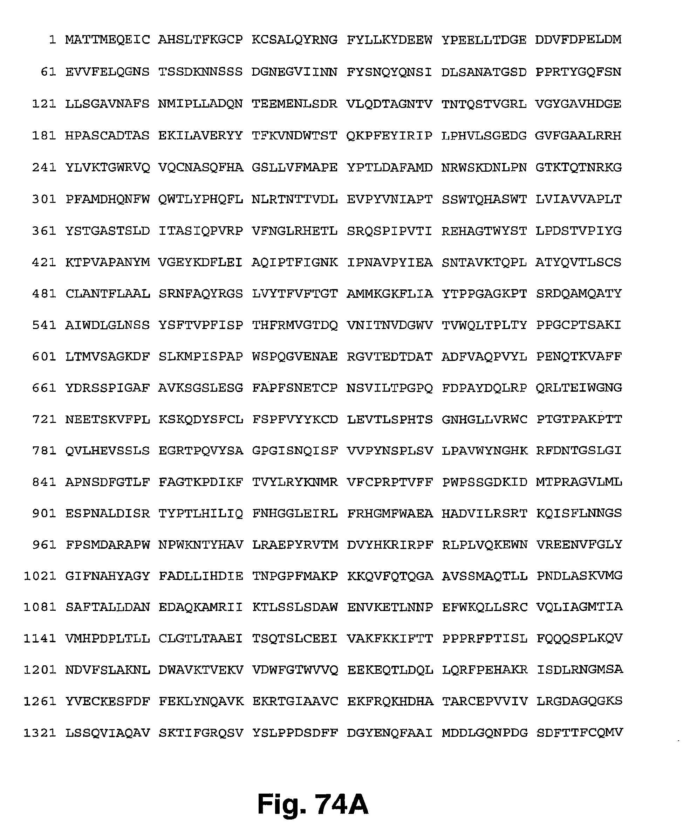

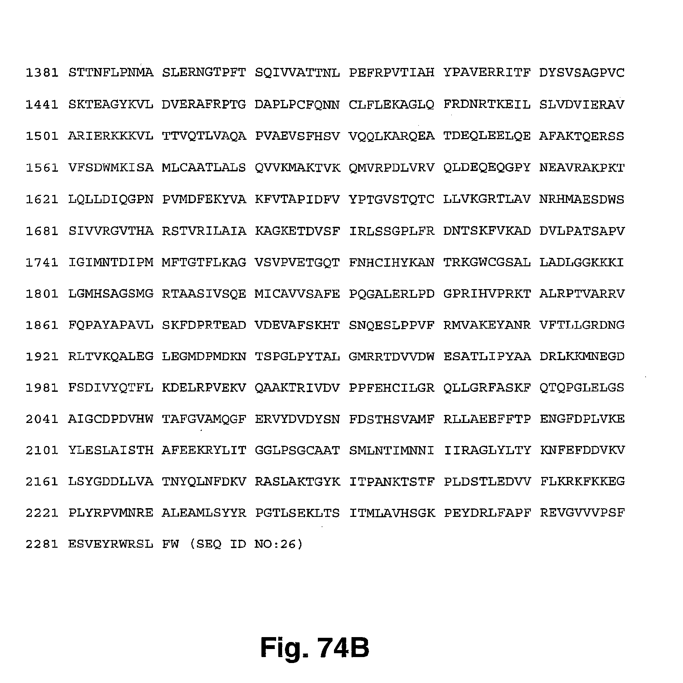

**Figure 74** lists the amino acid sequence (SEQ ID NO:26) for EMCV-Da (Accession P17594).

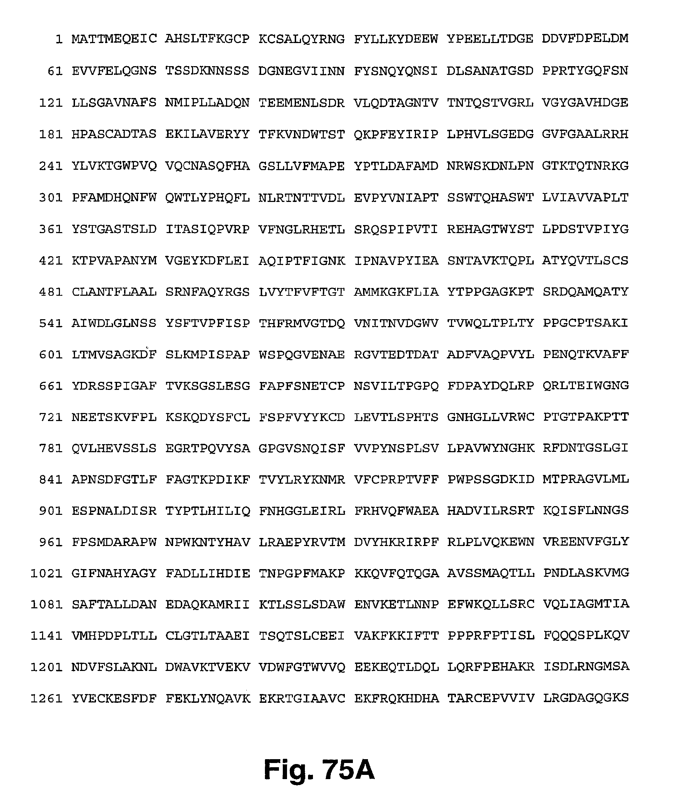

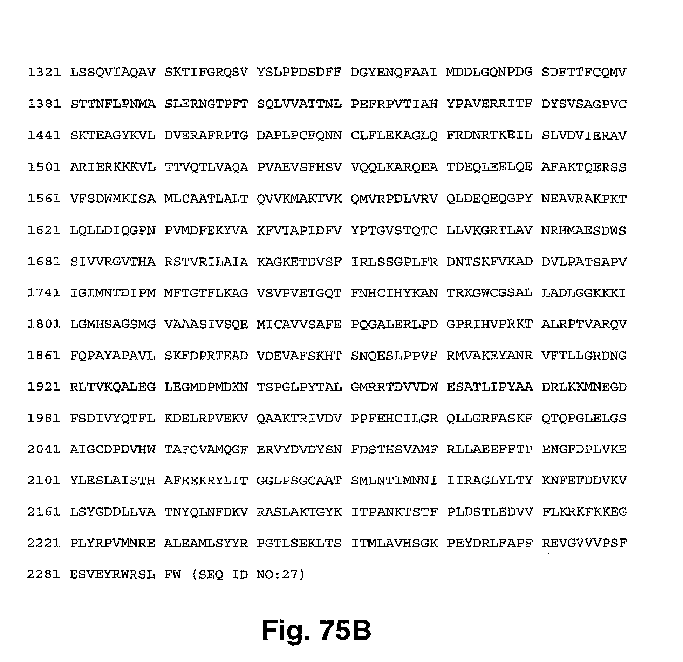

**Figure 75** lists the amino acid sequence (SEQ ID NO:27) for EMCV-Db.

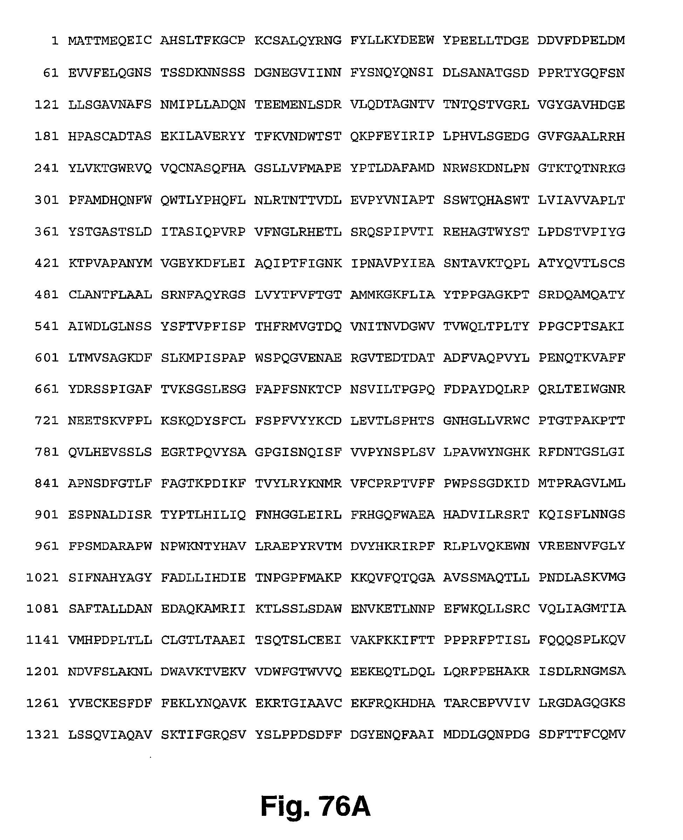

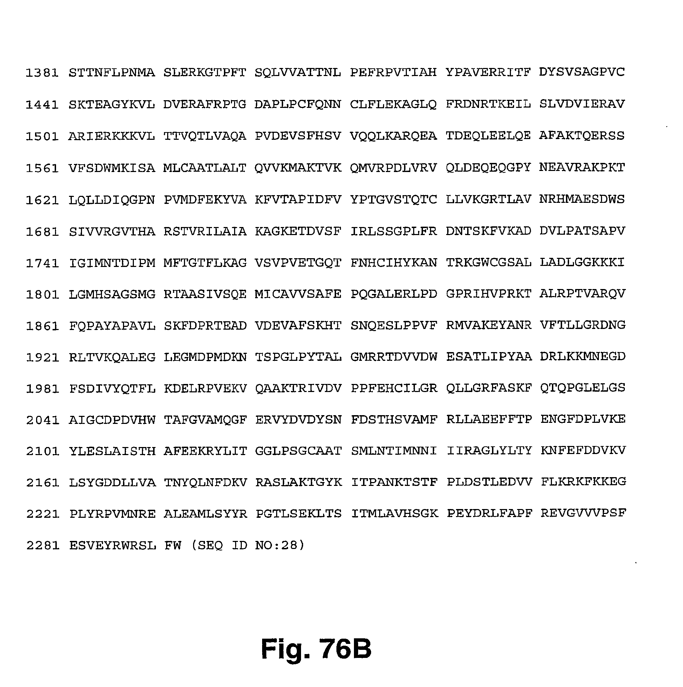

**Figure 76** lists the amino acid sequence (SEQ ID NO:28) for EMCV-PV2 (Accession CAA60776).

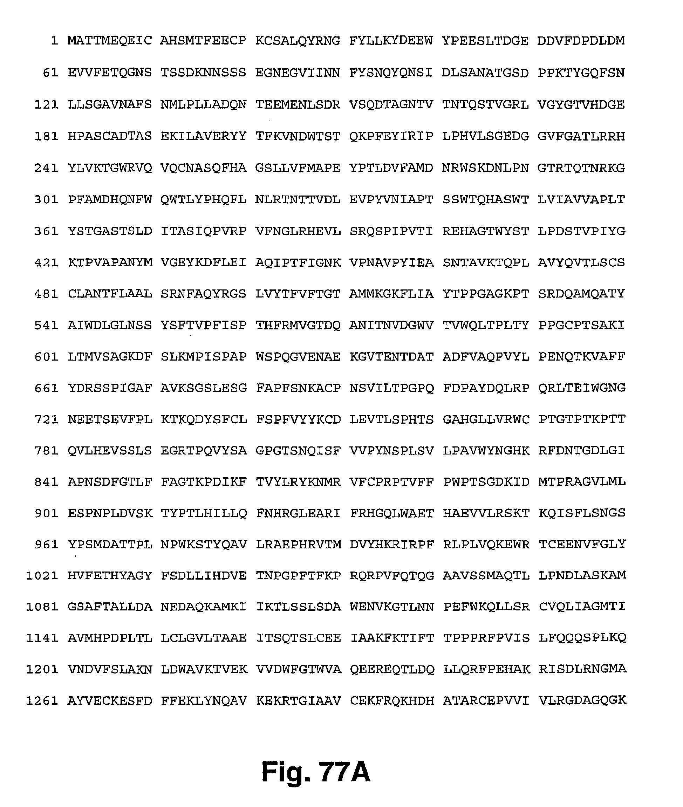

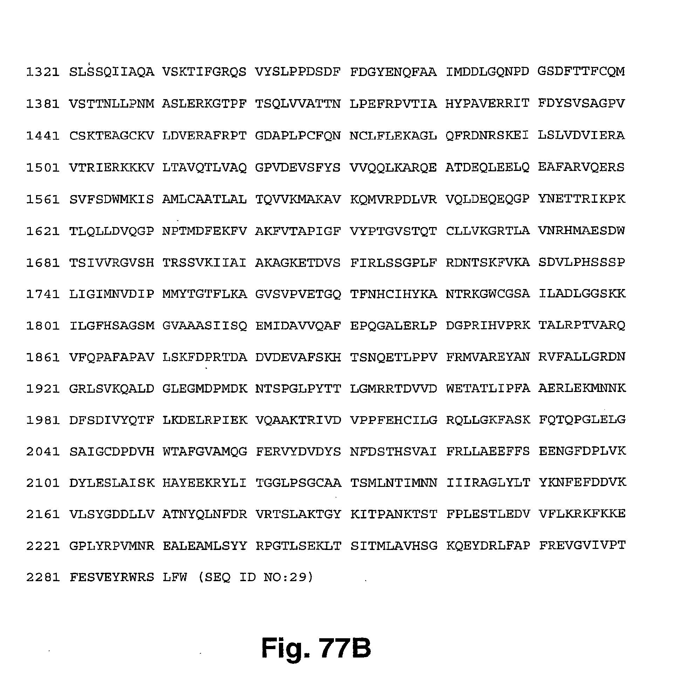

**Figure 77** lists the amino acid sequence (SEQ ID NO:29) for EMCV-mengo (Accession AAA46547).

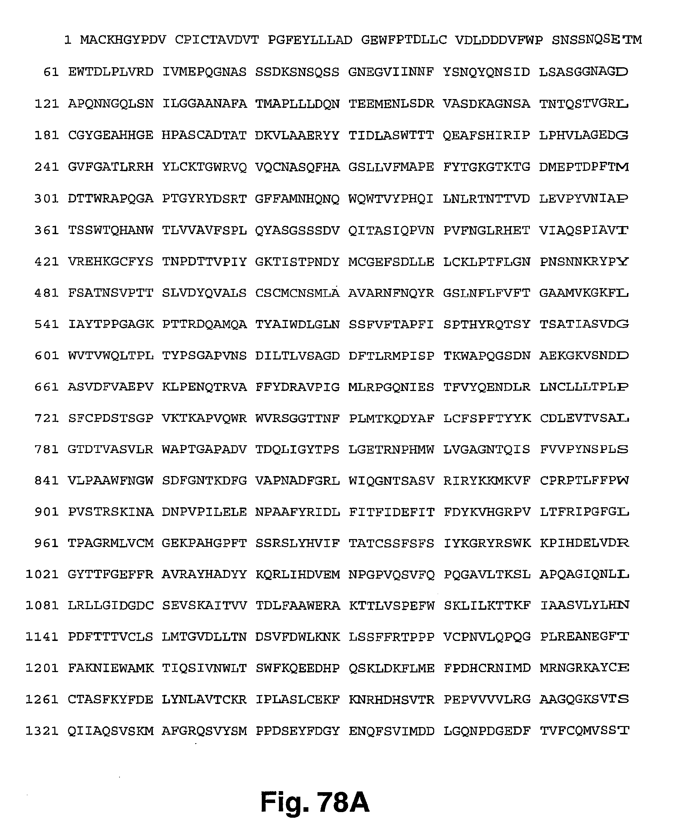

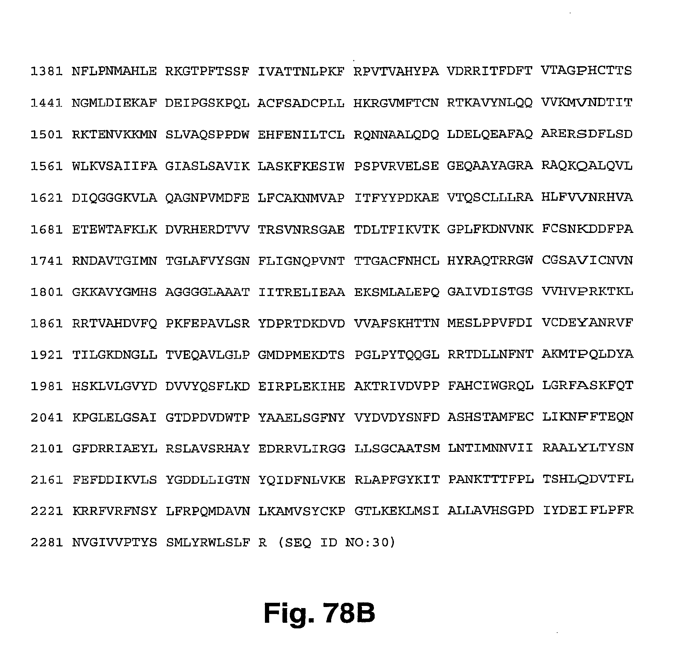

**Figure 78** lists the amino acid sequence (SEQ ID NO:30) for TMEV/DA (Accession AAA47928).

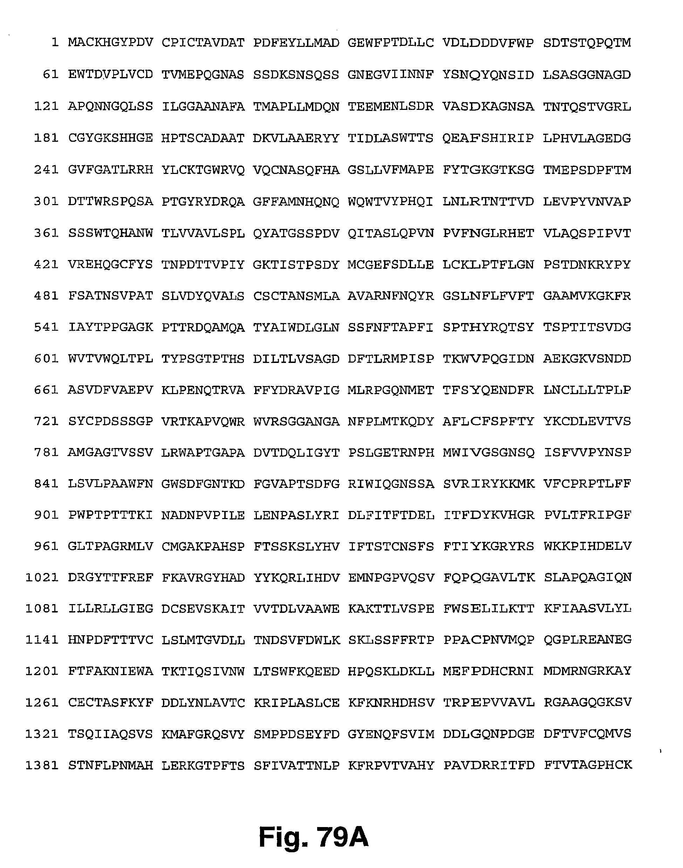

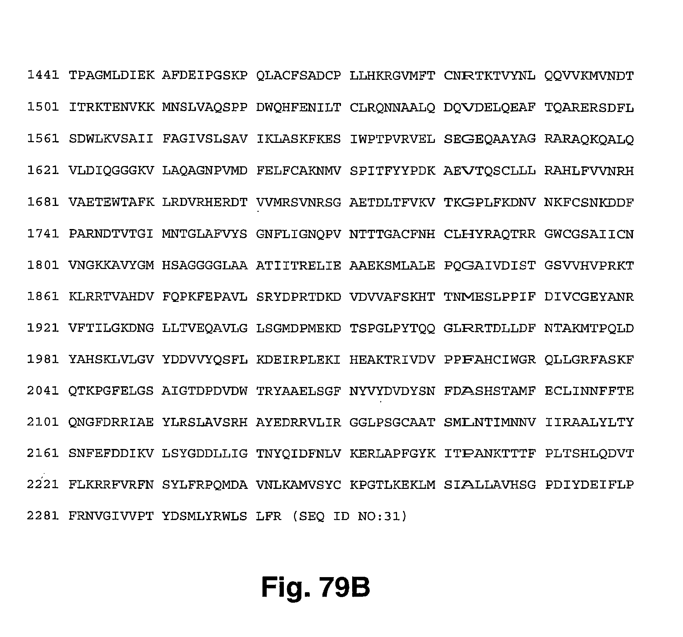

**Figure 79** lists the amino acid sequence (SEQ ID NO:31) for TMEV/GDVII (Accession AAA47929).

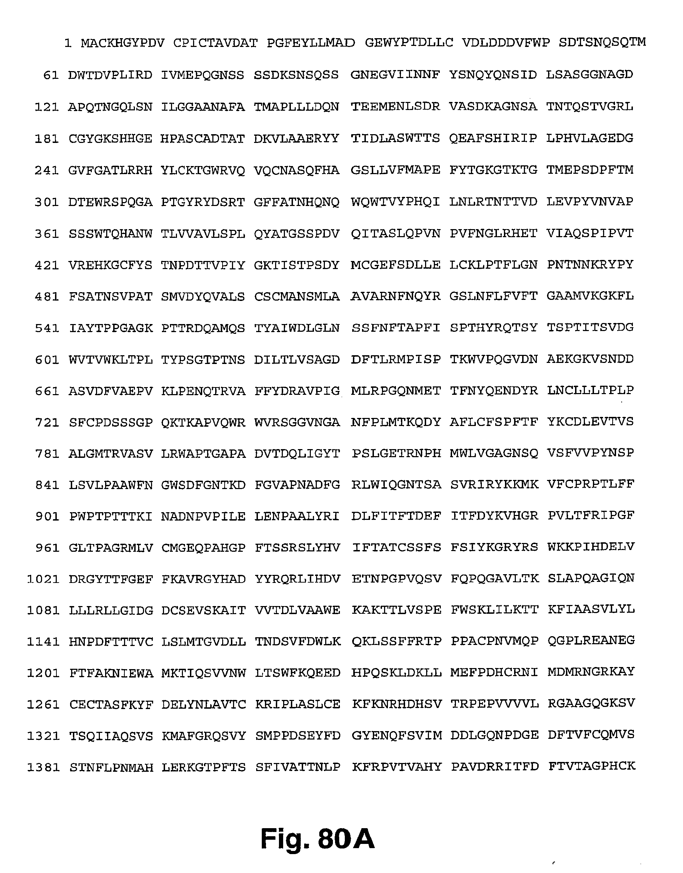

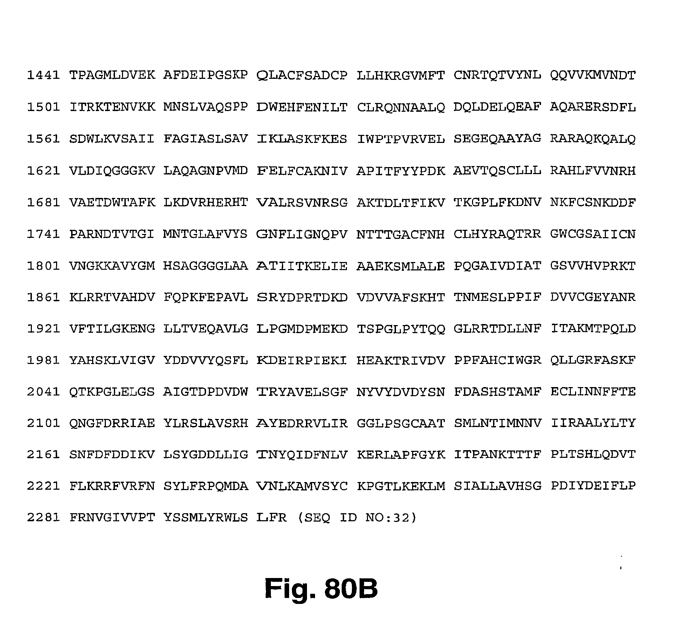

**Figure 80** lists the amino acid sequence (SEQ ID NO:32) for TMEV/BeAn8386 (Accession AAA47930).

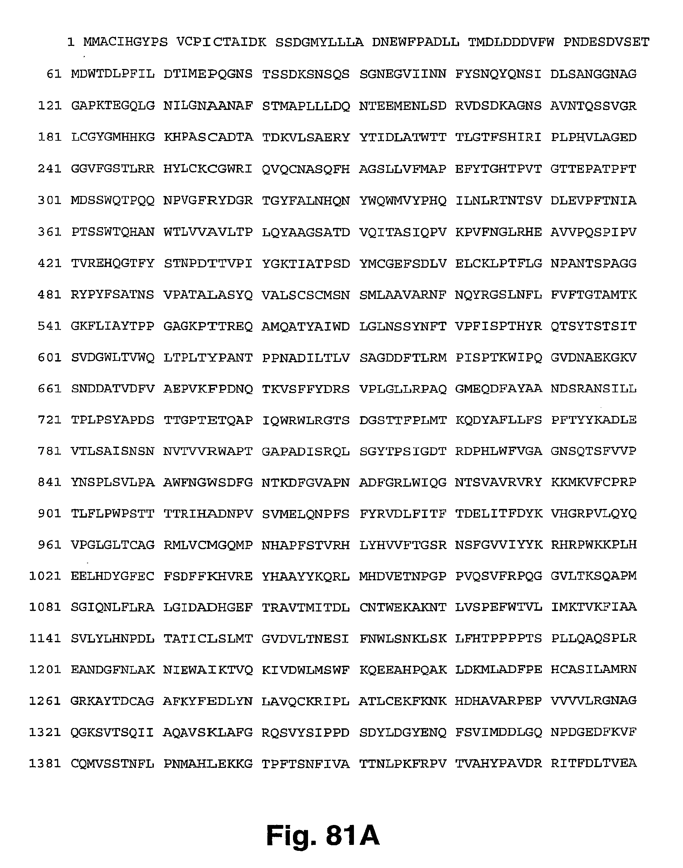

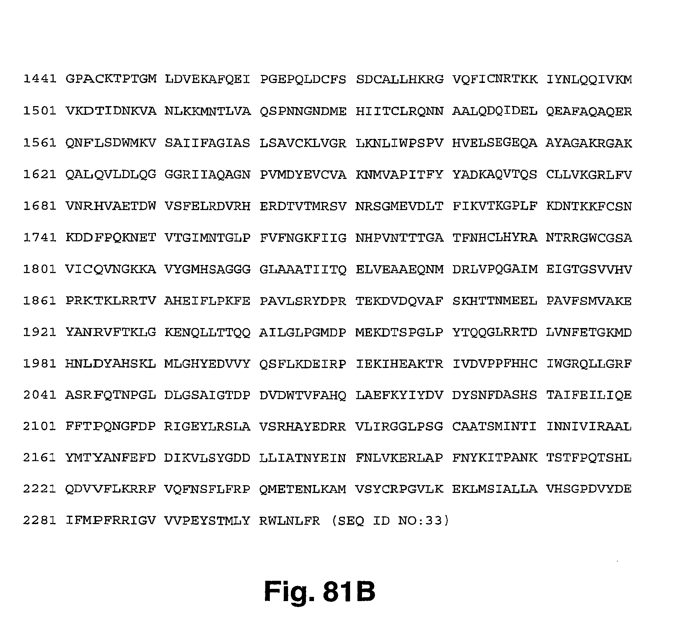

**Figure 81** lists the amino acid sequence (SEQ ID NO:33) for TLV-NGS910 (Accession BAC58035).

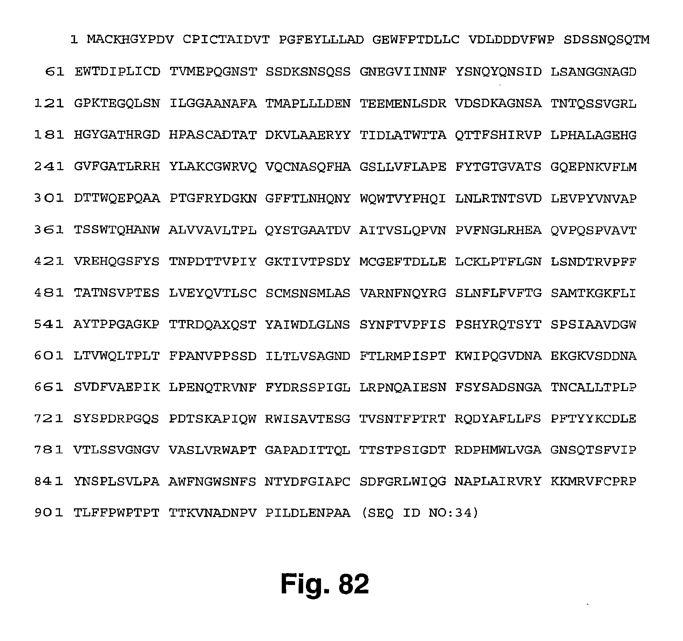

**Figure 82** lists the amino acid sequence (SEQ ID NO:34) for VHEV/Siberia-55 (Accession AAA47931).

## DETAILED DESCRIPTION OF THE INVENTION

The terms "virus," "viral particle," "virus particle," "vector particle," "viral vector particle," and "virion" are used interchangeably and are to be understood broadly - for example - as meaning infectious viral particles that are formed when, *e.g.,* a viral vector of the invention is transduced or transfected into an appropriate cell or cell line for the generation of infectious particles.

The terms "derivative," "mutant," "variant" and "modified" are used interchangeably to generally indicate that a derivative, mutant, variant or modified virus can have a nucleic acid or amino acid sequence difference in respect to a template viral nucleic acid or amino acid sequence. For example, a SVV derivative, mutant, variant or modified SVV may refer to a SW that has a nucleic acid or amino acid sequence difference with respect to the wild-type SVV nucleic acid or amino acid sequence of ATCC Patent Deposit Number PTA-5343.

As used herein, the terms "cancer," "cancer cells," "neoplastic cells," "neoplasia," "tumor," and "tumor cells," are used interchangeably, and refer to cells that exhibit relatively autonomous growth, so that they exhibit an aberrant growth phenotype characterized by a significant loss of control of cell proliferation. Neoplastic cells can be malignant or benign. According to the present invention, one type of preferred tumor cells are those with neurotropic properties.

The terms "identical" or percent "identity" in the context of two or more nucleic acid or protein sequences, refer to two or more sequences or subsequences that are the same or have a specified percentage of amino acid residues or nucleotides that are the same, when compared and aligned for maximum correspondence, as measured using a sequence comparison algorithm such as Protein-Protein BLAST (Protein-Protein BLAST of GenBank databases (Altschul, S.F., Gish, W., Miller, W., Myers, E.W. & Lipman, D.J. (1990) "Basic local alignment search tool." J. Mol. Biol. 215:403-410)) or by visual inspection. The BLAST algortihm is described in Altschul et al., J. Mol. Biol., 215:403-410 (1990), and publicly available BLAST software is provided through the National Center for Biotechnology Information (NCBI) (http://www.ncbi.nlm.nih.gov/).

For example, as used herein, the term "at least 90% identical to" refers to percent identities from 90 to 100 relative to the reference polypeptides (or polynucleotides). Identity at a level of 90% or more is indicative of the fact that, assuming for exemplification purposes a test and reference polypeptide length of 100 amino acids are compared, no more than 10% (*i.e.,* 10 out of 100) amino acids in the test polypeptide differs from that of the reference polypeptide. Similar comparisons can be made between a test and reference polynucleotide. Such differences can be represented as point mutations randomly distributed over the entire length of an amino acid sequence or they can be clustered in one or more locations of varying length up to the maximum allowable, e.g., 10 out of 100 amino acid difference (90% identity). Differences are defined as nucleic acid or amino acid substitutions, insertions or deletions. At the level of identities above about 85-90%, the result should be independent of the program and gap paramaters set; such high levels of identity can be assessed readily, often without relying on software.

In the context of the present invention, the term "isolated" refers to a nucleic acid molecule, polypeptide, virus or cell that, by the hand of man, exists apart from its native environment. An isolated nucleic acid molecule or polypeptide may exist in a purified form or may exist in a non-native environment, such as, for example, a recombinant host cell. An isolated virus or cell may exist in a purified form, such as in a cell culture, or may exist in a non-native environment such as, for example, a recombinant or xenogeneic organism.

The term "naturally occurring" or "wildtype" is used to describe an entity that can be found in nature as distinct from being artificially produced by man. For example, a protein or nucleotide sequence present in an organism (including a virus), which can be isolated from a source in nature and which has not been intentionally modified by man in the laboratory, is naturally occurring.

The concepts of "high stringency," "intermediate stringency" or "low stringency" refer to nucleic acid hybridization conditions. High stringency conditions refers to conditions that require a greater identity between a target's nucleic acid sequence and a probe's nucleic acid sequence in order for annealing or hybridization to occur between the target and the probe. Low stringency conditions refer to conditions that require a lower identity between a target's nucleic acid sequence and a probe's nucleic acid sequence in order for annealing or hybridization to occur between the target and the probe. Stringency conditions can be controlled by the salt concentration of the buffer or by the temperature at which the hybridization is carried out, where higher salt concentrations result in less stringent conditions and where higher temperatures result in more stringent conditions. Although stringency conditions will vary based on the length and nucleic acid content of the sequences undergoing hybridization, representative conditions of high, intermediate and low stringency are described in the following exemplary conditions. A commonly used hybridization buffer is SSC (sodium chloride sodium citrate) with a 20X stock concentration corresponding to 0.3 M trisodium citrate and 3 M NaCl. For high stringency conditions, the working concentration of SSC can be 0.1X - 0.5X (1.5 - 7.5 mM trisodium citrate, 15 - 75 mM NaCl) with the hybridization temperature set at 65°C. Intermediate conditions typically utilize a 0.5X - 2X SSC concentration (7.5 - 30 mM trisodium citrate, 75 - 300 mM NaCl) at a temperature of 55 - 62°C. Hybridizations conducted under low stringency conditions can use a 2X - 5X SSC concentration (30 - 75 mM trisodium citrate, 300 - 750 mM NaCl) at a temperature of 50 - 55°C. Note that these conditions are merely exemplary and are not to be considered limitations.

Seneca Valley Virus (SVV):

SVV is a novel, heretofore undiscovered RNA virus, most closely related to members from the *Cardiovirus* genus in the *Picornaviridae* family. Thus, for purposes of the present invention, SVV is considered to be a member of the *Cardiovirus* genus and the *Picornaviridae* family. Cardioviruses are distinguished from other picornaviruses by special features of their genome organization, common pathological properites, and the dissociability of their virions at pHs between 5 and 7 in 0.1M NaCl (Scraba, D. et al., "Cardioviruses (Picornaviridae)," in Encyclopedia of Virology, 2nd Edition, R.G. Webseter and A. Granoff, Editors, 1999). The results of sequence analyses between SVV and other Cardioviruses are discussed hereinbelow.

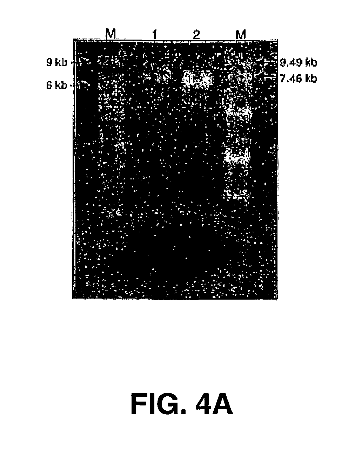

The genome of SW consists of one single-stranded positive (+) sense strand RNA molecule having a predicted size of about 7.5 kb (see Figure 4A). As SW is a picornavirus, it has a number of features that are conserved in all picornaviruses: (i) genomic RNA is infectious, and thus can be transfected into cells to bypass the virus-receptor binding and entry steps in the viral life cycle; (ii) a long (about 600-1200 bp) untranslated region (UTR) at the 5' end of the genome and a shorter 3' untranslated region (about 50-100 bp); (iii) the 5' UTR contains a clover-leaf secondary structure known as the internal ribosome entry site (IRES); (iv) the rest of the genome encodes a single polyprotein and (v) both ends of the genome are modified, the 5' end by a covalently attached small, basic protein, "Vpg," and the 3' end by polyadenylation.

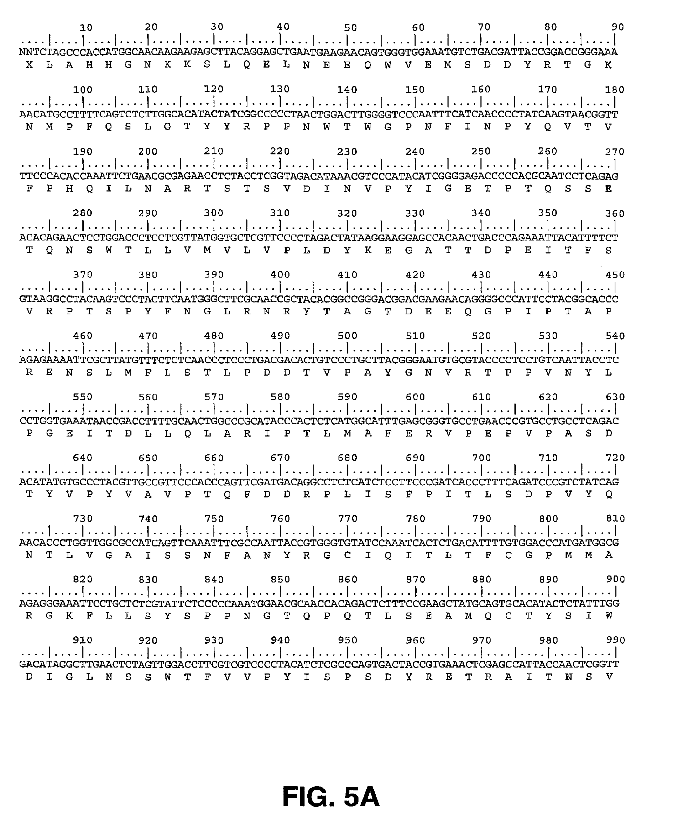

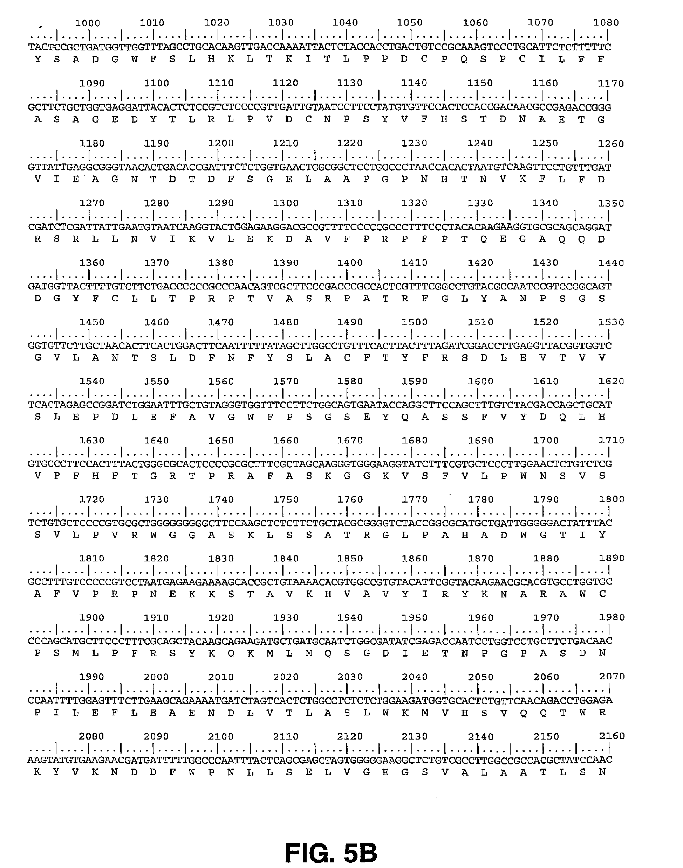

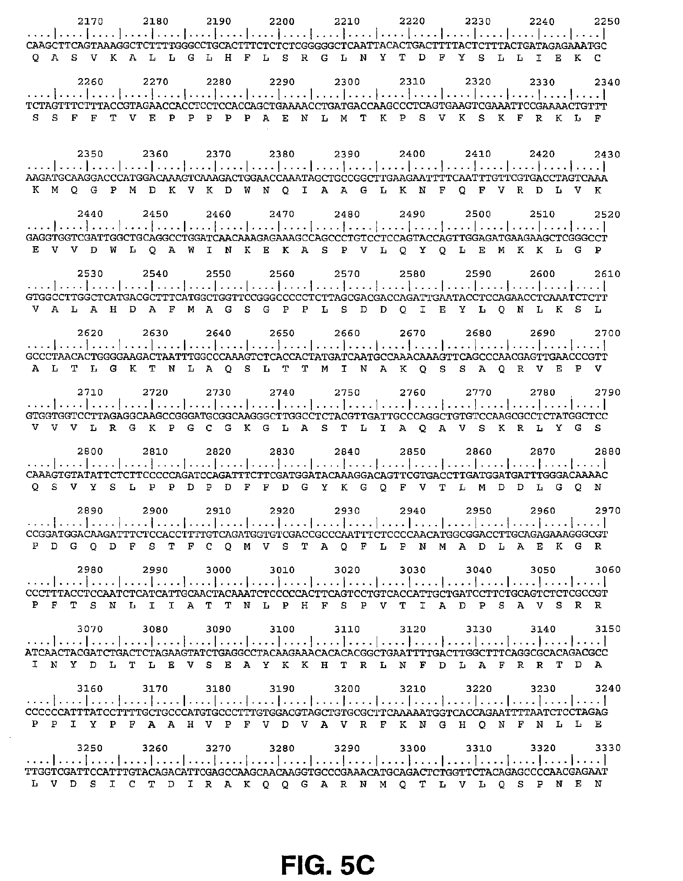

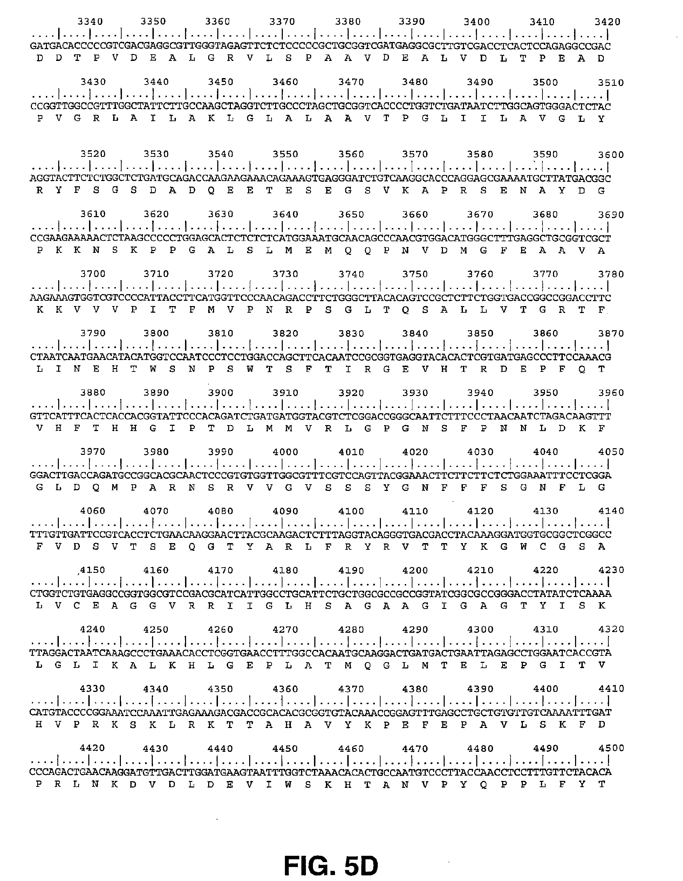

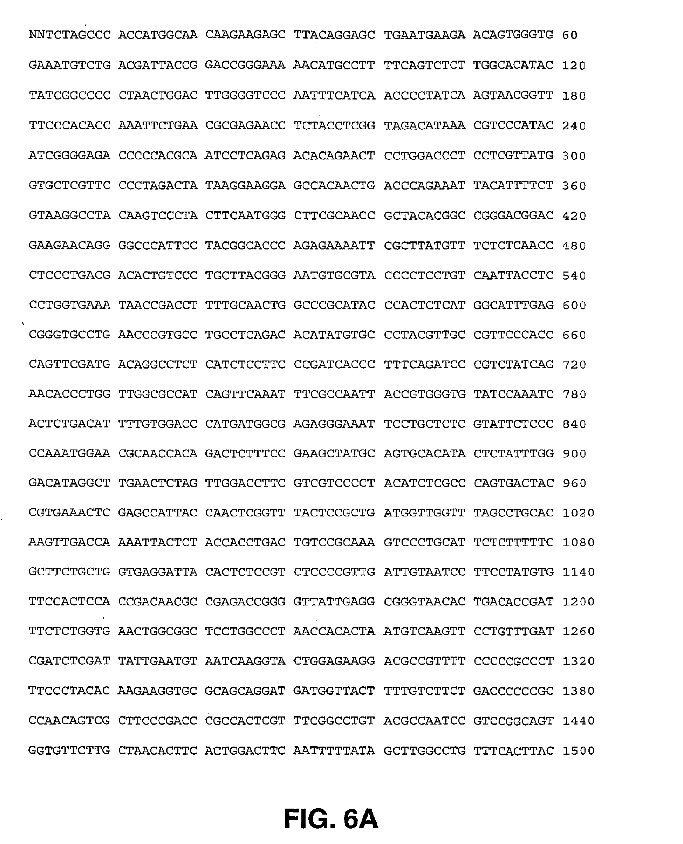

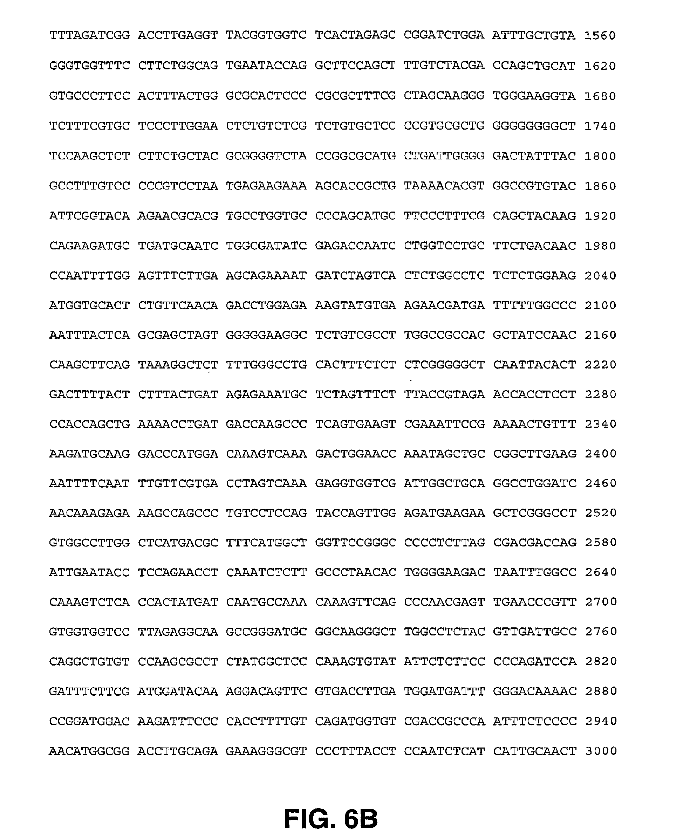

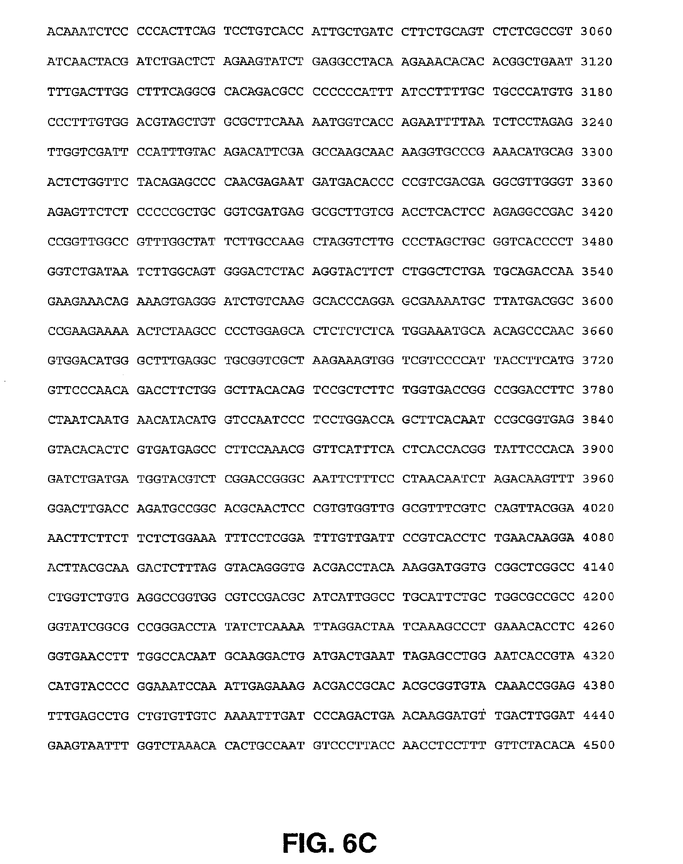

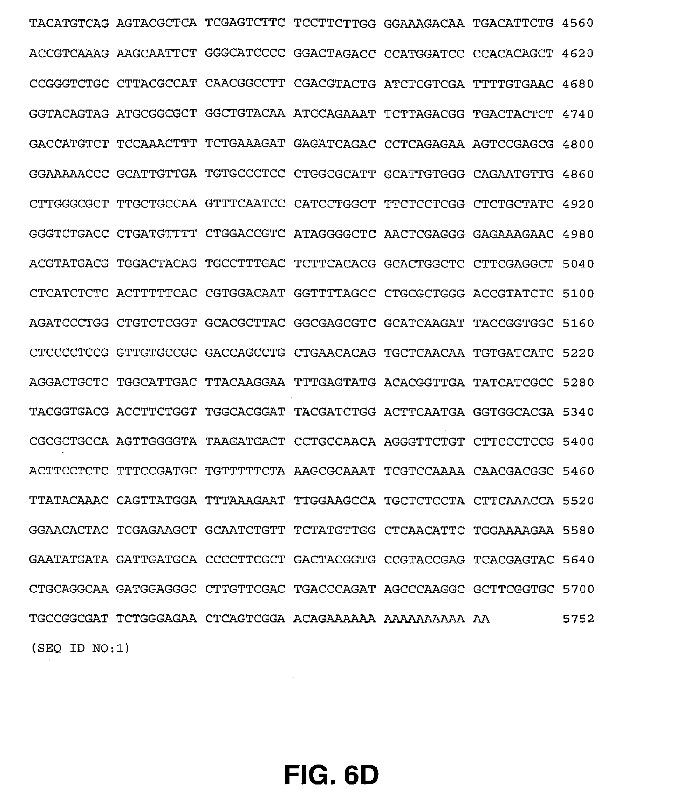

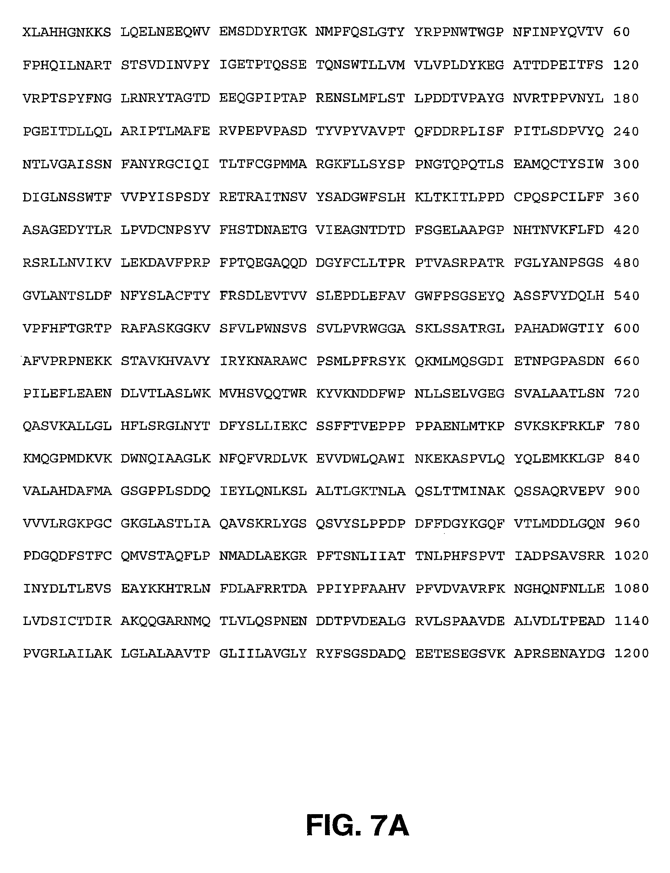

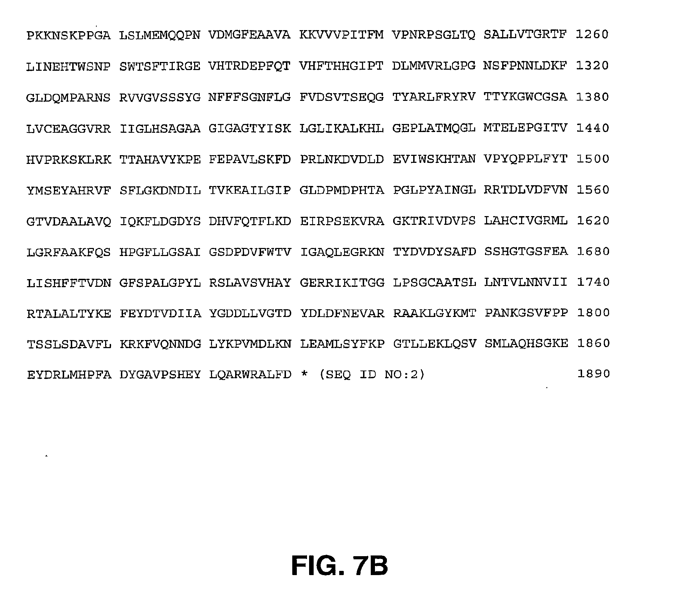

The present invention provides the isolated SW virus (ATCC Patent Deposit number PTA-5343) and the complete genomic content of SVV therefrom. Presently, the largest SVV genomic fragment that has been sequenced is an isolated SVV nucleic acid, derived from the PTA-5343 isolate, that comprises the majority of the SVV genomic sequence, and is listed in Figures 5A-5E and Figures 6A-6D, and has the designation of SEQ ID NO:1 herein. Translation of this nucleotide sequence shows that the majority of the single polyprotein of SVV is encoded by SEQ ID NO:1. The amino acid sequence encoded by nucleotides 1 to 5673 of SEQ ID NO: 1 is listed in Figures 5A-E and Figures 7A-7B has the designation of SEQ ID NO:2 herein. The present invention therefore provides isolated portions of SEQ ID NO: 1, including SEQ ID NOs:3, 5, 7, 9, 11, 13, 15, 17, 19 and 21, that can be subcloned into expression vectors such that polypeptides encoded by these portions of SEQ ID NO: 1 can be isolated.

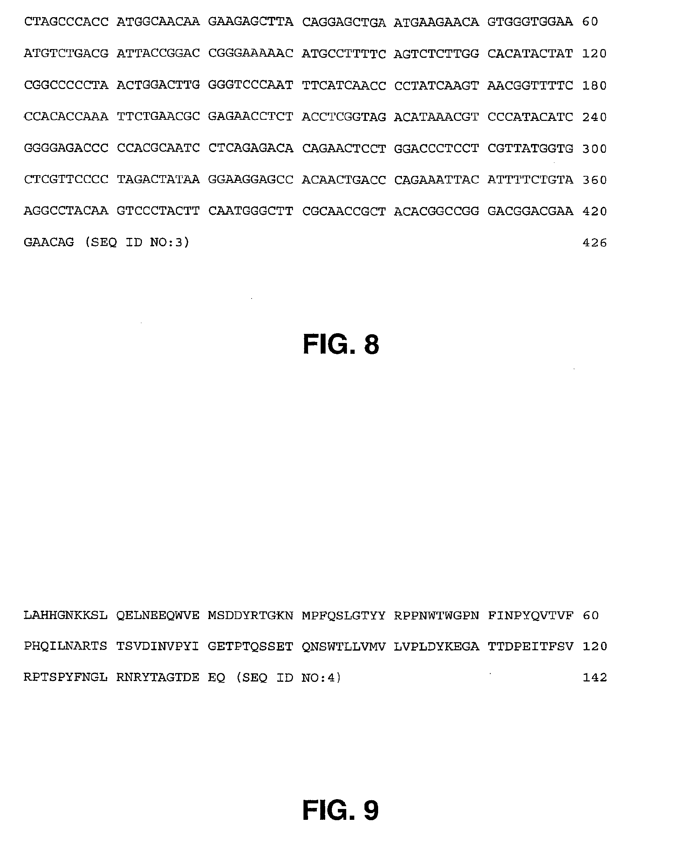

An isolated partial SVV VP2 (1B) protein may be with the amino acid sequence of SEQ ID NO:4, as listed in Figure 9 (which corresponds to amino acids 2-143 of SEQ ID NO:2). The amino acid sequence of the partial SVV VP2 protein is encoded by the nucleic acid sequence of SEQ ID NO:3, as listed in Figure 8 (which corresponds to nucleotides 4-429 of SEQ ID NO: 1).

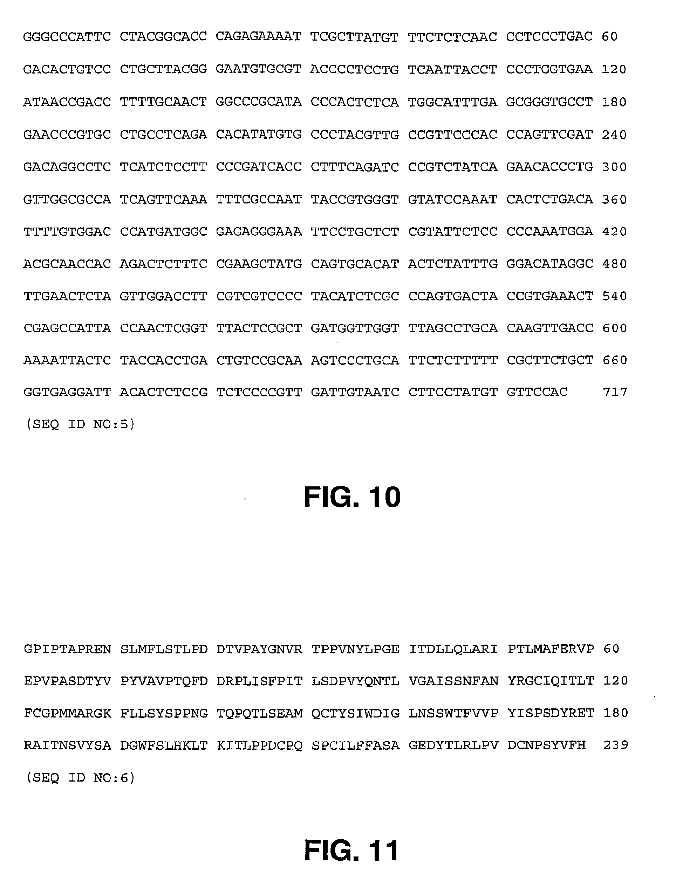

An isolated SVV VP3 (1C) protein may be provided with the amino acid sequence of SEQ ID NO:6, as listed in Figure 11 (which corresponds to amino acids 144-382 of SEQ ID NO:2). The amino acid sequence of the SVV VP3 protein is encoded by the nucleic acid sequence of SEQ ID NO:5, as listed in Figure 10 (which corresponds to nucleotides 430-1146 of SEQ ID NO:1).

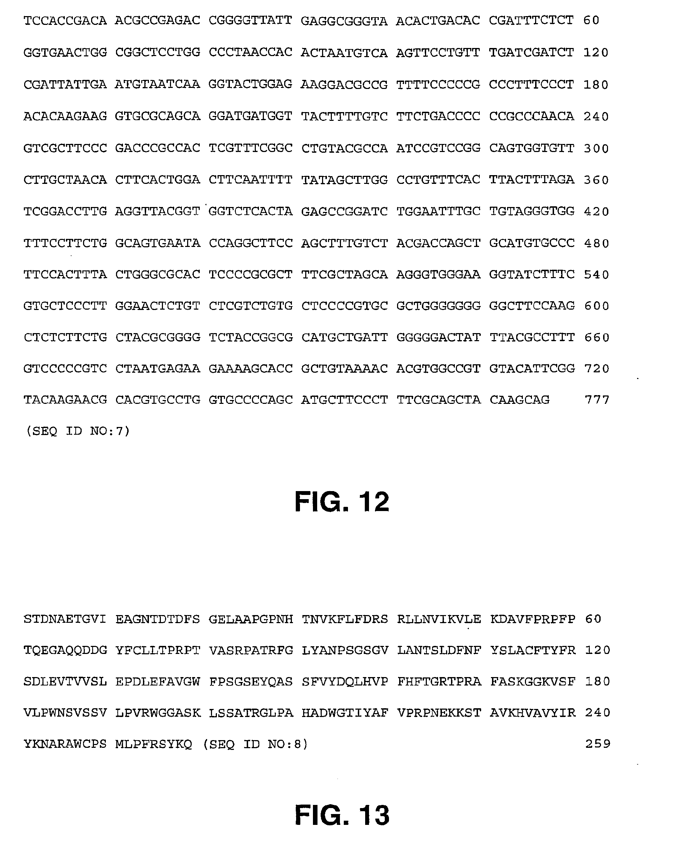

An isolated SVV VP1 (ID) protein may be provided with the amino acid sequence of SEQ ID NO:8, as listed in Figure 13 (which corresponds to amino acids 383-641 of SEQ ID NO:2). The amino acid sequence of the SVV VP1 protein is encoded by the nucleic acid sequence of SEQ ID NO:7, as listed in Figure 12 (which corresponds to nucleotides 1147-1923 of SEQ ID NO:1).

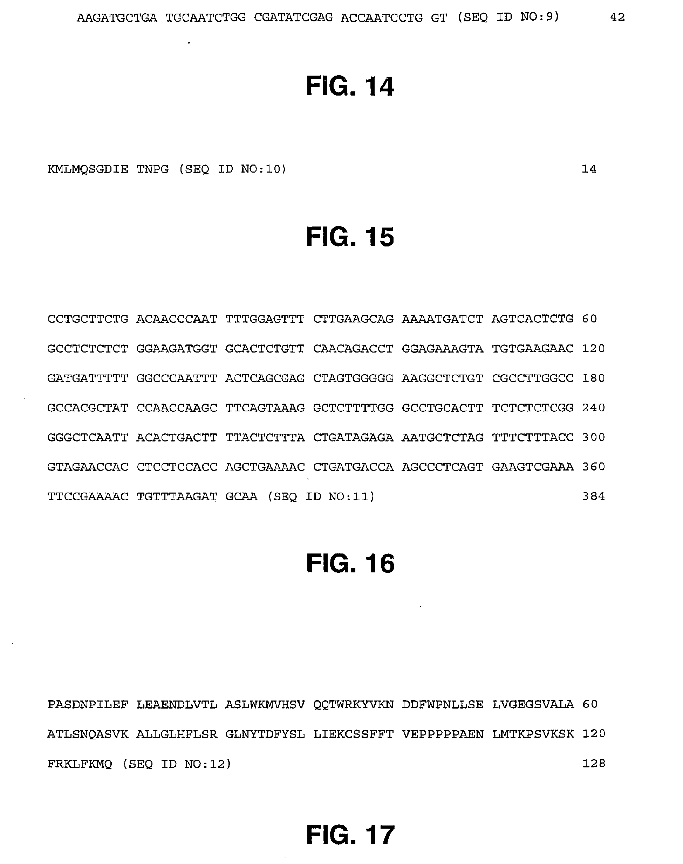

An isolated SVV 2A protein may be provided with the amino acid sequence of SEQ ID NO:10, as listed in Figure 15 (which corresponds to amino acids 642-655 of SEQ ID NO:2). The amino acid sequence of the SVV 2A protein is encoded by the nucleic acid sequence of SEQ ID NO:9, as listed in Figure 14 (which corresponds to nucleotides 1924-1965 of SEQ ID NO:1).

An isolated SVV 2B protein may be provided with the amino acid sequence of SEQ ID NO: 12, as listed in Figure 17 (which corresponds to amino acids 656-783 of SEQ ID NO:2). The amino acid sequence of the SW 2B protein is encoded by the nucleic acid sequence of SEQ ID NO: 11, as listed in Figure 16 (which corresponds to nucleotides 1966-2349 of SEQ ID NO:1).

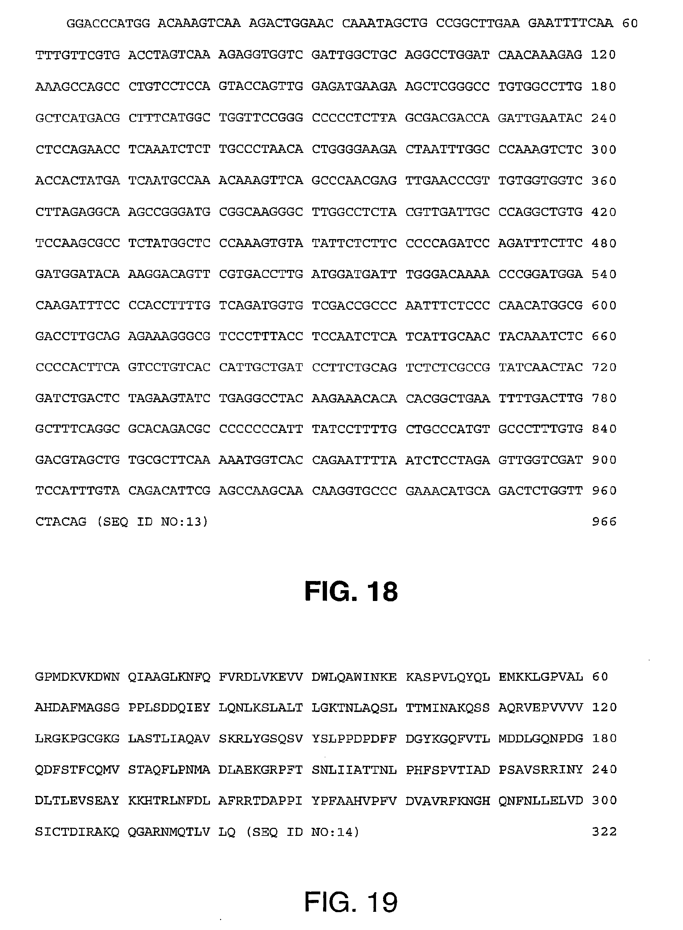

An isolated SVV 2C protein may be provided with the amino acid sequence of SEQ ID NO: 14, as listed in Figure 19 (which corresponds to amino acids 784-1105 of SEQ ID NO:2). The amino acid sequence of the SW 2B protein is encoded by the nucleic acid sequence of SEQ ID NO: 13, as listed in Figure 18 (which corresponds to nucleotides 2350-3315 of SEQ ID NO:1).

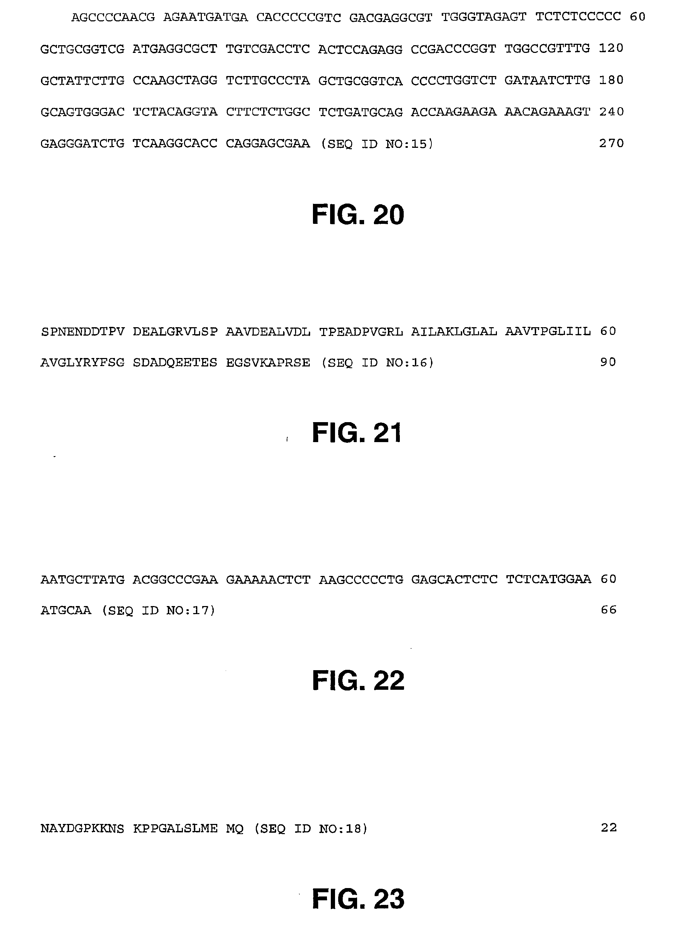

An isolated SVV 3A protein may be provided with the amino acid sequence of SEQ ID NO:16, as listed in Figure 21 (which corresponds to amino acids 1106-1195 of SEQ ID NO:2). The amino acid sequence of the SVV 3A protein is encoded by the nucleic acid sequence of SEQ ID NO: 15, as listed in Figure 20 (which corresponds to nucleotides 3316-3585 of SEQ ID NO:1).

An isolated SVV 3B (VPg) protein may be provided with the amino acid sequence of SEQ ID NO:18, as listed in Figure 23 (which corresponds to amino acids 1196-1217 of SEQ ID NO:2). The amino acid sequence of the SW 3B protein is encoded by the nucleic acid sequence of SEQ ID NO: 17, as listed in Figure 22 (which corresponds to nucleotides 3586-3651 of SEQ ID NO:1).

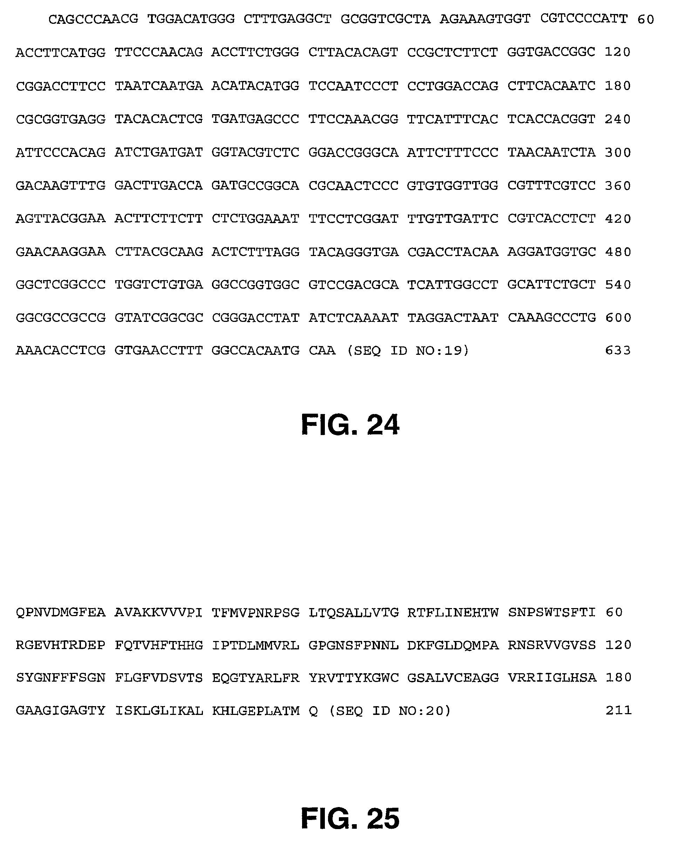

An isolated SVV 3C ("pro" or "protease") protein may be provided with the amino acid sequence of SEQ ID NO: 20, as listed in Figure 25 (which corresponds to amino acids 1218-1428 of SEQ ID NO:2). The amino acid sequence of the SW 3C protein is encoded by the nucleic acid sequence of SEQ ID NO: 19, as listed in Figure 24 (which corresponds to nucleotides 3652-4284 of SEQ ID NO: 1).

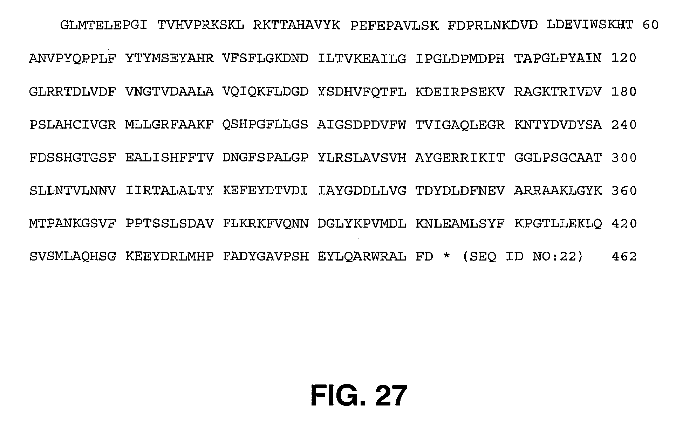

An isolated SVV 3D ("pol" or "polymerase") protein may be provided with the amino acid sequence of SEQ ID NO:22, as listed in Figure 27 (which corresponds to amino acids 1429-1890 of SEQ ID NO:2). The amino acid sequence of the SW 3C protein is encoded by the nucleic acid sequence of SEQ ID NO: 19, as listed in Figure 24 (which corresponds to nucleotides 4285-5673 of SEQ ID NO:1; nucleotides 5671-5673, "tga," code for a stop-codon, which is depicted in the amino acid sequence listings as an asterisk "*").

The nucleic acids of the present invention include both RNA and DNA forms, and implicitly, the complementary sequences of the provided listings.

Thus, the isolated SW nucleic acid depicted by SEQ ID NO: 1 has a length of 5,752 nucleotides that encodes a polypeptide with the amino acid sequence depicted by SEQ ID NO:2. The SVV genomic sequence is translated as a single polyprotein that is cleaved into various downstream "translation products."

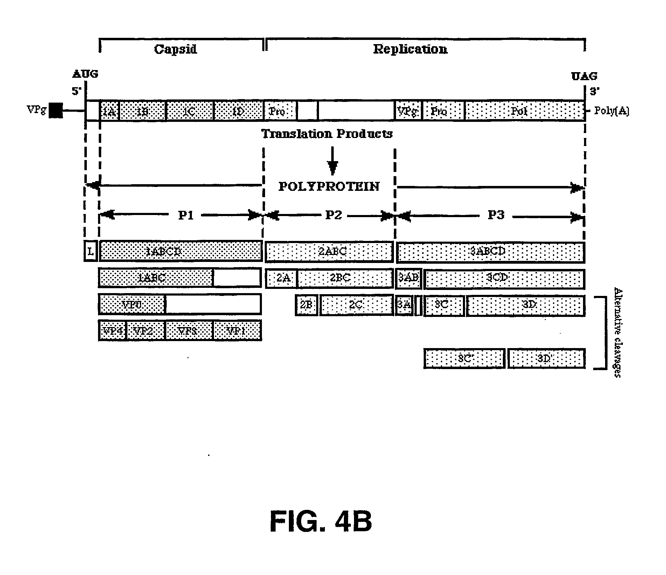

The majority of the full-length SVV polyprotein amino acid sequence is encoded by nucleotides 1-5673 of SEQ ID NO: 1. The polyprotein is cleaved into three precursor proteins, P1, P2 and P3 (see Figure 4B). P1, P2 and P3 are further cleaved into smaller products. The cleavage products of the structural region P1 (1ABCD; or the capsid region) are 1ABC, VP0, VP4, VP2, VP3 and VP1. The cleavage products of the non-structural protein P2 (2ABC) are 2A, 2BC, 2B and 2C. The cleavage products of the non-structural region P3 polyprotein (3ABCD) are 3AB, 3CD, 3A, 3C, 3D, 3C', and 3D'. Isolated nucleic acids may comprise: (i) the sequence of 2ABC (nucleotides 1924-3315 of SEQ ID NO:1) and the protein encoded therefrom; (ii) the sequence of 2BC (nucleotides 1966-3315 of SEQ ID NO: 1) and the protein encoded therefrom; (iii) the sequence of 3ABCD (nucleotides 3316-5673 of SEQ ID NO:1) and the protein encoded therefrom; (iv) the sequence of 3AB (nucleotides 3316-3651 of SEQ ID NO:1) and the protein encoded therefrom; and (v) the sequence of 3CD (nucleotides 3652-5673 of SEQ ID NO: 1) and the protein encoded therefrom.

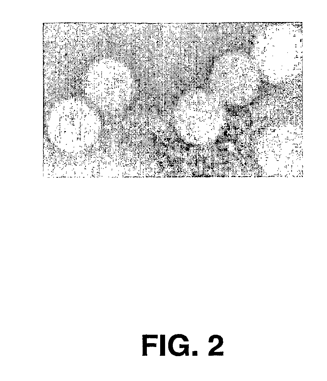

The basic capsid structure of picornaviruses consists of a densely packed icosahedral arrangement of 60 protomers, each consisting of 4 polypeptides, VP1, VP2, VP3 and VP4, all of which are derived from the cleavage of the original protomer, VP0. The SVV virus particle is about 27 nm in diameter *(see* Figure 2), which is consistent with the size of other picornavirus particles, which are about 27-30 nm in diameter.

The kinetics of picornavirus replication is rapid, the cycle being completed in about 5-10 hours (typically by about 8 hours) *(see* Figure 68 for a schematic of the picornavirus replication cycle). Upon receptor binding, the genomic RNA is released from the particle into the cytoplasm. Genomic RNA is then translated directly by polysomes, but in about 30 minutes after infection, cellular protein synthesis declines sharply, almost to zero. This phenomenon is called "shutoff," and is a primary cause of cytopathic effects (cpe). Shutoff appears to be due to cleavage of the host cell's 220 kDa cap-binding complex (CBC) that is involved in binding the m7G cap structure at the 5' end of all eukaryotic mRNA during initiation of translation. The cleavage of the CBC appears to be caused by the 2A protease.

The 5' UTR contains the IRES. Normally, eukaryotic translation is initiated when ribosomes bind to the 5' methylated cap and then scans along the mRNA to find the first AUG initiation codon. The IRES overcomes this process and allows Picornavirus RNA's to continue to be translated after degradation of CBC.

The virus polyprotein is initially cleaved by the 2A protease into polyproteins P1, P2 and P3 *(see* Figure 4B). Further cleavage events are then carried out by 3C, the main picornavirus protease. One of the cleavage products made by 3C is the virus RNA-dependent RNA polymerase (3D), which copies the genomic RNA to produce a negative (-) sense strand. The (-) sense strand forms the template for the (+) strand (genomic) RNA synthesis. Some of the (+) strands are translated to produce additional viral proteins are some (+) strands are packaged into capsids to form new virus particles.

The (+) strand RNA genome is believed to be packaged into preformed capsids, although the molecular interactions between the genome and the capsid are not clear. Empty capsids are common in all picornavirus infections. The capsid is assembled by cleavage of the P1 polyprotein precursor into a protomer consisting of VP0, VP3, and VP1, which join together enclosing the genome. Maturation and infectivity of the virus particle relies on an internal autocatalytic cleavage of VP0 into VP2 and VP4. Release of newly formed virus particles occurs when the cell lyses.

The present invention also provides an isolated virus having all the identifying characteristics and nucleic acid sequence of ATCC Patent Deposit number PTA-5343. Viruses of the present invention can be directed to the PTA-5343 isolate, variants, homologues, derivatives and mutants of the PTA-5343 isolate, and variants, homologues, derivatives and mutants of other picornaviruses that are modified in respect to sequences of SVV (both wild-type as disclosed herein and mutant) that are determined to be responsible for its oncolytic properties.

The present invention further provides antibodies that are specific against: the isolated SW having the ATTC Patent Deposit number PTA-5343, and epitopes from the isolated SW proteins having the amino acid sequence SEQ ID NO: 2. The invention also includes antibodies that are specific against epitopes from the proteins that are encoded by fragments or portions of SEQ ID NO: 1.

Comparative analyses of the RNA sequences from a variety of cardiovirus isolates have shown >45% nucleotide identity between genomes. Cardioviruses can be subclassified into the EMC-like viruses ("EMCV" - such as, Mengo, B, R; and also MM, ME, Columbia-SK), the Theiler's-like viruses ("TMEV" - such as, BeAn, DA and GD VII strains), and the Vilyuisk viruses.

In analyzing the SW sequence to other viruses, it appears that SW is a cardiovirus (see Example 4 and Figures referenced therein). If EMCV and TMEV are taken as the standard cardioviruses, SW is clearly not a typical cardiovirus. However, even these two viruses have their differences, notably in the 5' UTR (Pevear et al., 1987, J. Virol., 61: 1507-1516). Phylogenetically SW clusters with EMCV and TMEV in much of its polyprotein (P1, 2C, $3C^{pro}$ and $3D^{pol}$ regions; see Figures 31-37), indicating that SVV is most likely a cardiovirus.

Methods for Treating Cancer:

The present invention provides methods for cancer therapy using viruses modified in view of the oncolytic properties of SW, including picornaviruses, derivatives, variants, mutants or homologues thereof. The present invention shows that wild-type SW (*i.e.,* ATTC Patent Deposit number PTA-5343) has the ability to selectively kill some types of tumors. For example, SVV can selectively kill tumor cells that have neurotropic or neuroendocrine properties, including small cell lung cancer (SCLC) and neuroblastomas. Other examples of neuroendocrine tumors that are contemplated to be treated by the viruses of the present invention include, but are not limited to: adrenal pheochromocytomas, gastrinomas (causing Zollinger-Ellison syndrome), glucagonomas, insulinomas, medullary carcinomas (including medullary thyroid carcinoma), multiple endocrine neoplasia syndromes, pancreatic endocrine tumors, paragangliomas, VIPomas (vasoactive intestinal polypeptide tumor), islet cell tumors, and pheochromocytoma.

Also encompassed in the present invention are the four types of neuroendocrine lung tumors. The most serious type, small cell lung cancer (SCLC), is among the most rapidly growing and spreading of all cancers. Large cell neuroendocrine carcinoma is a rare cancer that, with the exception of the size of the cells forming the cancer, is very similar to SCLC in its prognosis and in how patients are treated. Carcinoid tumors, also known as carcinoids, comprise the other 2 types of lung neuroendocrine cancer. These two types are typical carcinoid and atypical carcinoid.

Not being bound by theory, the ability of SVV to specifically kill tumor cells may include, but is not limited to: selective replication, apoptosis, lysis via tumor-selective cell entry, tumor-selective translation, tumor-selective proteolysis, tumor-selective RNA replication, and combinations thereof.

SVV has many advantageous characteristics over other oncolytic viruses, including modified adenoviruses, for example: (i) SVV has a very high selectivity for cancers with neural properties, including SCLC, Wilms' tumor, retinoblastoma, and neuroblastoma - for example, SW shows a greater than 10,000-fold selectivity toward neuroendocrine tumor cells; (ii) SVV has been shown to have a 1,000 fold better cell-killing specificity than chemotherapy treatments; (iii) SVV exhibits no overt toxicity in mice following systemic administration with as high as $10^{14}$ viral particles per kilogram; (iv) the efficacy of SW is very robust in that 100% of large pre-established tumors can be eradicated in mice, with no recurrence of tumor growth; (v) SVV can be purified to high titer and can be produced at more than 200,000 particles per cell in permissive cell lines; (vi) SVV has a small size (the SW virus particle is less than 30 nm in diameter) enabling better penetration and spread in tumors than other oncolytic viruses, (vii) SW replicates quickly (less than 12 hours) and (viii) no modification of SW is necessary for its use as a specific anti-tumor agent.

Further, initial studies *(see* Example 6) indicate some additional factors that make SVV an advantageous tool for oncolytic viral therapy: (i) human serum samples do not contain neutralizing antibodies directed against SVV; (ii) SW is not inhibited by complement; and (iii) SVV is not inhibited by hemagglutination. All of these factors contribute to the fact that SVV exhibits a longer circulation time *in vivo* than other oncolytic viruses (for example, *see* Example 7).

SVV possesses potent antitumor activity against tumor cell-types with neural characteristics. SVV does not exhibit cytolytic activity against normal human, rat mouse, bovine or ovine cell lines or non-neural tumor cell lines. Further SVV is not cytotoxic to primary human hepatocytes. Table 1 below summarizes studies that have been conducted to determine the *in vitro* cytolytic potency of SVV against selected tumor cell types.

**Table 1: SVV Cytolytic Potency Against Selected Tumor Cell-Types**

| Cell Line | Cell Type | $EC_{50}$ (VP/cell) |
|---|---|---|
| H446 | Human SCLC | 0.0012 |
| PER.C6 | Human Embryonic Retinoblast | 0.02 |
| H69AR | SCLC-Multidrug Resistant | 0.035 |
| 293 | AD5 DNA Transformed Human Kidney | 0.036 |
| Y79 | Human Retinoblastoma | 0.00035 |

(continued)

| Cell Line | Cell Type | $EC_{50}$ (VP/cell) |
|---|---|---|
| IMR32 | Human Brain Neuroblastsoma | 0.035 |
| D283 | Med Human Brain Cerebellar Medulloblastoma | 0.25 |
| SK-N-AS | Human Brain Neuroblastoma | 0.474 |
| N1E-115 | Mouse Neuroblastoma | 0.0028 |
| BEKPCB3E1 | Bovine embryonic Kidney cells transformed with Ad5E1 | 0.99 |
| H1299 | Human non-SCLC | 7.66 |
| ST | Porcine Testis | 5.9 |
| DMS153 | Human SCLC | 9.2 |
| BEK | Bovine Embryonic Kidney | 17.55 |
| M059K | Human Brain Malignant Glioblastoma | 1,061 |
| PK15 | Porcine Kidney | 1,144 |
| FBRC | Fetal Bovine Retina | 10,170 |
| HCN-1A | Human Brain | 23,708 |
| H460 | Human LCLC | >30,000 (inactive) |
| Neuro 2A | Mouse Neuroblastoma | >30,000 (inactive) |
| DMS79 | Human SCLC | >30,000 (inactive) |
| H69 | Human SCLC | >30,000 (inactive) |
| C8D30 | Mouse Brain | >30,000 (inactive) |
| MRC-5 | Human Fetal Lung Fibroblast | >30,000 (inactive) |
| HMVEC | Neonatal vascular endothelial cells | >30,000 (inactive) |
| HMVEC | Adult vascular endothelial cells | >30,000 (inactive) |
| A375-S2 | Human Melanoma | >30,000 (inactive) |
| SK-MEL-28 | Melanoma | >30,000 (inactive) |
| PC3 | Human prostate cancer | >30,000 (inactive) |
| PC3M2AC6 | Human prostate cancer | >30,000 (inactive) |
| LnCap | Human Prostate cancer | >30,000 (inactive) |
| DU145 | Human prostate cancer | >30,000 (inactive) |

Murine studies *(see* Examples) show that SW can specifically kill tumors with great efficacy and specificity *in vivo.* These *in vivo* studies show that SVV has a number of advantages over other oncolytic viruses. For example, one important factor affecting the ability of an oncolytic tumor virus to eradicate established tumors is viral penetration. In studies with adenoviral vectors, Ad5 based vectors had no effect on SCLC tumor development in athymic mice. Based on immunohistochemical results, adenovirus did not appear to penetrate the established tumors. In contrast, SVV was able to eliminate H446 SCLC tumors in athymic nude mice following a single systemic administration. SVV has a small size (<30 nm in diameter) enabling better penetration and spread in tumor tissue than other viruses, and thus, the small size of SW may contribute to its ability to successfully penetrate and eradicate established tumors.

Chemoresistance is a major issue facing any patient that receives chemotherapy as a facet of cancer therapy. Patients that become chemoresistant often, if not always, have a much poorer prognosis and may be left with no alternative therapy. It is well known that one of the major causes of chemoresistance is the expression, over expression, or increased activity of a family of proteins called Multiple Drug Resistant proteins (MRPs). Applicants have found that a sensitivity of certain tumor cells for SVV is also correlated with the chemoresistant state of cancer cells and MRP expression. H69 is a chemosensitive (adriamycin) cell line that is resistant to SVV *in vitro,* whereas H69AR is a chemoresistant cell line that overexpresses MRPs and is sensitive to SVV *(see* Table 1). Evidence indicates that overexpression of MRPs, including MDR, correlates with sensitivity of cells to SVV killing. Thus, in one embodiment, the present invention provides a method for treating cancer wherein SVV kills cells overexpressing an MRP.

The present invention also provides methods for treating diseases that are a result of abnormal cells, such as abnormally proliferative cells. The method comprises contacting said abnormal cells with SVV in a manner that results in the destruction of a portion or all of the abnormal cells. SVV can be used to treat a variety of diseases that are a result of abnormal cells. Examples of these diseases include, but are not limited to, cancers wherein the tumor cells display neuroendocrine features and neurofibromatosis.

Neuroendocrine tumors can be identified by a variety of methods. For example, neuroendocrine tumors produce and secrete a multitude of peptide hormones and amines. Some of these substances cause a specific clinical syndrome: carcinoid, Zollinger-Ellison, hyperglycemic, glucagonoma and WDHA syndrome. Specific markers for these syndromes are basal and/or stimulated levels of urinary 5-HIAA, serum or plasma gastrin, insulin, glucagon and vasoactive intestinal polypeptide, respectively. Some carcinoid tumors and about one third of endocrine pancreatic tumors do not present any clinical symptoms and are called 'nonfunctioning' tumors. Therefore, general tumor markers such as chromogranin A, pancreatic polypeptide, serum neuron-specific enolase and subunits of glycoprotein hormones have been used for screening purposes in patients without distinct clinical hormone-related symptoms. Among these general tumor markers chromogranin A, although its precise function is not yet established, has been shown to be a very sensitive and specific serum marker for various types of neuroendocrine tumors. This is because it may also be elevated in many cases of less well-differentiated tumors of neuroendocrine origin that do not secrete known hormones. At the moment, chromogranin A is considered the best general neuroendocrine serum or plasma marker available both for diagnosis and therapeutic evaluation and is increased in 50-100% of patients with various neuroendocrine tumors. Chromogranin A serum or plasma levels reflect tumor load, and it may be an independent marker of prognosis in patients with midgut carcinoids.

The present invention also provides a pharmaceutical composition comprising the virus of the invention and a pharmaceutically acceptable carrier. Such compositions, which can comprise an effective amount of SW in a pharmaceutically acceptable carrier, are suitable for local or systemic administration to individuals in unit dosage forms, sterile parenteral solutions or suspensions, sterile non-parenteral solutions or oral solutions or suspensions, oil in water or water in oil emulsions, and the like. Formulations for parenteral and non-parenteral drug delivery are known in the art. Compositions also include lyophilized and/or reconstituted forms of SVV. Acceptable pharmaceutical carriers are, for example, saline solution, protamine sulfate (Elkins-Sinn, Inc., Cherry Hill, NJ), water, aqueous buffers, such as phosphate buffers and Tris buffers, or Polybrene (Sigma Chemical, St. Louis, MO) and phosphate-buffered saline and sucrose. The selection of a suitable pharmaceutical carrier is deemed to be apparent to those skilled in the art from the teachings contained herein. These solutions are sterile and generally free particulate matter other than SW. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate, etc. Excipients that enhance infection of cells by SW may be included.

SW is administered to a host or subject in an amount that is effective to inhibit, prevent or destroy the growth of the tumor cells through replication of the virus in the tumor cells. Methods that utilize SW for cancer therapy include systemic, regional or local delivery of the virus at safe, developable, and tolerable doses to elicit therapeutically useful destruction of tumor cells. Even following systemic administration, the therapeutic index for SW is at least 10, preferably at least 100 or more preferably at least 1000. In general, SVV is administered in an amount of between $1x10^8$ and $1x10^{14}$ vp/kg. The exact dosage to be administered is dependent upon a variety of factors including the age, weight, and sex of the patient, and the size and severity of the tumor being treated. The viruses may be administered one or more times, which may be dependent upon the immune response potential of the host. Single or multiple administrations of the compositions can be carried out with dose levels and pattern being selected by the treating physician. If necessary, the immune response may be diminished by employing a variety of immunosuppressants, so as to permit repetitive administration and/or enhance replication by reducing the immune response to the viruses. Anti-neoplastic viral therapy of the present invention may be combined with other anti-neoplastic protocols. Delivery can be achieved in a variety of ways, employing liposomes, direct injection, catheters, topical application, inhalation, etc. Further, a DNA copy of the SVV genomic RNA, or portions thereof, can also be a method of delivery, where the DNA is subsequently transcribed by cells to produce SVV virus particles or particular SVV polypeptides.

A therapeutically effective dose refers to that amount of the virus that results in amelioration of symptoms or a prolongation of survival in a patient. Toxicity and therapeutic efficacy of viruses can be determined by standard procedures in cell cultures or experimental animals, e.g., for determining the $LD_{50}$ (the dose lethal to 50% of the population of animals or cells; for viruses, the dose is in units of vp/kg) and the $ED_{50}$ (the dose - vp/kg - therapeutically effective in 50% of the population of animals or cells) or the $EC_{50}$ (the effective concentration - vp/cell (see Table 1 for example) - in 50% of the population of animals or cells). The dose ratio between toxic and therapeutic effects is the therapeutic index and it can be expressed as the ratio between $LD_{50}$ and $ED_{50}$ or $EC_{50}$. Viruses which exhibit high therapeutic indices are preferred. The data obtained from these cell culture assays and animal studies can be used in formulating a range of dosage for use in human. The dosage of viruses lies preferably within a range of circulating concentrations that include the $ED_{50}$ or $EC_{50}$ with little or no toxicity. The dosage may vary within this range depending upon the dosage form employed and the route of administration utilize

Methods for Producing the Viruses of the Present Invention:

Methods for producing the present viruses to very high titers and yields are additional aspects of the invention. As stated, SVV can be purified to high titer and can be produced at more than 200,000 particles per cell in permissive cell lines. Cells that are capable of producing high quantities of viruses include, but are not limited to, PER.C6 *(*Fallaux et al., Human Gene Therapy, 9:1909-1917, 1998), H446 (ATCC# HTB-171) and the other cell lines listed in Table 1 where the $EC_{50}$ value is less than 10.

For example, the cultivation of picornaviruses can be conducted as follows. The virus of interest is plaque purified once and a well-isolated plaque is picked and amplified in a permissive cell line, such as PER.C6. A crude virus lysate (CVL) from the infected cells can be made by multiple cycles of freeze and thaw, and used to infect large numbers of permissive cells. The permissive cells can be grown in various tissue culture flasks, for example, 50x150 $cm^2$ flasks using various media, such as Dulbecco's modified Eagle medium (DMEM, Invitrogen, Carlsbad, CA)) containing 10% fetal bovine serum (Biowhitaker, Walkersvile, MD) and 10 mM magnesium chloride (Sigma, St Louis, MO). The infected cells can be harvested between 24-48 hours after infection or when complete cytopathic effects (CPE) are noticed, and are collected by centrifugation at 1500 rpm for 10 minutes at 4°C. The cell pellet is resuspended in the cell culture supernatant and is subjected to multiple cycles of freeze and thaw. The resulting CVL is clarified by centrifugation at 1500 rpm for 10 minutes at 4°C. Virus can be purified by gradient centrifugation. For example, two rounds of CsCl gradients can suffice for SVV purification: a one-step gradient (density of CsCl 1.24 g/ml and 1.4 g/ml) followed by one continuous gradient centrifugation (density of CsCl 1.33 g/ml). The purified virus concentration is determined spectrophotometrically, assuming 1A260 = 9.5 x $10^{12}$ particles (Scraba D.G., and Palmenberg, A.C. 1999. Cardioviruses (Picornaviridae). In:Encyclopedia of Virology, Second edition, R.G. Webster and A Granoff Eds). Titers of purified virus are also determined by a standard plaque assay using PER.C6 cells. The yield of SW from PER.C6 cells are greater than 200, 000 particles per cell with particles to PFU ratio of about 100. The yields of SVV from other permissive cells (H446-ATCC# HTB-171) may be at least this high or higher.

In addition, several steps in a commercially attractive large scale Good Manufacturing Processes (GMP) are applicable to the purification of SW. The invention also contemplates methods for purifying SW that are based on methods for purifying adenoviruses. These methods include isolating SVV based on its density, since SVV has a very similar density to adenovirus and can be co-purified with adenovirus.

Methods for Detecting and Studying Tumors:

Methods may be provided for detecting tumor or neoplastic cells in a patient using the viruses of the present invention. Cellular samples can be obtained from a patient and screened by incubating the sample with an epitope-tagged SVV (or other tumor-specific viruses provided by the invention, *i.e.,* tumor-specific mutant cardioviruses), and then screening the sample for bound SVV by detecting the epitope tag. Alternatively, the sample can be screened by detecting whether the SVV causes any cellular lysis. If SVV does cause cellular lysis, or if SW can bind specifically to cells in the sample, this would indicate the possibility that the sample contains neoplastic or tumor cells known to be capable of being bound and/or infected by SVV.

Additionally, SVV can be used in a method for detecting a tumor cell *in vivo.* In such a method, epitope-tagged SVV can first be inactivated in a manner where SW can still bind to tumor cells specifically but cannot replicate. Tumor cells that have bound SVV can be detected by assaying for the epitope tag. Detection of the epitope tag can be accomplished by antibodies that specifically bind the epitope, where the antibodies are either labeled (for example, fluorescently) or where the antibodies can then be detected by labeled secondary antibodies.

The present methods of detection encompass detecting any type of tumor or neoplastic cell that is specifically targeted by any virus of the present invention. Specific tumor types include, for example, neuroendocrine-type tumors, such as retinoblastomas, SCLC, neuroblastomas glioblastomas and medulloblastomas.

SVV may be used as a tool to study tumor cells. SVV selectively destroys some tumor cell types, and has very little, if any, toxic effects on non-tumor cells. Because of these characteristics, SW can be used to study tumors and possibly discover a new tumor specific gene and/or pathway. In other words, there is some characteristic of the tumor cells that allows replication of SVV, wherein normal cells do not exhibit said characteristic. Upon identification of a new tumor specific gene and/or pathway, therapeutic antibodies or small molecules can then be designed or screened to determine whether these reagents are anti-tumor agents.

Methods may be provided for identifying all types of cancers that respond to SW. A method for identifying SW-responsive cells may comprise obtaining cells, contacting said cells with SVV and detecting cell killing or detecting viral replication. Cell killing can be detected using various methods known to one skilled in the art (*e.g.,* MTS assay, *see* High-Throughput section herein). Methods of detecting virus replication are also known to one skilled in the art (e.g., observance of CPE, plaque assay, DNA quantification methods, FACS to detect quantity of virus in the tumor cells, RT-PCR assays to detect viral RNA, etc.). In one embodiment, the cells are cancer cells. Examples of cancer cells include, but are not limited to, established tumor cell lines and tumor cells obtained from a mammal. In one embodiment, the mammal is a human. In a further embodiment, the cells are cancer cells obtained from a human cancer patient.

The method for identifying SW-responsive cancer cells may be used to discover tumor cell lines or tumor tissues that are permissive for SVV replication. Also, by determining the characteristics of permissive tumor cells, one may be able to identify characteristics of tumor cells that cause the cells to be selectively killed by SW. The discovery of these characteristics could lead to new targets for cancer drugs. Also, the methods for identifying SVV responsive cancer cells could be used as a screen for human cancer patients who would benefit from treatment with SVV.

Since the natural host of SW has not yet been determined, there is a need for an assay to detect SW.

In one embodiment, the detection assay is based on antibodies specific to SVV polypeptide epitopes. In another embodiment, the detection assay is based on the hybridization of nucleic acids. In one embodiment, RNA is isolated from SW, labeled (e.g., radioactive, chemiluminsecence, fluorescence, etc.) to make a probe. RNA is then isolated from test material, bound to nitrocellulose (or a similar or functionally equivalent substrate), probed with the labeled SVV RNA, and the amount of bound probe detected. Also, the RNA of the virus may be directly or indirectly sequenced and a PCR assay developed based on the sequences. In one embodiment, the PCR assay is a real time PCR assay.

Methods for Making Viruses with Altered Tropism:

The present invention provides methods for constructing SW mutants (or variants or derivatives) where these mutants have an altered cell-type tropism. Specifically, SW mutants are selected for their ability to specifically bind and/or kill tumor or neoplastic cells that are known to be resistant to wild-type SVV binding.

The native or wild-type SW has a simple genome and structure that allow the modification of the native virus to target other cancer indications. These new derivatives have an expanded tropism toward non-neural cancers and still maintain the high therapeutic index found in the native SVV. One possible means of targeting is the inclusion of tissue-specific peptides or ligands onto the virus.

To select cancer-targeting viral candidates, the present invention provides methods to construct and screen an oncolytic virus library with a genetic insertion that encodes a random peptide sequence in the capsid region of the native SVV. A random peptide library with a diversity of $10^8$ is believed to be sufficient and should yield peptides that specifically direct the virus to tumor tissue.

Various studies have shown that tumor cells exhibit different characteristics from normal cells such as: (1) tumor cells have more permeable cell membranes; (2) tumors have specific stromal cell types such as angiogenic endothelial cells which have previously been shown to express cell type specific receptors; and (3) tumor cells differentially express certain receptors, antigens and extracellular matrix proteins (Arap, W. et al., Nat. Med., 2002, 8(2): 121-127; Kolonin, M. et al., Curr. Opin. Chem. Biol., 2001, 5(3): 308-313; St. Croix, B. et al., Science, 2000, 289(5482): 1997-1202). These studies demonstrated that tumor and normal tissues are distinct at the molecular level and targeted drug delivery and treatment of cancer is feasible. Specifically, several peptides selected by homing to blood vessels in mouse models have been used for targeted delivery of cytotoxic drugs (Arap, W. et al., Science, 1998, 279(5349): 377-380), pro-apoptotic peptides (Ellerby, H.M. et al., Nat. Med., 1999, 17(8): 768-774), metalloprotease inhibitor (Koivunen, E. et al., Nat. Biotechnol, 1999, 17(8): 768-774), cytokine (Curnis, F. et al., Nat. Biotechnol., 2000, 18(11): 1185-1190), fluorophores (Hong. F.D. and Clayman, G.L., Cancer Res., 2000, 60(23): 6551-6556) and genes (Trepel, M. et al., Hum. Gene Ther., 2000, 11(14): 1971-1981). The tumor-targeting peptides have proven to increase the efficacy and lower the toxicity of the parental drugs.

A library of SVV derivatives can be generated by the insertion of a random peptide sequence into the capsid region of the virus. As shown in Figure 57, a vector is first generated that contains the SW capsid region, *i.e.,* "pSVV capsid." This capsid vector can then be mutagenized, for example, by cutting the vector with a restriction enzyme that cuts DNA at random positions, *i.e.,* CviJI (a blunt cutter). The vector is cut at numerous positions, and DNA that has been cut only once by CviJI can be isolated by gel-purification *(see* Figure 57). This isolated population of DNA contains a plurality of species that have been cut in the capsid region at different locations. This population is then incubated with oligonucleotides and ligase, such that a percentage of the oligonucleotides will be ligated into the capsid region of the vector at a number of different positions. In this manner, a library of mutant SVV capsids can be generated.

The oligonucleotides that are inserted into the capsid encoding region can be either random oligonucleotides, non-random oligonucleotides *(i.e.,* the sequence of the oligonucleotide is pre-determined), or semi-random *(i. e.,* a portion of the oligonucleotide is pre-determined and a portion has a random sequence). The non-random aspect of the contemplated oligonucleotides can comprise an epitope-encoding region. Contemplated epitopes include, but are not limited to, c-myc - a 10 amino acid segment of the human protooncogene myc (EQKLISEEDL (SEQ ID NO: 35); HA - haemoglutinin protein from human influenza hemagglutinin protein (YPYDVPDYA (SEQ ID NO: 36)); and $His_6$ (SEQ ID NO: 116) - a sequence encoding for six consecutive histidines.

The library of mutant capsid polynucleotides (for example, "pSVV capsid library" in Figure 57) can then be digested with restriction enzymes such that only the mutant capsid encoding region is excised. This mutant capsid encoding region is then ligated into a vector containing the full-length genomic sequence minus the capsid encoding region *(see* Figure 58, for example). This ligation generates a vector having a full-length genomic sequence, where the population (or library) of vectors comprise a plurality of mutant capsids. In Figure 58, this library of SVV mutants comprising different capsids is denoted as "pSVVFL capsid." The pSVVFL capsid vector library is then linearized and reverse-transcribed in order to generate mutant SVV RNA *(see* Figure 59). The mutant SVV RNA is then transfected into a permissive cell line such that those SVV sequences that do not possess a dehabilitating mutation in its capsid are translated by the host cells to produce a plurality of mutant SW particles. In Figure 59, the plurality of mutant SW particles are denoted as a "SVV capsid library."

The peptide encoded by the oligonucleotide inserted into the capsid encoding region can serve as a targeting moiety for specific viral infection. The viruses that target a specific type of cancer would selectively infect only those cancer cells that have a receptor to the peptide, replicate in those cells, kill those cells, and spread to only those same types of cells. This methodology enables the identification of novel tumor-targeting peptides and ligands, tumor-selective receptors, therapeutic SVV derivatives and other virus derivatives, including picornavirus derivatives.

*In vitro and in vivo* screening of SVV mutant libraries have several advantages over other technologies such as peptide bead libraries and phage display. Unlike these other technologies, the desirable candidate here, *i.e.* an SVV derivative that selectively binds to a cancer cell, will replicate *in situ.* This replication-based library approach has numerous advantages over prior methods of discovering new cell binding moieties, such as phage display. First, the screening of a SVV library is based on replication. Only the desired viral derivatives can replicate in the target tissue, in this case certain cancer cells. The screening/selection process will yield very specific viral candidates that have both the targeting peptide moiety and may be a cancer therapeutic itself. On the contrary, phage display screens will only result in binding events and yields only the targeting peptide candidates. Thus, SW library screening offers a much faster and selective approach. Second, during *in vitro* or *in vivo* phage display screening, phages recovered from the target cells have to be amplified in bacteria, while SVV derivatives can be directly recovered and purified from infected cells (or from the culture supernatant of lytically infected cells). Third, SVV has a smaller genome that renders easier manipulability; thus it is possible to randomly insert the genetic information into the capsid region to ensure an optimized insertion. Therefore, construction and screening of the SW library has a high possibility to produce highly effective viral derivatives. These derivatives are designed and screened to specifically infect cancers with non-neural properties.

The insertion of oligonucleotides into the capsid encoding region will result in the generation of some defective mutants. Mutants may be defective in the sense that the insertion of an oligonucleotide sequence can result in a stop codon, such that the viral polyprotein will not be produced. Also, mutants may be defective in the sense that the insertion of an oligonucleotide sequence may result in the alteration of the capsid structure such that capsid can no longer be assembled. To decrease the probability that the insertion of oligonucleotide sequences may result in stop codon or untenable capsid structure, random oligonucleotides can be designed such that they do not encode for stop codons or for certain amino acids using methods such as TRIM.

To determine whether there is an optimal insertion point in the capsid region for oligonucleotides, one can generate an RGD-SVV library *(see* Example 16). The polynucleotide encoding the SW capsid is randomly cut, for example, with CviJI. The randomly linearized capsid polynucleotides are then ligated to oligonucleotides encoding at least the RGD amino acid sequence (arginine-glycine-aspartic acid). These RGD-capsid sequences are then ligated into SW full-length sequence vectors that are missing the capsid sequence. RGD-SVV derivatives viruses are produced and tested for their ability to infect and replicate in certain integrin-expressing cell lines (as the RGD peptide has been shown to target entities to integrin receptors). The RGD-SVV derivatives that are successful in infecting the integrin-expressing cell lines are then analyzed to determine whether there is a predominant insertion site for the RGD oligonucleotide. This site can then be used for site-directed insertion of random, non-random or semi-random oligonucleotides.

Further, in comparing portions of the capsid encoding region between SW and other picornaviruses *(see* Figure 28), there are various non-boxed regions between the viruses where the sequence similarity is at its lowest. These regions may be important in contributing to the different tropisms between the viruses. Thus, these regions may be candidate locations for oligonucleotide insertion mutagenesis of the SVV capsid (and for other viral capsids).

<u>Inactivated SVV as a Tumor-Specific Therapeutic:</u>

Since SVV and SVV-capsid derivatives can target specific tumor cell-types and/or tissues, the SVV particle itself can be used as a delivery vehicle for therapeutics. In such a method, the need for the oncolytic abilities of SW becomes optional, as the delivered therapeutic can kill the targeted tumor cell.

For example, the wild-type SVV can be inactivated such that the virus no longer lyses infected cells, but where the virus can still specifically bind and enter targeted tumor cell-types. There are many standard methods known in the art to inactivate the replicative functions of viruses. For example, whole virus vaccines are inactivated by formalin or β-propiolactone such that the viruses cannot replicate. The wild-type SVV may itself contain peptides that cause the apoptosis of cells. Alternatively, SVV can be irradiated. However, irradiated viruses should first be tested to ensure that they are still capable of specifically targeting tumor cells, as certain irradiation conditions may cause protein, and thus capsid, alterations. Further, mutant SVVs can be generated where the packaging signal sequence is deleted. These SVV mutants are able to specifically bind and enter target cells, but replicated SW genomic RNA will not be packaged and assembled into capsids. However, this method may prove to be useful as initial entry of these mutant SVVs will cause host-protein synthesis shut-off such that tumor-cell death is still achieved.

Derivative SVVs having mutant capsids can also be inactivated and used to kill cancer cells. Derivative SVVs with oligonucleotides encoding epitope tags inserted into the capsid region can be used as vehicles to deliver toxins to tumor cells. As described herein, derivative SVVs can be randomly mutagenized and screened for tumor-specific tropisms. Toxins can be attached to the epitope tags, such that the virus delivers the toxin to tumor cells. Alternatively, therapeutic antibodies that specifically bind to the epitope tag can be used, such that the virus delivers the therapeutic antibody to the tumor cell.

High-Throughput Screening:

High-throughput methods may be provided for screening viruses that have the ability to specifically infect different cell-lines. The specificity of infection can be detected by assaying for cytopathic effects. For example, a number of different tumor cell-lines can be grown in different wells of a multi-well plate that is amenable for high-throughput screening, for example a 384 well-plate. To each well, a sample of virus is added to test whether the cells are killed by virus-mediated lysis. From those wells that show cytopathic effects, the media is collected such that any viruses in the media can be amplified by infecting permissive cell lines in flasks or large tissue culture plates. The viruses are grown such that the RNA can be isolated and the sequence analyzed to determine sequence mutations that may be responsible for providing a tumor cell-type specific tropism for a virus.

Various colorimetric and fluorometric methods can quickly assay cytopathic effects, including fluorescent-dye based assays, ATP-based assays, MTS assays and LDH assays. Fluorescent-dye based assays can include nucleic acid stains to detect dead-cell populations, as cell-impermeant nucleic acid stains can specifically detect dead-cell populations. If it is desired to simultaneously detect both live-cell and dead-cell populations, nucleic acid stains can be used in combination with intracellular esterase substrates, membrane-permeant nucleic acid stains, membrane potential-sensitive probes, organelle probes or other cell-permeant indicators to detect the live-cell population. For example, Invitrogen (Carlsbad, CA) offers various SYTOX™ nucleic acid stains that only penetrate cells with compromised plasma membranes. Ethidium bromide and propidium iodide can also be used to detect dead or dying cells. These stains are high-affinity nucleic acid stains that can be detected by any light-absorbance reader

For example, lysis can be based on the measurement of lactate dehydrogenase (LDH) activity released from the cytosol of damaged cells into the supernatant. To detect the presence of LDH in cell culture supernatants, a substrate mixture can be added such that LDH will reduce the tetrazolium salt INT to formazan by a coupled enzymatic reaction. The formazan dye can then be detected by a light-absorbance reader. Alternatively, an MTS assay [3-(4,5-dimethylthiazol-2-yl)-5(3-carboxymethoxyphenyl)-2-(4-sulfophenyl)-2H-tetrazolium, inner salt] using phenzine methosulfate (PMS) as the electron coupling reagent can also be used to detect cytotoxicity. Promega (Madison, WI) offers a CellTiter 96® $AQ_{ueous}$ One Solution Cell Proliferation Assay kit where the solution reagent is added directly to culture wells, incubated for 1-4 hours and then absorbance is recorded at 490 nm. The quantity of formazan product as measured by the amount of 490 nm absorbance is directly proportional to the number of living cells in culture.

There are numerous high-throughput devices for reading light-absorbance. For example, SpectraMax Plus 384 Absorbance Platereader (Molecular Devices) can detect wavelengths from 190-1000 nm in 1 nm increments. The device can read 96-well microplates in 5 seconds and 384-well microplates in 16 seconds for ultra fast sample throughput.

Virus replication can also be assayed as an indication of successful infection, and such detection methods can be used in a high-throughput manner. For example, real-time RT-PCR methods can be used to detect the presence of virus transcripts in cell-culture supernatants. Upon reverse-transcription of viral RNA into cDNA, the cDNA can be amplified and detected by PCR with the use of doublestranded DNA-binding dyes (for example, SYBR® Green, Qiagen GmbH, Germany). The amount of PCR product can then be directly measured using a fluorimeter.

Viruses from the wells showing cytopathic effects are grown up and tested in *further in vitro* (re-testing of tumor and normal cell lines) and *in vivo* models (testing whether the virus can kill explanted tumors in mice).

Antibodies:

The present invention is also directed to antibodies that specifically bind to the viruses of the present invention, including the proteins of the viruses. Antibodies of the present invention include naturally occurring as well as non-naturally occurring antibodies, including, for example, single chain antibodies, chimeric antibodies, bifunctional antibodies and humanized antibodies, as well as antigen-binding fragments thereof. Such non-naturally occurring antibodies can be constructed using solid phase peptide synthesis, can be produced recombinantly or can be obtained, for example, by screening combinatorial libraries consisting of variable heavy chains and variable light chains (Huse et al., Science 246:1275-1281, 1989). These and other methods of making, for example, chimeric, humanized, CDR-grafted, single chain, and bifunctional antibodies are well known to those skilled in the art (Winter and Harris, Immunol. Today 14:243-246, 1993; Ward et al., Nature 341:544-546, 1989; Harlow and Lane, Antibodies: A laboratory manual, Cold Spring Harbor Laboratory Press, 1988); Hilyard et al., Protein Engineering: A practical approach, IRL Press 1992; Borrabeck, Antibody Engineering, 2d ed., Oxford University Press 1995). Antibodies of the invention include intact molecules as well as fragments thereof, such as Fab, F(ab')2, and Fv which are capable of binding to an epitopic determinant present in a polypeptide of the present invention.

Where a peptide portion of a SW polypeptide of the invention (*i.e.,* any peptide fragment from SEQ ID NO:2) or peptide portion of another viral polypeptide of the invention used as an immunogen for antibody generation is non-immunogenic, it can be made immunogenic by coupling the hapten to a carrier molecule such as bovine serum albumin (BSA) or keyhole limpet hemocyanin (KLH), or by expressing the peptide portion as a fusion protein. Various other carrier molecules and methods for coupling a hapten to a carrier molecule are well known in the art (for example, by Harlow and Lane, *supra,* 1988). Methods for raising polyclonal antibodies, for example, in a rabbit, goat, mouse or other mammal, are well known in the art (see, for example, Green et al., "Production of Polyclonal Antisera," in Immunochemical Protocols, Manson, ed., Humana Press 1992, pages 1-5; Coligan et al., "Production of Polyclonal Antisera in Rabbits, Rats, Mice and Hamsters," in Curr. Protocols Immunol. (1992), section 2.4.1).

Monoclonal antibodies also can be obtained using methods that are well known and routine in the art (Kohler and Milstein, Nature 256:495, 1975; Coligan *et al., supra,* 1992, sections 2.5.1-2.6.7; Harlow and Lane, *supra,* 1988). For example, spleen cells from a mouse immunized with a virus, viral polypeptide or fragment thereof, can be fused to an appropriate myeloma cell line to produce hybridoma cells. Cloned hybridoma cell lines can be screened using, for example, labeled SVV polypeptide to identify clones that secrete monoclonal antibodies having the appropriate specificity, and hybridomas expressing antibodies having a desirable specificity and affinity can be isolated and utilized as a continuous source of the antibodies. Polyclonal antibodies similarly can be isolated, for example, from serum of an immunized animal. Such antibodies, in addition to being useful for performing a method of the invention, also are useful, for example, for preparing standardized kits. A recombinant phage that expresses, for example, a single chain antibody also provides an antibody that can used for preparing standardized kits. Monoclonal antibodies, for example, can be isolated and purified from hybridoma cultures by a variety of well established techniques, including, for example, affinity chromatography with Protein-A SEPHAROSE gel, size exclusion chromatography, and ion exchange chromatography (Barnes et al., in Meth. Mol. Biol. 10:79-104, Humana Press 1992); Coligan *et al., supra,* 1992, see sections 2.7.1-2.7.12 and sections 2.9.1-2.9.3).

An antigen-binding fragment of an antibody can be prepared by proteolytic hydrolysis of a particular antibody, or by expression of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol-reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly (see, for example, Goldenberg, U.S. Pat. Nos. 4,036,945 and 4,331,647; Nisonhoff et al., Arch. Biochem. Biophys. 89:230. 1960; Porter, Biochem. J. 73:119, 1959; Edelman et al., Meth. Enzymol., 1:422 (Academic Press 1967); Coligan *et al., supra,* 1992, see sections 2.8.1-2.8.10 and 2.10.1-2.10.4).

Another example of an antigen binding fragment of an antibody is a peptide coding for a single complementarity determining region (CDR). CDR peptides can be obtained by constructing polynucleotides encoding the CDR of an antibody of interest. Such polynucleotides can be prepared, for example, using the polymerase chain reaction to synthesize a variable region encoded by RNA obtained from antibody-producing cells (for example, Larrick et al., Methods: A Companion to Methods in Enzymology 2:106, 1991).

The antibodies of the invention are suited for use, for example, in immunoassays in which they can be utilized in liquid phase or bound to a solid phase carrier. In addition, the antibodies in these immunoassays can be detectably labeled in various ways. Examples of types of immunoassays which can utilize antibodies of the invention are competitive and non-competitive immunoassays in either a direct or indirect format. Examples of such immunoassays are the radioimmunoassay (RIA) and the sandwich (immunometric) assay. Detection of the antigens using the antibodies of the invention can be done utilizing immunoassays which are run in either the forward, reverse, or simultaneous modes, including immunohistochemical assays on physiological samples. Those of skill in the art will know, or can readily discern, other immunoassay formats without undue experimentation.

There are many different labels and methods of labeling antibodies known to those of ordinary skill in the art. Examples of the types of labels which can be used in the present invention include enzymes, radioisotopes, fluorescent compounds, colloidal metals, chemiluminescent compounds, phosphorescent compounds, and bioluminescent compounds. Those of ordinary skill in the art will know of other suitable labels for binding to the antibody, or alternatively to the antigen, or will be able to ascertain such, using routine experimentation.

As various changes can be made in the above methods and compositions without departing from the scope of the invention as defined in the appended claims, it is intended that all subject matter contained in the above description, or shown in the accompanying drawings be interpreted as illustrative, and not in a limiting sense.

## EXAMPLES

The examples described below are provided to illustrate the present invention and are not included for the purpose of limiting the invention.

### Example 1

Amplification and Purification of Virus

Cultivation of SW in PER.C6 cells: SVV is plaque purified once and a well isolated plaque is picked and amplified in PER.C6 cells (Fallaux et al., 1998). A crude virus lysate (CVL) from SVV infected PER.C6 cells is made by three cycles of freeze and thaw and used to infect PER.C6 cells. PER.C6 cells are grown in 50 x 150 $cm^2$ T.C. flasks using Dulbecco's modified Eagle medium (DMEM, Invitrogen, Carlsbad, CA, USA)) containing 10% fetal bovine serum (Biowhitaker, Walkersvile, MD, USA) and 10 mM magnesium chloride (Sigma, St Louis, MO, USA). The infected cells harvested 30 hr after infection when complete CPE is noticed and are collected by centrifugation at 1500 rpm for 10 minutes at 4°C. The cell pellet is resuspended in the cell culture supernatant (30 ml) and is subjected to three cycles of freeze and thaw. The resulting CVL is clarified by centrifugation at 1500 rpm for 10 minutes at 4°C. Virus is purified by two rounds of CsCl gradients: a one-step gradient (density of CsCl 1.24 g/ml and 1.4 g/ml) followed by one continuous gradient centrifugation (density of CsCl 1.33 g/ml). The purified virus concentration is determined spectrophotometrically, assuming $1A_{260} = 9.5 \times 10^{12}$ particles (Scraba D.G., and Palmenberg, A.C. 1999. Cardioviruses (Picornaviridae). In:Encyclopedia of Virology, Second edition, R.G. Webster and A Granoff Eds). Titers of purified virus are also determined by a standard plaque assay using PER.C6 cells. The yield of SVV from PER.C6 cells are greater than 200, 000 particles per cell with particles to PFU ratio of about 100. The yields of SVV from other permissive cells (H446-ATCC# HTB-171) may be at least this high or higher.

### Example 2

Electron Microscopy

SVV is mounted onto formvar carbon-coated grids using the direct application method, stained with uranyl acetate, and examined in a transmission electron microscope. Representative micrographs of the virus are taken at high magnification. For the transmission electron microscope, ultra-thin sections of SVV-infected PER.C6 cells are cut from the embedded blocks, and the resulting sections are examined in the transmission electron microscope.

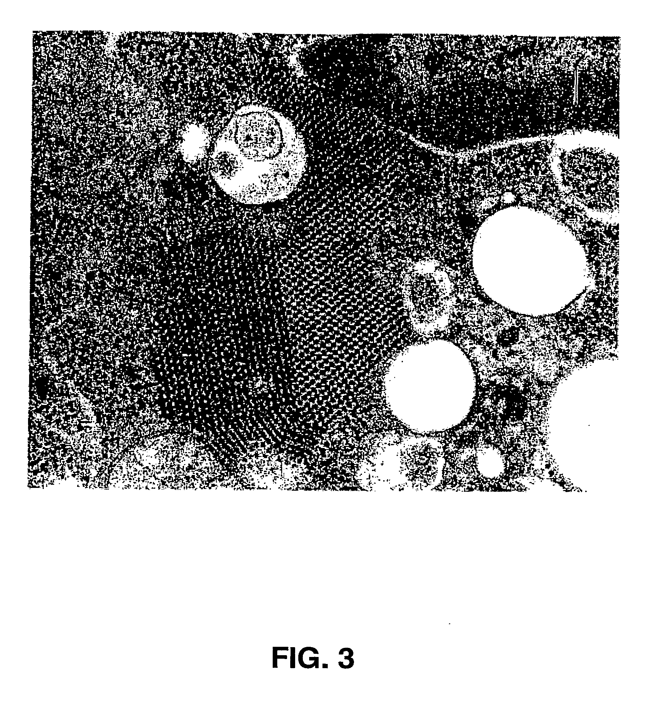

The purified SVV particles are spherical and about 27 nm in diameter, appearing singly or in small aggregates on the grid. A representative picture of SVV is shown in Figure 2. In some places, broken viral particles and empty capsids with stain penetration are also seen. Ultrastructural studies of infected PER.C6 cells revealed crystalline inclusions in the cytoplasm. A representative picture of PER.C6 cells infected with SVV is shown in Figure 3. The virus infected cells revealed a few large vesicular bodies (empty vesicles).

### Example 3

Nucleic Acid Isolation of SVV

RNA Isolation: SW genomic RNA was extracted using guanidium thiocyanate and a phenol extraction method using Trizol (Invitrogen). Isolation was performed according to the supplier's recommendations. Briefly, 250 μl of the purified SVV was mixed with 3 volumes TRIZOL and 240 μl of chloroform. The aqueous phase containing RNA was precipitated with 600 μl isopropanol. The RNA pellet was washed twice with 70% ethanol, dried and dissolved in DEPC-treated water. The quantity of RNA extracted was estimated by optical density measurements at 260 nm. An aliquot of RNA was resolved through a 1.25% denaturing agarose gel (Cambrex Bio Sciences Rockland Inc., Rockland, ME USA) and the band was visualized by ethidium bromide staining and photographed (Figure 4).

cDNA synthesis: cDNA of the SVV genome was synthesized by RT-PCR. Synthesis of cDNA was performed under standard conditions using 1 μg of RNA, AMV reverse transcriptase, and random 14-mer oligonucleotide or oligo-dT. Fragments of the cDNA were amplified, cloned into plasmids and the clones are sequenced

**Example 4**

SVV Sequence Analysis:

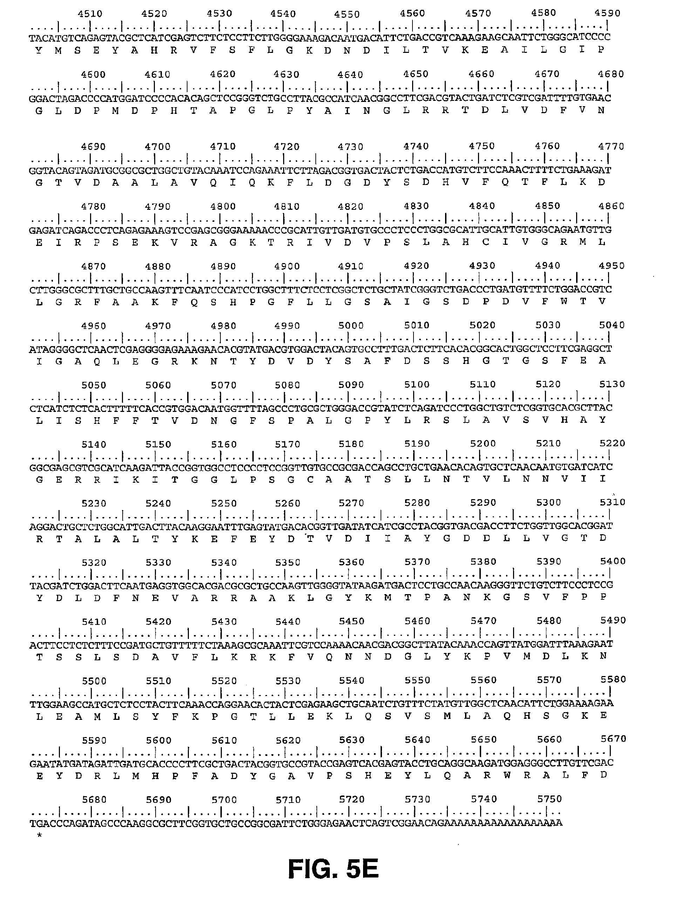

The nucleotide sequence of SVV SEQ ID NO: 1 was analyzed to determine its evolutionary relationship to other viruses. The translated product (SEQ ID NO:2) for this ORF was picornavirus-like and reached from the middle of VP2 to the termination codon at the end of the 3D polymerase and was 1890 amino acids in length (Fig. 5A-5E and 7A-7B). The 3' untranslated region (UTR), nucleotides 5671-5734, which follows the ORF is 64 nucleotides (nt) in length, including the termination codon and excluding the poly(A) tail of which 18 residues are provided (Fig. 5E).

Preliminary comparisons (not shown) of three partial genome segments of SW had revealed that SVV was most closely related members of the genus *Cardiovirus* (family *Picornaviridae*). Therefore an alignment of the polyprotein sequences of SVV, encephalomyocarditis virus (EMCV; species *Encephalomyocarditis virus,* Theiler's murine encephalomyelitis virus (TMEV; species *Theilovirus*), Vilyuisk human encephalomyelitis virus (VHEV; species *Theilovirus)* and a rat TMEV-like agent (TLV; species *Theilovirus)* was constructed (Fig. 28). From this alignment, the SW polyprotein processing was compared to the polyprotein processing of the most closely related members of the Cardiovirus genus. Cleavage sites between the individual polypeptides is demarcated by the "/" character in Fig. 28.

In picornaviruses, most polyprotein cleavages are carried out by one or more virus-encoded proteases, although in cardio-, aphtho-, erbo- and teschoviruses the cleavage between P1-2A and 2B is carried out by a poorly understood *cis*-acting mechanism related to the 2A sequence itself and critically involving the sequence "NPG/P", where "/" represents the break between the 2A and 2B polypeptides (Donnelly et al., 1997, J. Gen. Virol. 78: 13-21). One of the parechoviruses, Ljungan virus, has this sequence (NPGP SEQ ID NO: 111) present upstream of a typical parechovirus 2A and is either an additional 2A or is the C-terminal end of the P1 capsid region. In all nine currently recognised picornavirus genera, $3C^{pro}$ carries out all but the *cis*-acting self-cleaving reactions (i.e. 2A cleaves at its N-terminus in entero- and rhinoviruses and L cleaves at its C-terminus in aphthoviruses and erboviruses). The post-assembly cleavage of the capsid polypeptide VP0 to VP4 and VP2 is not carried out by $3C^{pro}$, but by an unknown mechanism which may involve the virus RNA. The VP0 cleavage does not occur in parechoviruses and kobuviruses. The normal cardiovirus $3C^{pro}$ cleavage site has either a glutamine (Q) or glutamate (E) at the -1 position and glycine (G), serine (S), adenine (A) or asparagine (N) at the +1 position (Table 2). The cleavages of the SW polyprotein conform to this pattern except for the VP3/VP1 site which is histidine (H)/serine (S) (Table 2); however, H/S is probably present as the cleavage site between 3A and $3B^{VPg}$ in at least one strain of equine rhinitis A virus (ERAV; genus *Aphthovirus)* (Wutz et al., 1996, J. Gen. Virol. 77:1719-1730).

**Table 2. Cleavage sites of SW and cardioviruses**

| Between | | SVV | EMCV | TMEV | Rat TLV | VHEV |
|---|---|---|---|---|---|---|
| L | VP4 | Not known | LQ/GN | PQ/GN | PQ/GN | PQ/GN |
| VP4 | VP2 | Not known | LA/DQ | LL/DQ<br>LM/DQ | LL/DQ | LL/DE |
| VP2 | VP3 | EQ/GP | RQ/SP | AQ/SP | PQ/SP | PQ/SP |
| VP3 | VP1 | FH/ST | PQ/GV | PQ/GV<br>PQ/GI<br>PQ/GS | PQ/GV | PQ/GV |
| VP1 | 2A | KQ/KM | LE/SP | LE/NP | LQ/NP | LE/NP |
| 2A | 2B | NPG/P* | NPG/P* | NPG/P* | NPG/P* | Nk |
| 2B | 2C | MQ/GP | QQ/SP | PQ/GP | AQ/SP | Nk |
| 2C | 3A | LQ/SP | AQ/GP<br>AQ/AP | AQ/SP | AQ/SP | Nk |
| 3A | 3B | SE/NA | EQ/GP | EQ/AA | EQ/AA | Nk |
| 3B | 3C | MQ/QP | IQ/GP<br>VQ/GP | IQ/GG | IQ/GG | Nk |

(continued)

| Between | | SVV | EMCV | TMEV | Rat TLV | VHEV |
|---|---|---|---|---|---|---|
| 3C | 3D | MQ/GL | PQ/GA | PQ/GA | PQ/GA | Nk |

*, the break between 2A and 2B is not a cleavage event.

Primary cleavages (P1/P2 and P2/P3) of SVV: These primary cleavage events are predicted to occur in a similar fashion to cardio-, aphtho-, erbo- and teschoviruses, involving separation of P1-2A from 2B by a novel mechanism involving the sequence NPG/P and a traditional cleavage event by $3C^{pro}$ between 2BC and P3 (Table 2).

P1 cleavages: Cleavages within the SVV P1 capsid coding region were relatively easy to predict by alignment with sequence with EMCV and TMEV (Table 2).

P2 cleavages: The 2C protein is involved in RNA synthesis. The 2C polypeptide of SVV contains NTP-binding motifs GxxGxGKS/T (domain A) and hyhyhyxxD (in which hy is any hydrophobic residue; domain B) present in putative helicases and all picornavirus 2Cs (Fig. 29).

P3 cleavages: Prediction of the P3 cleavage sites was also relatively straightforward. Little is known about the function of the 3A polypeptide. However, all picornavirus 3A proteins contain a putative transmembrane alpha-helix. Primary sequence identity is low in this protein between SVV and cardioviruses (See Fig. 28 between positions 1612 to 1701).

The genome-linked polypeptide, VPg, which is encoded by the 3B region, shares few amino acids in common with the other cardioviruses, however, the third residue is a tyrosine, consistent with its linkage to the 5' end of the virus genome (Rothberg et al., 1978). See Fig. 28 between positions 1703 and 1724.

The three-dimensional structure of four picornavirus 3C cysteine proteases have been solved and the active-site residues identified (HAV, Allaire et al., 1994, Nature, 369: 72-76; Bergmann et al., 1997, J. Virol., 71: 2436-2448; PV-1, Mosimann et al., 1997, J. Mol. Biol., 273: 1032-1047; HRV-14, Matthews et al., 1994, Cell, 77: 761-771; and HRV-2, Matthews et al., 1999, Proc. Natl. Acad. Sci. USA, 96: 11000-11007). The cysteine bolded in Fig. 29 is the nucleophile, while the first bolded histidine is the general base and the specificity for glutamine residues is defined mainly by the second bolded histidine; all three residues are conserved in the SVV sequence (Fig. 29) and all other known picornaviruses (Fig. 28; for 3C sequence comparison see between positions 1726 and 1946).

The 3D polypeptide is the major component of the RNA-dependent RNA polymerase and SVV contains motifs conserved in picorna-like virus RNA-dependent RNA polyerases, i.e. KDEL/IR (SEQ ID NO: 113), PSG, YGDD (SEQ ID NO: 114) and FLKR (SEQ ID NO: 115) (Fig. 3: Fig. 28 between positions 1948 and 2410).

Myristoylation of the N-terminus of P1: In most picornaviruses the P1 precursor polypeptide is covalently bound by its N-terminal glycine residue (when present the N-terminal methionine is removed) to a molecule of myristic acid via an amide linkage (Chow et al., 1987, Nature, 327: 482-486). Consequently the cleavage products VP0 and VP4 which contain the P1 N-terminus are also myristoylated. This myristoylation is carried out by myristoyl transferase which recognises an eight amino acid signal beginning with glycine. In picornaviruses, a five residue consensus sequence motif, G-x-x-x-T/S, has been identified (Palmenberg, 1989, In Molecular Aspects of Picornavirus Infection and Detection, pp. 211-241, Ed. Semler & Ehrenfeld, Washington D.C., Amer. Soc. for Micro.). Parechoviruses *(Human parechovirus,* and *Ljungan virus)* as well as not having a maturation cleavage of VP0 are apparently not myristoylated, however, there appears to be some type of molecule blocking the N-terminus of VP0 for these viruses.

*Comparisons of the individual SW polypeptides with the public sequence databases*

Each of the SW polypeptides (SEQ ID NOs: 4, 6, 8, 10, 12, 14, 16, 18, 20 and 22) were compared to the public protein sequence databases using the FASTA online program at the European Bioinformatics Institute (EBI; http://www.ebi.ac.uk/). The results (best matches) of these comparisons are shown in Table 3. The capsid polypeptides (VP2, VP3 and VP1) taken as a whole, along with 2C, $3C^{pro}$ and $3D^{pol}$ are most closely related to members of the cardiovirus genus, however, the short predicted 2A sequence is closer to that of Ljungan virus (genus *Parechovirus).* A more detailed comparison of the SVV 2A nucleotide sequence with similar sequences is shown in Fig. 28 (see also Fig. 70 for 2A-like NPG/P (SEQ ID NO: 111) protein comparison).

**Table 3. Database matches of individual predicted polypeptides of Seneca Valley virus**

| SVV polypeptide | Length (aa) | % identity | % identity ungapped | aa overlap | Organism | Matched protein |
|---|---|---|---|---|---|---|
| L (Leader) | No data | - | - | - | - | - |
| VP4 (1A) | No data | - | - | - | - | - |

(continued)

| SVV polypeptide | Length (aa) | % identity | % identity ungapped | aa overlap | Organism | Matched protein |
|---|---|---|---|---|---|---|
| VP2 (1B) | >142 | 42.857 | 44.037 | 112 | TMEVWW | VP2 |
| | | ~51 | - | -80 | EMCV BEL-2887A/91 | VP2 |
| VP3 (1C) | 239 | 44.068 | 46.637 | 236 | EMCV ATCC VR-129B | VP3 |
| VP1 (1D) | 259 | 31.086 | 36.404 | 267 | EMCV M100/1/02 | VP1 |
| 2A | 14 | 71.429 | 71.429 | 14 | Ljungan virus 174F | 2A1 |
| 2B | 128 | 39.286 | 41.509 | 56 | *Ureaplasma urealyticum* | Multiple banded antigen |
| 2C | 322 | 38.602 | 40.190 | 329 | EMCV PV21 | 2C |
| 3A | 90 | 37.838 | 41.791 | 74 | *Chlorobium tepidum* TLS* | Enolase 2† |
| $3B^{VPg}$ | 22 | No matches | - | - | - | - |
| $3C^{pro}$ | 211 | 37.089 | 38.537 | 213 | EMCV-R | 3C protease |
| $3D^{pol}$ | 462 | 58.009 | 58.515 | 462 | EMCV-PV21 | 3D polymerase |

* a photosynthetic, anaerobic, green-sulfur bacterium
t 2-phosphoglycerate dehydratase 2) (2-phospho-D-glycerate hydro-lyase 2

The significance of the matches of SW 2B with *Ureaplasma urealyticum* multiple banded antigen or 3A with *Chlorobium tepidum* endolase 2 is not clear, however, these relationships maybe worthy of further investigation.

*Phylogenetic comparison of SW polypeptides with other picornaviruses*

Those SW polypeptides which could be aligned with the cardioviruses (VP2, VP3, VP1, 2C, 3C and 3D) were compared with the same proteins of representative members of each of the picornavirus species (Table 4). The programs BioEdit v5.0.9 (Hall, 1999, Nucl. Acids. Symp. Ser., 41: 95-98) and Clustal X v1.83 (Thompson et al., 1997, Nucl. Acids Res., 25:4876-4882) were used to make the alignments and to construct distance matrices and unrooted Neighbor-joining trees according to the algorithm of Saitou and Nei (Satiou and Nei, 1987, Mol. Biol. Evol., 4: 406-425). Confidence limits on branches were accessed by bootstrap resampling (1000 pseudo-replicates). The trees were drawn using TreeView 1.6.6 (Page, 1996) (Figs. 31 to 37). The distance matrices used to construct the trees used values corrected for multiple substitutions, while Figures 38-44 show the actual percentage amino acid identities. Table 4 shows the current classification of the family *Picornaviridae* and the representative virus sequences used in these comparisons.

**Table 4. The taxonomic classification of the picornaviruses used in the comparisons with SW.**

| Genus | Species | Representative virus | Abbrev. | Acc. No. |
|---|---|---|---|---|
| *Enterovirus* | *Poliovirus* | Poliovirus 1 | PV-1 | V01149 |
| | *Human enterovirus A* | Coxsackievirus A16 | CV-A16 | U05876 |
| | *Human enterovirus B* | Coxsackievirus B5 | CV-B5 | X67706 |
| | *Human enterovirus C* | Coxsackievirus A21 | CV-A21 | D00538 |
| | *Human enterovirus D* | Enterovirus 70 | EV-70 | D00820 |
| | *Simian enterovirus A* | Simian enterovirus A1 | SEV-A | AF201894 |
| | *Bovine enterovirus* | Bovine enterovirus 1 | BEV-1 | D00214 |
| | *Porcine enterovirus B* | Porcine enterovirus 9 | PEV-9 | AF363453 |
| New genus? | Not yet designated | Simian virus 2* | SV2 | AY064708 |
| | *Porcine enterovirus A* | Porcine enterovirus 8* | PEV-8 | AF406813 |
| *Rhinovirus* | *Human rhinovirus A* | Human rhinovirus 2 | HRV-2 | X02316 |
| | *Human rhinovirus B* | Human rhinovirus 14 | HRV-14 | K02121 |
| *Cardiovirus* | *Encephalomyocarditis virus* | Encephalomyocarditis virus | EMCV | M81861 |

(continued)

| Genus | Species | Representative virus | Abbrev. | Acc. No. |
|---|---|---|---|---|
| | *Theilovirus* | Theiler's murine encephalomyelitis virus | TMEV | M20562 |
| *Aphthovirus* | *Foot-and-mouth disease virus* | Foot-and-mouth disease virus O | FMDV-O | X00871 |
| | *Equine rhinitis A virus* | Equine rhinitis A virus | ERAV | X96870 |
| *Hepatovirus* | *Hepatitis A virus* | Hepatitis A virus | HAV | M14707 |
| | Avian encephalomyelitis-like viruses | Avian encephalomyelitis virus | AEV | AJ225173 |
| *Parechovirus* | *Human parechovirus* | Human parechovirus 1 | HPeV-1 | L02971 |
| | *Ljungan virus* | Ljungan virus | LV | AF327920 |
| *Kobuvirus* | *Aichi virus* | Aichi virus | AiV | AB040749 |
| | *Bovine kobuvirus* | Bovine kobuvirus | BKV | AB084788 |
| *Erbovirus* | *Equine rhinitis B virus* | Equine rhinitis B virus 1 | ERBV-1 | X96871 |
| *Teschovirus* | *Porcine teschovirus* | Porcine teschovirus 1 | PTV-1 | AJ011380 |

* the current taxonomic status of SV2 and PEV-8 places them in the enterovirus genus, however, it has been suggested that they may be reclassified in a new genus (Krumbholz et al., 2002; Oberste et al., 2003).

The trees of the individual capsid proteins (Figs. 31 to 33) are not all representative of the tree produced when the data from all tree polypeptides is combined (Fig. 34). This is probably the result of difficulties in aligning the capsid polypeptides, particularly when they are not full length as is the case for VP2 (Fig. 31). However, the P1, 2C, $3C^{pro}$ and $3D^{pol}$ trees are all in agreement and show that SW clusters with EMCV and TMEV.

*Seneca Valley virus as a member of the cardiovirus genus*

Clearly the $3D^{pol}$ of SVV is related to the cardioviruses, almost as closely as EMCV and TMEV are to each other (Fig. 37; Fig. 44). In the other polypeptides which are generally considered as being relatively conserved in picornaviruses, 2C and 3C, SVV is also most closely related to the cardioviruses although it is not as closely related to EMCV and TMEV as they are to each other (Fig. 42 and Fig. 43, respectively). In the outer capsid proteins (taken as a whole), SVV is also most closely related to the cardioviruses and has approximately the same relationship as the two aphthovirus species, *Foot-and-mouth disease virus* and *Equine rhinitis A virus* (~33%). SVV diverges greatly from the cardioviruses in the 2B and 3A polypeptides and has no detectable relationship with any known picornavirus. However, this is not without precedent; avian encephalomyelitis virus differs considerably from hepatitis A virus (HAV) in 2A, 2B and 3A (Marvil et al., 1999, J. Gen. Virol., 80:653-662) but is tentatively classified within the genus *Hepatovirus* along with HAV.

Seneca Valley virus is clearly not a typical cardiovirus if EMCV and TMEV are taken as the standard. However, even these two viruses have their differences, notably in the 5' UTR (Pevear et al., 1987, J. Gen. Virol., 61: 1507-1516). However, phylogenetically SVV clusters with EMCV and TMEV in much of its polyprotein (P1, 2C, $3C^{pro}$ and $3D^{pol}$ regions). Ultimately, the taxonomic position of SVV within the Picornaviridae will be decided by the Executive Committee (EC) of the International Committee for the Taxonomy of Viruses (ICTV) following recommendations by the *Picornaviridae* Study Group and supporting published material. There are two options: i) include SVV as a new species in the cardiovirus genus; or ii) assign SVV to a new genus. At this stage, and for purposes of the present invention, SVV is in the cardiovirus genus.

**Example 4**

SDS-PAGE and N-Terminal Sequence Analysis of SVV Capsid Proteins

Purified SW is subjected to electrophoresis using NuPAGE pre-cast Bis-Tris polyacrylamide mini-gel electrophoresis system (Novex, San Diego, Ca, USA). One half of the gel is visualized by silver stain while the other half is used to prepare samples for amino acid sequencing of the N-termini of the capsid proteins. Prior to transfer of proteins to membrane, the gel is soaked in 10 mM CAPS buffer, pH 11, for 1 hour, and a PVDF membrane (Amersham) is wetted in methanol. Proteins are transferred to the PVDF membrane. After transfer, proteins are visualized by staining with Amido black for approximately 1 minute, and bands of interest are excised with a scalpel and air dried. The proteins can be subjected to automated N-terminal sequence determination by Edman degradation using a pulsed phase sequencer.

Three major structural proteins of the purified SVV are shown in Figure 45 (approximately 36 kDa, 31 kDa, and 27 kDa).

**Example 5**

Assay for Neutralization Antibodies to SVV in Human Serum Samples

Preexisting antibodies to particular viral vectors may limit the use of such vectors for systemic delivery applications such as for treatment of metastatic cancer, because preexisting antibodies may bind to systemically delivered vectors and neutralize them before the vectors have a chance to transduce the targeted tissue or organ. Therefore, it is desirable to ensure that humans do not carry neutralization antibodies to viral vectors selected for systemic delivery. To determine whether human sera samples contain SVV-specific neutralizing antibodies, neutralization assays are carried out using randomly collected human sera samples.

Tissue culture infective dose 50: One day before the experiment, 180 μl of PER.C6 cell suspension containing $1x10^4$ cells are plated in 96-well tissue culture dish. The crude virus lysate (CVL) of SVV is diluted in log steps from $10^{-0}$ to $10^{-11}$ in DMEM medium (Dulbecco's Modified Eagle's Medium) and 20 μl of each dilution is transferred to three wells of a Falcon 96-well tissue culture plate containing PER.C6 cells. The plates are incubated at 37°C in 5% $CO_2$ and read at 3 days for microscopic evidence of cytopathic effect (CPE), and the tissue culture infective dose 50 ($TCID_{50}$) is calculated.

Neutralization assay: First, 40 μl of medium is placed in all the wells and then 40 μl of heat-inactivated serum is added to the first well and mixed by pipeting, making a 1:4 dilution used for screening purposes. 40 μl is then transferred to the next well to perform a two-fold dilution of the serum samples. 40 μl of SVV virus, containing 100 $TCID_{50}$, is added to wells containing diluted serum samples. Plates are incubated at 37°C for 1 hour. 40 μl of the mix is taken and transferred to a plate containing PER.C6 cells ($1x10^4$ cells/160 μl/well). The plates are incubated at 37°C for 3 days. After this time, the cultures are read microscopically for CPE.

In a representative neutralization assay performed as described above, twenty-two human sera samples randomly collected from USA, Europe and Japan were examined for SVV specific neutralizing antibodies. The serum samples were serially diluted and mixed with a fixed amount of SVV containing 100 $TCID_{50}$. Serum-virus mixtures were then used to infect PER.C6 cells and incubated for 24 hours. Neutralizing antibody titer was determined as the reciprocal of the highest dilution of serum able to block CPE formation. In this experiment, no dilution of serum blocked CPE formation indicating that the human serum samples did not contain SVV neutralizing antibodies.

Further SVV infection of PER.C6 was not inhibited by incubation with human blood *(see* Example 6), indicating that SVV infection was not inhibited by complement or by hemagglutination. As a result, SVV exhibits a longer circulation time *in vivo* than other oncolytic viruses, which is a significant problem with the use of oncolytic adenoviruses.

**Example 6**

Binding of SVV to Human Erythrocytes and Hemagglutination

Various viral serotypes have been shown to cause *in vitro* hemagglutination of erythrocytes isolated from blood of various animal species. Hemagglutination or binding to erythrocytes may cause toxicity *in vivo* and may also affect *in vivo* biodistribution and the efficacy of a viral vector. Therefore, it is desirable to analyze the erythrocyte agglutination properties of a viral vector selected for systemic administration to treat metastatic cancers.

Hemagglutination assay: To determine whether SVV causes agglutination of human erythrocytes, hemagglutination assays are carried out in U-bottom 96-well plates. Purified SVV is serially diluted in 25 μl PBS (Phosphate Buffered Saline) in duplicates, and an equal volume of 1% erythrocyte suspension is added to each well. Blood samples used for isolation of erythrocytes are obtained from healthy individuals with heparin as an anticoagulant. Erythrocytes are prepared by washing the blood three times in cold PBS to remove the plasma and the white blood cells. After the last wash, erythrocytes are suspended in PBS to make a 1% (V/V) cell suspension. The virus and erythrocytes are gently mixed and the plates are incubated at room temperature for 1 hour and monitored for a hemagglutination pattern.

Whole blood inactivation assay: To rule out direct inactivation of SVV by blood components, aliquots of virus are incubated with heparinized human blood belonging to A, B, AB and O blood groups or PBS for 30 minutes or 1 hour at room temperature prior to separation of plasma, after which PER.C6 cells are infected and titers are calculated.

In representative assays performed as described above, no hemagglutination of human erythrocytes of different blood groups (A, B, AB and O) was seen at any tested dilutions of SW. A slight increase in the virus titer is noticed when SVV is mixed with blood human samples and incubated for 30 minutes and 1 hour, indicating that the virus is not inactivated by blood components but becomes more infectious under tested conditions.

**Example 7**

*In vivo* Clearance

Blood circulation time: To determine the blood circulation time and the amount of the virus in the tumor, H446 tumor bearing nude mice were treated with SVV at a dose of $1x10^{12}$ vp/kg by tail vein injection. The mice were bled at 0, 1, 3, 6, 24,48, 72 hours and 7 days (189 hours) post-injection and the plasma was separated from the blood immediately after collection, diluted in infection medium, and used to infect PER.C6 cells. The injected mice were sacrificed at 6, 24, 48, 72 hours and 7 days post-injection and the tumors were collected. The tumors were cut into small sections and suspended in one ml of medium and subjected to three cycles of freeze and thaw to release the virus from the infected cells. Serial log dilutions of supernatants were made and assayed for titer on PER.C6 cells. SVV titers were expressed as pfu/ml. The tumor sections were also subjected to H&E staining and immunohistochemistry to detect the virus capsid proteins in the tumor.

The circulating levels of virus particles in the blood were determined based on the assumption that 7.3% of mouse body weight is blood. In representative assays performed as essentially as described above, within 6 hours of virus administration, the circulating levels of SVV reduced to zero particles and SVV was not detectable at later time points (Fig. 46A). In the tumor, SVV was detectable at 6 hours post-injection, after which the amount of the virus increased steadily by two logs (Fig. 46B). The virus was detectable in the tumor as late as 7 days postinjection (Fig. 46B). The tumor sections when subjected to immunohistochemistry, revealed SVV proteins in the tumor cells (Figure 47, top panels). When stained by H&E, the tumor sections revealed several rounded tumor cells (Figure 47, bottom panels).

SVV also exhibits a substantially longer resident time in the blood compared to similar doses of i.v. adenovirus. Following a single i.v. dose, SW remains present in the blood for up to 6 hours (Figure 46C; Figure 46C is a duplication of Figure 46A for comparison purposes to Figure 46D), whereas adenovirus is cleared from the blood in about an hour (Figure 46D).

**Example 8**

Tumor Cell Selectivity

*In vitro* cell killing activity of SVV: To determine the susceptibility of human, bovine, porcine, and mouse cells, normal and tumor cells were obtained from various sources and infected with SVV. All cell types were cultured in media and under the conditions recommended by the supplier. Primary human hepatocytes may be purchased from In Vitro Technologies (Baltimore, MD) and cultured in Hepatocye Culture Media (HCM™, BioWhittaker/Clonetics Inc., San Diego, CA).

*In vitro* cytopathic assay: To determine which types of cells are susceptible to SVV infection, monolayers of proliferating normal cells and tumor cells were infected with serial dilutions of purified SVV. The cells were monitored for CPE and compared with uninfected cells. Three days following infection, a MTS cytotoxic assay is performed and lethal dose-50 percent ($LD_{50}$) values in particles per cell are calculated. See Tables 5 and 6 below.

Table 5. Cell lines with $EC_{50}$ values less than 100

| **Cell lines with EC50 < 1** | **EC50 number** |
|---|---|
| H446 (human sclc) | 0.001197 |
| PERC6 | 0.01996 |
| H69AR (sclc-multidrug resisitant) | 0.03477 |
| 293 (human kidney transformed with ad5E1) | 0.03615 |
| Y79 (human retinoblastoma) | 0.0003505 |
| IMR32 (human brain; neuroblastoma) | 0.03509 |
| D283med (human brain; cerebellum; medulloblastoma) | 0.2503 |
| SK-N-AS (human brain; neuroblastoma) | 0.474 |
| N1E-115 (mouse neuroblastoma) | 0.002846 |
| SK-NEP-1 (kidney, wilms' tumor, pleural effusion, human) | 0.03434 |
| BEKPCB3E1 (bovine embryonic kidney cells transformed with ad5E1 | 0.99 |

(continued)

| Cell lines with EC50 < 1 | EC50 number |
|---|---|
| | |
| **Cell Lines with EC50 < 10 (1-10)** | **EC50 number** |
| H1299 (human-non sclc) | 7.656 |
| ST (pig testes) | 5.929 |
| DMS 153 (human sclc) | 9.233 |
| | |
| **Cell lines with EC50 < 100 (10-100)** | **EC50 number** |
| BEK (bovine embryonic kidney) | 17.55 |

Table 6. Cell lines with $EC_{50}$ values more than 1000

| | | |
|---|---|---|
| M059K (human brain; malignant glioblastoma) | HUVEC (human vein endothelial cells) | CMT-64 (mouse-sclc) |
| KK (human glioblastoma) | HAEC (human aortic endothelial cells) | LLC-1 (mouse-LCLC)) |
| U-118MG (human glioblatoma) | WI38 (human lung fibroblast) | RM-1 (mouse-prostate) |
| DMS 79 (human sclc) | MRC-5 (human lung fibroblast) | RM-2 (mouse-prostate) |
| H69 (human sclc) | IMR90 (human lung fibroblast) | RM-9 (mouse-prostate) |
| DMS 114 (human sclc) | HMVEC (human microvascular endothelial cells-adult) | MLTC-1 (mouse-testes) |
| DMS 53 (human sclc) | HMVEC (human microvascular endothelial cells-neonatal) | KLN-205 (mouse-sqcc) |
| H460 (human-LCLC) | HCN-1A (human brain) | CMT-93 (mouse-rectal) |
| A375-S2 (human melanoma) | HRCE (human renal cortical epithelial cells) | B16F0 (mouse melanoma) |
| SK-MEL-28 (human melanoma) | | Neuro-2A (mouse neuroblastoma) |
| PC3 (human prostate) | | C8D30 (mouse brain) |
| PC3M2AC6 (human prostate) | | PK15 (pig-kidney) |
| LNCaP (human prostate) | | FBRC (fetal bovine retina) |
| DU145 (human prostate) | | MDBK (bovine kidney) |
| Hep3B (human liver carcinoma) | | CSL 503 (sheep lung cells transformed with ad5E1) |
| Hep2G (human liver carcinoma) | | OFRC (ovine fetal retina cells) |
| SW620 (human-colon) | | |
| SW839 (human kidney) | | |
| 5637 (human bladder) | | |
| HeLa S3 | | |
| S8 | | |

The MTS assay was performed according to the manufacturer's instructions (CellTiter 96® $AQ_{ueous}$ Assay by Promega, Madison, WI). The CellTiter 96® $AQ_{ueous}$ Assay preferably uses the tetrazolium compound (3-(4,5-dimethylthiazol-2-yl)-5-(3-carboxymethoxyphenyl)-2-(4-sulfophenyl)-2H-tetrazolium, inner salt; MTS) and an electron coupling reagent, phenazine methosulfate (PMS). Contact-inhibited normal human cells evaluated in the study include: HUVEC (human umbilical vein endothelial cells), HAEC (human aortic endothelial cells, Clonetics/BioWhittaker # CC-2535), Wi38 (normal human embryo lung fibroblasts, ATCC # CCL-75), IMR90 (human normal lung fibroblasts, ATCC CCL-186), MRC-5 (human normal lung fibroblasts, ATCC, # CCL-171) and HRCE (human renal cortical epithelial cells, CloneticsBioWhittaker # CC-2554).

SVV does not produce CPE in any of the above contact-inhibited normal cells. No virus-induced CPE was seen in the following human tumor cell lines: Hep3B (ATCC # HB-8064), HepG2 (human hepatocellular carcinoma, ATCC # HB-8065), LNCaP (human prostate carcinoma, ATCC # CRL-10995), PC3M-2AC6, SW620 (human colorectal adenocarcinoma, ATCC # CCL-227), SW 839 (human kidney adenocarcinoma, ATCC # HTB-49), 5637 (human urinary bladder carcinoma, ATCC # HTB-9), DMS-114 (small cell lung cancer, ATCC # CRL-2066), DMS 153 (human small cell lung cancer, ATCC # CRL-2064), A549 (human lung carcinoma, ATCC # CCL-185), HeLa S3 (human cervical adenocarcinoma, ATCC # CCL-2.2), NCI-H460 (human large cell lung cancer, ATCC # HTB-177), KK (glioblastoma), and U-118 MG (human glioblastoma, ATCC # HTB-15). Note - the cell lines in Table 6 with $EC_{50}$ values greater than 1000 are most likely not permissive for SW replication and/or virion production; although the possibility remains that SVV can bind and enter into these cells but CPE is not observed because SVV replication cannot occur inside the cell or that replication does occur but CPE is not observed because there is some other post-entry block (*i.e.,* no packaging of replicated SVV genomes into virions). However, considering the absence of CPE in these cell lines, these cell-lines, and potentially tumor-types thereof, are good candidates to test which cell and tumor-types are permissive or non-permissive for SW replication. Although wild-type SVV is tumor-specific, and has been shown to target neuroendocrine tumors, including small cell lung cancer and neuroblastomas, there may be individual patients that have types of etiologies such that SVV is not permissive in their form of neuroendocrine tumor. Therefore, the invention does contemplate the generation of SVV derivatives that can kill tumor cell-types isolated from individual patients where the tumors are non-permissive to the wild-type SVV, and the tumor-types isolated from these individuals can include, for example, glioblastoma, lymphoma, small cell lung cancer, large cell lung cancer, melanoma, prostate cancer, liver carcinoma, colon cancer, kidney cancer, colon cancer, bladder cancer, rectal cancer and squamous cell lung cancer.

SVV-mediated cytotoxicity on primary human hepatocytes (In Vitro Technologies) was determined by LDH release assay (CytoTox® 96 Non-Radioactive Cytotoxicity Assay, Promega, # G1780). Primary human hepatocytes plated in collagen coated 12-well plates were infected with SVV at 1, 10 and 100 and 1000 particles per cell (ppc). After 3 hours of infection, the infection medium was replaced with 2 ml of growth medium and incubated for 3 days in a $CO_2$ incubator. The cell associated lactate dehydrogenase (LDH) and LDH in the culture supernatant was measured separately. Percent cytotoxicity is determined as a ratio of LDH units in supernatant over maximal cellular LDH plus supernatant LDH.

$$\text{Percent cytotoxicity} = \frac{\text{LDH units in culture supernatant X 100}}{\text{Sum of LDH units in supernatant and cell lysate}}$$

The data shown in Figure 48 illustrates the absence of SVV mediated hepatoxicity at all tested multiplicity of infections.

**Example 10**

Virus Production Assay

To assess the replicative abilities of SVV, several selected contact-inhibited normal cells and actively dividing tumor cells were infected with SVV at one virus particle per cell (ppc). After 72 hours, cells and the medium were subjected to three freeze-thaw cycles and centrifuged to collect the supernatant. Serial log dilutions of supernatants were made and assayed for titer on PER.C6 cells. For each cell line, the efficiency of SVV replication was expressed as pfu/ml (Figure 49).

**Example 10**

Toxicity

The maximum tolerated dose (MTD) is defined as the dosage immediately preceding the dose at which animals (e.g. mice) demonstrate a dose limiting toxicity (DLT) after the treatment with SVV. DLT is defined as the dose at which the animals exhibit a loss in body weight, symptoms, and mortality attributed to SVV administration during the entire duration of the study. Neutralizing antibodies to SVV were assessed at baseline, day 15, and day 21. Neutralization assays were carried as described earlier.

Escalating doses ($1x10^8$ - $1x10^{14}$vp/kg) of SVV were administered intravenously into both immune deficient nude and caesarean derived-1 (CD-1) out-bred immune competent mice purchased from Harlan Sprague Dawley (Indianapolis, IN, USA) to determine the MTD with 10 mice per dose level. The virus was well-tolerated at all tested dose levels without exhibiting any clinical symptoms and without loss in body weight (Figure 50). Mice were bled at day 15 and 21 and the sera was monitored for the presence of SVV-specific neutralizing antibodies in neutralization assays. SVV injected CD1 mice develop neutralizing antibodies and the titers range from 1/1024 to greater than 1/4096.

Another toxicity study was conducted on the immunocompetent mouse strain (A/J). It has been demonstrated that SVV exhibits cell killing activity and replication in N1E-115 cells (see Table 1). The murine cell line N1E-115 (a neuroblastoma cell line, *i.e.,* neuroendocrine cancer) is derived from the A/J mouse strain. Thus, a syngeneic mouse model was established where N1E-115 cells were implanted subcutaneously in A/J mice to form tumors, and the mice were then treated with SVV to investigate its efficacy and toxicity.

In the A/J study, mice were i.v. injected with SVV to determine whether A/J mice can tolerate systemic administration of SVV. Blood hematology results were obtained to look for signs of toxicity, and serum chemistry results can also be obtained. The study design is shown in Table 7 below:

Table 7: All Study Design

| Group # | Animals (Female) | Test Article | Dosage Level (particles/kg) | Dosage Volume (mL/kg) | Dosing regimen | Necropsy Day |
|---|---|---|---|---|---|---|
| 1 | 5 | Vehicle | 0 | 10 | IV on Day 1 | Day 15 |
| 2 | 5 | SVV | $10^8$ | 10 | IV on Day 1 | Day 15 |
| 3 | 5 | SVV | $10^{11}$ | 10 | IV on Day 1 | Day 15 |
| 4 | 5 | SVV | $10^{14}$ | 10 | IV on Day 1 | Day 15 |

The A/J mice were 8-10 week old females obtained from The Jackson Laboratory (Bar Harbor, Maine). SVV was prepared by storing isolated virions at -80°C until use. SVV was prepared fresh by thawing on ice and diluting with HBSS (Hank's balanced salt solution). SVV was diluted to concentrations of $10^7$ particles/mL for group 2, $10^{10}$ particles/mL, for group 3, and $10^{13}$ particle/mL for group 4. HBSS was used as the vehicle control for group 1. All dosing solutions were kept on wet ice until dosing.

SVV was administered to animals intravenous injection via the tail vein at a dose volume of 10 mL/kg body weight. Animals were weighed on the day of dosing and dose volumes were adjusted based on body weight *(i.e.,* a 0.0200 kg mouse gets 0.200 mL of dosing solution). Mice were monitored twice daily for morbidity and mortality. Mice were weighed twice weekly. Information relating to moribund animals and animals exhibiting any unusual symptoms (physically or behaviorally) are recorded immediately.

Post-mortem observations and measurements entail the collection of blood from all surviving animals at terminal sacrifice for standard hematology and serum chemistry (AST, ALT, BUN, CK, LDH). The following organs are to be collected at sacrifice: brain, heart, lung, kidney, liver, and gonads. Half of each organ sample is snap frozen on dry ice and the other half will be placed in formalin.

Initial blood hematology results (CBC, differential) were obtained two weeks after SVV injection and the results are summarized below in Table 8 below. Five mice were tested from each test group (see Table 7):

Table 8: A/J Toxicity Results - Blood Hematology

| | Test Group 1 | Test Group 2 | Test Group 3 | Test Group 4 |
|---|---|---|---|---|
| **Body Weight Result ± SD (g):**<br>Day 0 | 21.48 ± 0.88 | 21.98 ± 1.93 | 22.58 ± 0.87 | 21.04 ± 1.67 |
| Day 14 | 20.26 ± 0.93 | 20.92 ± 1.71 | 21.44 ± 0.84 | 21.26 ± 1.45 |
| **CBC Wet (Result ± SD (ref range)):**<br>White blood count (THSN/UL) | 3.63 ± 1.57 (2.60-10.69) | 4.5 ± 1.57 (2.60-10.69) | 4.26 ± 0.94 (2.60-10.69) | 4.72 ± 0.62 (2.60-10.69) |

(continued)

| | | | | |
|---|---|---|---|---|
| **CBC Wet (Result ± SD (ref range)):** | | | | |
| Red blood count (MILL/UL) | 9.87 ± 0.03 (6.4-9.4) | 9.49 ± 0.07 (6.4-9.4) | 9.76 ± 0.37 (6.4-9.4) | 9.71 ± 0.32 (6.4-9.4) |
| Hemoglobin (GM/DL) | 15.37 ± 0.06 (11.5-16.1) | 14.78 ± 0.29 (11.5-16.1) | 15.12 ± 0.66 (11.5-16.1) | 15.02 ± 0.63 (11.5-16.1) |
| Hematocrit (%) | 46.03 ± 0.40 (36.1-49.5) | 44.52 ± 0.49 (36.1-49.5) | 45.7 ± 1.82 (36.1-49.5) | 45.28 ± 1.69 (36.1-49.5) |
| MCV (FL) | 46.67 ± 0.58 (45.4-60.3) | 47.00 ± 0.0 (45.4-60.3) | 47.0 ± 0.0 (45.4-60.3) | 46.6 ± 0.55 (45.4-60.3) |
| MHC (PICO GM) | 15.57 ± 0.06 (14.1-19.3) | 15.70 ± 0.17 (14.1-19.3) | 15.37 ± 0.06 (14.1-19.3) | 15.43 ± 0.15 (14.1-19.3) |
| MCHC (%) | 33.37 ± 0.12 (25.4-34.1) | 33.14 ± 0.48 (25.4-34.1) | 33.08 ± 0.22 (25.4-34.1) | 33.14 ± 0.25 (25.4-34.1) |
| Platelet (THSN/UL) | 885.33 ± 28.6 (592-2972) | 758.2 ± 146.2 (592-2972) | 874.8 ± 56.7 (592-2972) | 897.2 ± 105.4 (592-2972) |
| **Differential (Result ± SD (ref** range)): | | | | |
| Bands (THSN/UL) | 0.0 (0.0-0.1) | 0.0 (0.0-0.1) | 0.0 (0.0-0.1) | 0.0 (0.0-0.1) |
| Seg. Neutrophils (THSN/UL) | 0.92 ± 0.27 (0.13-2.57) | 1.16 ± 0.37 (0.13-2.57) | 1.09 ± 0.38 (0.13-2.57) | 0.96 ± 0.20 (0.13-2.57) |
| Lymphocytes (THSN/UL) | 2.64 ± 1.26 (1.43-9.94) | 2.98 ± 1.41 (1.43-9.94) | 3.10 ± 0.56 (1.43-9.94) | 3.70 ± 0.41 (1.43-9.94) |
| Monocytes (THSN/UL) | 0.06 ± 0.04 (0.0-0.39) | 0.15 ± 0.05 (0.0-0.39) | 0.06 ± 0.03 (0.0-0.39) | 0.05 ± 0.02 (0.0-0.39) |
| Eosinophils (THSN/UL) | 0.01 ± 0.01 (0.0-0.24) | 0.01 ± 0.01 (0.0-0.24) | 0.01 ± 0.01 (0.0-0.24) | 0.003 ± 0.01 (0.0-0.24) |
| Basophils (THSN/UL) | 0.0 (0.0-0.0) | 0.004 ± 0.005 (0.0-0.0) | 0.0 (0.0-0.0) | 0.0 (0.0-0.0) |
| Atypical Lympho. (THSN/UL) | 0.0 (0.0-0.0) | 0.0 (0.0-0.0) | 0.0 (0.0-0.0) | 0.0 (0.0-0.0) |
| Metamyelocytes (THSN/UL) | 0.0 (0.0-0.0) | 0.0 (0.0-0.0) | 0.0 (0.0-0.0) | 0.0 (0.0-0.0) |
| Myelocytes (THSN/UL) | 0.0 (0.0-0.0) | 0.0 (0.0-0.0) | 0.0 (0.0-0.0) | 0.0 (0.0-0.0) |
| NRBC (/100WBC) | 0.0 (0.0-0.0) | 0.0 (0.0-0.0) | 0.0 (0.0-0.0) | 0.0 (0.0-0.0) |
| **Other (Result ± SD (ref range)):** | | | | |
| AST (SGOT) (U/L) | 1762.8 ± 1129.8 (72-288) | 899.0 ± 234.6 (72-288) | 779.8 ± 312.2 (72-288) | 843.2 ± 653.4 (72-288) |
| ALT (SGPT) (U/L) | 2171.8 ± 2792.9 (24-140) | 535.2 ± 272.8 (24-140) | 555 ± 350.8 (24-140) | 380.2 ± 385.7 (24-140) |
| BUN (MG/DL) | 27.2 ± 0.8 (9-28) | 24.8 ± 1.9 (9-28) | 24.6 ± 5.5 (9-28) | 28.2 ± 12.8 (9-28) |
| Creatine phosphokinase (U/L) | 28312.8 ± 20534.4 (0-800) | 12194.4 ± 4049.2 (0-800) | 10157 ± 5420.5 (0-800) | 11829 ± 10363.9 (0-800) |
| LDH (U/L) | 6650.2 ± 4788.6 (260-680) | 3661.6 ± 933.6 (260-680) | 3450.8 ± 972.6 (260-680) | 2808.4 ± 1709.1 (260-680) |
| Hemolytic Index (MG/DL HGB) | 706.6 ± 423.4 (0-70) | 477.6 ± 195.7 (0-70) | 589.6 ±198.6 (0-70) | 496.4 ± 321.1 (0-70) |

These results show that there are no abnormalities in blood hematology profiles obtained from mice treated with low, medium and high doses of SVV compared to blood hematology profiles obtained from untreated mice. From this study, it can be concluded that there are no measureable signs of toxicity following systemic administration of SVV, indicating that SVV is tolerated by A/J mice following i.v. injection.

**Example 11**

Efficacy

Athymic female nude mice (nu/nu) aged 6-7 weeks purchased from Harlan Sprague Dawley (Indianapolis, IN) were used in efficacy studies. Mice were injected subcutaneously with $5x10^6$ H446 cells into the right flank using manual restraint. Tumor sizes were measured regularly, and the volumes were calculated using the formula $\pi/6$ x W x $L^2$, where L= length and W = width of the tumor. When the tumors reach approximately 100-150 $mm^3$, mice (n=10) were randomly divided into groups. Mice were injected with escalating doses of SW by tail vein injections at a dose volume of 10 ml/kg. A control group of mice was injected with an equivalent volume of HBSS. Dose escalation proceeds from 1x $10^7$ to $1x10^{13}$ particles per kilogram body weight. Antitumoral efficacy was determined by measuring tumor volumes twice weekly following SVV administration. Complete response was defined as complete disappearance of xenograft; partial response as regression of the tumor volume by equal to or more than 50%; and no response as continuous growth of tumor as in the control group.

Tumors from mice treated with HBSS grew rapidly and the tumor volumes reached more than 2000 $mm^3$ by study day 20 (Figure 51; *see* line with open diamond). In contrast, mice given one systemic injection of SVV at all tested doses (with the exception of the lowest dose) became tumor free by study day 20. In the lowest dose group, 8 mice became tumor free, one mouse had a very large tumor and the other had a small palpable tumor (25 $mm^3$) by study day 31. To evaluate the antitumor activity of SVV on large sized tumors, five mice from HBSS group bearing tumors >2000 $mm^3$ were systemically injected with a single dose of $1x10^{11}$ vp/kg on study day 20. For the duration of the follow-up period (11 days of after SVV injection), a dramatic regression of the tumor volumes were noted (Figure 51).

Figure 52 shows a picture of mice that were "untreated" with SW (*i.e.*, treated with HBSS) or "treated" with SW. As can be seen, the untreated mice had very large tumors and the treated mice showed no visible signs of tumor. Further, for unsacrified mice treated with SVV, no tumor regrowth was observed for the duration of the study, 200 days.

*In vitro* efficacy data for SVV for specific tumor cell lines is shown in Tables 1 and 5. The data shows that SW specifically infects particular tumor cell types and does not infect normal adult cells, a significant advantage over any other known oncolytic virus. SW has been shown to have 1,000 times better cell killing specificity than chemotherapy treatments (cell killing specifity values for SW have been shown to be greater than 10,000, whereas cell killing specificity values for chemotherapy are around 10).

Specific cytotoxic activity of SW was demonstrated in H446 human SCLC cells. Following a two-day incubation with increasing concentrations of SVV, cell viability was determined. The results are shown in Figure 53. Figure 53 shows cell survival following incubation of SVV with either H446 SCLC tumor cells (top graph) or normal human H460 cells (bottom graph). SW specifically killed the tumor cells with an $EC_{50}$ of approximately $10^{-3}$ particles per cell. In contrast, normal human cells were not killed at any concentration of SVV. Further, as summarized in Tables 1-3, SW was also cytotoxic toward a number of other tumor cell lines, including SCLC-multidrug resistant tumor cells. The $EC_{50}$ values for SVV cytotoxicity for the other tumor cell lines ranged from $10^{-3}$ to greater than 20,000 particles per cell. SVV was non-cytotoxic against a variety other non-neural tumors and normal human tissues. Additionally, SVV was not cytotoxic to primary human hepatocytes, as measured by LDH release at up to 1000 particles per cell *(see* Figure 48).

**Example 12**

Biodistribution and Pharmacokinetic Study in Rodents

Pharmacokinetic and biodistribution study of SVV is performed in normal mice and immunocompromised athymic nude mice bearing H446 SCLC tumors. This study evaluates the biodistribution, elimination and persistence of SVV following a single intravenous administration to both normal and immunocompromised tumor-bearing mice. Groups of mice each receive a single i.v. dose of control buffer or one of three doses of SVV ($10^8$, $10^{10}$, or $10^{12}$ vp/kg) and are monitored for clinical signs. Blood samples are obtained from groups of 5 mice at 1, 6, 24 and 48 hours post dose, and at 1, 2, 4, and 12 weeks post dose. Dose levels include a known low efficacious dose and two higher dose levels to determine linearity of virus elimination. Groups of mice are sacrificed at 24 hours, and 2, 4 and 12 weeks post dose. Selected tissues, including liver, heart, lung, spleen, kidney, lymph nodes, bone marrow, brain and spinal cord tissues are aseptically collected and tested for the presence of SVV RNA using a validated RT-PCR assay.

Samples of urine and feces are obtained at sacrifice, at 24 hours, and at 2, 4 and 12 weeks post dose and are examined for the presence of infectious virus. The design of the experiments in this Example are shown in Table 9 below:

Table 9: Biodistribtuion of SVV in CD-1 Mice and Athymic Nude Mice Bearing SCLC Tumors

| Group | Treatment | Dose Level (vp/kg) | Route | # of Mice/Timepoint for Blood Sampling | # of Mice/Timepoint for PCR Tissue Distribution |
|---|---|---|---|---|---|
| Normal CD-1 Mice | | | | | |
| 1 | Saline | 0 | i.v. | 5 | 5 |
| 2 | SVV | $10^8$ | i.v. | 5 | 5 |
| 3 | SVV | $10^{10}$ | i.v. | 5 | 5 |
| 4 | SVV | $10^{12}$ | i.v. | 5 | 5 |
| Athymic Tumor Bearing Mice | | | | | |
| 5 | Saline | 0 | i.v. | 5 | 5 |
| 6 | SVV | $10^8$ | i.v. | 5 | 5 |
| 7 | SVV | $10^{10}$ | i.v. | 5 | 5 |
| 8 | SVV | $10^{12}$ | i.v. | 5 | 5 |

Acute i.v. toxicology studes were also performed in both normal and immunocompromised athymic nude mice bearing H446 SCLC tumors. Preliminary i.v. studies in normal and SCLC tumor bearing mice indicate safety of SVV at doses up to $10^{14}$ vp/kg. No adverse clinical signs were observed and there was no loss of body weight up to 2 weeks following a single i.v. dose of $10^{14}$ vp/kg.

**Example 13**

Viral Transmission Study in Normal Adult and Pregnant Mice

The purpose of this Example is to determine if SVV is transmissible following cohabitation of noninfected normal mice with mice injected with a high concentration of SVV. Because SW does not replicate in normal, non-tumor bearing mice, tumor bearing mice can also be injected with high concentrations of SVV and subsequently exposed to normal, healthy animals to better simulate the clinical scenario. A secondary purpose is to assess the potential transmissibility of SVV from an infected female to an uninfected pregnant DAM, and subsequently to the developing fetus.

Three groups of five naive male and female CD-1 mice are exposed to a single mouse of the same sex infected with either $10^8$, $10^{10}$ or $10^{12}$ vp/kg, and are monitored for the presence of SVV by blood sampling.

Similarly, an SVV exposed female is co-mingled with a number of timed pregnant females, and the ability of the virus to transmit from the infected female to an uninfected pregnant female, and subsequently to the developing fetus is determined.

**Example 14**

Non-Human Primate Studies

The safety, toxicity and toxicokinetics of SVV are also determined in non-human primates. In a dose range-finding phase, individual monkeys receive a single i.v. dose of SW at $10^8$ vp/kg and are closely monitored for clinical signs of infection or toxicity. If this dose is well tolerated, additional animals are treated with a higher i.v. dose until a dose of $10^{12}$ vp/kg is achieved. Subsequently, the main study consists of groups of three male and female monkeys, and each monkey is dosed once weekly for six weeks with either vehicle alone or one of three doses of SVV and monitored for signs of toxicity. An additional two monkeys per sex are dosed with the vehicle alone and with the high dose level of SVV for six weeks, and are allowed an additional four weeks recovery prior to sacrifice.

Blood samples are obtained following dosing during week 1 and week 6. Clinical pathological and hematology blood samples are obtained prior to the initial dose and prior to sacrifice. Additional blood samples are obtained following each dose for assessing the presence of neutralizing antibodies to SW.

Surviving monkeys are euthanized and subjected to a full gross necropsy and a full tissue list is collected from the main study and recovery monkeys. Tissues from the control and high dose groups are evaluated histopathologically.

Urine and fecal samples are collected following dosing on weeks 1 and 6 and are evaluated for presence of infectious SVV. The overall design of this Example is shown in Table 10 below.

Table10: Multiple Dose Toxicology Study of SVV in Primates

| **Dose Range-finding Phase** | | | | | | | |
|---|---|---|---|---|---|---|---|
| Group | Treatment | Dose (vp/kg) | Route | Males | | Females | |
| 1 | SVV | $10^{8}$ | IV | 1 | | 1 | |
| 2 | SVV | $10^{10*}$ | IV | 1 | | 1 | |
| 3 | SVV | $10^{12*}$ | IV | 1 | | 1 | |
| | | | | | | | |
| **Main Phase Recovery** | | | | | | **Main Phase** | |
| Group | Treatment | Dose (vp/kg) | Route | Male | Female | Male | Female |
| 1 | Control | - | IV | 3 | 3 | 2 | 2 |
| 2 | SVV | $10^{8*}$ | IV | 3 | 3 | - | - |
| 3 | SVV | $10^{10*}$ | IV | 3 | 3 | - | - |
| 4 | SVV | $10^{12*}$ | IV | 3 | 3 | 2 | 2 |
| *Doses can vary based on results of Dose Rage-finding phase | | | | | | | |

## Example 15

Construction of a Full-Length and Functional Genomic SVV Plasmid

To date, only about 1.5-2 Kb of the 5' genomic sequence of SVV remains to be sequenced, representing the nucleotide region covering the 5' UTR, 1A (VP4) and part of 1B (VP2). Additional SVV cDNAs are prepared from isolated SW of ATCC deposit number PTA-5343. SVV particles are infected into a permissive cell line, such as PER.C6, and viruses are isolated. Viral RNA is then recovered from the virus particles such that cDNA copies are made therefrom. Individual cDNA clones are sequenced, such that selected cDNA clones are combined into one full-length clone in a plasmid having a T7 promoter upstream of the 5' end of the SVV sequence. The full-length SVV from this plasmid is reverse-transcribed, by utilizing T7 polymerase and an *in vitro* transcription system, in order to generate full-length RNA *(see* Figure 55). The full-length RNA is then transfected into permissive cell lines to test the infectivity of the full-length clone *(see* Figure 55).

The methodology is as follows. *RNA Isolation:* SVV genomic RNA is extracted using guanidium thiocyanate and a phenol extraction method using Trizol (Invitrogen). Briefly, 250 μl of the purified SVV is mixed with 3 volumes Trizol and 240 μl of chloroform. The aqueous phase containing RNA is precipitated with 600 μl isopropanol. The RNA pellet is washed twice with 70% ethanol, dried and dissolved in DEPC-treated water. The quantity of RNA extracted is estimated by optical density measurements at 260 nm. An aliquot of RNA is resolved through a 1.25% denaturing agarose gel (Cambrex Bio Sciences Rockland Inc., Rockland, ME USA) and the band visualized by ethidium bromide staining and photographed.

*cDNA synthesis:* cDNA of the SVV genome is synthesized by RT-PCR. Synthesis of cDNA is performed under standard conditions using 1 μg of RNA, AMV reverse transcriptase, and random 14-mer oligonucleotide or oligo-dT. Fragments of the cDNA are amplified, cloned into the plasmid and the clones are sequenced. It is possible that more extensive measures are necessary to sequence the extreme 5' end of the genome.

*Closing of full length genome:* Once the sequence is known routine molecular biology enables the construction of a full-length clone of SVV downstream of a T7 polymerase promoter (for example, *see* Figure 54).

*Recovery of SVV:* The plasmid with the full-length genome of SVV is reverse-transcribed following standard protocols. The viral RNA (100 ng) is used to transfect H446 cells, a cell line known to be permissive for the native SVV, but the most efficient cell line for viral RNA transfection can be empirically determined among a variety of cell lines.

**Example 16**

Construction of an RGD-Displaying SW Library

To find the optimal insertion position for the construction of SW capsid mutants generated with random with oligonucleotides encoding random peptide sequences, a simple model system (RGD) is employed. RGD (arginine, glycine, aspartic acid) is a short peptide ligand that binds to integrins. A successful RGD-SVV derivative should contain the following characteristics: (1) the genetic insertion should not alter any of SVV's unique and desirable properties; and (2) a successful RGD derivative virus should have tropism toward aVb5 integrin containing cells.

A SW plasmid containing just the contiguous capsid region will be singly cleaved at random positions and a short model peptide sequence, referred to as RGD, will be inserted at each position. The virus SVV-RGD library will be constructed from this plasmid library utilizing the general technology described in Figures 56 and 57.

Random insertion of the cRGD oligonucleotide into the capsid region is conducted. In brief, a plasmid is constructed that just encodes the contiguous 2.1 Kb capsid region of SVV (*see* Figure 56, "pSWcapsid"). A single random cleavage is made in pSVVcapsid by partially digesting the plasmid utilizing either CviJI or an endonuclease V method as described below *(see* Figure 57). After isolating the single cleaved plasmid the RGD oligonucleotide will be inserted to create a pSVVcapsid-RGD library.

The restriction enzyme CviJI has several advantages over other random cleavage methods such as sonication or shearing. First, as CviJI is a blunt ended cutter no repair is necessary. Second, CviJI has been demonstrated to cleave at random locations such that no hot spots will occur. The procedure is also simple and rapid. Briefly, the concentration of CviJI and/or time of digestion are increasingly lowered until the majority of cleaved DNA is a linearized plasmid, *i.e.* a single cleavage. This can be observed by standard agarose gel electrophoresis as depicted in Figure 57. The band is then isolated, purified and ligated with the RGD oligo.

Another method that may be utilized to randomally cleave DNA is the endonuclease V method (Kiyazaki, K., Nucleic Acids Res., 2002, 30(24): e139). Endonuclease V nicks uracil-containing DNA at the second or third phosphodiester bond 3' to uracil sites. This method is also expected to randomly cleave DNA, the frequency is simply determined by adjusting the concentration of dUTP in the polymerase chain reaction. Although the cleavage sites are always two or three bases downstream of a thymidine (substituted by uracil) site, this method is expected to produce much fewer hot and cold spots than other methodologies.

The randomly linearized plasmids are ligated with the cRGD oligonucleotides. The resultant pSVV capsid library is then amplified, such that a population of polynucleotides encoding the capsid region with randomly inserted cRGD regions can be purified *(see* Figures 57 and 58). The population of capsid polynucleotides is then subcloned into a vector containing the full-length SVV sequence minus the capsid region, such that a library of full-length SVV sequences are generated (where the library manifests sequence diversity in the capsid region as the cRGD sequence is randomly inserted). This library is then reverse transcribed into RNA, and the RNA is transfected into a permissive cell line to generate a population of SW particles having different capsids *(see* Figure 59). Once this RGD-SVV population of virus particles is recovered ("RGD-SVV library"), a number of viruses *(i.e.,* 10 or more) will be randomly picked for sequencing to confirm the insertion of the RGD sequence and diversity of insertion site.

*In vitro selection of the RGD-displaying SW library.* The SVV-RGD library is screened to determine which insertion site enabled an expanded tropism of SVV. The RGD-SVV library is allowed to infect $\alpha_v\beta_5$ integrin-expressing NSCLC lines (non-small cell lung cancer cell lines, i.e., A549 expressing $\alpha_v\beta_5$). Only those SW derivatives that contain a functional and properly displayed RGD motif can infect these cells and replicate.

*In vitro* screening is carried out by a high throughput automation system (TECAN) that is capable of liquid handling, concurrent incubation of 20 cell lines and measurement in 384-well plates *(see* Figure 62 and Figure 63). The cells are harvested 30 hr after infection when complete CPE is noticed and then cells are collected by centrifugation at 1500 rpm for 10 minutes at 4°C. The cell pellets are then resuspended in the cell culture supernatant and subjected to three cycles of freeze and thaw. The resulting suspension is clarified by centrifugation at 1500 rpm for 10 minutes at 4°C. Virus is purified by two rounds of CsCl gradients: a one-step gradient (density of CsCl 1.24 g/ml and 1.4 g/ml) followed by one continuous gradient centrifugation (density of CsCl 1.33 g/ml). The purified virus concentration is determined spectrophotometrically, assuming $1A_{260} = 9.5 \times 10^{12}$ particles (Scraba, D.G. and Palmenberg, A.C., 1999). The process may be repeated multiple times until a sufficient amount of virus is recovered from $\alpha_V\beta_5$ cells.

*Analysis of recovered RGD-SW derivatives.* A small pool of individual RGD-displaying SVV derivatives (about 10-50 different derivataives) are analyzed. The viral mixture is diluted and single viral particles are expanded for analysis. Each derivative is tested to determine whether they have gained the ability to infect $\alpha_v\beta_5$-expressing cells efficiently and specifically. The capsid region of each derivative with this property is then be sequenced to determine the site of RGD insertion. The recovered cRGD-displaying SVV derivatives should possess the following properties: (1) the original properties of the virus are still intact; and (2) the derivatives have gained the ability to infect cells that express high levels of integrins that bind to RGD. This approach aims to identify one or more sites that enable an expanded tropism with RGD insertion, such that random oligonucleotides can be inserted into these sites to generate SVV derivatives with altered tropism.

The sequenced cRGD-SVV derivatives are numbered and ranked by their binding abilities to integrin. To test the binding activity, recombinant $\beta_2$ integrin is immobilized on a 96-well microtiter plate in PBS, washed twice with PBS, blocked with 3% BSA in PBS, and then incubated with a unique RGD-displaying virus. The native virus without peptide insertions is used as a negative control. After 1-5 hr of incubation, the wells are washed at least three times with PBS. Then, the viruses that are bound to the plate will be detected by anti-SVV antibodies. RGD peptide or antibodies against integrin should be able to compete with the binding of the RGD-SVV derivatives to the integrin-bound plate.

The cRGD-SVV derivatives (20) that have the strongest binding to integrin are analyzed to determine the 'successful' location(s) of cRGD oligonucleotide insertion. The insertion sites provide insights into the tropism of SVV. Based on the analysis of the insertion sites and other known structures, an ideal location to place a random peptide library can be determined (this method is an alternative method for generating SVV derivatives, where oligonucleotides (known sequence or random sequence) are inserted into random locations in the capsid). SVV derivatives generated with random sequence oligonucleotides are constructed in essentially the same manner as described above for the RGD-SVV library, except for two additional and novel methodologies. To avoid unwanted stop codons and deleterious amino acid insertions (e.g. cysteines or prolines) within a desired coding region, TRIM (trinucleotide-mutagenesis) technology developed by Morphosys (Munich, Germany) can be used to generate random oligonucleotides for capsid insertion. TRIM utilizes tri-nucleotides which only code for amino acids at the desired position (Virnekas, B. et al., Nucleic Acids Res, 1994, 22(25): 5600-5607). The random-peptide displaying SVV with a diversity of $10^8$ is believed to be sufficient and expected to yield peptides that specifically direct the virus to targeted tumor tissues. Random-peptide displaying SW is tested *in vitro* as described above, or *in vivo* using tumor-bearing mice.

## Claims

1. An isolated nucleic acid comprising a nucleic acid sequence having at least 95% sequence identity to SEQ ID NOs: 1, 3, 5, 7, 9, 11, 13, 15, 17, 19, 21, or a contiguous portion of any one of these sequences that is at least 50 nucleotides in length.

2. The isolated nucleic acid of claim 1, wherein the nucleic acid is RNA or DNA.

3. An isolated polypeptide comprising an amino acid sequence having at least 95% sequence identity to SEQ ID NOs 2, 4, 6, 8, 10, 12, 14, 16, 18, 20, 22, or an amino acid sequence encoded by the nucleic acid of claim 1.

4. An isolated Seneca Valley virus, comprising identifying characteristics of ATCC Patent Deposit number PTA-5343, wherein the identifying characteristics are: (i) a single stranded RNA genome (positive (+) sense strand) of about 7.5 kilobases (kb); (ii) a diameter of about 27 nanometers; (iii) a capsid comprising at least 3 proteins comprising a 31 kDa capsid protein comprising an amino acid sequence that is at least 80%, 85%, 90%, 95%, or 99% identical to SEQ ID NO: 8; a 36 kDa capsid protein comprising an amino acid sequence that is at least 80%, 85%, 90%, 95%, or 99% identical to SEQ ID NO: 4; and a 27 kDa capsid protein comprising an amino acid sequence that is at least 80%, 85%, 90%, 95%, or 99% identical to SEQ ID NO: 6; and (iv) a buoyant density of 1.34 g/mL on a cesium chloride gradient; and (v) replication competence in tumor cells.

5. An isolated Seneca Valley virus, in accordance with claim 4, having a genome comprising a sequence that is at least 95%, 90%, 85%, or 80% identical to SEQ ID NO:1.

6. The virus of claim 4, comprising the following characteristics: replication competence in tumor cells, tumor-cell tropism, and lack of cytolysis in normal cells.

7. The virus of claim 6, wherein said virus is replication competent in tumor cell types having neuroendocrine properties.

8. A pharmaceutical composition comprising an effective amount of the virus of any one of claims 4 to 7 and a pharmaceutically acceptable carrier.

9. An isolated antibody which specifically binds to the polypeptide of claim 3 having at least 95% sequence identity to SEQ ID NO 2, to the isolated virus of claim 4 or to the isolated virus of claim 5 having a genome comprising a sequence that is at least 95% identical to SEQ ID NO: 1.

**10.** A virus for use to treat cancer, wherein the virus has a genomic sequence that comprises a sequence that is at least 95% identical to SEQ ID NO: 1, 3, 5, 7, 9, 11, 13, 15, 17, 19 or 21.

**11.** Use of a virus for the manufacture of a medicament for the treatment of cancer, wherein the virus has a genomic sequence that comprises a sequence that is at least 95% identical to SEQ ID NO: 1, 3, 5, 7, 9, 11, 13, 15, 17, 19 or 21.

**12.** The virus of any one of claims 4 to 7, for use in a treatment comprising killing an abnormally proliferative cell.

**13.** Use of the virus of any one of claims 4 to 7, for the manufacture of a medicament for a treatment comprising killing an abnormally proliferative cell.

**14.** A method for making an oncolytic virus, the method comprising:

(a) comparing a Seneca Valley virus genomic sequence with a test virus genomic sequence, wherein the Seneca Valley virus genomic sequence comprises a sequence that is at least 95% identical to SEQ ID NO:1;
(b) identifying at least a first amino acid difference between a polypeptide encoded by the Seneca Valley virus genomic sequence and a polypeptide encoded by the test virus genomic sequence;
(c) mutating the test virus genomic sequence such that the polypeptide encoded by the test virus genomic sequence has at least one less amino acid difference to the polypeptide encoded by the Seneca Valley virus genomic sequence;
(d) transfecting the mutated test virus genomic sequence into a tumor cell; and
(e) determining whether the tumor cell is lytically infected by the mutated test virus genomic sequence.

**15.** The method of claim 14, wherein the test virus is a picornavirus.

**16.** The method of claim 14, wherein the test virus is a cardiovirus.

**17.** The method of claim 14, wherein the amino acid differences are between a Seneca Valley virus capsid protein and a test virus capsid protein.

**18.** The method of claim 14, wherein mutating the test virus genomic sequence comprises mutating a cDNA having the test virus genomic sequence.

**19.** The method of claim 14, wherein transfecting the mutated test virus genomic sequence comprises transfecting RNA, wherein the RNA is generated from the cDNA having the mutated test virus genomic sequence.

**20.** A method for making a mutant virus having an altered cell-type tropism, the method comprising:

(a) creating a library of viral mutants comprising a plurality of nucleic acid sequences, wherein the library is created from a parental sequence comprising a sequence that is at least 95% identical to SEQ ID NO:1;
(b) transfecting the library of viral mutants into a permissive cell, such that a plurality mutant viruses are produced;
(c) isolating the plurality of mutant viruses;
(d) incubating a non-permissive cell with the isolated plurality of mutant viruses; and
(e) recovering a mutant virus that was produced in the non-permissive cell, thereby making a mutant virus having an altered tropism.

**21.** The method of claim 20, further comprising:

(f) incubating the recovered mutant virus in the non-permissive cell; and
(g) recovering a mutant virus that that was produced in the non-permissive cell.

**22.** The method of claim 21, further comprising iteratively repeating steps (f) and (g).

**23.** The method of claim 21, wherein the incubating is conducted in a multi-well high-throughput platform wherein the platform comprises a different non-permissive cell-type in each well.

**24.** The method of claim 23, further comprising screening the platform to identify which wells contain a mutant virus that kills the cells.

**25.** The method of claim 24, wherein the screening is conducted by analyzing light absorbance in each well.

**26.** The method of claim 20, wherein the non-permissive cell is a tumor cell.

**27.** The method of claim 20, wherein creating the library of viral mutants comprises:

(i) providing a polynucleotide having a sequence identical to a portion of a genomic sequence of a virus;
(ii) mutating the polynucleotide in order to generate a plurality of different mutant polynucleotide sequences; and
(iii) ligating the plurality of mutated polynucleotides into a vector having the genomic sequence of the virus except for the portion of the genomic sequence of the virus that the polynucleotide in step (i) contains, thereby creating the library of viral mutants.

**28.** The method of claim 27, wherein the mutating of step (ii) is conducted by random insertion of nucleotides into the polynucleotide.

**29.** The method of claim 27, wherein the mutating of step (ii) is conducted in a capsid encoding region of the polynucleotide.

**30.** The method of claim 28, wherein the random insertion of nucleotides is conducted by trinucleotide-mutagenesis (TRIM).

**31.** The method of claim 28, wherein at least a portion of the nucleotides inserted into the polynucleotide encodes an epitope tag.

**32.** An isolated Seneca Valley virus having ATCC Patent Deposit number PTA-5343.

**Patentansprüche**

**1.** Isolierte Nukleinsäure, eine Nukleinsäuresequenz aufweisend, die mindestens 95 % Sequenzidentität zu SEQ ID NOs: 1, 3, 5, 7, 9, 11, 13, 15, 17, 19, 21 oder einem zusammenhängenden Abschnitt einer dieser Sequenzen, der mindestens 50 Nukleotide lang ist, aufweist.

**2.** Isolierte Nukleinsäure nach Anspruch 1, wobei die Nukleinsäure RNA oder DNA ist.

**3.** Isoliertes Polypeptid, eine Aminosäuresequenz mit mindestens 95 % Sequenzidentität zu SEQ ID NOs 2, 4, 6, 8, 10, 12, 14, 16, 18, 20, 22 oder eine Aminosäuresequenz aufweisend, die von der Nukleinsäure nach Anspruch 1 kodiert wird.

**4.** Isolierter Seneca-Valley-Virus, aufweisend:

kennzeichnende Merkmale von ATCC Patenthinterlegungsnummer PTA-5343, wobei die kennzeichnenden Merkmale sind: (i) ein einzelsträngiges RNA-Genom (positiv- (+)-strängig) von etwa 7,5 Kilobasen (kb); (ii) ein Durchmesser von etwa 27 Nanometern; (iii) ein Kapsid, das mindestens 3 Proteine aufweist, nämlich ein 31 kDa-Kapsidprotein, das eine Aminosäuresequenz umfasst, die zu mindestens 80 %, 85 %, 90 %, 95 % oder 99 % identisch ist mit SEQ ID NO: 8; ein 36 kDa-Kapsidprotein, das eine Aminosäuresequenz umfasst, die zu mindestens 80 %, 85 %, 90 %, 95 % oder 99 % identisch ist zu SEQ ID NO: 4; und ein 27 kDa-Kapsidprotein, das eine Aminosäuresequenz aufweist, die zu mindestens 80 %, 85%, 90 %, 95 % oder 99 % identisch ist zu SEQ ID NO: 6; und (iv) eine Schwimmdichte von 1,34 g/ml auf einem Cäsiumchloridgadienten; und (v) Replikationskompetenz in Turmorzellen.

**5.** Isolierter Seneca-Valley-Virus nach Anspruch 4 mit einem Genom, das eine Sequenz aufweist, die zu mindestens 95 %, 90 % oder 80 % identisch ist zu SEQ ID NO:1.

**6.** Virus nach Anspruch 4, die folgenden Merkmale aufweisend: Replikationskompetenz in Turmorzellen, Tumorzellentropismus und Fehlen von Cytolyse in normalen Zellen.

**7.** Virus nach Anspruch 6, wobei das Virus in Tumorzellen, die neuroendokrine Eigenschaften haben, replikationskompetent ist.

8. Pharmazeutische Zusammensetzung, eine wirksame Menge des Virus nach einem der Ansprüche 4 bis 7 und einen pharmazeutisch annehmbaren Träger aufweisend.

9. Isolierter Antikörper, der sich spezifisch an das Polypeptid nach Anspruch 3, das mindestens 95 % Sequenzidentität zu SEQ ID NO 2 aufweist, an das isolierte Virus nach Anspruch 4 oder an das isolierte Virus nach Anspruch 5 bindet, das ein Genom mit einer Sequenz aufweist, die zu mindestens 95 % identisch ist mit SEQ ID NO: 1.

10. Virus zur Verwendung bei der Behandlung von Krebs, wobei das Virus eine Genomsequenz mit einer Sequenz aufweist, die zu mindestens 95 % identisch ist zu SEQ ID NO: 1, 3, 5, 7, 9, 11, 13, 15, 17, 19 oder 21.

11. Verwendung eines Virus zur Herstellung eines Medikaments zur Behandlung von Krebs, wobei das Virus eine Genomsequenz aufweist, die zu mindestens 95 % identisch ist zu SEQ ID NO: 1, 3, 5, 7, 9, 11, 13, 15, 17, 19 oder 21.

12. Virus nach einem der Ansprüche 4 bis 7 zur Verwendung bei einer Behandlung, die das Abtöten einer anomal proliferativen Zelle umfasst.

13. Verwendung des Virus nach einem der Ansprüche 4 bis 7 zur Herstellung eines Medikaments für eine Behandlung, die das Abtöten einer anomal proliferativen Zelle umfasst.

14. Verfahren zur Herstellung eines onkolytischen Virus, wobei das Verfahren umfasst:

(a) Vergleichen einer Genomsequenz des Seneca-Valley-Virus mit einer Genomsequenz eines zu testenden Virus, wobei die Genomsequenz des Seneca-Valley-Virus eine Sequenz umfasst, die zu mindestens 95 % identisch ist mit SEQ ID NO:1;
(b) Identifizieren mindestens eines Aminosäureunterschieds zwischen einem Polypeptid, das von der Genomsequenz des Seneca-Valley-Virus kodiert wird, und einem Polypeptid, das von der Genomsequenz des zu testenden Virus kodiert wird.
(c) Hervorbringen einer Mutation der Genomsequenz des zu testenden Virus auf solche Weise, dass das Polypeptid, das von der Genomsequenz des zu testenden Virus kodiert wird, mindestens einen Aminosäureunterschied weniger zu dem Polypeptid aufweist, das von der Genomsequenz des Seneca-Valley-Virus kodiert wird;
(d) Transfizieren der mutierten Genomsequenz des zu testenden Virus in eine Tumorzelle; und
(e) Bestimmen, ob die Tumorzelle lytisch durch die mutierte Genomsequenz des zu testenden Virus infiziert worden ist.

15. Verfahren nach Anspruch 14, wobei das zu testende Viurs ein Picornavirus ist.

16. Verfahren nach Anspruch 14, wobei das zu testende Viurs ein Cardiovirus ist.

17. Verfahren nach Anspruch 14, wobei die Aminosäureunterschiede zwischen einem Kapsidprotein des Seneca-Valley-Virus und einem Kapsidprotein eines gesteten Virus bestehen.

18. Verfahren nach Anspruch 14, wobei das Hervorbringen einer Mutation der Genomsequenz des zu testenden Virus das Hervorbringen einer Mutation einer cDNA umfasst, welche die Genomsequenz des zu testenden Virus aufweist.

19. Verfahren nach Anspruch 14, wobei das Transfizieren der mutierten Genomsequenz des zu testenden Virus das Transfizieren von RNA umfasst, wobei die RNA aus der cDNA erzeugt wird, welche die mutierte Genomsequenz des zu testenden Virus aufweist.

20. Verfahren zur Herstellung eines mutierten Virus mit einem veränderten Zelltyptropismus, wobei das Verfahren umfasst:

(a) Erzeugen einer Bibliothek aus viralen Mutanten, die eine Mehrzahl von Nukleinsäuresequenzen aufweisen, wobei die Bibliothek aus einer Muttersequenz erzeugt wird, die eine Sequenz umfasst, die zu mindestens 95 % identisch ist zu SEQ ID NO:1;
(b) Transfizieren der Bibliothek von viralen Mutanten in eine permissive Zelle, so dass mehrere mutierte Viren erzeugt werden;
(c) Isolieren der mehreren mutierten Viren;
(d) Inkubieren einer nicht-permissiven Zelle mit den mehreren mutierten Viren; und
(e) Gewinnen eines mutierten Virus, das in der nicht-permissiven Zelle erzeugt worden ist, wodurch ein mutiertes Virus mit einem veränderten Tropismus hergestellt wird.

**21.** Verfahren nach Anspruch 20, ferner umfassend:

(f) Inkubieren des gewonnenen mutierten Virus in der nicht-permissiven Zelle; und
(g) Gewinnen eines mutierten Virus, das in der nicht-permissiven Zelle erzeugt worden ist.

**22.** Verfahren nach Anspruch 21, ferner das iterative Wiederholen der Schritte (f) und (g) umfassend.

**23.** Verfahren nach Anspruch 21, wobei das Inkubieren auf einer Plattform mit mehreren Vertiefungen und mit hohem Durchsatz durchgeführt wird, wobei die Plattform in jeder Vertiefung einen anderen nicht-permissiven Zelltyp aufweist.

**24.** Verfahren nach Anspruch 23, ferner das Durchmustern der Plattform umfassend, um zu bestimmen, welche Vertiefungen ein mutiertes Virus enthalten, welches die Zellen abtötet.

**25.** Verfahren nach Anspruch 24, wobei die Durchmusterung durch die Analyse der Lichtextinktion in jeder Vertiefung durchgeführt wird.

**26.** Verfahren nach Anspruch 20, wobei die nicht-permissive Zelle eine Tumorzelle ist.

**27.** Verfahren nach Anspruch 20, wobei die Erzeugung der Bibliothek von viralen Mutanten umfasst:

(i) Bereitstellen eines Polynukleotids mit einer Sequenz, die zu einem Abschnitt einer Genomsequenz eines Virus identisch ist;
(ii) Hervorbringen einer Mutation des Polynukleotids, um mehrere verschiedene mutierte Polynukleotidsequenzen zu erzeugen; und
(iii) Ligieren der mehreren mutierten Polynukleotidsequenzen in einen Vektor, der die Genomsequenz des Virus aufweist, abgesehen von dem Abschnitt der Genomsequenz des Virus, den das Polynukleotid in Schritt (i) enthält, wodurch die Bibliothek von viralen Mutanten gebildet wird.

**28.** Verfahren nach Anspruch 27, wobei die Mutation von Schritt (ii) durch zufällige Inserierung von Nukleotiden in das Polynukleotid hervorgebracht wird.

**29.** Verfahren nach Anspruch 27, wobei die Mutation von Schritt (ii) in einer Kapsidkodierungsregion des Polynukleotids hervorgebracht wird.

**30.** Verfahren nach Anspruch 28, wobei die zufällige Inserierung von Nukleotiden durch Trinukleotidmutagenese (TRIM) durchgeführt wird.

**31.** Verfahren nach Anspruch 28, wobei zumindest ein Abschnitt der Nukleotide, die in das Polynukleotid inseriert werden, ein Epitop-Tag kodieren.

**32.** Isolierter Seneca-Valley-Virus mit der ATCC-Patenthinterlegungsnummer PTA-5343.

## Revendications

**1.** Un acide nucléique isolé contenant une séquence d'acides nucléiques possédant une identité de séquence d'au moins 95% avec SEQ ID n°1, 3, 5, 7, 9, 11, 13, 15, 17, 19, 21, ou une partie contiguë de l'une quelconque de ces séquences qui possède une longueur d'au moins 50 nucléotides.

**2.** L'acide nucléique isolé selon la Revendication 1, où l'acide nucléique est ARN ou ADN.

**3.** Un polypeptide isolé contenant une séquence d'acides aminés possédant une identité de séquence d'au moins 95% avec SEQ ID n°2, 4, 6, 8, 10, 12, 14, 16, 18, 20, 22, ou une séquence d'acides aminés codée par l'acide nucléique selon la Revendication 1.

**4.** Un virus de Seneca Valley isolé, comprenant des caractéristiques d'identification du brevet ATCC Numéro de dépôt PTA-5343, où les caractéristiques d'identification sont : (i) un génome d'ARN à simple brin (brin dans le sens positif (+)) d'environ 7,5 kilobases (kb), (ii) un diamètre d'environ 27 nanomètres, (iii) une capside contenant au moins trois protéines contenant une protéine de capside de 31 kDa contenant une séquence d'acides aminés qui est au moins à 80%, 85%, 90%, 95% ou 99% identique à SEQ ID n°8, une protéine de capside de 36 kDa contenant une séquence d'acides aminés qui est au moins à 80%, 85%, 90%, 95% ou 99% identique à SEQ ID n°4, et une protéine de capside de 27 kDa contenant une séquence d'acides aminés qui est au moins à 80%, 85%, 90%, 95% ou 99% identique à SEQ ID n° 6, et (iv) une densité de flottaison de 1,34 g/mL sur un gradient de chlorure de césium, et (v) une compétence de réplication dans des cellules tumorales.

**5.** Un virus de Seneca Valley isolé selon la Revendication 4, possédant un génome contenant une séquence qui est au moins à 95%, 90%, 85% ou 80% identique à SEQ ID n°1.

**6.** Le virus selon la Revendication 4, comprenant les caractéristiques suivantes : compétence de réplication dans des cellules tumorales, tropisme de cellule tumorale et absence de cytolyse dans des cellules normales.

**7.** Le virus selon la Revendication 6, où ledit virus est compétent pour la réplication dans des types de cellule tumorale possédant des propriétés neuroendocriniennes.

**8.** Une composition pharmaceutique contenant une quantité efficace du virus selon l'une quelconque des Revendications 4 à 7 et un vecteur pharmaceutiquement acceptable.

**9.** Un anticorps isolé qui se lie spécifiquement au polypeptide selon la Revendication 3 possédant une identité de séquence d'au moins 95% avec SEQ ID n°2, au virus isolé selon la Revendication 4 ou au virus isolé selon la Revendication 5 possédant une génome contenant une séquence qui est au moins à 95% identique à SEQ ID n°1.

**10.** Un virus pour une utilisation dans le traitement d'un cancer, où le virus possède une séquence génomique qui contient une séquence qui est au moins à 95% identique à SEQ ID n°1, 3, 5, 7, 9, 11, 13, 15, 17, 19 ou 21.

**11.** L'utilisation d'un virus pour la fabrication d'un médicament pour le traitement d'un cancer, où le virus possède une séquence génomique qui contient une séquence qui est au moins à 95% identique à SEQ ID n°1, 3, 5, 7, 9, 11, 13, 15, 17, 19 ou 21.

**12.** Le virus selon l'une quelconque des Revendications 4 à 7, pour une utilisation dans un traitement comprenant l'élimination d'une cellule anormalement proliférative.

**13.** L'utilisation du virus selon l'une quelconque des Revendications 4 à 7, pour la fabrication d'un médicament pour un traitement comprenant l'élimination d'une cellule anormalement proliférative.

**14.** Un procédé de fabrication d'un virus oncolytique, le procédé comprenant :

(a) la comparaison d'une séquence génomique de virus de Seneca Valley à une séquence génomique de virus d'essai, où la séquence génomique de virus de Seneca Valley contient une séquence qui est au moins à 95% identique à SEQ ID n°1.
(b) l'identification d'au moins une première différence d'acides aminés entre un polypeptide codé par la séquence génomique de virus de Seneca Valley et un polypeptide codé par la séquence génomique de virus d'essai,
(c) la mutation de la séquence génomique de virus d'essai de sorte que le polypeptide codé par la séquence génomique de virus d'essai possède au moins une différence d'acides aminés de moins que le polypeptide codé par la séquence génomique de virus de Seneca Valley,
(d) la transfection de la séquence génomique de virus d'essai mutée en une cellule tumorale, et
(e) la détermination si la cellule tumorale est infectée de manière lytique par la séquence génomique de virus d'essai mutée.

**15.** Le procédé selon la Revendication 14, où le virus d'essai est un picornavirus.

16. Le procédé selon la Revendication 14, où le virus d'essai est un cardiovirus.

17. Le procédé selon la Revendication 14, où les différences d'acides aminés sont entre une protéine de capside de virus de Seneca Valley et une protéine de capside de virus d'essai.

18. Le procédé selon la Revendication 14, où la mutation de la séquence génomique de virus d'essai comprend la mutation d'un ADNc possédant la séquence génomique de virus d'essai.

19. Le procédé selon la Revendication 14, où la transfection de la séquence génomique de virus d'essai mutée comprend la transfection d'un ARN, où l'ARN est généré à partir de l'ADNc possédant la séquence génomique de virus d'essai mutée.

20. Un procédé de fabrication d'un virus mutant possédant un tropisme de type cellulaire altéré, le procédé comprenant :

(a) la création d'une bibliothèque de mutants viraux comprenant une pluralité de séquences d'acides nucléiques, où la bibliothèque est créée à partir d'une séquence parentale contenant une séquence qui est au moins à 95% identique à SEQ ID n°1,
(b) la transfection de la bibliothèque de mutants viraux en une cellule permissive, de sorte qu'une pluralité de virus mutants soit produite,
(c) l'isolation de la pluralité de virus mutants,
(d) l'incubation d'une cellule non permissive avec la pluralité de virus mutants isolés, et
(e) la récupération d'un virus mutant qui a été produit dans la cellule non permissive, réalisant ainsi un virus mutant possédant un tropisme altéré.

21. Le procédé selon la Revendication 20, comprenant en outre :

(f) l'incubation du virus mutant récupéré dans la cellule non permissive, et
(g) la récupération d'un virus mutant qui a été produit dans la cellule non permissive.

22. Le procédé selon la Revendication 21, comprenant en outre la répétition itérative des opérations (f) et (g).

23. Le procédé selon la Revendication 21, où l'incubation est conduite dans une plate-forme haute capacité multipuits où la plate-forme comprend un type différent de cellule non permissive dans chaque puits.

24. Le procédé selon la Revendication 23, comprenant en outre l'analyse de la plate-forme de façon à identifier quels puits contiennent un virus mutant qui élimine les cellules.

25. Le procédé selon la Revendication 24, où l'analyse est conduite par une analyse de l'absorbance de la lumière dans chaque puits.

26. Le procédé selon la Revendication 20, où la cellule non permissive est une cellule tumorale.

27. Le procédé selon la Revendication 20, où la création de la bibliothèque de mutants viraux comprend :

(i) la fourniture d'un polynucléotide possédant une séquence identique à une partie d'une séquence génomique d'un virus,
(ii) la mutation du polynucléotide de façon à générer une pluralité de séquences de polynucléotides mutants différentes, et
(iii) la ligature de la pluralité de polynucléotides mutés en un vecteur possédant la séquence génomique du virus sauf pour la partie de la séquence génomique du virus que contient le polynucléotide à l'opération (i), créant ainsi la bibliothèque de mutants viraux.

28. Le procédé selon la Revendication 27, où la mutation de l'opération (ii) est conduite par une insertion aléatoire de nucléotides dans le polynucléotide.

29. Le procédé selon la Revendication 27, où la mutation de l'opération (ii) est conduite dans une zone de codage de capside du polynucléotide.

30. Le procédé selon la Revendication 28, où l'insertion aléatoire de nucléotides est conduite par une mutagénèse de trinucléotides (TRIM).

31. Le procédé selon la Revendication 28, où au moins une partie des nucléotides insérés dans le polynucléotide code une étiquette épitopique.

32. Un virus de Seneca Valley isolé possédant le Numéro de dépôt PTA-5343 de brevet ATCC.

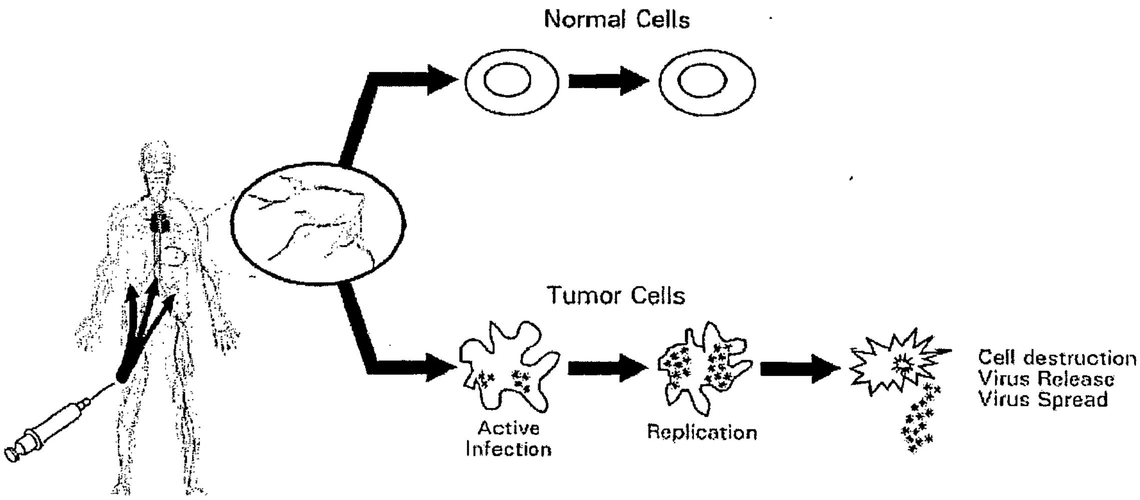
Normal Cells
Tumor Cells
Active Infection
Replication
Cell destruction
Virus Release
Virus Spread

FIG. 1

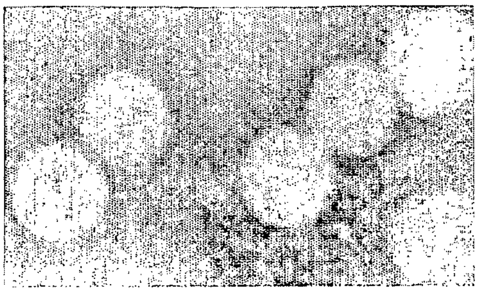

FIG. 2

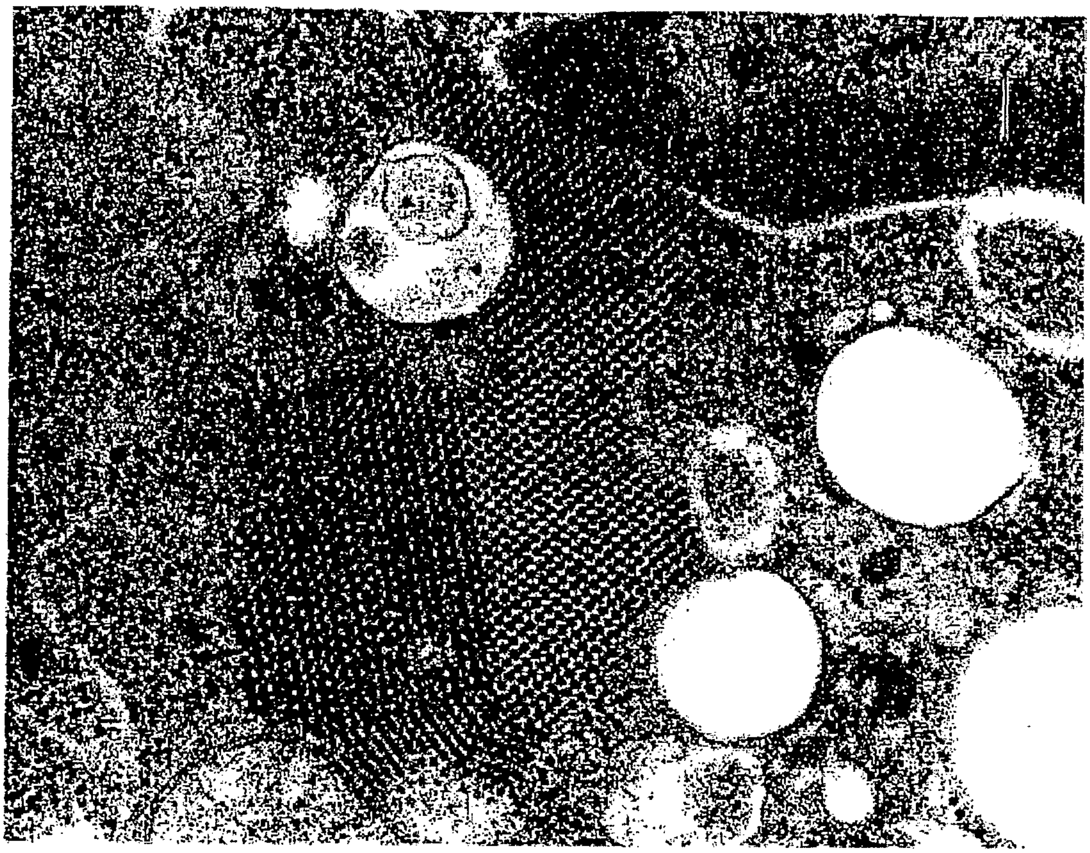

FIG. 3

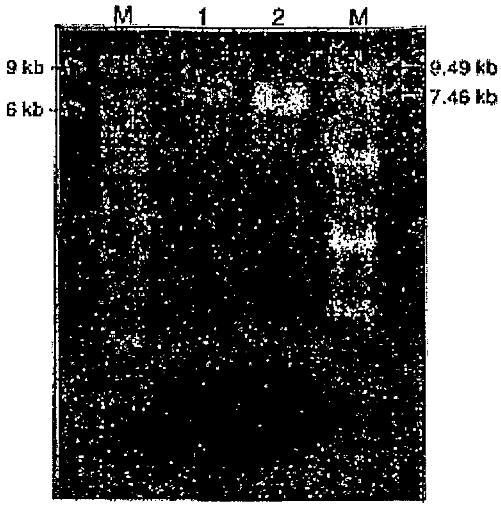


FIG. 4A

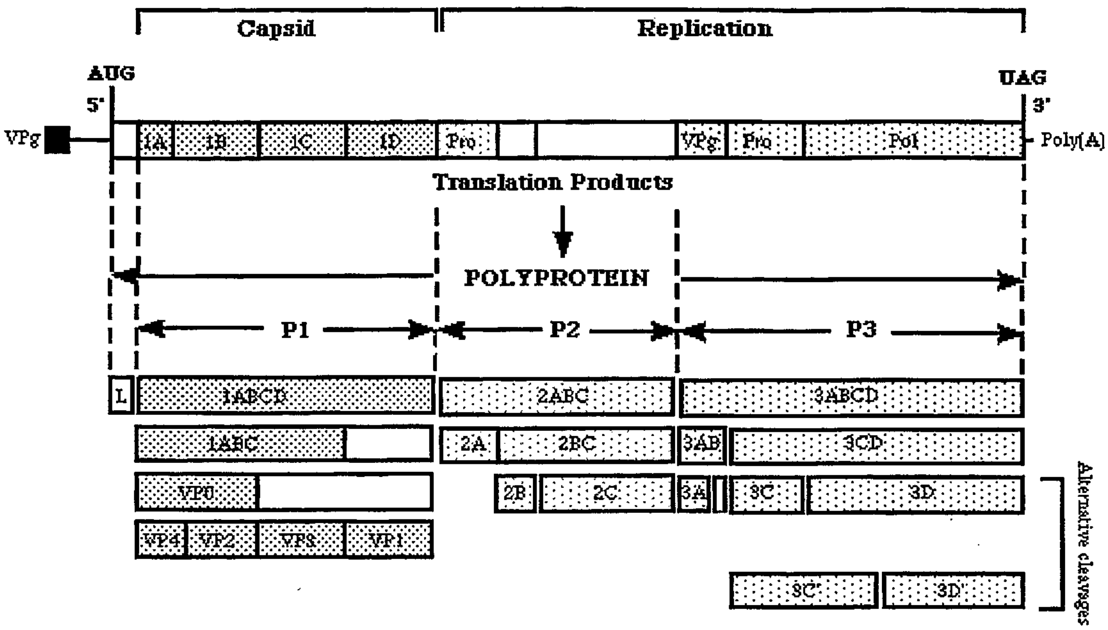
Capsid
Replication
AUG
5'
UAG
3'
VPg
1A
1B
1C
1D
Pro
VPg
Pro
Pol
Poly(A)
Translation Products
POLYPROTEIN
P1
P2
P3
L
1ABCD
2ABC
3ABCD
1ABC
2A
2BC
3AB
3CD
VP0
2B
2C
3A
3C
3D
VP4
VP2
VP3
VP1
3C'
3D'
Alternative cleavages

FIG. 4B

```
        10        20        30        40        50        60        70        80        90
....|....|....|....|....|....|....|....|....|....|....|....|....|....|....|....|....|....|
NNTCTAGCCCACCATGGCAACAAGAAGAGCTTACAGGAGCTGAATGAAGAACAGTGGGTGGAAATGTCTGACGATTACCGGACCGGGAAA
 X  L  A  H  H  G  N  K  K  S  L  Q  E  L  N  E  E  Q  W  V  E  M  S  D  D  Y  R  T  G  K

       100       110       120       130       140       150       160       170       180
....|....|....|....|....|....|....|....|....|....|....|....|....|....|....|....|....|....|
AACATGCCTTTTCAGTCTCTTGGCACATACTATCGGCCCCCTAACTGGACTTGGGGTCCCAATTTCATCAACCCCTATCAAGTAACGGTT
 N  M  P  F  Q  S  L  G  T  Y  Y  R  P  P  N  W  T  W  G  P  N  F  I  N  P  Y  Q  V  T  V

       190       200       210       220       230       240       250       260       270
....|....|....|....|....|....|....|....|....|....|....|....|....|....|....|....|....|....|
TTCCCACACCAAATTCTGAACGCGAGAACCTCTACCTCGGTAGACATAAACGTCCCATACATCGGGGAGACCCCCACGCAATCCTCAGAG
 F  P  H  Q  I  L  N  A  R  T  S  T  S  V  D  I  N  V  P  Y  I  G  E  T  P  T  Q  S  S  E

       280       290       300       310       320       330       340       350       360
....|....|....|....|....|....|....|....|....|....|....|....|....|....|....|....|....|....|
ACACAGAACTCCTGGACCCTCCTCGTTATGGTGCTCGTTCCCCTAGACTATAAGGAAGGAGCCACAACTGACCCAGAAATTACATTTTCT
 T  Q  N  S  W  T  L  L  V  M  V  L  V  P  L  D  Y  K  E  G  A  T  T  D  P  E  I  T  F  S

       370       380       390       400       410       420       430       440       450
....|....|....|....|....|....|....|....|....|....|....|....|....|....|....|....|....|....|
GTAAGGCCTACAAGTCCCTACTTCAATGGGCTTCGCAACCGCTACACGGCCGGGACGGACGAAGAACAGGGGCCCATTCCTACGGCACCC
 V  R  P  T  S  P  Y  F  N  G  L  R  N  R  Y  T  A  G  T  D  E  E  Q  G  P  I  P  T  A  P

       460       470       480       490       500       510       520       530       540
....|....|....|....|....|....|....|....|....|....|....|....|....|....|....|....|....|....|
AGAGAAAATTCGCTTATGTTTCTCTCAACCCTCCCTGACGACACTGTCCCTGCTTACGGGAATGTGCGTACCCCTCCTGTCAATTACCTC
 R  E  N  S  L  M  F  L  S  T  L  P  D  D  T  V  P  A  Y  G  N  V  R  T  P  P  V  N  Y  L

       550       560       570       580       590       600       610       620       630
....|....|....|....|....|....|....|....|....|....|....|....|....|....|....|....|....|....|
CCTGGTGAAATAACCGACCTTTTGCAACTGGCCCGCATACCCACTCTCATGGCATTTGAGCGGGTGCCTGAACCCGTGCCTGCCTCAGAC
 P  G  E  I  T  D  L  L  Q  L  A  R  I  P  T  L  M  A  F  E  R  V  P  E  P  V  P  A  S  D

       640       650       660       670       680       690       700       710       720
....|....|....|....|....|....|....|....|....|....|....|....|....|....|....|....|....|....|
ACATATGTGCCCTACGTTGCCGTTCCCACCCAGTTCGATGACAGGCCTCTCATCTCCTTCCCGATCACCCTTTCAGATCCCGTCTATCAG
 T  Y  V  P  Y  V  A  V  P  T  Q  F  D  D  R  P  L  I  S  F  P  I  T  L  S  D  P  V  Y  Q

       730       740       750       760       770       780       790       800       810
....|....|....|....|....|....|....|....|....|....|....|....|....|....|....|....|....|....|
AACACCCTGGTTGGCGCCATCAGTTCAAATTTCGCCAATTACCGTGGGTGTATCCAAATCACTCTGACATTTTGTGGACCCATGATGGCG
 N  T  L  V  G  A  I  S  S  N  F  A  N  Y  R  G  C  I  Q  I  T  L  T  F  C  G  P  M  M  A

       820       830       840       850       860       870       880       890       900
....|....|....|....|....|....|....|....|....|....|....|....|....|....|....|....|....|....|
AGAGGGAAATTCCTGCTCTCGTATTCTCCCCCAAATGGAACGCAACCACAGACTCTTTCCGAAGCTATGCAGTGCACATACTCTATTTGG
 R  G  K  F  L  L  S  Y  S  P  P  N  G  T  Q  P  Q  T  L  S  E  A  M  Q  C  T  Y  S  I  W

       910       920       930       940       950       960       970       980       990
....|....|....|....|....|....|....|....|....|....|....|....|....|....|....|....|....|....|
GACATAGGCTTGAACTCTAGTTGGACCTTCGTCGTCCCCTACATCTCGCCCAGTGACTACCGTGAAACTCGAGCCATTACCAACTCGGTT
 D  I  G  L  N  S  S  W  T  F  V  V  P  Y  I  S  P  S  D  Y  R  E  T  R  A  I  T  N  S  V
```

# FIG. 5A

```
        1000      1010      1020      1030      1040      1050      1060      1070      1080
....|....|....|....|....|....|....|....|....|....|....|....|....|....|....|....|....|....|
TACTCCGCTGATGGTTGGTTTAGCCTGCACAAGTTGACCAAAATTACTCTACCACCTGACTGTCCGCAAAGTCCCTGCATTCTCTTTTTC
 Y  S  A  D  G  W  F  S  L  H  K  L  T  K  I  T  L  P  P  D  C  P  Q  S  P  C  I  L  F  F

        1090      1100      1110      1120      1130      1140      1150      1160      1170
....|....|....|....|....|....|....|....|....|....|....|....|....|....|....|....|....|....|
GCTTCTGCTGGTGAGGATTACACTCTCCGTCTCCCCGTTGATTGTAATCCTTCCTATGTGTTCCACTCCACCGACAACGCCGAGACCGGG
 A  S  A  G  E  D  Y  T  L  R  L  P  V  D  C  N  P  S  Y  V  F  H  S  T  D  N  A  E  T  G

        1180      1190      1200      1210      1220      1230      1240      1250      1260
....|....|....|....|....|....|....|....|....|....|....|....|....|....|....|....|....|....|
GTTATTGAGGCGGGTAACACTGACACCGATTTCTCTGGTGAACTGGCGGCTCCTGGCCCTAACCACACTAATGTCAAGTTCCTGTTTGAT
 V  I  E  A  G  N  T  D  T  D  F  S  G  E  L  A  A  P  G  P  N  H  T  N  V  K  F  L  F  D

        1270      1280      1290      1300      1310      1320      1330      1340      1350
....|....|....|....|....|....|....|....|....|....|....|....|....|....|....|....|....|....|
CGATCTCGATTATTGAATGTAATCAAGGTACTGGAGAAGGACGCCGTTTTCCCCCGCCCTTTCCCTACACAAGAAGGTGCGCAGCAGGAT
 R  S  R  L  L  N  V  I  K  V  L  E  K  D  A  V  F  P  R  P  F  P  T  Q  E  G  A  Q  Q  D

        1360      1370      1380      1390      1400      1410      1420      1430      1440
....|....|....|....|....|....|....|....|....|....|....|....|....|....|....|....|....|....|
GATGGTTACTTTTGTCTTCTGACCCCCCGCCCAACAGTCGCTTCCCGACCCGCCACTCGTTTCGGCCTGTACGCCAATCCGTCCGGCAGT
 D  G  Y  F  C  L  L  T  P  R  P  T  V  A  S  R  P  A  T  R  F  G  L  Y  A  N  P  S  G  S

        1450      1460      1470      1480      1490      1500      1510      1520      1530
....|....|....|....|....|....|....|....|....|....|....|....|....|....|....|....|....|....|
GGTGTTCTTGCTAACACTTCACTGGACTTCAATTTTTATAGCTTGGCCTGTTTCACTTACTTTAGATCGGACCTTGAGGTTACGGTGGTC
 G  V  L  A  N  T  S  L  D  F  N  F  Y  S  L  A  C  F  T  Y  F  R  S  D  L  E  V  T  V  V

        1540      1550      1560      1570      1580      1590      1600      1610      1620
....|....|....|....|....|....|....|....|....|....|....|....|....|....|....|....|....|....|
TCACTAGAGCCGGATCTGGAATTTGCTGTAGGGTGGTTTCCTTCTGGCAGTGAATACCAGGCTTCCAGCTTTGTCTACGACCAGCTGCAT
 S  L  E  P  D  L  E  F  A  V  G  W  F  P  S  G  S  E  Y  Q  A  S  S  F  V  Y  D  Q  L  H

        1630      1640      1650      1660      1670      1680      1690      1700      1710
....|....|....|....|....|....|....|....|....|....|....|....|....|....|....|....|....|....|
GTGCCCTTCCACTTTACTGGGCGCACTCCCCGCGCTTTCGCTAGCAAGGGTGGGAAGGTATCTTTCGTGCTCCCTTGGAACTCTGTCTCG
 V  P  F  H  F  T  G  R  T  P  R  A  F  A  S  K  G  G  K  V  S  F  V  L  P  W  N  S  V  S

        1720      1730      1740      1750      1760      1770      1780      1790      1800
....|....|....|....|....|....|....|....|....|....|....|....|....|....|....|....|....|....|
TCTGTGCTCCCCGTGCGCTGGGGGGGGGCTTCCAAGCTCTCTTCTGCTACGCGGGGTCTACCGGCGCATGCTGATTGGGGGACTATTTAC
 S  V  L  P  V  R  W  G  G  A  S  K  L  S  S  A  T  R  G  L  P  A  H  A  D  W  G  T  I  Y

        1810      1820      1830      1840      1850      1860      1870      1880      1890
....|....|....|....|....|....|....|....|....|....|....|....|....|....|....|....|....|....|
GCCTTTGTCCCCCGTCCTAATGAGAAGAAAAGCACCGCTGTAAAACACGTGGCCGTGTACATTCGGTACAAGAACGCACGTGCCTGGTGC
 A  F  V  P  R  P  N  E  K  K  S  T  A  V  K  H  V  A  V  Y  I  R  Y  K  N  A  R  A  W  C

        1900      1910      1920      1930      1940      1950      1960      1970      1980
....|....|....|....|....|....|....|....|....|....|....|....|....|....|....|....|....|....|
CCCAGCATGCTTCCCTTTCGCAGCTACAAGCAGAAGATGCTGATGCAATCTGGCGATATCGAGACCAATCCTGGTCCTGCTTCTGACAAC
 P  S  M  L  P  F  R  S  Y  K  Q  K  M  L  M  Q  S  G  D  I  E  T  N  P  G  P  A  S  D  N

        1990      2000      2010      2020      2030      2040      2050      2060      2070
....|....|....|....|....|....|....|....|....|....|....|....|....|....|....|....|....|....|
CCAATTTTGGAGTTTCTTGAAGCAGAAAATGATCTAGTCACTCTGGCCTCTCTCTGGAAGATGGTGCACTCTGTTCAACAGACCTGGAGA
 P  I  L  E  F  L  E  A  E  N  D  L  V  T  L  A  S  L  W  K  M  V  H  S  V  Q  Q  T  W  R

        2080      2090      2100      2110      2120      2130      2140      2150      2160
....|....|....|....|....|....|....|....|....|....|....|....|....|....|....|....|....|....|
AAGTATGTGAAGAACGATGATTTTTGGCCCAATTTACTCAGCGAGCTAGTGGGGGAAGGCTCTGTCGCCTTGGCCGCCACGCTATCCAAC
 K  Y  V  K  N  D  D  F  W  P  N  L  L  S  E  L  V  G  E  G  S  V  A  L  A  A  T  L  S  N
```

FIG. 5B

```
         2170      2180      2190      2200      2210      2220      2230      2240      2250
....|....|....|....|....|....|....|....|....|....|....|....|....|....|....|....|....|....|
CAAGCTTCAGTAAAGGCTCTTTTGGGCCTGCACTTTCTCTCGGGGGCTCAATTACACTGACTTTTACTCTTTACTGATAGAGAAATGC
 Q  A  S  V  K  A  L  L  G  L  H  F  L  S  R  G  L  N  Y  T  D  F  Y  S  L  L  I  E  K  C

         2260      2270      2280      2290      2300      2310      2320      2330      2340
....|....|....|....|....|....|....|....|....|....|....|....|....|....|....|....|....|....|
TCTAGTTTCTTTACCGTAGAACCACCTCCTCCACCAGCTGAAAACCTGATGACCAAGCCCTCAGTGAAGTCGAAATTCCGAAAACTGTTT
 S  S  F  F  T  V  E  P  P  P  P  P  A  E  N  L  M  T  K  P  S  V  K  S  K  F  R  K  L  F

         2350      2360      2370      2380      2390      2400      2410      2420      2430
....|....|....|....|....|....|....|....|....|....|....|....|....|....|....|....|....|....|
AAGATGCAAGGACCCATGGACAAAGTCAAAGACTGGAACCAAATAGCTGCCGGCTTGAAGAATTTTCAATTTGTTCGTGACCTAGTCAAA
 K  M  Q  G  P  M  D  K  V  K  D  W  N  Q  I  A  A  G  L  K  N  F  Q  F  V  R  D  L  V  K

         2440      2450      2460      2470      2480      2490      2500      2510      2520
....|....|....|....|....|....|....|....|....|....|....|....|....|....|....|....|....|....|
GAGGTGGTCGATTGGCTGCAGGCCTGGATCAACAAAGAGAAAGCCAGCCCTGTCCTCCAGTACCAGTTGGAGATGAAGAAGCTCGGGCCT
 E  V  V  D  W  L  Q  A  W  I  N  K  E  K  A  S  P  V  L  Q  Y  Q  L  E  M  K  K  L  G  P

         2530      2540      2550      2560      2570      2580      2590      2600      2610
....|....|....|....|....|....|....|....|....|....|....|....|....|....|....|....|....|....|
GTGGCCTTGGCTCATGACGCTTTCATGGCTGGTTCCGGGCCCCCTCTTAGCGACGACCAGATTGAATACCTCCAGAACCTCAAATCTCTT
 V  A  L  A  H  D  A  F  M  A  G  S  G  P  P  L  S  D  D  Q  I  E  Y  L  Q  N  L  K  S  L

         2620      2630      2640      2650      2660      2670      2680      2690      2700
....|....|....|....|....|....|....|....|....|....|....|....|....|....|....|....|....|....|
GCCCTAACACTGGGGAAGACTAATTTGGCCCAAAGTCTCACCACTATGATCAATGCCAAACAAAGTTCAGCCCAACGAGTTGAACCCGTT
 A  L  T  L  G  K  T  N  L  A  Q  S  L  T  T  M  I  N  A  K  Q  S  S  A  Q  R  V  E  P  V

         2710      2720      2730      2740      2750      2760      2770      2780      2790
....|....|....|....|....|....|....|....|....|....|....|....|....|....|....|....|....|....|
GTGGTGGTCCTTAGAGGCAAGCCGGGATGCGGCAAGGGCTTGGCCTCTACGTTGATTGCCCAGGCTGTGTCCAAGCGCCTCTATGGCTCC
 V  V  V  L  R  G  K  P  G  C  G  K  G  L  A  S  T  L  I  A  Q  A  V  S  K  R  L  Y  G  S

         2800      2810      2820      2830      2840      2850      2860      2870      2880
....|....|....|....|....|....|....|....|....|....|....|....|....|....|....|....|....|....|
CAAAGTGTATATTCTCTTCCCCCAGATCCAGATTTCTTCGATGGATACAAAGGACAGTTCGTGACCTTGATGGATGATTTGGGACAAAAC
 Q  S  V  Y  S  L  P  P  D  P  D  F  F  D  G  Y  K  G  Q  F  V  T  L  M  D  D  L  G  Q  N

         2890      2900      2910      2920      2930      2940      2950      2960      2970
....|....|....|....|....|....|....|....|....|....|....|....|....|....|....|....|....|....|
CCGGATGGACAAGATTTCTCCACCTTTTGTCAGATGGTGTCGACCGCCCAATTTCTCCCCAACATGGCGGACCTTGCAGAGAAAGGGCGT
 P  D  G  Q  D  F  S  T  F  C  Q  M  V  S  T  A  Q  F  L  P  N  M  A  D  L  A  E  K  G  R

         2980      2990      3000      3010      3020      3030      3040      3050      3060
....|....|....|....|....|....|....|....|....|....|....|....|....|....|....|....|....|....|
CCCTTTACCTCCAATCTCATCATTGCAACTACAAATCTCCCCCACTTCAGTCCTGTCACCATTGCTGATCCTTCTGCAGTCTCTCGCCGT
 P  F  T  S  N  L  I  I  A  T  T  N  L  P  H  F  S  P  V  T  I  A  D  P  S  A  V  S  R  R

         3070      3080      3090      3100      3110      3120      3130      3140      3150
....|....|....|....|....|....|....|....|....|....|....|....|....|....|....|....|....|....|
ATCAACTACGATCTGACTCTAGAAGTATCTGAGGCCTACAAGAAACACACACGGCTGAATTTTGACTTGGCTTTCAGGCGCACAGACGCC
 I  N  Y  D  L  T  L  E  V  S  E  A  Y  K  K  H  T  R  L  N  F  D  L  A  F  R  R  T  D  A

         3160      3170      3180      3190      3200      3210      3220      3230      3240
....|....|....|....|....|....|....|....|....|....|....|....|....|....|....|....|....|....|
CCCCCCATTTATCCTTTTGCTGCCCATGTGCCCTTTGTGGACGTAGCTGTGCGCTTCAAAAATGGTCACCAGAATTTTAATCTCCTAGAG
 P  P  I  Y  P  F  A  A  H  V  P  F  V  D  V  A  V  R  F  K  N  G  H  Q  N  F  N  L  L  E

         3250      3260      3270      3280      3290      3300      3310      3320      3330
....|....|....|....|....|....|....|....|....|....|....|....|....|....|....|....|....|....|
TTGGTCGATTCCATTTGTACAGACATTCGAGCCAAGCAACAAGGTGCCCGAAACATGCAGACTCTGGTTCTACAGAGCCCCAACGAGAAT
 L  V  D  S  I  C  T  D  I  R  A  K  Q  Q  G  A  R  N  M  Q  T  L  V  L  Q  S  P  N  E  N
```

FIG. 5C

```
      3340      3350      3360      3370      3380      3390      3400      3410      3420
....|....|....|....|....|....|....|....|....|....|....|....|....|....|....|....|....|....|
GATGACACCCCCGTCGACGAGGCGTTGGGTAGAGTTCTCTCCCCCGCTGCGGTCGATGAGGCGCTTGTCGACCTCACTCCAGAGGCCGAC
 D  D  T  P  V  D  E  A  L  G  R  V  L  S  P  A  A  V  D  E  A  L  V  D  L  T  P  E  A  D

      3430      3440      3450      3460      3470      3480      3490      3500      3510
....|....|....|....|....|....|....|....|....|....|....|....|....|....|....|....|....|....|
CCGGTTGGCCGTTTGGCTATTCTTGCCAAGCTAGGTCTTGCCCTAGCTGCGGTCACCCCTGGTCTGATAATCTTGGCAGTGGGACTCTAC
 P  V  G  R  L  A  I  L  A  K  L  G  L  A  L  A  A  V  T  P  G  L  I  I  L  A  V  G  L  Y

      3520      3530      3540      3550      3560      3570      3580      3590      3600
....|....|....|....|....|....|....|....|....|....|....|....|....|....|....|....|....|....|
AGGTACTTCTCTGGCTCTGATGCAGACCAAGAAGAAACAGAAAGTGAGGGATCTGTCAAGGCACCCAGGAGCGAAAATGCTTATGACGGC
 R  Y  F  S  G  S  D  A  D  Q  E  E  T  E  S  E  G  S  V  K  A  P  R  S  E  N  A  Y  D  G

      3610      3620      3630      3640      3650      3660      3670      3680      3690
....|....|....|....|....|....|....|....|....|....|....|....|....|....|....|....|....|....|
CCGAAGAAAAACTCTAAGCCCCCTGGAGCACTCTCTCTCATGGAAATGCAACAGCCCAACGTGGACATGGGCTTTGAGGCTGCGGTCGCT
 P  K  K  N  S  K  P  P  G  A  L  S  L  M  E  M  Q  Q  P  N  V  D  M  G  F  E  A  A  V  A

      3700      3710      3720      3730      3740      3750      3760      3770      3780
....|....|....|....|....|....|....|....|....|....|....|....|....|....|....|....|....|....|
AAGAAAGTGGTCGTCCCCATTACCTTCATGGTTCCCAACAGACCTTCTGGGCTTACACAGTCCGCTCTTCTGGTGACCGGCCGGACCTTC
 K  K  V  V  V  P  I  T  F  M  V  P  N  R  P  S  G  L  T  Q  S  A  L  L  V  T  G  R  T  F

      3790      3800      3810      3820      3830      3840      3850      3860      3870
....|....|....|....|....|....|....|....|....|....|....|....|....|....|....|....|....|....|
CTAATCAATGAACATACATGGTCCAATCCCTCCTGGACCAGCTTCACAATCCGCGGTGAGGTACACACTCGTGATGAGCCCTTCCAAACG
 L  I  N  E  H  T  W  S  N  P  S  W  T  S  F  T  I  R  G  E  V  H  T  R  D  E  P  F  Q  T

      3880      3890      3900      3910      3920      3930      3940      3950      3960
....|....|....|....|....|....|....|....|....|....|....|....|....|....|....|....|....|....|
GTTCATTTCACTCACCACGGTATTCCCACAGATCTGATGATGGTACGTCTCGGACCGGGCAATTCTTTCCCTAACAATCTAGACAAGTTT
 V  H  F  T  H  H  G  I  P  T  D  L  M  M  V  R  L  G  P  G  N  S  F  P  N  N  L  D  K  F

      3970      3980      3990      4000      4010      4020      4030      4040      4050
....|....|....|....|....|....|....|....|....|....|....|....|....|....|....|....|....|....|
GGACTTGACCAGATGCCGGCACGCAACTCCCGTGTGGTTGGCGTTTCGTCCAGTTACGGAAACTTCTTCTTCTCTGGAAATTTCCTCGGA
 G  L  D  Q  M  P  A  R  N  S  R  V  V  G  V  S  S  S  Y  G  N  F  F  F  S  G  N  F  L  G

      4060      4070      4080      4090      4100      4110      4120      4130      4140
....|....|....|....|....|....|....|....|....|....|....|....|....|....|....|....|....|....|
TTTGTTGATTCCGTCACCTCTGAACAAGGAACTTACGCAAGACTCTTTAGGTACAGGGTGACGACCTACAAAGGATGGTGCGGCTCGGCC
 F  V  D  S  V  T  S  E  Q  G  T  Y  A  R  L  F  R  Y  R  V  T  T  Y  K  G  W  C  G  S  A

      4150      4160      4170      4180      4190      4200      4210      4220      4230
....|....|....|....|....|....|....|....|....|....|....|....|....|....|....|....|....|....|
CTGGTCTGTGAGGCCGGTGGCGTCCGACGCATCATTGGCCTGCATTCTGCTGGCGCCGCCGGTATCGGCGCCGGGACCTATATCTCAAAA
 L  V  C  E  A  G  G  V  R  R  I  I  G  L  H  S  A  G  A  A  G  I  G  A  G  T  Y  I  S  K

      4240      4250      4260      4270      4280      4290      4300      4310      4320
....|....|....|....|....|....|....|....|....|....|....|....|....|....|....|....|....|....|
TTAGGACTAATCAAAGCCCTGAAACACCTCGGTGAACCTTTGGCCACAATGCAAGGACTGATGACTGAATTAGAGCCTGGAATCACCGTA
 L  G  L  I  K  A  L  K  H  L  G  E  P  L  A  T  M  Q  G  L  M  T  E  L  E  P  G  I  T  V

      4330      4340      4350      4360      4370      4380      4390      4400      4410
....|....|....|....|....|....|....|....|....|....|....|....|....|....|....|....|....|....|
CATGTACCCCGGAAATCCAAATTGAGAAAGACGACCGCACACGCGGTGTACAAACCGGAGTTTGAGCCTGCTGTGTTGTCAAAATTTGAT
 H  V  P  R  K  S  K  L  R  K  T  T  A  H  A  V  Y  K  P  E  F  E  P  A  V  L  S  K  F  D

      4420      4430      4440      4450      4460      4470      4480      4490      4500
....|....|....|....|....|....|....|....|....|....|....|....|....|....|....|....|....|....|
CCCAGACTGAACAAGGATGTTGACTTGGATGAAGTAATTTGGTCTAAACACACTGCCAATGTCCCTTACCAACCTCCTTTGTTCTACACA
 P  R  L  N  K  D  V  D  L  D  E  V  I  W  S  K  H  T  A  N  V  P  Y  Q  P  P  L  F  Y  T
```

FIG. 5D

```
       4510      4520      4530      4540      4550      4560      4570      4580      4590
....|....|....|....|....|....|....|....|....|....|....|....|....|....|....|....|....|....|
TACATGTCAGAGTACGCTCATCGAGTCTTCTCCTTCTTGGGGAAAGACAATGACATTCTGACCGTCAAAGAAGCAATTCTGGGCATCCCC
 Y  M  S  E  Y  A  H  R  V  F  S  F  L  G  K  D  N  D  I  L  T  V  K  E  A  I  L  G  I  P

       4600      4610      4620      4630      4640      4650      4660      4670      4680
....|....|....|....|....|....|....|....|....|....|....|....|....|....|....|....|....|....|
GGACTAGACCCCATGGATCCCCACACAGCTCCGGGTCTGCCTTACGCCATCAACGGCCTTCGACGTACTGATCTCGTCGATTTTGTGAAC
 G  L  D  P  M  D  P  H  T  A  P  G  L  P  Y  A  I  N  G  L  R  R  T  D  L  V  D  F  V  N

       4690      4700      4710      4720      4730      4740      4750      4760      4770
....|....|....|....|....|....|....|....|....|....|....|....|....|....|....|....|....|....|
GGTACAGTAGATGCGGCGCTGGCTGTACAAATCCAGAAATTCTTAGACGGTGACTACTCTGACCATGTCTTCCAAACTTTTCTGAAAGAT
 G  T  V  D  A  A  L  A  V  Q  I  Q  K  F  L  D  G  D  Y  S  D  H  V  F  Q  T  F  L  K  D

       4780      4790      4800      4810      4820      4830      4840      4850      4860
....|....|....|....|....|....|....|....|....|....|....|....|....|....|....|....|....|....|
GAGATCAGACCCTCAGAGAAAGTCCGAGCGGGAAAAACCCGCATTGTTGATGTGCCCTCCCTGGCGCATTGCATTGTGGGCAGAATGTTG
 E  I  R  P  S  E  K  V  R  A  G  K  T  R  I  V  D  V  P  S  L  A  H  C  I  V  G  R  M  L

       4870      4880      4890      4900      4910      4920      4930      4940      4950
....|....|....|....|....|....|....|....|....|....|....|....|....|....|....|....|....|....|
CTTGGGCGCTTTGCTGCCAAGTTTCAATCCCATCCTGGCTTTCTCCTCGGCTCTGCTATCGGGTCTGACCCTGATGTTTTCTGGACCGTC
 L  G  R  F  A  A  K  F  Q  S  H  P  G  F  L  L  G  S  A  I  G  S  D  P  D  V  F  W  T  V

       4960      4970      4980      4990      5000      5010      5020      5030      5040
....|....|....|....|....|....|....|....|....|....|....|....|....|....|....|....|....|....|
ATAGGGGCTCAACTCGAGGGGAGAAAGAACACGTATGACGTGGACTACAGTGCCTTTGACTCTTCACACGGCACTGGCTCCTTCGAGGCT
 I  G  A  Q  L  E  G  R  K  N  T  Y  D  V  D  Y  S  A  F  D  S  S  H  G  T  G  S  F  E  A

       5050      5060      5070      5080      5090      5100      5110      5120      5130
....|....|....|....|....|....|....|....|....|....|....|....|....|....|....|....|....|....|
CTCATCTCTCACTTTTTCACCGTGGACAATGGTTTTAGCCCTGCGCTGGGACCGTATCTCAGATCCCTGGCTGTCTCGGTGCACGCTTAC
 L  I  S  H  F  F  T  V  D  N  G  F  S  P  A  L  G  P  Y  L  R  S  L  A  V  S  V  H  A  Y

       5140      5150      5160      5170      5180      5190      5200      5210      5220
....|....|....|....|....|....|....|....|....|....|....|....|....|....|....|....|....|....|
GGCGAGCGTCGCATCAAGATTACCGGTGGCCTCCCCTCCGGTTGTGCCGCGACCAGCCTGCTGAACACAGTGCTCAACAATGTGATCATC
 G  E  R  R  I  K  I  T  G  G  L  P  S  G  C  A  A  T  S  L  L  N  T  V  L  N  N  V  I  I

       5230      5240      5250      5260      5270      5280      5290      5300      5310
....|....|....|....|....|....|....|....|....|....|....|....|....|....|....|....|....|....|
AGGACTGCTCTGGCATTGACTTACAAGGAATTTGAGTATGACACGGTTGATATCATCGCCTACGGTGACGACCTTCTGGTTGGCACGGAT
 R  T  A  L  A  L  T  Y  K  E  F  E  Y  D  T  V  D  I  I  A  Y  G  D  D  L  L  V  G  T  D

       5320      5330      5340      5350      5360      5370      5380      5390      5400
....|....|....|....|....|....|....|....|....|....|....|....|....|....|....|....|....|....|
TACGATCTGGACTTCAATGAGGTGGCACGACGCGCTGCCAAGTTGGGGTATAAGATGACTCCTGCCAACAAGGGTTCTGTCTTCCCTCCG
 Y  D  L  D  F  N  E  V  A  R  R  A  A  K  L  G  Y  K  M  T  P  A  N  K  G  S  V  F  P  P

       5410      5420      5430      5440      5450      5460      5470      5480      5490
....|....|....|....|....|....|....|....|....|....|....|....|....|....|....|....|....|....|
ACTTCCTCTCTTTCCGATGCTGTTTTTCTAAAGCGCAAATTCGTCCAAAACAACGACGGCTTATACAAACCAGTTATGGATTTAAAGAAT
 T  S  S  L  S  D  A  V  F  L  K  R  K  F  V  Q  N  N  D  G  L  Y  K  P  V  M  D  L  K  N

       5500      5510      5520      5530      5540      5550      5560      5570      5580
....|....|....|....|....|....|....|....|....|....|....|....|....|....|....|....|....|....|
TTGGAAGCCATGCTCTCCTACTTCAAACCAGGAACACTACTCGAGAAGCTGCAATCTGTTTCTATGTTGGCTCAACATTCTGGAAAAGAA
 L  E  A  M  L  S  Y  F  K  P  G  T  L  L  E  K  L  Q  S  V  S  M  L  A  Q  H  S  G  K  E

       5590      5600      5610      5620      5630      5640      5650      5660      5670
....|....|....|....|....|....|....|....|....|....|....|....|....|....|....|....|....|....|
GAATATGATAGATTGATGCACCCCTTCGCTGACTACGGTGCCGTACCGAGTCACGAGTACCTGCAGGCAAGATGGAGGGCCTTGTTCGAC
 E  Y  D  R  L  M  H  P  F  A  D  Y  G  A  V  P  S  H  E  Y  L  Q  A  R  W  R  A  L  F  D

       5680      5690      5700      5710      5720      5730      5740      5750
....|....|....|....|....|....|....|....|....|....|....|....|....|....|....|....|..
TGACCCAGATAGCCCAAGGCGCTTCGGTGCTGCCGGCGATTCTGGGAGAACTCAGTCGGAACAGAAAAAAAAAAAAAAAAA
 *
```

FIG. 5E

NNTCTAGCCC ACCATGGCAA CAAGAAGAGC TTACAGGAGC TGAATGAAGA ACAGTGGGTG 60
GAAATGTCTG ACGATTACCG GACCGGGAAA AACATGCCTT TTCAGTCTCT TGGCACATAC 120
TATCGGCCCC CTAACTGGAC TTGGGGTCCC AATTTCATCA ACCCCTATCA AGTAACGGTT 180
TTCCCACACC AAATTCTGAA CGCGAGAACC TCTACCTCGG TAGACATAAA CGTCCCATAC 240
ATCGGGGAGA CCCCCACGCA ATCCTCAGAG ACACAGAACT CCTGGACCCT CCTCGTTATG 300
GTGCTCGTTC CCCTAGACTA TAAGGAAGGA GCCACAACTG ACCCAGAAAT TACATTTTCT 360
GTAAGGCCTA CAAGTCCCTA CTTCAATGGG CTTCGCAACC GCTACACGGC CGGGACGGAC 420
GAAGAACAGG GGCCCATTCC TACGGCACCC AGAGAAAATT CGCTTATGTT TCTCTCAACC 480
CTCCCTGACG ACACTGTCCC TGCTTACGGG AATGTGCGTA CCCCTCCTGT CAATTACCTC 540
CCTGGTGAAA TAACCGACCT TTTGCAACTG GCCCGCATAC CCACTCTCAT GGCATTTGAG 600
CGGGTGCCTG AACCCGTGCC TGCCTCAGAC ACATATGTGC CCTACGTTGC CGTTCCCACC 660
CAGTTCGATG ACAGGCCTCT CATCTCCTTC CCGATCACCC TTTCAGATCC CGTCTATCAG 720
AACACCCTGG TTGGCGCCAT CAGTTCAAAT TTCGCCAATT ACCGTGGGTG TATCCAAATC 780
ACTCTGACAT TTTGTGGACC CATGATGGCG AGAGGGAAAT TCCTGCTCTC GTATTCTCCC 840
CCAAATGGAA CGCAACCACA GACTCTTTCC GAAGCTATGC AGTGCACATA CTCTATTTGG 900
GACATAGGCT TGAACTCTAG TTGGACCTTC GTCGTCCCCT ACATCTCGCC CAGTGACTAC 960
CGTGAAACTC GAGCCATTAC CAACTCGGTT TACTCCGCTG ATGGTTGGTT TAGCCTGCAC 1020
AAGTTGACCA AAATTACTCT ACCACCTGAC TGTCCGCAAA GTCCCTGCAT TCTCTTTTTC 1080
GCTTCTGCTG GTGAGGATTA CACTCTCCGT CTCCCCGTTG ATTGTAATCC TTCCTATGTG 1140
TTCCACTCCA CCGACAACGC CGAGACCGGG GTTATTGAGG CGGGTAACAC TGACACCGAT 1200
TTCTCTGGTG AACTGGCGGC TCCTGGCCCT AACCACACTA ATGTCAAGTT CCTGTTTGAT 1260
CGATCTCGAT TATTGAATGT AATCAAGGTA CTGGAGAAGG ACGCCGTTTT CCCCCGCCCT 1320
TTCCCTACAC AAGAAGGTGC GCAGCAGGAT GATGGTTACT TTTGTCTTCT GACCCCCCGC 1380
CCAACAGTCG CTTCCCGACC CGCCACTCGT TTCGGCCTGT ACGCCAATCC GTCCGGCAGT 1440
GGTGTTCTTG CTAACACTTC ACTGGACTTC AATTTTTATA GCTTGGCCTG TTTCACTTAC 1500

# FIG. 6A

TTTAGATCGG ACCTTGAGGT TACGGTGGTC TCACTAGAGC CGGATCTGGA ATTTGCTGTA 1560
GGGTGGTTTC CTTCTGGCAG TGAATACCAG GCTTCCAGCT TTGTCTACGA CCAGCTGCAT 1620
GTGCCCTTCC ACTTTACTGG GCGCACTCCC CGCGCTTTCG CTAGCAAGGG TGGGAAGGTA 1680
TCTTTCGTGC TCCCTTGGAA CTCTGTCTCG TCTGTGCTCC CCGTGCGCTG GGGGGGGGCT 1740
TCCAAGCTCT CTTCTGCTAC GCGGGGTCTA CCGGCGCATG CTGATTGGGG GACTATTTAC 1800
GCCTTTGTCC CCCGTCCTAA TGAGAAGAAA AGCACCGCTG TAAAACACGT GGCCGTGTAC 1860
ATTCGGTACA AGAACGCACG TGCCTGGTGC CCCAGCATGC TTCCCTTTCG CAGCTACAAG 1920
CAGAAGATGC TGATGCAATC TGGCGATATC GAGACCAATC CTGGTCCTGC TTCTGACAAC 1980
CCAATTTTGG AGTTTCTTGA AGCAGAAAAT GATCTAGTCA CTCTGGCCTC TCTCTGGAAG 2040
ATGGTGCACT CTGTTCAACA GACCTGGAGA AAGTATGTGA AGAACGATGA TTTTTGGCCC 2100
AATTTACTCA GCGAGCTAGT GGGGGAAGGC TCTGTCGCCT TGGCCGCCAC GCTATCCAAC 2160
CAAGCTTCAG TAAAGGCTCT TTTGGGCCTG CACTTTCTCT CTCGGGGGCT CAATTACACT 2220
GACTTTTACT CTTTACTGAT AGAGAAATGC TCTAGTTTCT TTACCGTAGA ACCACCTCCT 2280
CCACCAGCTG AAAACCTGAT GACCAAGCCC TCAGTGAAGT CGAAATTCCG AAAACTGTTT 2340
AAGATGCAAG GACCCATGGA CAAAGTCAAA GACTGGAACC AAATAGCTGC CGGCTTGAAG 2400
AATTTTCAAT TTGTTCGTGA CCTAGTCAAA GAGGTGGTCG ATTGGCTGCA GGCCTGGATC 2460
AACAAAGAGA AAGCCAGCCC TGTCCTCCAG TACCAGTTGG AGATGAAGAA GCTCGGGCCT 2520
GTGGCCTTGG CTCATGACGC TTTCATGGCT GGTTCCGGGC CCCCTCTTAG CGACGACCAG 2580
ATTGAATACC TCCAGAACCT CAAATCTCTT GCCCTAACAC TGGGGAAGAC TAATTTGGCC 2640
CAAAGTCTCA CCACTATGAT CAATGCCAAA CAAAGTTCAG CCCAACGAGT TGAACCCGTT 2700
GTGGTGGTCC TTAGAGGCAA GCCGGGATGC GGCAAGGGCT TGGCCTCTAC GTTGATTGCC 2760
CAGGCTGTGT CCAAGCGCCT CTATGGCTCC CAAAGTGTAT ATTCTCTTCC CCCAGATCCA 2820
GATTTCTTCG ATGGATACAA AGGACAGTTC GTGACCTTGA TGGATGATTT GGGACAAAAC 2880
CCGGATGGAC AAGATTTCCC CACCTTTTGT CAGATGGTGT CGACCGCCCA ATTTCTCCCC 2940
AACATGGCGG ACCTTGCAGA GAAAGGGCGT CCCTTTACCT CCAATCTCAT CATTGCAACT 3000

# FIG. 6B

ACAAATCTCC CCCACTTCAG TCCTGTCACC ATTGCTGATC CTTCTGCAGT CTCTCGCCGT 3060
ATCAACTACG ATCTGACTCT AGAAGTATCT GAGGCCTACA AGAAACACAC ACGGCTGAAT 3120
TTTGACTTGG CTTTCAGGCG CACAGACGCC CCCCCCATTT ATCCTTTTGC TGCCCATGTG 3180
CCCTTTGTGG ACGTAGCTGT GCGCTTCAAA AATGGTCACC AGAATTTTAA TCTCCTAGAG 3240
TTGGTCGATT CCATTTGTAC AGACATTCGA GCCAAGCAAC AAGGTGCCCG AAACATGCAG 3300
ACTCTGGTTC TACAGAGCCC CAACGAGAAT GATGACACCC CCGTCGACGA GGCGTTGGGT 3360
AGAGTTCTCT CCCCCGCTGC GGTCGATGAG GCGCTTGTCG ACCTCACTCC AGAGGCCGAC 3420
CCGGTTGGCC GTTTGGCTAT TCTTGCCAAG CTAGGTCTTG CCCTAGCTGC GGTCACCCCT 3480
GGTCTGATAA TCTTGGCAGT GGGACTCTAC AGGTACTTCT CTGGCTCTGA TGCAGACCAA 3540
GAAGAAACAG AAAGTGAGGG ATCTGTCAAG GCACCCAGGA GCGAAAATGC TTATGACGGC 3600
CCGAAGAAAA ACTCTAAGCC CCCTGGAGCA CTCTCTCTCA TGGAAATGCA ACAGCCCAAC 3660
GTGGACATGG GCTTTGAGGC TGCGGTCGCT AAGAAAGTGG TCGTCCCCAT TACCTTCATG 3720
GTTCCCAACA GACCTTCTGG GCTTACACAG TCCGCTCTTC TGGTGACCGG CCGGACCTTC 3780
CTAATCAATG AACATACATG GTCCAATCCC TCCTGGACCA GCTTCACAAT CCGCGGTGAG 3840
GTACACACTC GTGATGAGCC CTTCCAAACG GTTCATTTCA CTCACCACGG TATTCCCACA 3900
GATCTGATGA TGGTACGTCT CGGACCGGGC AATTCTTTCC CTAACAATCT AGACAAGTTT 3960
GGACTTGACC AGATGCCGGC ACGCAACTCC CGTGTGGTTG GCGTTTCGTC CAGTTACGGA 4020
AACTTCTTCT TCTCTGGAAA TTTCCTCGGA TTTGTTGATT CCGTCACCTC TGAACAAGGA 4080
ACTTACGCAA GACTCTTTAG GTACAGGGTG ACGACCTACA AAGGATGGTG CGGCTCGGCC 4140
CTGGTCTGTG AGGCCGGTGG CGTCCGACGC ATCATTGGCC TGCATTCTGC TGGCGCCGCC 4200
GGTATCGGCG CCGGGACCTA TATCTCAAAA TTAGGACTAA TCAAAGCCCT GAAACACCTC 4260
GGTGAACCTT TGGCCACAAT GCAAGGACTG ATGACTGAAT TAGAGCCTGG AATCACCGTA 4320
CATGTACCCC GGAAATCCAA ATTGAGAAAG ACGACCGCAC ACGCGGTGTA CAAACCGGAG 4380
TTTGAGCCTG CTGTGTTGTC AAAATTTGAT CCCAGACTGA ACAAGGATGT TGACTTGGAT 4440
GAAGTAATTT GGTCTAAACA CACTGCCAAT GTCCCTTACC AACCTCCTTT GTTCTACACA 4500

# FIG. 6C

```
TACATGTCAG AGTACGCTCA TCGAGTCTTC TCCTTCTTGG GGAAAGACAA TGACATTCTG 4560
ACCGTCAAAG AAGCAATTCT GGGCATCCCC GGACTAGACC CCATGGATCC CCACACAGCT 4620
CCGGGTCTGC CTTACGCCAT CAACGGCCTT CGACGTACTG ATCTCGTCGA TTTTGTGAAC 4680
GGTACAGTAG ATGCGGCGCT GGCTGTACAA ATCCAGAAAT TCTTAGACGG TGACTACTCT 4740
GACCATGTCT TCCAAACTTT TCTGAAAGAT GAGATCAGAC CCTCAGAGAA AGTCCGAGCG 4800
GGAAAAACCC GCATTGTTGA TGTGCCCTCC CTGGCGCATT GCATTGTGGG CAGAATGTTG 4860
CTTGGGCGCT TTGCTGCCAA GTTTCAATCC CATCCTGGCT TTCTCCTCGG CTCTGCTATC 4920
GGGTCTGACC CTGATGTTTT CTGGACCGTC ATAGGGGCTC AACTCGAGGG GAGAAAGAAC 4980
ACGTATGACG TGGACTACAG TGCCTTTGAC TCTTCACACG GCACTGGCTC CTTCGAGGCT 5040
CTCATCTCTC ACTTTTTCAC CGTGGACAAT GGTTTTAGCC CTGCGCTGGG ACCGTATCTC 5100
AGATCCCTGG CTGTCTCGGT GCACGCTTAC GGCGAGCGTC GCATCAAGAT TACCGGTGGC 5160
CTCCCCTCCG GTTGTGCCGC GACCAGCCTG CTGAACACAG TGCTCAACAA TGTGATCATC 5220
AGGACTGCTC TGGCATTGAC TTACAAGGAA TTTGAGTATG ACACGGTTGA TATCATCGCC 5280
TACGGTGACG ACCTTCTGGT TGGCACGGAT TACGATCTGG ACTTCAATGA GGTGGCACGA 5340
CGCGCTGCCA AGTTGGGGTA TAAGATGACT CCTGCCAACA AGGGTTCTGT CTTCCCTCCG 5400
ACTTCCTCTC TTTCCGATGC TGTTTTTCTA AAGCGCAAAT TCGTCCAAAA CAACGACGGC 5460
TTATACAAAC CAGTTATGGA TTTAAAGAAT TTGGAAGCCA TGCTCTCCTA CTTCAAACCA 5520
GGAACACTAC TCGAGAAGCT GCAATCTGTT TCTATGTTGG CTCAACATTC TGGAAAAGAA 5580
GAATATGATA GATTGATGCA CCCCTTCGCT GACTACGGTG CCGTACCGAG TCACGAGTAC 5640
CTGCAGGCAA GATGGAGGGC CTTGTTCGAC TGACCCAGAT AGCCCAAGGC GCTTCGGTGC 5700
TGCCGGCGAT TCTGGGAGAA CTCAGTCGGA ACAGAAAAAA AAAAAAAAAA AA         5752
(SEQ ID NO:1)
```

# FIG. 6D

XLAHHGNKKS LQELNEEQWV EMSDDYRTGK NMPFQSLGTY YRPPNWTWGP NFINPYQVTV 60
FPHQILNART STSVDINVPY IGETPTQSSE TQNSWTLLVM VLVPLDYKEG ATTDPEITFS 120
VRPTSPYFNG LRNRYTAGTD EEQGPIPTAP RENSLMFLST LPDDTVPAYG NVRTPPVNYL 180
PGEITDLLQL ARIPTLMAFE RVPEPVPASD TYVPYVAVPT QFDDRPLISF PITLSDPVYQ 240
NTLVGAISSN FANYRGCIQI TLTFCGPMMA RGKFLLSYSP PNGTQPQTLS EAMQCTYSIW 300
DIGLNSSWTF VVPYISPSDY RETRAITNSV YSADGWFSLH KLTKITLPPD CPQSPCILFF 360
ASAGEDYTLR LPVDCNPSYV FHSTDNAETG VIEAGNTDTD FSGELAAPGP NHTNVKFLFD 420
RSRLLNVIKV LEKDAVFPRP FPTQEGAQQD DGYFCLLTPR PTVASRPATR FGLYANPSGS 480
GVLANTSLDF NFYSLACFTY FRSDLEVTVV SLEPDLEFAV GWFPSGSEYQ ASSFVYDQLH 540
VPFHFTGRTP RAFASKGGKV SFVLPWNSVS SVLPVRWGGA SKLSSATRGL PAHADWGTIY 600
AFVPRPNEKK STAVKHVAVY IRYKNARAWC PSMLPFRSYK QKMLMQSGDI ETNPGPASDN 660
PILEFLEAEN DLVTLASLWK MVHSVQQTWR KYVKNDDFWP NLLSELVGEG SVALAATLSN 720
QASVKALLGL HFLSRGLNYT DFYSLLIEKC SSFFTVEPPP PPAENLMTKP SVKSKFRKLF 780
KMQGPMDKVK DWNQIAAGLK NFQFVRDLVK EVVDWLQAWI NKEKASPVLQ YQLEMKKLGP 840
VALAHDAFMA GSGPPLSDDQ IEYLQNLKSL ALTLGKTNLA QSLTTMINAK QSSAQRVEPV 900
VVVLRGKPGC GKGLASTLIA QAVSKRLYGS QSVYSLPPDP DFFDGYKGQF VTLMDDLGQN 960
PDGQDFSTFC QMVSTAQFLP NMADLAEKGR PFTSNLIIAT TNLPHFSPVT IADPSAVSRR 1020
INYDLTLEVS EAYKKHTRLN FDLAFRRTDA PPIYPFAAHV PFVDVAVRFK NGHQNFNLLE 1080
LVDSICTDIR AKQQGARNMQ TLVLQSPNEN DDTPVDEALG RVLSPAAVDE ALVDLTPEAD 1140
PVGRLAILAK LGLALAAVTP GLIIILAVGLY RYFSGSDADQ EETESEGSVK APRSENAYDG 1200

# FIG. 7A

```
PKKNSKPPGA LSLMEMQQPN VDMGFEAAVA KKVVVPITFM VPNRPSGLTQ SALLVTGRTF 1260
LINEHTWSNP SWTSFTIRGE VHTRDEPFQT VHFTHHGIPT DLMMVRLGPG NSFPNNLDKF 1320
GLDQMPARNS RVVGVSSSYG NFFFSGNFLG FVDSVTSEQG TYARLFRYRV TTYKGWCGSA 1380
LVCEAGGVRR IIGLHSAGAA GIGAGTYISK LGLIKALKHL GEPLATMQGL MTELEPGITV 1440
HVPRKSKLRK TTAHAVYKPE FEPAVLSKFD PRLNKDVDLD EVIWSKHTAN VPYQPPLFYT 1500
YMSEYAHRVF SFLGKDNDIL TVKEAILGIP GLDPMDPHTA PGLPYAINGL RRTDLVDFVN 1560
GTVDAALAVQ IQKFLDGDYS DHVFQTFLKD EIRPSEKVRA GKTRIVDVPS LAHCIVGRML 1620
LGRFAAKFQS HPGFLLGSAI GSDPDVFWTV IGAQLEGRKN TYDVDYSAFD SSHGTGSFEA 1680
LISHFFTVDN GFSPALGPYL RSLAVSVHAY GERRIKITGG LPSGCAATSL LNTVLNNVII 1740
RTALALTYKE FEYDTVDIIA YGDDLLVGTD YDLDFNEVAR RAAKLGYKMT PANKGSVFPP 1800
TSSLSDAVFL KRKFVQNNDG LYKPVMDLKN LEAMLSYFKP GTLLEKLQSV SMLAQHSGKE 1860
EYDRLMHPFA DYGAVPSHEY LQARWRALFD * (SEQ ID NO:2)                 1890
```

# FIG. 7B

CTAGCCCACC ATGGCAACAA GAAGAGCTTA CAGGAGCTGA ATGAAGAACA GTGGGTGGAA 60
ATGTCTGACG ATTACCGGAC CGGGAAAAAC ATGCCTTTTC AGTCTCTTGG CACATACTAT 120
CGGCCCCCTA ACTGGACTTG GGGTCCCAAT TTCATCAACC CCTATCAAGT AACGGTTTTC 180
CCACACCAAA TTCTGAACGC GAGAACCTCT ACCTCGGTAG ACATAAACGT CCCATACATC 240
GGGGAGACCC CCACGCAATC CTCAGAGACA CAGAACTCCT GGACCCTCCT CGTTATGGTG 300
CTCGTTCCCC TAGACTATAA GGAAGGAGCC ACAACTGACC CAGAAATTAC ATTTTCTGTA 360
AGGCCTACAA GTCCCTACTT CAATGGGCTT CGCAACCGCT ACACGGCCGG GACGGACGAA 420
GAACAG (SEQ ID NO:3) 426

**FIG. 8**

LAHHGNKKSL QELNEEQWVE MSDDYRTGKN MPFQSLGTYY RPPNWTWGPN FINPYQVTVF 60
PHQILNARTS TSVDINVPYI GETPTQSSET QNSWTLLVMV LVPLDYKEGA TTDPEITFSV 120
RPTSPYFNGL RNRYTAGTDE EQ (SEQ ID NO:4) 142

**FIG. 9**

GGGCCCATTC CTACGGCACC CAGAGAAAAT TCGCTTATGT TTCTCTCAAC CCTCCCTGAC 60
GACACTGTCC CTGCTTACGG GAATGTGCGT ACCCCTCCTG TCAATTACCT CCCTGGTGAA 120
ATAACCGACC TTTTGCAACT GGCCCGCATA CCCACTCTCA TGGCATTTGA GCGGGTGCCT 180
GAACCCGTGC CTGCCTCAGA CACATATGTG CCCTACGTTG CCGTTCCCAC CCAGTTCGAT 240
GACAGGCCTC TCATCTCCTT CCCGATCACC CTTTCAGATC CCGTCTATCA GAACACCCTG 300
GTTGGCGCCA TCAGTTCAAA TTTCGCCAAT TACCGTGGGT GTATCCAAAT CACTCTGACA 360
TTTTGTGGAC CCATGATGGC GAGAGGGAAA TTCCTGCTCT CGTATTCTCC CCCAAATGGA 420
ACGCAACCAC AGACTCTTTC CGAAGCTATG CAGTGCACAT ACTCTATTTG GGACATAGGC 480
TTGAACTCTA GTTGGACCTT CGTCGTCCCC TACATCTCGC CCAGTGACTA CCGTGAAACT 540
CGAGCCATTA CCAACTCGGT TTACTCCGCT GATGGTTGGT TTAGCCTGCA CAAGTTGACC 600
AAAATTACTC TACCACCTGA CTGTCCGCAA AGTCCCTGCA TTCTCTTTTT CGCTTCTGCT 660
GGTGAGGATT ACACTCTCCG TCTCCCCGTT GATTGTAATC CTTCCTATGT GTTCCAC 717
(SEQ ID NO:5)

# FIG. 10

GPIPTAPREN SLMFLSTLPD DTVPAYGNVR TPPVNYLPGE ITDLLQLARI PTLMAFERVP 60
EPVPASDTYV PYVAVPTQFD DRPLISFPIT LSDPVYQNTL VGAISSNFAN YRGCIQITLT 120
FCGPMMARGK FLLSYSPPNG TQPQTLSEAM QCTYSIWDIG LNSSWTFVVP YISPSDYRET 180
RAITNSVYSA DGWFSLHKLT KITLPPDCPQ SPCILFFASA GEDYTLRLPV DCNPSYVFH 239
(SEQ ID NO:6)

# FIG. 11

```
TCCACCGACA ACGCCGAGAC CGGGGTTATT GAGGCGGGTA ACACTGACAC CGATTTCTCT 60
GGTGAACTGG CGGCTCCTGG CCCTAACCAC ACTAATGTCA AGTTCCTGTT TGATCGATCT 120
CGATTATTGA ATGTAATCAA GGTACTGGAG AAGGACGCCG TTTTCCCCCG CCCTTTCCCT 180
ACACAAGAAG GTGCGCAGCA GGATGATGGT TACTTTTGTC TTCTGACCCC CCGCCCAACA 240
GTCGCTTCCC GACCCGCCAC TCGTTTCGGC CTGTACGCCA ATCCGTCCGG CAGTGGTGTT 300
CTTGCTAACA CTTCACTGGA CTTCAATTTT TATAGCTTGG CCTGTTTCAC TTACTTTAGA 360
TCGGACCTTG AGGTTACGGT GGTCTCACTA GAGCCGGATC TGGAATTTGC TGTAGGGTGG 420
TTTCCTTCTG GCAGTGAATA CCAGGCTTCC AGCTTTGTCT ACGACCAGCT GCATGTGCCC 480
TTCCACTTTA CTGGGCGCAC TCCCCGCGCT TTCGCTAGCA AGGGTGGGAA GGTATCTTTC 540
GTGCTCCCTT GGAACTCTGT CTCGTCTGTG CTCCCCGTGC GCTGGGGGGG GGCTTCCAAG 600
CTCTCTTCTG CTACGCGGGG TCTACCGGCG CATGCTGATT GGGGGACTAT TTACGCCTTT 660
GTCCCCCGTC CTAATGAGAA GAAAAGCACC GCTGTAAAAC ACGTGGCCGT GTACATTCGG 720
TACAAGAACG CACGTGCCTG GTGCCCCAGC ATGCTTCCCT TTCGCAGCTA CAAGCAG    777
(SEQ ID NO:7)
```

# FIG. 12

```
STDNAETGVI EAGNTDTDFS GELAAPGPNH TNVKFLFDRS RLLNVIKVLE KDAVFPRPFP 60
TQEGAQQDDG YFCLLTPRPT VASRPATRFG LYANPSGSGV LANTSLDFNF YSLACFTYFR 120
SDLEVTVVSL EPDLEFAVGW FPSGSEYQAS SFVYDQLHVP FHFTGRTPRA FASKGGKVSF 180
VLPWNSVSSV LPVRWGGASK LSSATRGLPA HADWGTIYAF VPRPNEKKST AVKHVAVYIR 240
YKNARAWCPS MLPFRSYKQ (SEQ ID NO:8)                               259
```

# FIG. 13

AAGATGCTGA TGCAATCTGG CGATATCGAG ACCAATCCTG GT (SEQ ID NO:9) 42

# FIG. 14

KMLMQSGDIE TNPG (SEQ ID NO:10) 14

# FIG. 15

CCTGCTTCTG ACAACCCAAT TTTGGAGTTT CTTGAAGCAG AAAATGATCT AGTCACTCTG 60
GCCTCTCTCT GGAAGATGGT GCACTCTGTT CAACAGACCT GGAGAAAGTA TGTGAAGAAC 120
GATGATTTTT GGCCCAATTT ACTCAGCGAG CTAGTGGGGG AAGGCTCTGT CGCCTTGGCC 180
GCCACGCTAT CCAACCAAGC TTCAGTAAAG GCTCTTTTGG GCCTGCACTT TCTCTCTCGG 240
GGGCTCAATT ACACTGACTT TTACTCTTTA CTGATAGAGA AATGCTCTAG TTTCTTTACC 300
GTAGAACCAC CTCCTCCACC AGCTGAAAAC CTGATGACCA AGCCCTCAGT GAAGTCGAAA 360
TTCCGAAAAC TGTTTAAGAT GCAA (SEQ ID NO:11) 384

# FIG. 16

PASDNPILEF LEAENDLVTL ASLWKMVHSV QQTWRKYVKN DDFWPNLLSE LVGEGSVALA 60
ATLSNQASVK ALLGLHFLSR GLNYTDFYSL LIEKCSSFFT VEPPPPPAEN LMTKPSVKSK 120
FRKLFKMQ (SEQ ID NO:12) 128

# FIG. 17

GGACCCATGG ACAAAGTCAA AGACTGGAAC CAAATAGCTG CCGGCTTGAA GAATTTTCAA 60

TTTGTTCGTG ACCTAGTCAA AGAGGTGGTC GATTGGCTGC AGGCCTGGAT CAACAAAGAG 120

AAAGCCAGCC CTGTCCTCCA GTACCAGTTG GAGATGAAGA AGCTCGGGCC TGTGGCCTTG 180

GCTCATGACG CTTTCATGGC TGGTTCCGGG CCCCCTCTTA GCGACGACCA GATTGAATAC 240

CTCCAGAACC TCAAATCTCT TGCCCTAACA CTGGGGAAGA CTAATTTGGC CCAAAGTCTC 300

ACCACTATGA TCAATGCCAA ACAAAGTTCA GCCCAACGAG TTGAACCCGT TGTGGTGGTC 360

CTTAGAGGCA AGCCGGGATG CGGCAAGGGC TTGGCCTCTA CGTTGATTGC CCAGGCTGTG 420

TCCAAGCGCC TCTATGGCTC CCAAAGTGTA TATTCTCTTC CCCCAGATCC AGATTTCTTC 480

GATGGATACA AAGGACAGTT CGTGACCTTG ATGGATGATT TGGGACAAAA CCCGGATGGA 540

CAAGATTTCC CCACCTTTTG TCAGATGGTG TCGACCGCCC AATTTCTCCC CAACATGGCG 600

GACCTTGCAG AGAAAGGGCG TCCCTTTACC TCCAATCTCA TCATTGCAAC TACAAATCTC 660

CCCCACTTCA GTCCTGTCAC CATTGCTGAT CCTTCTGCAG TCTCTCGCCG TATCAACTAC 720

GATCTGACTC TAGAAGTATC TGAGGCCTAC AAGAAACACA CACGGCTGAA TTTTGACTTG 780

GCTTTCAGGC GCACAGACGC CCCCCCCATT TATCCTTTTG CTGCCCATGT GCCCTTTGTG 840

GACGTAGCTG TGCGCTTCAA AAATGGTCAC CAGAATTTTA ATCTCCTAGA GTTGGTCGAT 900

TCCATTTGTA CAGACATTCG AGCCAAGCAA CAAGGTGCCC GAAACATGCA GACTCTGGTT 960

CTACAG (SEQ ID NO:13) 966

# FIG. 18

GPMDKVKDWN QIAAGLKNFQ FVRDLVKEVV DWLQAWINKE KASPVLQYQL EMKKLGPVAL 60

AHDAFMAGSG PPLSDDQIEY LQNLKSLALT LGKTNLAQSL TTMINAKQSS AQRVEPVVVV 120

LRGKPGCGKG LASTLIAQAV SKRLYGSQSV YSLPPDPDFF DGYKGQFVTL MDDLGQNPDG 180

QDFSTFCQMV STAQFLPNMA DLAEKGRPFT SNLIIATTNL PHFSPVTIAD PSAVSRRINY 240

DLTLEVSEAY KKHTRLNFDL AFRRTDAPPI YPFAAHVPFV DVAVRFKNGH QNFNLLELVD 300

SICTDIRAKQ QGARNMQTLV LQ (SEQ ID NO:14) 322

# FIG. 19

AGCCCCAACG AGAATGATGA CACCCCCGTC GACGAGGCGT TGGGTAGAGT TCTCTCCCCC 60

GCTGCGGTCG ATGAGGCGCT TGTCGACCTC ACTCCAGAGG CCGACCCGGT TGGCCGTTTG 120

GCTATTCTTG CCAAGCTAGG TCTTGCCCTA GCTGCGGTCA CCCCTGGTCT GATAATCTTG 180

GCAGTGGGAC TCTACAGGTA CTTCTCTGGC TCTGATGCAG ACCAAGAAGA AACAGAAAGT 240

GAGGGATCTG TCAAGGCACC CAGGAGCGAA (SEQ ID NO:15) 270

# FIG. 20

SPNENDDTPV DEALGRVLSP AAVDEALVDL TPEADPVGRL AILAKLGLAL AAVTPGLIIL 60

AVGLYRYFSG SDADQEETES EGSVKAPRSE (SEQ ID NO:16) 90

# FIG. 21

AATGCTTATG ACGGCCCGAA GAAAAACTCT AAGCCCCCTG GAGCACTCTC TCTCATGGAA 60

ATGCAA (SEQ ID NO:17) 66

# FIG. 22

NAYDGPKKNS KPPGALSLME MQ (SEQ ID NO:18) 22

# FIG. 23

CAGCCCAACG TGGACATGGG CTTTGAGGCT GCGGTCGCTA AGAAAGTGGT CGTCCCCATT 60

ACCTTCATGG TTCCCAACAG ACCTTCTGGG CTTACACAGT CCGCTCTTCT GGTGACCGGC 120

CGGACCTTCC TAATCAATGA ACATACATGG TCCAATCCCT CCTGGACCAG CTTCACAATC 180

CGCGGTGAGG TACACACTCG TGATGAGCCC TTCCAAACGG TTCATTTCAC TCACCACGGT 240

ATTCCCACAG ATCTGATGAT GGTACGTCTC GGACCGGGCA ATTCTTTCCC TAACAATCTA 300

GACAAGTTTG GACTTGACCA GATGCCGGCA CGCAACTCCC GTGTGGTTGG CGTTTCGTCC 360

AGTTACGGAA ACTTCTTCTT CTCTGGAAAT TTCCTCGGAT TTGTTGATTC CGTCACCTCT 420

GAACAAGGAA CTTACGCAAG ACTCTTTAGG TACAGGGTGA CGACCTACAA AGGATGGTGC 480

GGCTCGGCCC TGGTCTGTGA GGCCGGTGGC GTCCGACGCA TCATTGGCCT GCATTCTGCT 540

GGCGCCGCCG GTATCGGCGC CGGGACCTAT ATCTCAAAAT TAGGACTAAT CAAAGCCCTG 600

AAACACCTCG GTGAACCTTT GGCCACAATG CAA (SEQ ID NO:19) 633

# FIG. 24

QPNVDMGFEA AVAKKVVVPI TFMVPNRPSG LTQSALLVTG RTFLINEHTW SNPSWTSFTI 60

RGEVHTRDEP FQTVHFTHHG IPTDLMMVRL GPGNSFPNNL DKFGLDQMPA RNSRVVGVSS 120

SYGNFFFSGN FLGFVDSVTS EQGTYARLFR YRVTTYKGWC GSALVCEAGG VRRIIGLHSA 180

GAAGIGAGTY ISKLGLIKAL KHLGEPLATM Q (SEQ ID NO:20) 211

# FIG. 25

GGACTGATGA CTGAATTAGA GCCTGGAATC ACCGTACATG TACCCCGGAA ATCCAAATTG 60
AGAAAGACGA CCGCACACGC GGTGTACAAA CCGGAGTTTG AGCCTGCTGT GTTGTCAAAA 120
TTTGATCCCA GACTGAACAA GGATGTTGAC TTGGATGAAG TAATTTGGTC TAAACACACT 180
GCCAATGTCC CTTACCAACC TCCTTTGTTC TACACATACA TGTCAGAGTA CGCTCATCGA 240
GTCTTCTCCT TCTTGGGGAA AGACAATGAC ATTCTGACCG TCAAAGAAGC AATTCTGGGC 300
ATCCCCGGAC TAGACCCCAT GGATCCCCAC ACAGCTCCGG GTCTGCCTTA CGCCATCAAC 360
GGCCTTCGAC GTACTGATCT CGTCGATTTT GTGAACGGTA CAGTAGATGC GGCGCTGGCT 420
GTACAAATCC AGAAATTCTT AGACGGTGAC TACTCTGACC ATGTCTTCCA AACTTTTCTG 480
AAAGATGAGA TCAGACCCTC AGAGAAAGTC CGAGCGGGAA AAACCCGCAT TGTTGATGTG 540
CCCTCCCTGG CGCATTGCAT TGTGGGCAGA ATGTTGCTTG GGCGCTTTGC TGCCAAGTTT 600
CAATCCCATC CTGGCTTTCT CCTCGGCTCT GCTATCGGGT CTGACCCTGA TGTTTTCTGG 660
ACCGTCATAG GGGCTCAACT CGAGGGGAGA AAGAACACGT ATGACGTGGA CTACAGTGCC 720
TTTGACTCTT CACACGGCAC TGGCTCCTTC GAGGCTCTCA TCTCTCACTT TTTCACCGTG 780
GACAATGGTT TTAGCCCTGC GCTGGGACCG TATCTCAGAT CCCTGGCTGT CTCGGTGCAC 840
GCTTACGGCG AGCGTCGCAT CAAGATTACC GGTGGCCTCC CCTCCGGTTG TGCCGCGACC 900
AGCCTGCTGA ACACAGTGCT CAACAATGTG ATCATCAGGA CTGCTCTGGC ATTGACTTAC 960
AAGGAATTTG AGTATGACAC GGTTGATATC ATCGCCTACG GTGACGACCT TCTGGTTGGC 1020
ACGGATTACG ATCTGGACTT CAATGAGGTG GCACGACGCG CTGCCAAGTT GGGGTATAAG 1080
ATGACTCCTG CCAACAAGGG TTCTGTCTTC CCTCCGACTT CCTCTCTTTC CGATGCTGTT 1140
TTTCTAAAGC GCAAATTCGT CCAAAACAAC GACGGCTTAT ACAAACCAGT TATGGATTTA 1200
AAGAATTTGG AAGCCATGCT CTCCTACTTC AAACCAGGAA CACTACTCGA GAAGCTGCAA 1260
TCTGTTTCTA TGTTGGCTCA ACATTCTGGA AAAGAAGAAT ATGATAGATT GATGCACCCC 1320
TTCGCTGACT ACGGTGCCGT ACCGAGTCAC GAGTACCTGC AGGCAAGATG GAGGGCCTTG 1380
TTCGACTGA (SEQ ID NO:21) 1389

# FIG. 26

GLMTELEPGI TVHVPRKSKL RKTTAHAVYK PEFEPAVLSK FDPRLNKDVD LDEVIWSKHT 60

ANVPYQPPLF YTYMSEYAHR VFSFLGKDND ILTVKEAILG IPGLDPMDPH TAPGLPYAIN 120

GLRRTDLVDF VNGTVDAALA VQIQKFLDGD YSDHVFQTFL KDEIRPSEKV RAGKTRIVDV 180

PSLAHCIVGR MLLGRFAAKF QSHPGFLLGS AIGSDPDVFW TVIGAQLEGR KNTYDVDYSA 240

FDSSHGTGSF EALISHFFTV DNGFSPALGP YLRSLAVSVH AYGERRIKIT GGLPSGCAAT 300

SLLNTVLNNV IIRTALALTY KEFEYDTVDI IAYGDDLLVG TDYDLDFNEV ARRAAKLGYK 360

MTPANKGSVF PPTSSLSDAV FLKRKFVQNN DGLYKPVMDL KNLEAMLSYF KPGTLLEKLQ 420

SVSMLAQHSG KEEYDRLMHP FADYGAVPSH EYLQARWRAL FD * (SEQ ID NO:22) 462

# FIG. 27

```
                                   10        20        30        40        50        60        70        80        90        100
                           ....|....|....|....|....|....|....|....|....|....|....|....|....|....|....|....|....|....|....|....|
SVV                        ----------------------------------------------------------------------------------------------------
EMCV-R                     MATTMEQETCAHSLTFEECPKCSALQYRNG-FYLLKYDEEWYPEELLT-DGEDDVFDP---------------ELDHEVVFELQ/GNSTSSDKNNSSSEG
EMCV-PV21                  MATTMEQETCAHSLTFEECPKCSALQYRNG-FYLLKYDEEWYPEELLT-DGEDDVFDP---------------ELDHEVVFELQ/GNSTSSDKNNSSSEG
EMCV-B                     MATTMEQEICAHSLTLKGCPKCSALQYRNG-FYLLKYDEEWYPEELLT-DGEDDVFDP---------------ELDHEVVFELQ/GNSTSSDKNNSSSDG
EMCV-Da                    MATTMEQEICAHSLTFKGCPKCSALQYRNG-FYLLKYDEEWYPEELLT-DGEDDVFDP---------------ELDHEVVFELQ/GNSTSSDKNNSSSDG
EMCV-Db                    MATTMEQEICAHSLTFKGCPKCSALQYRNG-FYLLKYDEEWYPEELLT-DGEDDVFDP---------------ELDHEVVFELQ/GNSTSSDKNNSSSDG
EMCV-PV2                   MATTMEQEICAHSLTFKGCPKCSALQYRNG-FYLLKYDEEWYPEELLT-DGEDDVFDP---------------ELDHEVVFELQ/GNSTSSDKNNSSSDG
EMCV-Mengo                 MATTMEQEICAHSMTFEECPKCSALQYRNG-FYLLKYDEEWYPEESLT-DGEDDVFDP---------------DLDHEVVFETQ/GNSTSSDKNNSSSEG
TMEV/DA_[M20301]           MA-------CKHGYP-DVCPICTAVDVTPGFEYLLLADGEWYPTDLLCVDLDDDVFWPSNSSNQSETMEPTDLPLVRDTVMEPQ/GNASSSDKSNSQSSG
TMEV/GDVII_[M20562]        MA-------CKHGYP-DVCPICTAVDATPDFEYLLMADGEWYPTDLLCVDLDDDVFWPSDTSTQPQTMEPTDVPLVCDTVMEPQ/GNASSSDKSNSQSSG
TMEV/BeAn8386_[M16020]     MA-------CKHGYP-DVCPICTAVDATPGFEYLLMADGEWYPTDLLCVDLDDDVFWPSDTSNQSQTMDPTDVPLIRDIVMEPQ/GNSSSSDKSNSQSSG
TLV-NGS910                 MA-------CIHGYP-SVCPICTAIDKSSDGHYLLLADNEWYPADLLTHDLDDDVFWPNDESDVSETMDPTDLPPILDTIMEPQ/GNSTSSDKSNSQSSG
VHEV/Siberia-55_[M94869]   MA-------CKHGYP-DVCPICTAIDVTPGFEYLLLADGEWYPTDLLCVDLDDDVFWPSDSSNQSQTMEPTDIPLICDTVMEPQ/GNSTSSDKSNSQSSG

                                   110       120       130       140       150       160       170       180       190       200
                           ....|....|....|....|....|....|....|....|....|....|....|....|....|....|....|....|....|....|....|....|
SVV                        ----------------------------------------------------------------------------------------------------
EMCV-R                     NEGVIINNFYSNQYQNSIDLSAN-AAGSDPPRTYGQFSNLFSGAVNAFSNMLPLLA/DQNTEEMENLSDRVSQDTAGNTVTNTQSTVGRLVGYGTVHDGE
EMCV-PV21                  NEGVIINNFYSNQYQNSIDLSAN-AAGSDPPRTYGQFSNLFSGAVNAFSNMLPLLA/DQNTEEMENLSDRVSQDTAGNTVTNTQSTVGRLVGYGTVHDGE
EMCV-B                     NEGVIINNFYSNQYQNSIDLSAN-ATGSDPPRTYGQFSNLLSGAVNAFSNMIPLLA/DQNTEEMENLSDRVLQDTAGNTVTNTQSTVGRLVGYGAVHDGE
EMCV-Da                    NEGVIINNFYSNQYQNSIDLSAN-ATGSDPPRTYGQFSNLLSGAVNAFSNMIPLLA/DQNTEEMENLSDRVLQDTAGNTVTNTQSTVGRLVGYGAVHDGE
EMCV-Db                    NEGVIINNFYSNQYQNSIDLSAN-ATGSDPPRTYGQFSNLLSGAVNAFSNMIPLLA/DQNTEEMENLSDRVLQDTAGNTVTNTQSTVGRLVGYGAVHDGE
EMCV-PV2                   NEGVIINNFYSNQYQNSIDLSAN-ATGSDPPRTYGQFSNLLSGAVNAFSNMIPLLA/DQNTEEMENLSDRVLQDTAGNTVTNTQSTVGRLVGYGAVHDGE
EMCV-Mengo                 NEGVIINNFYSNQYQNSIDLSAN-ATGSDPPKTYGQFSNLLSGAVNAFSNMLPLLA/DQNTEEMENLSDRVSQDTAGNTVTNTQSTVGRLVGYGTVHDGE
TMEV/DA_[M20301]           NEGVIINNFYSNQYQNSIDLSASGGNAGDAPQNNGQLSNILGGAANAFATMAPLLL/DQNTEEMENLSDRVASDKAGNSATNTQSTVGRLCGYGEAHHGE
TMEV/GDVII_[M20562]        NEGVIINNFYSNQYQNSIDLSASGGNAGDAPQNNGQLSSILGGAANAFATMAPLLM/DQNTEEMENLSDRVASDKAGNSATNTQSTVGRLCGYGKSHHGE
TMEV/BeAn8386_[M16020]     NEGVIINNFYSNQYQNSIDLSASGGNAGDAPQTNGQLSNILGGAANAFATMAPLLL/DQNTEEMENLSDRVASDKAGNSATNTQSTVGRLCGYGKSHHGE
TLV-NGS910                 NEGVIINNFYSNQYQNSIDLSANGGNAGSAPKTEGQLSNILGNAANAFSTMAPLLL/DQNTEEMENLSDRVDSDKAGNSAVNTQSSVGRLCGYGNSHKGK
VHEV/Siberia-55_[M94869]   NEGVIINNFYSNQYQNSIDLSANGGNAGDGPKTEGQLSNILGGAANAFATMAPLLL/DENTEEMENLSDRVDSDKAGNSATNTQSSVGRLHGYGATHRGD

                                   210       220       230       240       250       260       270       280       290       300
                           ....|....|....|....|....|....|....|....|....|....|....|....|....|....|....|....|....|....|....|....|
SVV                        -----------------------------------------------------------------------------------LAEHGNKKSLQELNEEQ
EMCV-R                     HPASCADTASEKILAVERYYTFKVNDWTSTQKPFEYIRIPLPHVLSGEDGGVFGAALRRHYLVKTGWRVQVQCNASQFHAGSLLVFMAPEYPT-------
EMCV-PV21                  HPASCADTASEKILAVERYYTFKVNDWTSTQKPFEYIRIPLPHVLSGEDGGVFGAALRRHYLVKTGWRVQVQCNASQFHAGSLLVFMAPEYPT-------
EMCV-B                     HPASCADTASEKILAVERYYTFKVNDWTSTQKPFEYIRIPLPHVLSGEDGGVFGAALRRHYLVKTGWRVQVQCNASQFHAGSLLVFMAPEYPT-------
EMCV-Da                    HPASCADTASEKILAVERYYTFKVNDWTSTQKPFEYIRIPLPHVLSGEDGGVFGAALRRHYLVKTGWRVQVQCNASQFHAGSLLVFMAPEYPT-------
EMCV-Db                    HPASCADTASEKILAVERYYTFKVNDWTSTQKPFEYIRIPLPHVLSGEDGGVFGAALRRHYLVKTGWRVQVQCNASQFHAGSLLVFMAPEYPT-------
EMCV-PV2                   HPASCADTASEKILAVERYYTFKVNDWTSTQKPFEYIRIPLPHVLSGEDGGVFGAALRRHYLVKTGWRVQVQCNASQFHAGSLLVFMAPEYPT-------
EMCV-Mengo                 HPASCADTASEKILAVERYYTFKVNDWTSTQK-FEYIRIPLPHVLSGEDGGVFGATLRRHYLVKTGWRVQVQCNASQFHAGSLLVFMAPEYPT-------
TMEV/DA_[M20301]           HPASCADTATDKVLAAERYYTIDLASWTTTQEAFSHIRIPLPHVLAGEDGGVFGATLRRHYLCKTGWRVQVQCNASQFHAGSLLVFMAPEFYTGKGTKTG
TMEV/GDVII_[M20562]        HPTSCADAATDKVLAAERYYTIDLASWTTSQEAFSHIRIPLPHVLAGEDGGVFGATLRRHYLCKTGWRVQVQCNASQFHAGSLLVFMAPEFYTGKGTKSG
TMEV/BeAn8386_[M16020]     HPASCADTATDKVLAAERYYTIDLASWTTSQEAFSHIRIPLPHVLAGEDGGVFGATLRRHYLCKTGWRVQVQCNASQFHAGSLLVFMAPEFYTGKGTKTG
TLV-NGS910                 HPASCADTATDKVLSAERYYTIDLATWTTTLGTFSHIRIPLPHVLAGEDGGVFGSTLRRHYLCKCGWRIQVQCNASQFHAGSLLVFMAPEFYTGHTAVTG
VHEV/Siberia-55_[M94869]   HPASCADTATDKVLAAERYYTIDLATWTTAQTTFSHIRVPLPHALAGEHGGVFGATLRRHYLAKCGWRVQVQCNASQFHAGSLLVFLAPEFYTGTGVATG
```

# Fig. 28A

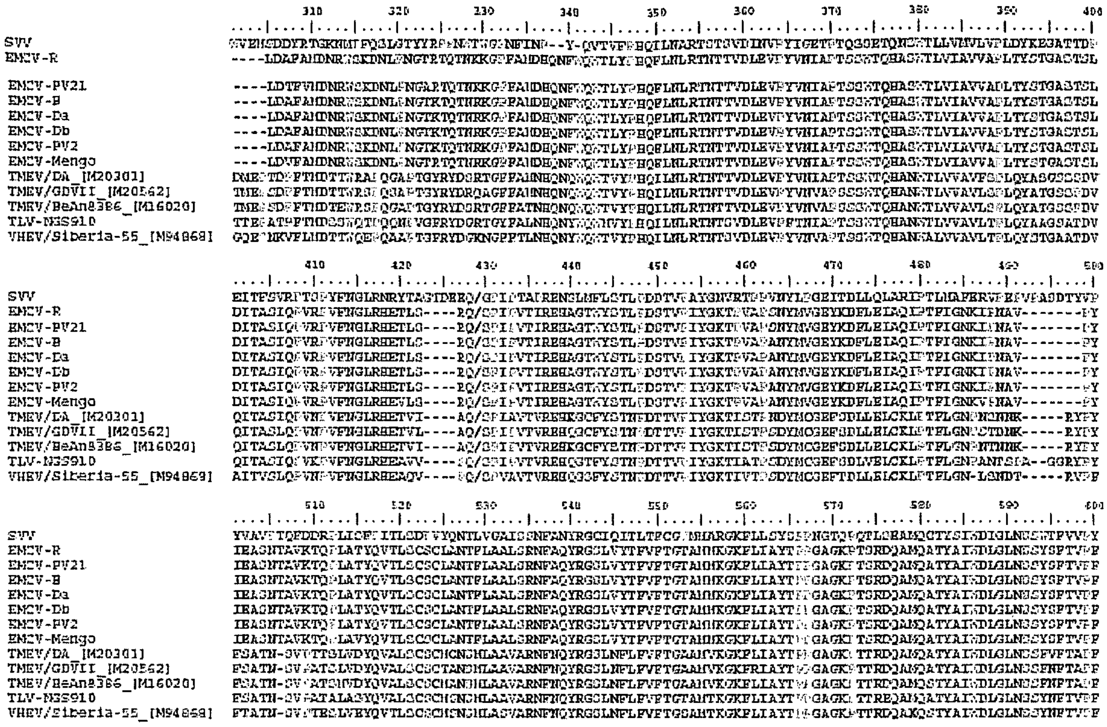
SVV
EMCV-R
EMCV-PV21
EMCV-B
EMCV-Da
EMCV-Db
EMCV-PV2
EMCV-Mengo
TMEV/DA_[M20301]
TMEV/GDVII_[M20562]
TMEV/BeAn8386_[M16020]
TLV-NGS910
VHEV/Siberia-55_[M94868]

Fig. 28B

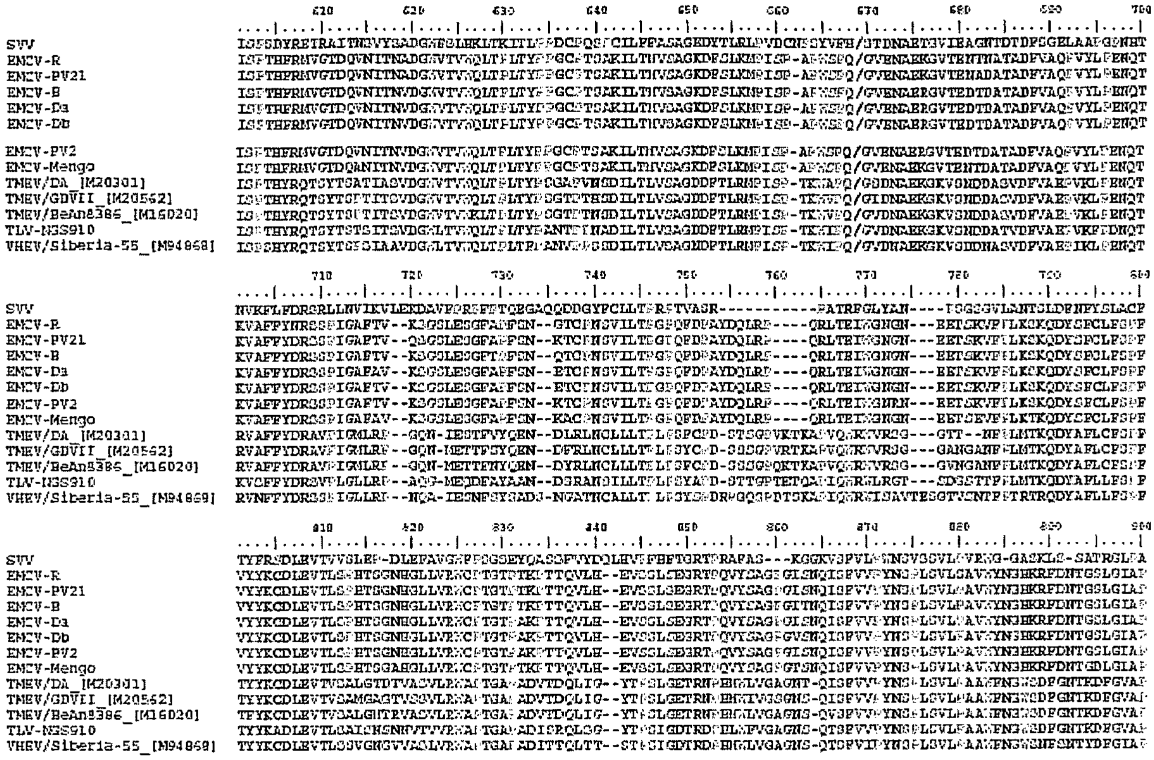
610 620 630 640 650 660 670 680 690 700
SVV
EMCV-R
EMCV-PV21
EMCV-B
EMCV-Da
EMCV-Db
EMCV-PV2
EMCV-Mengo
TMEV/DA_[M20301]
TMEV/GDVII_[M20562]
TMEV/BeAn8386_[M16020]
TLV-NGS910
VHEV/Siberia-55_[M94868]
710 720 730 740 750 760 770 780 790 800
810 820 830 840 850 860 870 880 890 900

# Fig. 28C

```
                                  910       920       930       940       950       960       970       980       990       1000
                          ....|....|....|....|....|....|....|....|....|....|....|....|....|....|....|....|....|....|....|....|
SVV                       HADSGTIYAFVFRFNEKKSTAVKHVAVYIRYKNARAKCPSHLPPRSYKQ-----------------/--------------------------------
EMCV-R                    NSDFGTLFFAGTKFD-------IKFTVYLRYKNKRVFCPRPTVFFPWPT-SGDKIDMTPRAGVLMLE/SPNALDISRTYPTLHVLIQFNHRGLEVRLFRH
EMCV-PV21                 NSDFGTLFFAGTKFD-------IKFTVYLRYKNMRVFCPRPTVFFPWPT-SGDKIDMTPRAGVLMLE/SPNALDISRTYPTLHVLIQFNHRGLEVRLFRH
EMCV-B                    NSDFGTLFFAGTKFD-------IKFTVYLRYKNMRVFCPRPTVFFPWPS-SGDKIDLTPRAGVLMLE/SPNALDISSTYPTLHILIQFNHGGLEIRLFRH
EMCV-Da                   NSDFGTLFFAGTKFD-------IKFTVYLRYKNMRVFCPRPTVFFPWPS-SGDKIDMTPRAGVLMLE/SPNALDISRTYPTLHILIQFNHGGLEIRLFRH
EMCV-Db                   NSDFGTLFFAGTKFD-------IKFTVYLRYKNMRVFCPRPTVFFPWPS-SGDKIDMTPRAGVLMLE/SPNALDISRTYPTLHILIQFNHGGLEIRLFRH
EMCV-PV2                  NSDFGTLFFAGTKFD-------IKFTVYLRYKNMRVFCPRPTVFFPWPS-SGDKIDMTPRAGVLMLE/SPNALDISRTYPTLHILIQFNHGGLEIRLFRH
EMCV-Mengo                NSDFGTLFFAGTKFD-------IKFTVYLRYKNMRVFCPRPTVFFPWPT-SGDKIDMTPRAGVLMLE/SPNPLDVSKTYPTLHILLQFNHRGLEARIFRH
TMEV/DA_[M20301]          HADFGRLWIQGNT----------SASVRIRYKKMKVFCPRPTLFFPWPTVSTRSKINADNPVPILELE/NPAAFYR------IDLFITFIDEFITFDYKVH
TMEV/GDVII_[M20562]       TSDFGRIWIQGNS----------SASVRIRYKKMKVFCPRPTLFFPWPTPTTTKINADNPVPILELE/NPASLYR------IDLFITFTDELITFDYKVH

TMEV/BeAn8386_[M16020]    HADFGRLWIQGNT----------SASVRIRYKKMKVFCPRPTLFFPWPTPTTTKINADNPVPILELE/NPAALYR------IDLFITFTDEFITFDYKVH
TLV-NGS910                HADFGRLWIQGNT----------SVAVRVRYKKMKVFCPRPTLFLPWPSTTTTRIHADNPVSVMELQ/NPFSFYR------VDLFITFTDELITFDYKVH
VHEV/Siberia-55_[M94868]  CSDFGRLWIQGNA----------PLAIRVRYKKMRVFCPRPTLFFPWPTPTTTKVNADNPVPILDLE/NPAA--------------------------

                                  1010      1020      1030      1040      1050      1060      1070      1080      1090      1100
                          ....|....|....|....|....|....|....|....|....|....|....|....|....|....|....|....|....|....|....|
SVV                       -------------------------------------------------------------------------------------------------KML
EMCV-R                    GHFWAETRADVILRSKTKQVSFLSNGNYPSHDSRAPWNPWKNTYQAVLRAEPCRVTMDIYYKRVRIPFRLPLVQKEWPVREENVFGLYRIFNAHYAGYFAD
EMCV-PV21                 GQFWAETRADVILRSKTKQVSFLSNGNYPSMDSRAPWNPWKNTYQAVLRAEPCRVTMDIYYKRVRIPFRLPLVQKEWRVREENVFGLYRIFNAHYAGYFAD
EMCV-B                    GMFWAEAHADVILRSRTKQISFLNNGSFPSHDARAPWNPWKNTYHAVLRAEPYRVTMDVYHKRIRIPFRLPLVQKEWNVREENVFGLYGIFNAHYAGYFAD
EMCV-Da                   GMFWAEAHADVILRSRTKQISFLNNGSFPSHDARAPWNPWKNTYHAVLRAEPYRVTMDVYHKRIRIPFRLPLVQKEWNVREENVFGLYGIFNAHYAGYFAD
EMCV-Db                   VQFWAEAHADVILRSRTKQISFLNNGSFPSHDARAPWNPWKNTYHAVLRAEPYRVTMDVYHKRIRIPFRLPLVQKEWNVREENVFGLYSIFNAHYAGYFAD
EMCV-PV2                  GQFWAEAHADVILRSRTKQISFLNNGSFPSHDARAPWNPWKNTYHAVLRAEPYRVTMDVYHKRIRIPFRLPLVQKEWNVREENVFGLYSIFNAHYAGYFAD
EMCV-Mengo                GQLWAETHAEVVLRSKTKQISFLSNGSYPSHDATTPLNPWKSTYQAVLRAEPHRVTMDVYHKRIRIPFRLPLVQKEWRTCEENVFGLYHVPETHYAGYFSD
TMEV/DA_[M20301]          GRFVLTFRIPGFGLTPAGRMLVCMG----EKPAHGPFTSSRSLYHVIFTATCSSFSFSIYKGRYRSWKKPIHDELVDRGYTTPGEFFRAVRAYHADYYKQ
TMEV/GDVII_[M20562]       GRFVLTFRIPGFGLTPAGRMLVCMG----AKPAHSPFTSSKSLYHVIFTSTCNSFSFTIYKGRYRSWKKPIHDELVDRGYTTPREFFKAVRGYHADYYKQ
TMEV/BeAn8386_[M16020]    GRPVLTFRLPGFGLTPAGRMLVCMG----EQPAHGPFTSSRSLYHVIFTATCSSFSFSIYKGRYRSWKKPIHDELVDRGYTTFGEFFKAVRGYHADYYRQ
TLV-NGS910                GRPVLQYQVPGLGLTCAGRMLVCMG----QHPNHAPFSTVRHLYHVVFTGSRHSFGVVIYYKRHRIPKKPLHEELHDYGFECFSDFFKHVREYHAAYYKQ
VHEV/Siberia-55_[M94868]  ----------------------------------------------------------------------------------------------------

                                  1110      1120      1130      1140      1150      1160      1170      1180      1190      1200
                          ....|....|....|....|....|....|....|....|....|....|....|....|....|....|....|....|....|....|....|
SVV                       HQSGDIETNPG/PASDNP--------------ILEFLEAEHDLVTLASLWHIVHSVQQTWRKYVKNDDFWPNLLSELVGE--------------------
EMCV-R                    LLIHDIETNPG/PFMFRPRKQ-VFQTQGAAVSSMAQTLLPNDLASKAMGSAFTALLDANEDAQKAMKIIKTLSSLSDAWENVKETLNNPEFWKQLLSRCV
EMCV-PV21                 LLIHDIETNPG/PFMFRPRKQ-VFQTQGAAVSSMAQTLLPNDLASKAMGSAFTALLDANEDARKAMKIIKTLSSLSDAWENVKETLNNPEFWKQLLSRCV
EMCV-B                    LLIHDIETNPG/PFMAKPKKQ-VFQTQGAAVSSMAQTLLPNDLASKVMGSAFTALLDANEDAQKAMRIIKTLSSLSDAWENVKETLNNPEFWKQLLSRCV
EMCV-Da                   LLIHDIETNPG/PFMAKPKKQ-VFQTQGAAVSSMAQTLLPNDLASKVMGSAFTALLDANEDAQKAMRIIKTLSSLSDAWENVKETLNNPEFWKQLLSRCV
EMCV-Db                   LLIHDIETNPG/PFMAKPKKQ-VFQTQGAAVSSMAQTLLPNDLASKVMGSAFTALLDANEDAQKAMRIIKTLSSLSDAWENVKETLNNPEFWKQLLSRCV
EMCV-PV2                  LLIHDIETNPG/PFMAKPKKQ-VFQTQGAAVSSMAQTLLPNDLASKVMGSAFTALLDANEDAQKAMRLIKTLSSLSDAWENVKETLNNPEFWKQLLSRCV
EMCV-Mengo                LLIHDVETNPG/PFTFKPRQRPVFQTQGAAVSSMAQTLLPNDLASKAMGSAFTALLDANEDAQKAMKIIKTLSSLSDAWENVKGTLNNPEFWKQLLSRCV
TMEV/DA_[M20301]          RLIHDVEMNPG/P------VQSVFQPQGAVLTKSLAPQAG-------IQNLLLRLLGIDGDCSEVSKAITVVTDLFAAWERAKTTLVSPEFWSKLILKTT
TMEV/GDVII_[M20562]       RLIHDVEMNPG/P------VQSVFQPQGAVLTKSLAPQAG-------IQNILLRLLGIEGDCSEVSKAITVVTDLVAAWEKAKTTLVSPEFWSELILETT
TMEV/BeAn8386_[M16020]    RLIHDVETNPG/P------VQSVFQPQGAVLTKSLAPQAG-------IQNLLLRLLGIDGDCSEVSKAITVVTDLVAAWEKAKTTLVSPEFWSKLILKTT
TLV-NGS910                RLMHDVETNPG/PP-----VQSVFRPQGGVLTKSQAPMSG-------IQNLFLRALGIDADHSEFTRAVTHITDLCNTWEKAKNTLVSPEFWTVLIMKTV
VHEV/Siberia-55_[M94868]  -----------/----------------------------------------------------------------------------------------

                                  1210      1220      1230      1240      1250      1260      1270      1280      1290      1300
                          ....|....|....|....|....|....|....|....|....|....|....|....|....|....|....|....|....|....|....|
SVV                       --GSVALAATLSNQASVKALLGLHFLSRGLNYTDFYSLLIEKCSSFFTVEPPPPPAENLMTKPSVKSKFRKLFKMQ/GPMDKVKDWNQIAAGLKNFQFVR
EMCV-R                    QLIAGMTIAVMHPDPLTLLCLGTLTAAEITSQTSLCEEIAAKFKTIFI-TPPP-------RFPTI-SLF----QQQ/SPL---KQVNDIFSLAKNLDWAV
EMCV-PV21                 QLIAGMTIAVMHPDPLTLLCLGTLTAAEITSQTSLCEEIAAKFKTIFI-TPPP-------RFPTI-SLF----QQQ/SPL---KQVNDPFSLAKNLDWAV
EMCV-B                    QLIAGMTIAVMHPDPLTLLCLGTLTAAEITSQTSLCEEIVAKFKKIFT-TPPP-------RFPTI-SLF----QQQ/SPL---KQVNDVFSLAKNLDWAV
EMCV-Da                   QLIAGMTIAVMHPDPLTLLCLGTLTAAEITSQTSLCEEIVAKFKKIFT-TPPP-------RF TI-SLF----QQQ/SPL---KQVNDVFSLAKNLDWAV
EMCV-Db                   QLIAGMTIAVMHPDPLTLLCLGTLTAAEITSQTSLCEEIVAKFKKIFT-TPPP-------RFPTI-SLF----QQQ/SPL---KQVNDVFSLAKNLDWAV
EMCV-PV2                  QLIAGMTIAVMHPDPLTLLCLGTLTAAEITSQTSLCEEIVAKFKKIFT-TPPP-------RFPTI-SLF----QQQ/SPL---KQVNDVFSLAKNLDWAV
EMCV-Mengo                QLIAGMTIAVMHPDPLTLLCLGVLTAAEITSQTSLCEEIAAKFKTIFT-TPPP-------RFPVI-SLF----QQQ/SPL---KQVNDVFSLAKNLDWAV
TMEV/DA_[M20301]          KFIAASVLYLHNPDFTTTVCLSLMTGVDLLTNDSVFDWLEKKLSSFFR-TPPP---------VCP-NVL----QPQ/GPL---READESFTPAKNIEWAM
TMEV/GDVII_[M20562]       KFIAASVLYLHNPDFTTTVCLSLMTGVDLLTNDSVFDWLESKLSSFFR-TPPP---------ACP-NVM----QPQ/GPL---READESFTPAKNIEWAT
TMEV/BeAn8386_[M16020]    KFIAASVLYLHNPDFTTTVCLSLMTGVDLLTNDSVFDWLEKKLSSFFR-TPPP---------ACP-NVM----QPQ/GPL---READESFTPAKNIEWAM
TLV-NGS910                KFIAASVLYLHNPDLTATICLSLMTGVDVLTNESIFNWLSNKLSKLFH-TPPP---------PTS-PLL----QAQ/SPL---READDSFNLAKNIEWAI
VHEV/Siberia-55_[M94868]  ------------------------------------------------------------------------/-----------------------
```

# Fig. 28D

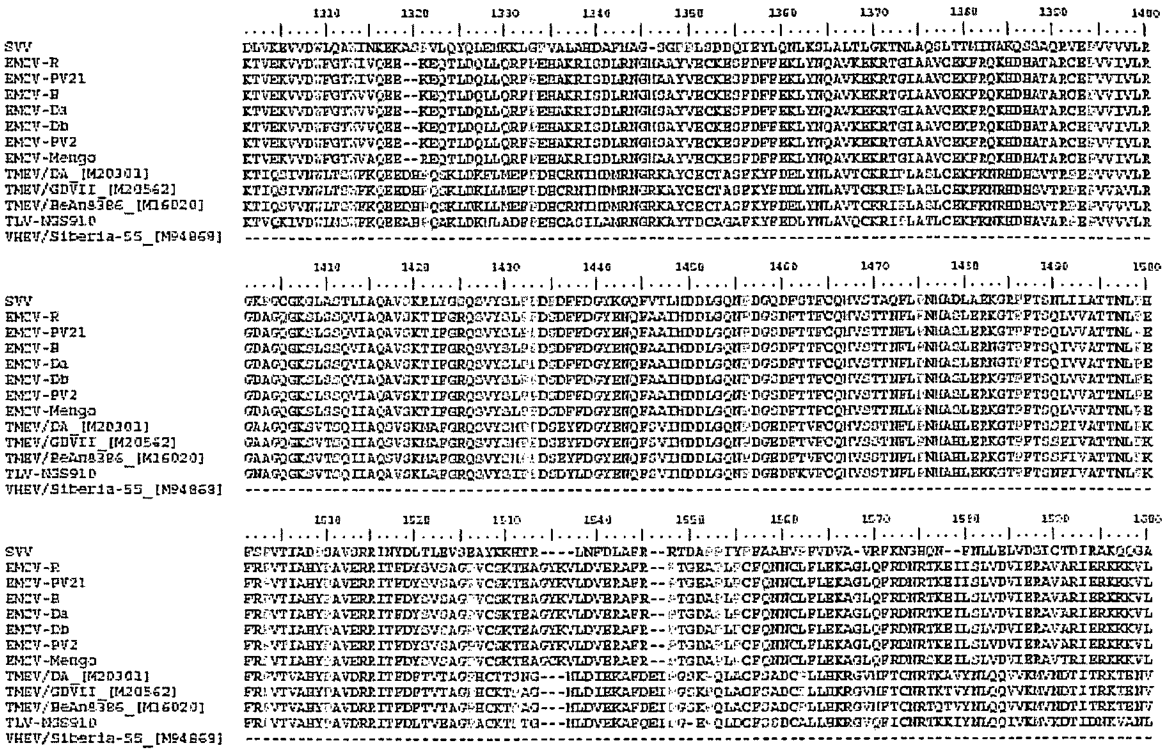
1310 1320 1330 1340 1350 1360 1370 1380 1390 1400
1410 1420 1430 1440 1450 1460 1470 1480 1490 1500
1510 1520 1530 1540 1550 1560 1570 1580 1590 1600
SVV
EMCV-R
EMCV-PV21
EMCV-B
EMCV-Da
EMCV-Db
EMCV-PV2
EMCV-Mengo
TMEV/DA_[M20301]
TMEV/GDVII_[M20562]
TMEV/BeAn8386_[M16020]
TLV-NJS910
VHEV/Siberia-55_[M94868]

Fig. 28E

```
                             1610      1620      1630      1640      1650      1660      1670      1680      1690      1700
                         ....|....|....|....|....|....|....|....|....|....|....|....|....|....|....|....|....|....|....|....|
SVV                      RHMQTLVLQ/SPMENDDTPVDEALGRVLSPAAVDEALVDLTPEADPVGRLAILAKLGLALAAVTPGLIILAVGLYRYFSGSDADQEETESEGSVKAPRSE
EMCV-R                   TTVQTLVAQ/GPVDEVSPHSVVQQLKARQQATDEQLEELQEAFAKVQERNSVFSDWLKISAMLCAATLALSQVVK--MAKAVKQMVKPDLVRVQLDEQEQ
EMCV-PV21                TTVQTLVAQ/APVDEVSPHSVVQQLKARQEATDEQLEELQEAFAKVQERNSVFSDWLKISAMLCAATLALSQVVK--MAKAVKQMVKPDLVRVQLDEQEQ
EMCV-B                   TTVQTLVAQ/APVDEVSPHSVVQQLKARQEATDEQLEELQEAFAKTQERSSVFSDWMKISAMLCAATLALSQVVK--MAKTVKQMVRPDLVRVQLDEQEQ
EMCV-Da                  TTVQTLVAQ/APVDEVSPHSVVQQLKARQEATDEQLEELQEAFAKTQERSSVFSDWMKISAMLCAATLALSQVVK--MAKTVKQMVRPDLVRVQLDEQEQ
EMCV-Db                  TTVQTLVAQ/APVDEVSPHSVVQQLKARQEATDEQLEELQEAFAKTQERSSVFSDWMKISAMLCAATLALTQVVK--MAKTVKQMVRPDLVRVQLDEQEQ
EMCV-PV2                 TTVQTLVAQ/APVDEVSPHSVVQQLKARQEATDEQLEELQEAFAKTQERSSVFSDWMKISAMLCAATLALTQVVK--MAKTVKQMVRPDLVRVQLDEQEQ
EMCV-Mengo               TAVQTLVAQ/GPVDEVSPYSVVQQLKARQEATDEQLEELQEAFARVQERSSVFSDWMKISAMLCAATLALTQVVK--MAKAVKQMVRPDLVRVQLDEQEQ
TMEV/DA_[M20301]         KKMNSLVAQ/SPPDWEEHFENILTCLRQNNAALQDQLDELQEAFAQARERSDFLSDWLKVSAIIFAGIASLSAVIK--LASKFKESIWPSPVRVELSEGEQ
TMEV/GDVII_[M20562]      KKMNSLVAQ/SPPDWQHFENILTCLRQNNAALQDQVDELQEAFTQARERSDFLSDWLKVSAIIFAGIVSLSAVIK--LASKFKESIWPTPVRVELSEGEQ
TMEV/BeAn8386_[M16020]   KKMNSLVAQ/SPPDWEEHFENILTCLRQNNAALQDQLDELQEAFAQARERSDFLSDWLKVSAIIFAGIASLSAVIK--LASKFKESIWPTPVRVELSEGEQ
TLV-NGS910               KKMNTLVAQ/SPNNGNDMEEIITCLRQNNAALQDQIDELQEAFAQAQERQNFLSDWMKVSAIIFAGIASLSAVCK--LVGRLKNLIWPSPVHVELSEGEQ
VHEV/Siberia-55_[M94868] ---------/------------------------------------------------------------------------------------------

                             1710      1720      1730      1740      1750      1760      1770      1780      1790      1800
                         ....|....|....|....|....|....|....|....|....|....|....|....|....|....|....|....|....|....|....|....|
SVV                      /NAYDGPKKNSKPPGALSIMEMQ/GP--------NVDMGFEAAVAKKVVKPITFMVPNRPSGLTQSALLVTGRTFLINEHTWSNPSWTSFTIRGEVHTRD
EMCV-R                   /GPYNETARVKP--KTLQLLDIQ/GP--------NPVMDFEKYVAKHVTAPIGFVYP--TGVSTQTCLLVRGRTLVVNRHMAES-DWTSIVVRGVTHARS
EMCV-PV21                /GPYNETARAKP--KTLQLLDIQ/GP--------NPVMDFEKYVAKHVTAPIDFVYP--TGVSTQTCLLVRGRTLAVNRHMAES-DWTSIVVRGVTHARS
EMCV-B                   /GPYNEAVRAKP--KTLQLLDIQ/GP--------NPVMDFEKYVAKFVTAPIDFVYP--TGVSTQTCLLVKGRTLAVNRHMAES-DWSSIVVRGVTHARS
EMCV-Da                  /GPYNEAVRAKP--KTLQLLDIQ/GP--------NPVMDFEKYVAKFVTAPIDFVYP--TGVSTQTCLLVKGRTLAVNRHMAES-DWSSIVVRGVTHARS
EMCV-Db                  /GPYNEAVRAKP--KTLQLLDIQ/GP--------NPVMDFEKYVAKFVTAPIDFVYP--TGVSTQTCLLVKGRTLAVNRHMAES-DWSSIVVRGVTHARS
EMCV-PV2                 /GPYNEAVRAKP--KTLQLLDIQ/GP--------NPVMDFEKYVAKFVTAPIDFVYP--TGVSTQTCLLVKGRTLAVNRHMAES-DWSSIVVRGVTHARS
EMCV-Mengo               /GPYNETTRIKP--KTLQLLDVQ/GP--------NPTMDFEKFVAKFVTAPIGFVYP--TGVSTQTCLLVKGRTLAVNRHMAES-DWTSIVVRGVSHTRS
TMEV/DA_[M20301]         /AAYAGRARAQK--QALQVLDIQ/GGGKVLAQAGNPVMDFELFCAKNMVAPITFYYPD-KAEVTQSCLLLRAHLFVVNRHVAET-EWTAFKLKDVRHERD
TMEV/GDVII_[M20562]      /AAYAGRARAQK--QALQVLDIQ/GGGKVLAQAGNPVMDFELFCAKNMVSPITFYYPD-KAEVTQSCLLLRAHLFVVNRHVAET-EWTAFKLRDVRHERD
TMEV/BeAn8386_[M16020]   /AAYAGRARAQK--QALQVLDIQ/GGGKVLAQAGNPVMDFELFCAKNIVAPITFYYPD-KAEVTQSCLLLRAHLFVVNRHVAET-DWTAFKLKDVRHERH
TLV-NGS910               /AAYAGAKRGAK--QALQVLDLQ/GGGRIIAQAGNPVMDYEVCVAKNMVAPITFYYAD-KAQVTQSCLLVKGRLFVVNRHVAET-DWVSFELRDVRHERD
VHEV/Siberia-55_[M94868] ----------------------/-----------------------------------------------------------------------------

                             1810      1820      1830      1840      1850      1860      1870      1880      1890      1900
                         ....|....|....|....|....|....|....|....|....|....|....|....|....|....|....|....|....|....|....|....|
SVV                      EPFQTVHFTHEGIPTDLMMVRLGPGNSFPNMLDKFG--LDQMPARNSRVVGVSSSYGNFFFSGNFLGFVDSVTSEQG-TYARLFRYRVTTYKGWCGSALV
EMCV-R                   -TVKILAIAKAGKETDVSFIRLSSGPLFRDNTSKFVKAGDVLPTGAAPVTGIMNTDIPMMYTGTFLKAGVSVPVETGQTFNHCIHYKANTRKGWCGSALL
EMCV-PV21                -TVKILAIAKAGKETDVSFIRLSSGPLFRDNTSKFVKAGDVLPTGAAPVTGIMNTDIPMMYTGTFLKAGVSVPVETGQTFNHCIHYKANTRKGWCGSALL
EMCV-B                   -TVRILAIAKAGKETDVSFIRLSSGPLFRDNTSKFVKADDVLPATSAPVIGIMNTDIPMMFTGTFLKAGVSVPVETGQTFNHCIHYKANTRKGWCGSALL
EMCV-Da                  -TVRILAIAKAGKETDVSFIRLSSGPLFRDNTSKFVKADDVLPATSAPVIGIMNTDIPMMFTGTFLKAGVSVPVETGQTFNHCIHYKANTRKGWCGSALL
EMCV-Db                  -TVRILAIAKAGKETDVSFIRLSSGPLFRDNTSKFVKADDVLPATSAPVIGIMNTDIPMMFTGTFLKAGVSVPVETGQTFNHCIHYKANTRKGWCGSALL
EMCV-PV2                 -TVRILAIAKAGKETDVSFIRLSSGPLFRDNTSKFVKADDVLPATSAPVIGIMNTDIPMMFTGTFLKAGVSVPVETGQTFNHCIHYKANTRKGWCGSALL
EMCV-Mengo               -SVKIIAIAKAGKETDVSFIRLSSGPLFRDNTSKFVKADDVLPHSGSPLIGIMNVDIPMMYTGTFLKAGVSVPVETGQTFNHCIHYKANTRKGWCGSAIL
TMEV/DA_[M20301]         -TVVTRSVNRSGAETDLTFIKVTKGPLFKDNVNKFCSNKDDFPARNDAVTGIMNTGLAFVYSGNFLIGNQPVNITTGACPNHCLHYRAQTRRGWCGSAVI
TMEV/GDVII_[M20562]      -TVVMRSVNRSGAETDLTFVKVTKGPLFKDNVNKFCSNKDDFPARNDTVTGIMNTGLAFVYSGNFLIGNQPVNITTGACPNHCLHYRAQTRRGWCGSAII
TMEV/BeAn8386_[M16020]   -TVALRSVNRSGAKTDLTFIKVTKGPLFKDNVNKFCSNKDDFPARNDTVTGIMNTGLAFVYSGNFLIGNQPVNITTGACPNHCLHYRAQTRRGWCGSAII
TLV-NGS910               -TVTMRSVNRSGMEVDLTFIKVTKGPLFKDNTKKFCSNKDDFPQKNETVTGIMNTGLPFVFNGKFIIGNHPVNTTTGATPNHCLHYRANTRRGWCGSAVI
VHEV/Siberia-55_[M94868] ----------------------------------------------------------------------------------------------------
```

# Fig. 28F

```
                                   1910      1920      1930      1940      1950      1960      1970      1980      1990      2000
                               ....|....|....|....|....|....|....|....|....|....|....|....|....|....|....|....|....|....|....|....|
SVV                            CEAGGVRRIIGLHSAGAAGIGAGTYIGKLGLIKALKHLGEPLATMQ/GLMTELEPGITVHVPRKSKLRKTTAHAVYKPEPEPAVLSKFDPRLNKDVDLDE
EMCV-R                         ADLGGPKKILGIHSAGSMGIAAASIVSQEMIRAVVN-----AFEPQ/GALERLPDGPRIHVPRKTALRPTVARQVFQPAYAPAVLSKFDPRT--EADVDE
EMCV-PV21                      ADLGGPKKILGIHSAGSMGIAAASIVSQEMIRAVVN-----AFEPQ/GALERLPDGPRIHVPRKTALRPTVARQVFQPAYAPAVLSKFDPRT--EADVDE
EMCV-B                         ADLGGRKKILGMHSAGSMGRTAASIVSQEMICAVVS-----AFEPQ/GALERLPDGPRIHVPRKTALRPTVARRVFQPAYAPAVLSKFDPRT--EADVDE
EMCV-Da                        ADLGGRKKILGMHSAGSMGRTAASIVSQEMICAVVS-----AFEPQ/GALERLPDGPRIHVPRKTALRPTVARRVFQPAYAPAVLSKFDPRT--EADVDE
EMCV-Db                        ADLGGRKKILGMHSAGSMGVAAASIVSQEMICAVVS-----AFEPQ/GALERLPDGPRIHVPRKTALRPTVARQVFQPAYAPAVLSKFDPRT--EADVDE

EMCV-PV2                       ADLGGRKKILGMHSAGSMGRTAASIVSQEMICAVVS-----AFEPQ/GALERLPDGPRIHVPRKTALRPTVARQVFQPAYAPAVLSKFDPRT--EADVDE
EMCV-Mengo                     ADLGGPKKILGFHSAGSMGVAAASIISQEMIDAVVQ-----AFEPQ/GALERLPDGPRIHVPRKTALRPTVARQVFQPAFAPAVLSKFDPRT--DADVDE
TMEV/DA_[M20301]               CNVNGKKAVYGMHSAGGGGLAAATIITRELIEAAEKSH--LALEPQ/GAIVDISTGSVVHVPRKTKLRRTVAHDVFQPKPEPAVLSRYDPRT--DKDVDV
TMEV/GDVII_[M20562]            CNVNGKKAVYGMHSAGGGGLAAATIITRELIEAAEKSH--LALEPQ/GAIVDISTGSVVHVPRKTKLRRTVAEDVFQPKPEPAVLSRYDPRT--DKDVDV
TMEV/BeAn8386_[M16020]         CNVNGKKAVYGMHSAGGGGLAAATIITKELIEAAEKSH--LALEPQ/GAIVDIATGSVVHVPRKTKLRRTVAHDVFQPKPEPAVLSRYDPRT--DKDVDV
TLV-NGS910                     CQVNGKKAVYGMHSAGGGGLAAATIITQELVEAAEQNH--DRLVPQ/GAIMEIGTGSVVHVPRKTKLRRTVAHEIFLPKPEPAVLSRYDPRT--EKDVDQ
VHEV/Siberia-55_[M94868]       ----------------------------------------------/-----------------------------------------------------

                                   2010      2020      2030      2040      2050      2060      2070      2080      2090      2100
                               ....|....|....|....|....|....|....|....|....|....|....|....|....|....|....|....|....|....|....|....|
SVV                            VIESKHTANPVYQPDLFYTYMSEYAHRVFSFLGKDNDILTVKEAILGIPGLDPMDPHTAPGLPYAINGLRRTDLVDPVNGTVDAALAVQIQKFLDGDYSD
EMCV-R                         VAFSKHTSNQESLPPVFRHVAKEYANRVFTLLGKDNGRLTVKQALEGLEGMDPMDRNTSPGLPYTALGMRRTDVVDWESATLIPFAAERLPKMNEGDFSE
EMCV-PV21                      VAFSKHTSNQESLPPVFRHVAKEYANRVFTLLGKDNGRLTVKQALEGLEGMDPMDRNTSPGLPYTALGMRRTDVVDWESATLIPFAAERLPKMNEGDFSE
EMCV-B                         VAFSKHTSNQESLPPVFRHVAKEYANRVFTLLGRDNGRLTVKQALEGLEGMDPMDKNTSPGLPYTALGMRRTDVVDWESATLIPYAADRLKKMNEGDFSD
EMCV-Da                        VAFSKHTSNQESLPPVFRHVAKEYANRVFTLLGRDNGRLTVKQALEGLEGMDPMDKNTSPGLPYTALGMRRTDVVDWESATLIPYAADRLKKMNEGDFSD
EMCV-Db                        VAFSKHTSNQESLPPVFRHVAKEYANRVFTLLGRDNGRLTVKQALEGLEGMDPMDKNTSPGLPYTALGMRRTDVVDWESATLIPYAADRLKKMNEGDFSD
EMCV-PV2                       VAFSKHTSNQESLPPVFRHVAKEYANRVFTLLGRDNGRLTVKQALEGLEGMDPMDKNTSPGLPYTALGMRRTDVVDWESATLIPYAADRLKKMNEGDFSD
EMCV-Mengo                     VAFSKHTSNQETLPPVFRHVARRYANRVFALLGRDNGRLSVKQALDGLEGMDPMDKNTSPGLPYTTLGMRRTDVVDWETATLIPFAAERLEKMNNKDFSD
TMEV/DA_[M20301]               VAFSKHTTNHESLPPVFDIVCDEYANRVFTILGKDNGLLTVEQAVLGLPGMDPMEKDTSPGLPYTQQGLRRTDLLNFNTAKMTPQLDYAHSKLVLGVYDD
TMEV/GDVII_[M20562]            VAFSKHTTNHESLPPIFDIVCGEYANRVFTILGKDNGLLTVEQAVLGLSGMDPMEKDTSPGLTYTQQGLRRTDLLDFNTAKMTPQLDYAHSKLVLGVYDD
TMEV/BeAn8386_[M16020]         VAFSKHTTNHESLPPIFDVVCGEYANRVFTILGKENGLLTVEQAVLGLPGMDPMEKDTSPGLPYTQQGLRRTDLLNFITAKMTPQLDYAHSKLVIGVYDD
TLV-NGS910                     VAFSKHTTNHEPLPPAVPSHVAKEYANRVFTKLGKENQLLTTQQAILGLPGMDPMEKDTSPGLPYTQQGLRRTDLVNFETGKMDPNLDYAHSKLMLGEYED
VHEV/Siberia-55_[M94868]       ----------------------------------------------------------------------------------------------------

                                   2110      2120      2130      2140      2150      2160      2170      2180      2190      2200
                               ....|....|....|....|....|....|....|....|....|....|....|....|....|....|....|....|....|....|....|....|
SVV                            HVFQTFLKDEIRPSEKVRAGKTRIVDVPGLAHCIVGRMLLGRFAAKFQSHPGPLLGSAIGSDPDVEWTVIGAQLEGRKNTYIDVDYSAFDSSHGTGSFEAL
EMCV-R                         VVYQTFLKDELRPIEKVQAAKTRIVDVPPFEHCILGRQLLGKFASKFQTQPGLELGSAIGCDPDVHWTAFGVAMQGFERVYDVDYSNFDSTHSVAMFRLL
EMCV-PV21                      VVYQTFLKDELRPIEKVQAAKTRIVDVPPFEHCILGRQLLGKFASKFQTQPGLELGSAIGCDPDVHWTAFGVAMQGFERVYDVDYSNFDSTHSVAMFRLL
EMCV-B                         IVYQTFLKDELRPVEKVQAAKTRIVDVPPFEHCILGRQLLGRFASKFQTQPGLELGSAIGCDPDVHWTAFGVAMQGFERVYDVDYSNFDSTHSVAMFRLL
EMCV-Da                        IVYQTFLKDELRPVEKVQAAKTRIVDVPPFEHCILGRQLLGRFASKFQTQPGLELGSAIGCDPDVHWTAFGVAMQGFERVYDVDYSNFDSTHSVAMFRLL
EMCV-Db                        IVYQTFLKDELRPVEKVQAAKTRIVDVPPFEHCILGRQLLGRFASKFQTQPGLELGSAIGCDPDVHWTAFGVAMQGFERVYDVDYSNFDSTHSVAMFRLL
EMCV-PV2                       IVYQTFLKDELRPVEKVQAAKTRIVDVPPFEHCILGRQLLGRFASKFQTQPGLELGSAIGCDPDVHWTAFGVAMQGFERVYDVDYSNFDSTHSVAMFRLL
EMCV-Mengo                     IVYQTFLKDELRPIEKVQAAKTRIVDVPPFEHCILGRQLLGKFASKFQTQPGLELGSAIGCDPDVHWTAFGVAMQGFERVYDVDYSNFDSTHSVAIFRLL
TMEV/DA_[M20301]               VVYQSFLKDEIRPLEKIHEAKTRIVDVPPFAHCIMGRQLLGRFASKFQTKPGLELGSAIGTDPDVDWTPYAAELSGFNYVYDVDYSNFDASHSTAMFECL
TMEV/GDVII_[M20562]            VVYQSFLKDEIRPLEKIHEAKTRIVDVPPFAHCIMGRQLLGRFASKFQTKPGFELGSAIGTDPDVDWTRYAAELSGFNYVYDVDYSNFDASHSTAMFECL
TMEV/BeAn8386_[M16020]         VVYQSFLKDEIRPIEKIHEAKTRIVDVPPFAHCIMGRQLLGRFASKFQTKPGLELGSAIGTDPDVDWTRYAVELSGFNYVYDVDYSNFDASHSTAMFECL
TLV-NGS910                     VVYQSFLKDEIRPIEKIHEAKTRIVDVPPFHHCIMGRQLLGRFASRFQTNPGLDLGSAIGTDPDVDWTVFAHQLAEFKYIYDVDYSMFDASHSTAIFEIL
VHEV/Siberia-55_[M94868]       ----------------------------------------------------------------------------------------------------
```

# Fig. 28G

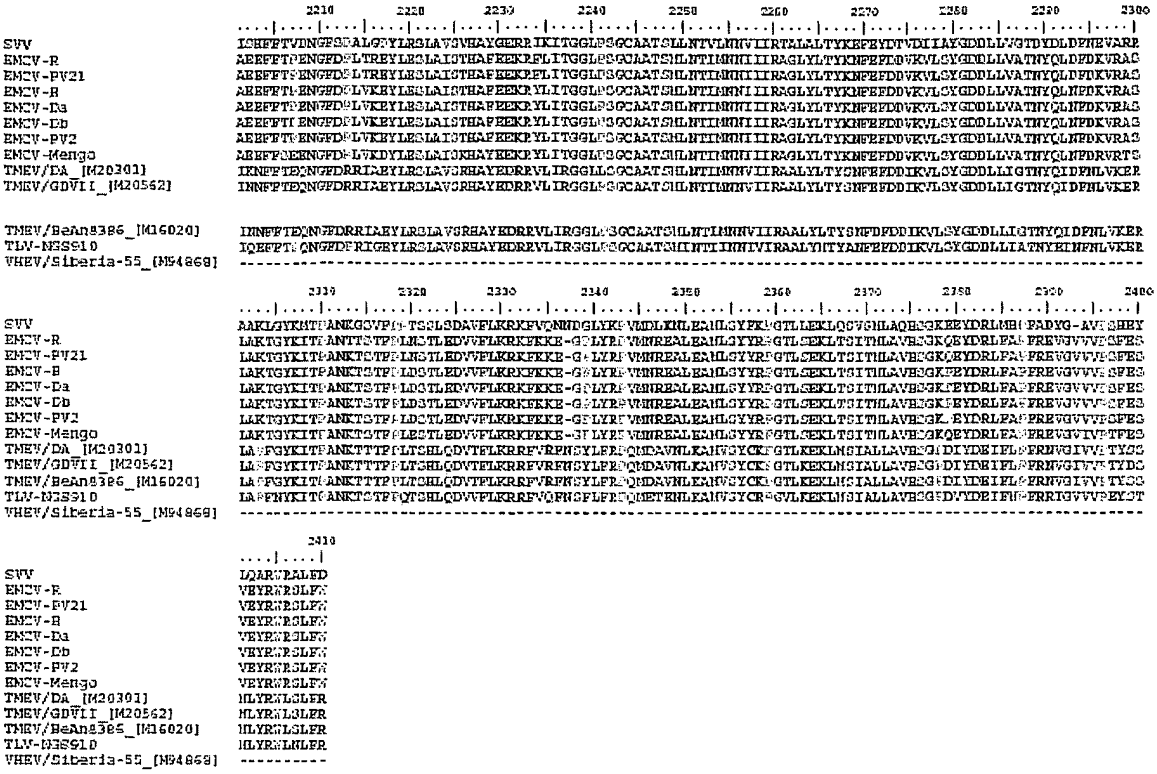
SVV
EMCV-R
EMCV-PV21
EMCV-B
EMCV-Da
EMCV-Db
EMCV-PV2
EMCV-Mengo
TMEV/DA_[M20301]
TMEV/GDVII_[M20562]
TMEV/BeAn8386_[M16020]
TLV-NGS910
VHEV/Siberia-55_[M94868]

Fig. 28H

| Polypeptide | aa | Predicted sequence |
|---|---|---|
| L Leader | ? | No data |
| VP4 (1A) | ? | No data |
| VP2 (1B) | >142 | LAHHGNKKSLQELNEEQWVEMSDDYRTGKNMPFQSLGTYYRPPNWTWGPN<br>FINPYQVTVFPHQILNARTSTSVDINVPYIGETPTQSSETQNSWTLLVMV<br>LVPLDYKEGATTDPEITFSVRPTSPYFNGLRNRYTAGTDEEQ |
| VP3 (1C) | 239 | GPIPTAPRENSLMFLSTLPDDTVPAYGNVRTPPVNYLPGEITDLLQLARI<br>PTLMAFERVPEPVPASDTYVPYVAVPTQFDDRPLISFPITLSDPVYQNTL<br>VGAISSNFANYRGCIQITLTFCGPMMARGKFLLSYSPPNGTQPQTLSEAM<br>QCTYSIWDIGLNSSWTFVVPYISPSDYRETRAITNSVYSADGWFSLHKLT<br>KITLPPDCPQSPCILFFASAGEDYTLRLPVDCNPSYVFH |
| VP1 (1D) | 259 | STDNAETGVIEAGNTDTDFSGELAAPGPNHTNVKFLFDRSRLLNVIKVLE<br>KDAVFPRPFPTQEGAQQDDGYFCLLTPRPTVASRPATRFGLYANPSGSGV<br>LANTSLDFNFYSLACFTYFRSDLEVTVVSLEPDLEFAVGWFPSGSEYQAS<br>SFVYDQLHVPFHFTGRTPRAFASKGGKVSFVLPWNSVSSVLPVRWGGASK<br>LSSATRGLPAHADWGTIYAFVPRPNEKKSTAVKHVAVYIRYKNARAWCPS<br>MLPFRSYKQ |
| 2A | 14 | KMLMQSGDIET**NPG** |
| 2B | 128 | **P**ASDNPILEFLEAENDLVTLASLWKMVHSVQQTWRKYVKNDDFWPNLLSE<br>LVGEGSVALAATLSNQASVKALLGLHFLSRGLNYTDFYSLLIEKCSSFFT<br>VEPPPPPAENLMTKPSVKSKFRKLFKMQ |
| 2C | 322 | GPMDKVKDWNQIAAGLKNFQFVRDLVKEVVDWLQAWINKEKASPVLQYQL<br>EMKKLGPVALAHDAFMAGSGPPLSDDQIEYLQNLKSLALTLGKTNLAQSL<br>TTMINAKQSSAQRVEPVVVVLR**G**KP**G**C**GKG**LASTLIAQAVSKRLYGSQSV<br>YSLPPDPDFFDGYKGQF**VTLMDD**LGQNPDGQDFSTFCQMVSTAQFLPNMA<br>DLAEKGRPFTSNLIIATTNLPHFSPVTIADPSAVSRRINYDLTLEVSEAY<br>KKHTRLNFDLAFRRTDAPPIYPFAAHVPFVDVAVRFKNGHQNFNLLELVD<br>SICTDIRAKQQGARNMQTLVLQ |
| 3A | 90 | SPNENDDTPVDEALGRVLSPAAVDEALVDLTPEADPVGRLAILAKLGLAL<br>AAVTPGLIILAVGLYRYFSGSDADQEETESEGSVKAPRSE |
| 3B (VPg) | 22 | NA**Y**DGPKKNSKPPGALSLMEMQ |
| 3C (pro) | 211 | QPNVDMGFEAAVAKKVVVPITFMVPNRPSGLTQSALLVTGRTFLINE**H**TW<br>SNPSWTSFTIRGEVHTRDEPFQTVHFTHHGIPTDLMMVRLGPGNSFPNNL<br>DKFGLDQMPARNSRVVGVSSSYGNFFFSGNFLGFVDSVTSEQGTYARLFR<br>YRVTTYKGW**C**GSALVCEAGGVRRIIGL**H**SAGAAGIGAGTYISKLGLIKAL<br>KHLGEPLATMQ |
| 3D (pol) | 462 | GLMTELEPGITVHVPRKSKLRKTTAHAVYKPEFEPAVLSKFDPRLNKDVD<br>LDEVIWSKHTANVPYQPPLFYTYMSEYAHRVFSFLGKDNDILTVKEAILG<br>IPGLDPMDPHTAPGLPYAINGLRRTDLVDFVNGTVDAALAVQIQKFLDGD<br>YSDHVFQTFL**KDEIR**PSEKVRAGKTRIVDVPSLAHCIVGRMLLGRFAAKF<br>QSHPGFLLGSAIGSDPDVFWTVIGAQLEGRKNTYDVDYSAFDSSHGTGSF<br>EALISHFFTVDNGFSPALGPYLRSLAVSVHAYGERRIKITGGL**PSG**CAAT<br>SLLNTVLNNVIIRTALALTYKEFEYDTVDIIA**YGDD**LLVGTDYDLDFNEV<br>ARRAAKLGYKMTPANKGSVFPPTSSLSDAV**FLKR**KFVQNNDGLYKPVMDL<br>KNLEAMLSYFKPGTLLEKLQSVSMLAQHSGKEEYDRLMHPFADYGAVPSH<br>EYLQARWRALFD |

# Fig. 29

| Genus | Species | Abbrev. | Serotype |
|---|---|---|---|
| *Enterovirus* | *Poliovirus* | PV-1 | = Poliovirus 1 |
| | *Human enterovirus A* | CV-A16 | = Coxsackievirus A16 |
| | *Human enterovirus B* | CV-B5 | = Coxsackievirus B5 |
| | *Human enterovirus C* | CV-A21 | = Coxsackievirus A21 |
| | *Human enterovirus D* | EV-70 | = Enterovirus 70 |
| | *Simian enterovirus A* | SEV-A | = Simian enterovirus A1 |
| | *Bovine enterovirus* | BEV-1 | = Bovine enterovirus 1 |
| | *Porcine enterovirus B* | PEV-9 | = Porcine enterovirus 9 |
| *Rhinovirus* | *Human rhinovirus A* | HRV-2 | = Human rhinovirus 2 |
| | *Human rhinovirus B* | HRV-14 | = Human rhinovirus 14 |
| New genus? | Not yet designated | SV2 | = Simian virus 2 |
| | *Porcine enterovirus A* | PEV-8 | = Porcine enterovirus 8 |
| *Cardiovirus* | *Encephalomyocarditis virus* | EMCV | = Encephalomyocarditis virus |
| | *Theilovirus* | TMEV | = Theiler's murine encephalomyelitis |
| | ? | virus | |
| *Aphthovirus* | *Foot-and-mouth disease virus* | SVV | = Seneca Valley virus |
| | *Equine rhinitis A virus* | FMDV-O | = Foot-and-mouth disease virus O |
| *Hepatovirus* | *Hepatitis A virus* | ERAV | = Equine rhinitis A virus |
| | Avian encephalomyelitis-like | HAV | = Hepatitis A virus |
| | viruses | AEV | = Avian encephalomyelitis virus |
| *Parechovirus* | *Human parechovirus* | HPeV-1 | = Human parechovirus 1 |
| | *Ljungan virus* | LV | = Ljungan virus |
| *Kobuvirus* | *Aichi virus* | AiV | = Aichi virus |
| | *Bovine kobuvirus* | BKV | = Bovine kobuvirus |
| *Erbovirus* | *Equine rhinitis B virus* | ERBV-1 | = Equine rhinitis B virus 1 |
| *Teschovirus* | *Porcine teschovirus* | PTV-1 | = Porcine teschovirus 1 |

Fig. 30

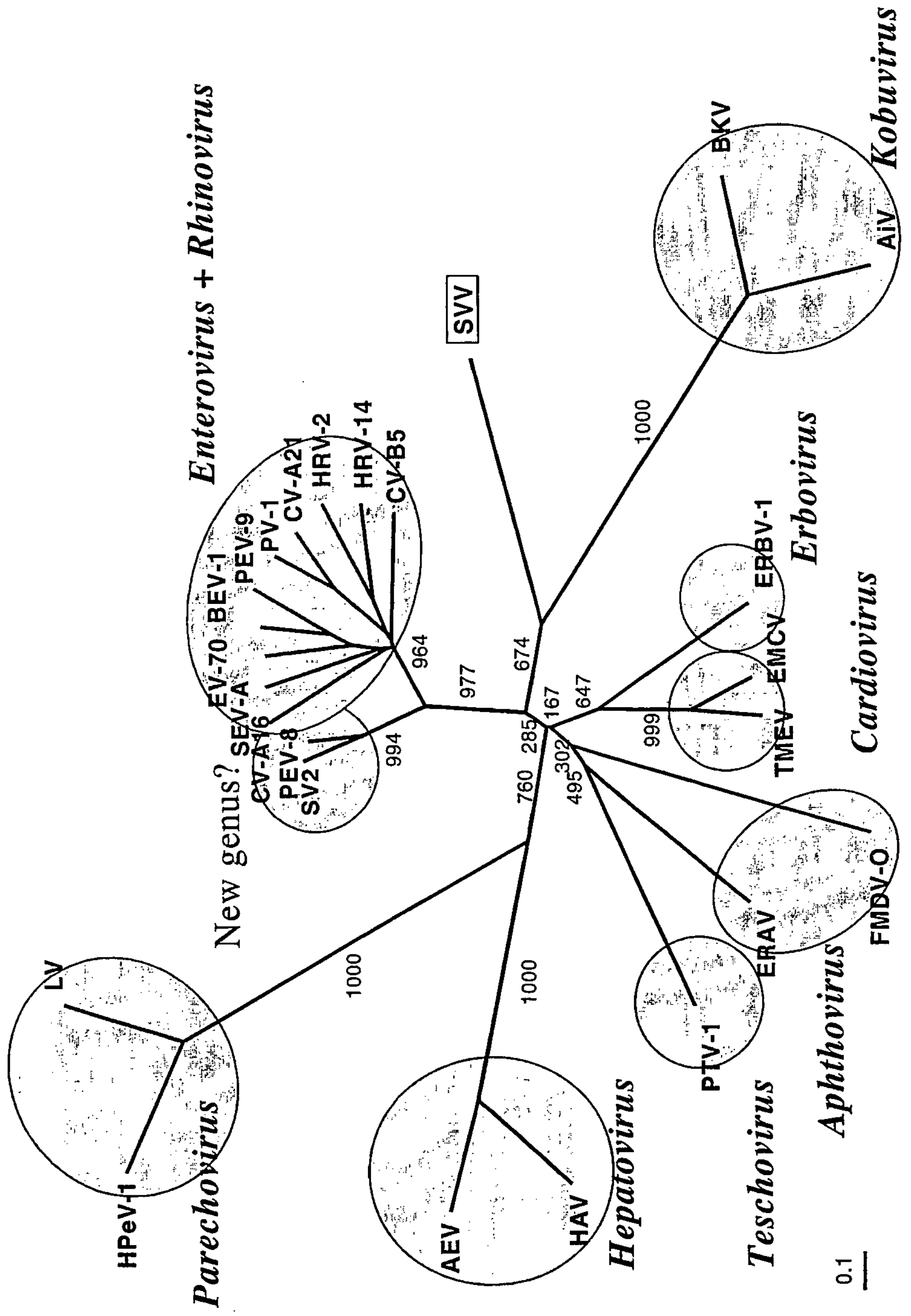
Enterovirus + Rhinovirus
EV-70
BEV-1
PEV-9
PV-1
CV-A21
HRV-2
HRV-14
CV-B5
SEV-A
CV-A16
PEV-8
SV2
New genus?
SVV
Kobuvirus
BKV
AiV
Erbovirus
ERBV-1
Cardiovirus
EMCV
TMEV
Aphthovirus
ERAV
FMDV-O
Teschovirus
PTV-1
Hepatovirus
AEV
HAV
Parechovirus
LV
HPeV-1
1000
1000
1000
964
977
674
994
285
167
647
999
302
495
760
0.1

Fig. 31

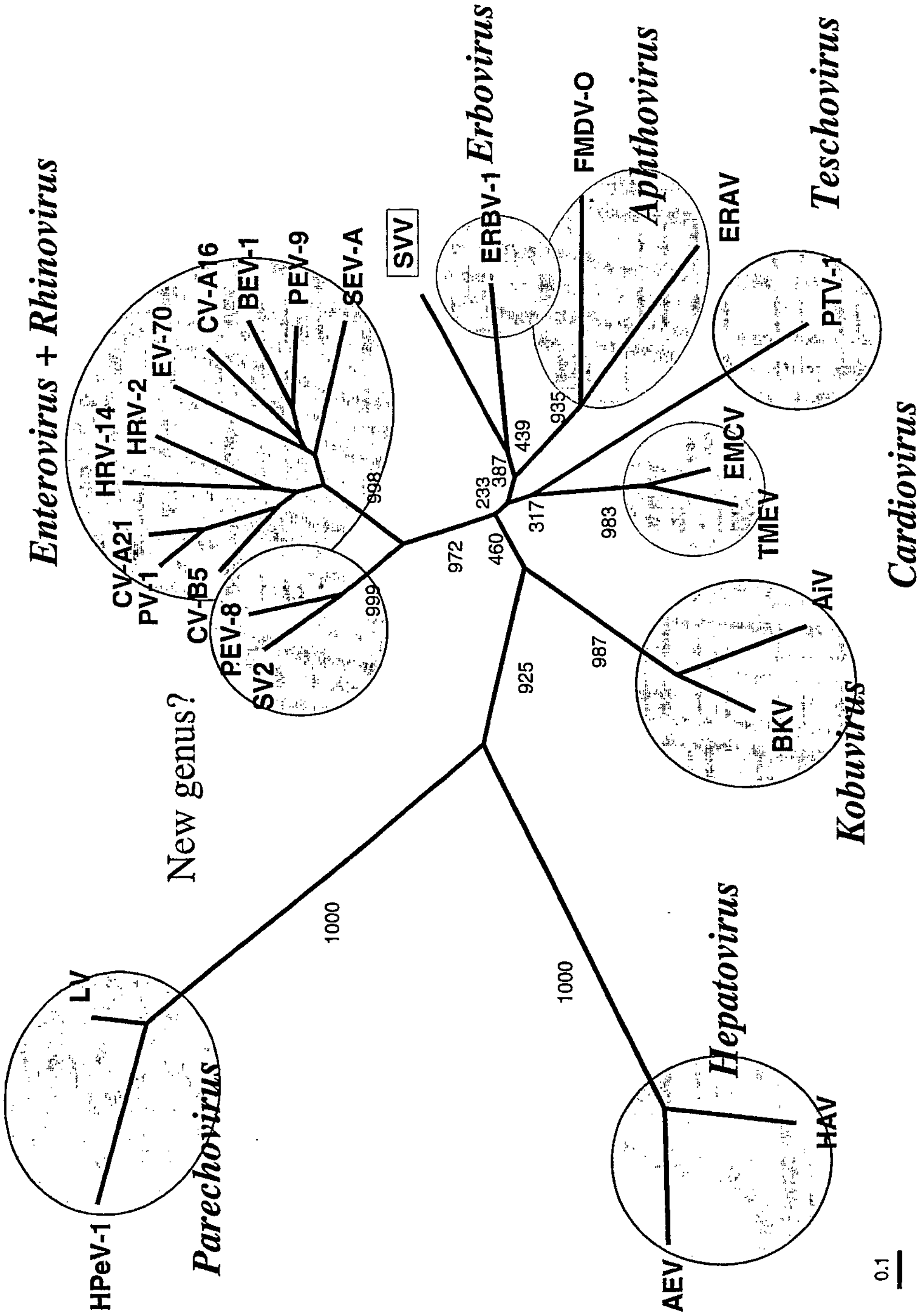
Enterovirus + Rhinovirus
HRV-14
HRV-2
EV-70
CV-A16
BEV-1
PEV-9
SEV-A
CV-A21
PV-1
CV-B5
PEV-8
SV2
998
999
New genus?
SVV
ERBV-1
Erbovirus
FMDV-O
Aphthovirus
ERAV
Teschovirus
PTV-1
935
439
387
233
972
460
317
983
EMCV
TMEV
Cardiovirus
AiV
987
925
BKV
Kobuvirus
1000
1000
LV
HPeV-1
Parechovirus
Hepatovirus
HAV
AEV
0.1

Fig. 32

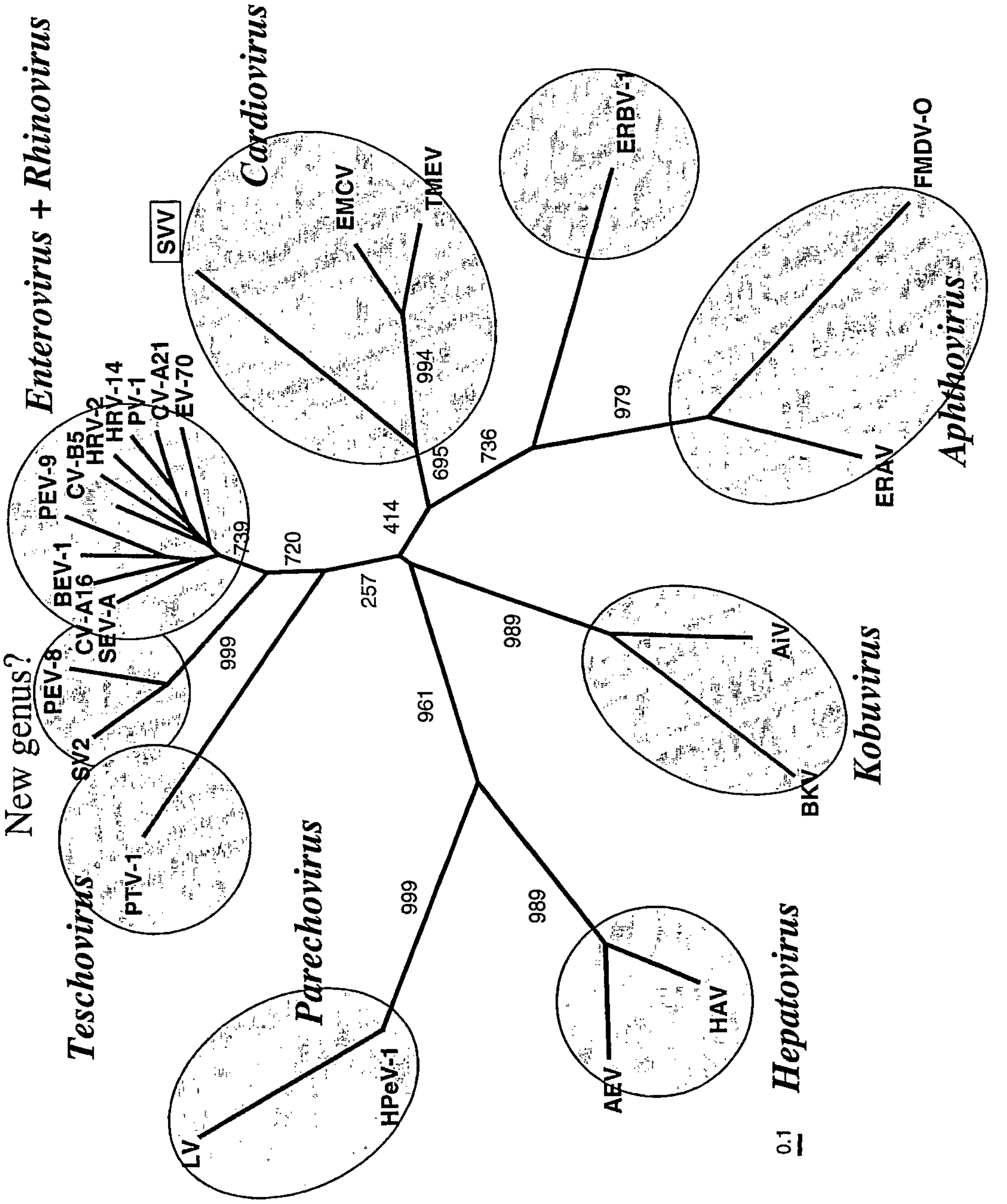

Enterovirus + Rhinovirus
Cardiovirus
SVV
EMCV
TMEV
ERBV-1
FMDV-O
Aphthovirus
ERAV
979
736
994
695
414
720
739
PEV-9
CV-B5
HRV-2
HRV-14
PV-1
CV-A21
EV-70
BEV-1
CV-A16
SEV-A
New genus?
PEV-8
SV2
999
257
PTV-1
Teschovirus
Parechovirus
999
LV
HPeV-1
961
989
AiV
BKV
Kobuvirus
989
AEV
HAV
Hepatovirus
0.1


Fig. 33

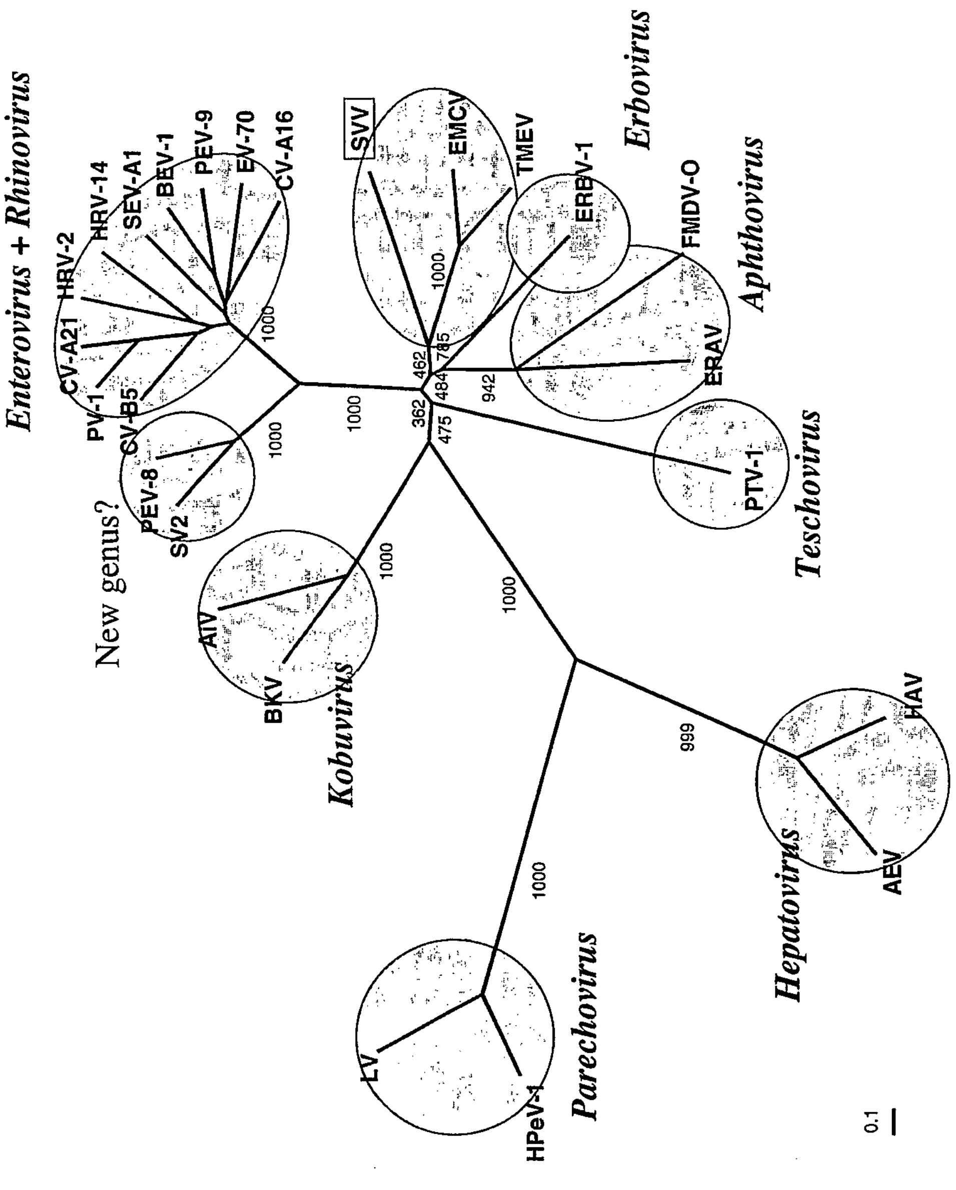
Enterovirus + Rhinovirus
CV-A21
PV-1
CV-B5
HRV-2
HRV-14
SEV-A1
BEV-1
PEV-9
EV-70
CV-A16
1000
1000
PEV-8
SV2
New genus?
1000
SVV
EMCV
1000
TMEV
ERBV-1
Erbovirus
FMDV-O
Aphthovirus
ERAV
462
785
484
942
362
475
PTV-1
Teschovirus
ATV
BKV
1000
Kobuvirus
1000
999
HAV
AEV
Hepatovirus
1000
LV
HPeV-1
Parechovirus
0.1

Fig. 34

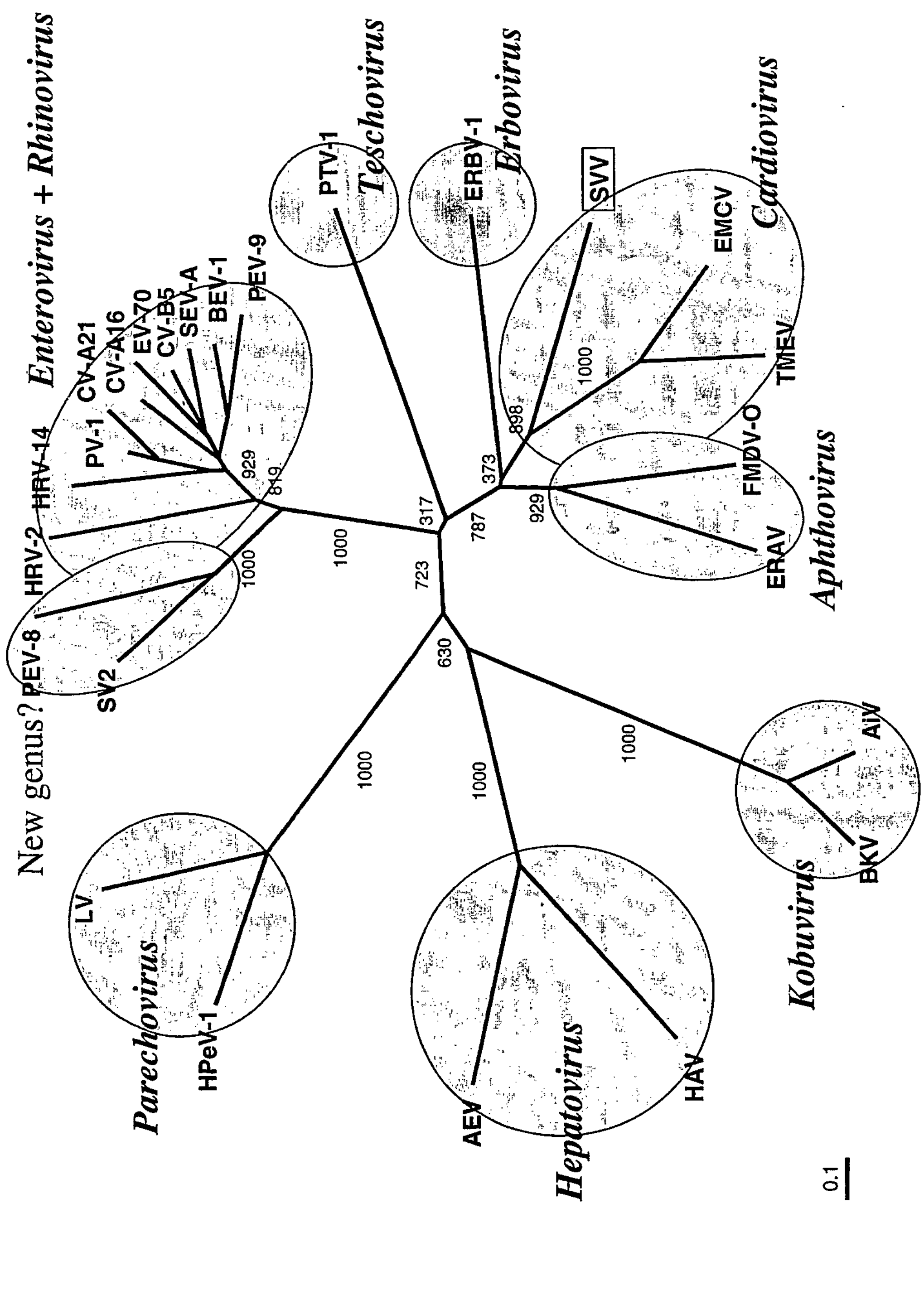
Enterovirus + Rhinovirus
HRV-14
PV-1
CV-A21
CV-A16
EV-70
CV-B5
SEV-A
BEV-1
PEV-9
HRV-2
PEV-8
SV2
New genus?
Teschovirus
PTV-1
Erbovirus
ERBV-1
Cardiovirus
SVV
EMCV
TMEV
Aphthovirus
FMDV-O
ERAV
Kobuvirus
AiV
BKV
Hepatovirus
AEV
HAV
Parechovirus
LV
HPeV-1
929
819
1000
317
373
898
1000
787
929
723
630
1000
1000
1000
1000
0.1

FIG. 35

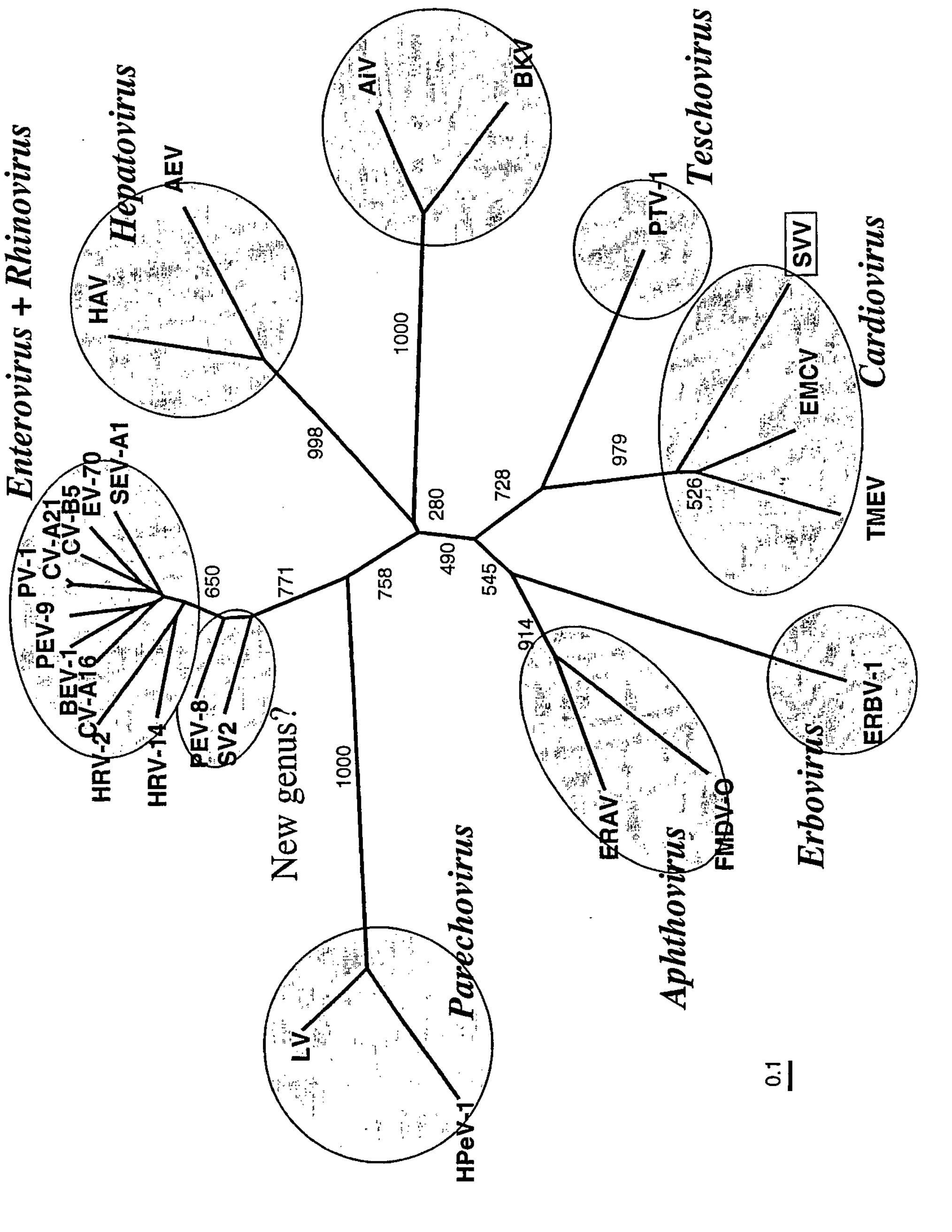

Enterovirus + Rhinovirus
PV-1
CV-A21
CV-B5
EV-70
SEV-A1
PEV-9
BEV-1
CV-A16
HRV-2
HRV-14
650
PEV-8
SV2
771
New genus?
Hepatovirus
HAV
AEV
998
AiV
BKV
1000
280
758
490
1000
Parechovirus
LV
HPeV-1
Teschovirus
PTV-1
728
979
Cardiovirus
SVV
EMCV
TMEV
526
545
914
Aphthovirus
ERAV
FMDV-O
Erbovirus
ERBV-1
0.1


FIG. 36

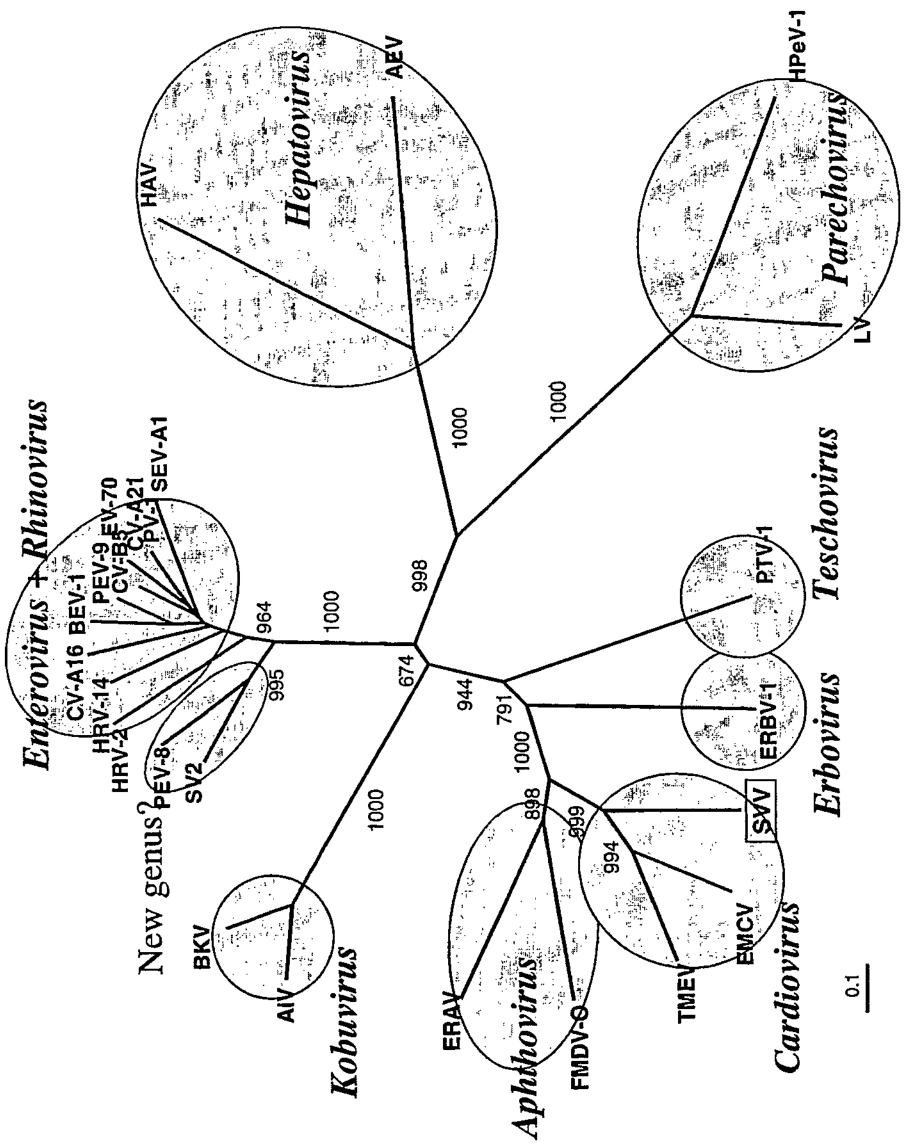

Hepatovirus
HAV
AEV
Parechovirus
HPeV-1
LV
1000
1000
998
Enterovirus + Rhinovirus
SEV-A1
PV-1
CV-A21
EV-70
CV-B5
PEV-9
BEV-1
CV-A16
HRV-14
HRV-2
964
1000
995
PEV-8
SV2
New genus?
674
1000
Kobuvirus
BKV
AIV
Teschovirus
PTV-1
944
Erbovirus
ERBV-1
791
1000
SVV
898
999
994
Aphthovirus
ERAV
FMDV-O
Cardiovirus
TMEV
EMCV
0.1


FIG. 37

| | Cardiovirus | | | Aphthovirus | | Erbo | Teschno | Enterovirus | | | | | | | | Rhinovirus | | Entero? | | Kobu | | Hepato | | Parecho | |
|---|---|---|---|---|---|---|---|---|---|---|---|---|---|---|---|---|---|---|---|---|---|---|---|---|---|
| | SVV | EMCV | TMEV | FMDV-O | ERAV | ERBV | PTV-1 | PV-1 | CV-A21 | CV-A16 | CV-B5 | EV-70 | SEV-A | BEV-1 | PEV-9 | HRV-14 | HRV-2 | SV2 | PEV-8 | AiV | BKV | HAV | AEV | HPeV-1 | LV |
| SVV | 100 | 33 | 35 | 24 | 26 | 30 | 24 | 24 | 24 | 29 | 29 | 30 | 35 | 25 | 27 | 26 | 25 | 31 | 31 | 27 | 21 | 20 | 19 | 18 | 18 |
| EMCV | 33 | 100 | 70 | 32 | 35 | 47 | 29 | 32 | 30 | 31 | 34 | 30 | 34 | 34 | 31 | 34 | 33 | 40 | 37 | 20 | 21 | 25 | 25 | 22 | 21 |
| TMEV | 35 | 70 | 100 | 33 | 34 | 45 | 31 | 35 | 34 | 30 | 32 | 31 | 34 | 33 | 31 | 31 | 28 | 36 | 37 | 21 | 21 | 25 | 25 | 23 | 21 |
| FMDV-O | 24 | 32 | 33 | 100 | 31 | 35 | 31 | 27 | 26 | 30 | 27 | 25 | 28 | 28 | 25 | 31 | 25 | 32 | 29 | 15 | 17 | 24 | 24 | 19 | 17 |
| ERAV | 26 | 35 | 34 | 31 | 100 | 35 | 36 | 32 | 31 | 28 | 35 | 30 | 31 | 28 | 27 | 29 | 30 | 39 | 34 | 22 | 22 | 20 | 24 | 18 | 22 |
| ERBV | 30 | 47 | 45 | 35 | 35 | 100 | 29 | 26 | 28 | 34 | 31 | 31 | 33 | 30 | 31 | 33 | 32 | 36 | 35 | 21 | 21 | 26 | 28 | 20 | 21 |
| PTV-1 | 24 | 29 | 31 | 31 | 36 | 29 | 100 | 30 | 31 | 27 | 29 | 26 | 30 | 30 | 30 | 30 | 26 | 28 | 30 | 18 | 22 | 25 | 22 | 19 | 22 |
| PV-1 | 24 | 32 | 35 | 27 | 32 | 26 | 30 | 100 | 71 | 52 | 52 | 51 | 55 | 54 | 48 | 53 | 51 | 42 | 44 | 21 | 23 | 19 | 21 | 18 | 20 |
| CV-A21 | 24 | 30 | 34 | 26 | 31 | 28 | 31 | 71 | 100 | 51 | 53 | 52 | 52 | 53 | 47 | 54 | 55 | 43 | 46 | 19 | 23 | 23 | 22 | 20 | 19 |
| CV-A16 | 29 | 31 | 30 | 30 | 28 | 34 | 27 | 52 | 51 | 100 | 52 | 53 | 53 | 54 | 52 | 52 | 48 | 49 | 48 | 18 | 19 | 22 | 22 | 19 | 17 |
| CV-B5 | 29 | 34 | 32 | 27 | 35 | 31 | 29 | 52 | 53 | 52 | 100 | 55 | 54 | 53 | 49 | 56 | 51 | 51 | 48 | 20 | 17 | 23 | 22 | 20 | 18 |
| EV-70 | 30 | 30 | 31 | 25 | 30 | 31 | 26 | 51 | 52 | 53 | 55 | 100 | 57 | 63 | 62 | 52 | 53 | 46 | 49 | 20 | 20 | 25 | 23 | 18 | 17 |
| SEV-A | 35 | 34 | 34 | 28 | 31 | 33 | 30 | 55 | 52 | 53 | 54 | 57 | 100 | 55 | 54 | 47 | 50 | 43 | 45 | 21 | 20 | 21 | 22 | 22 | 19 |
| BEV-1 | 25 | 34 | 33 | 28 | 28 | 30 | 30 | 54 | 53 | 54 | 53 | 63 | 55 | 100 | 67 | 54 | 52 | 45 | 46 | 20 | 23 | 21 | 21 | 16 | 16 |
| PEV-9 | 27 | 31 | 31 | 25 | 27 | 31 | 30 | 48 | 47 | 52 | 49 | 62 | 54 | 67 | 100 | 53 | 49 | 45 | 45 | 21 | 23 | 24 | 25 | 20 | 18 |
| HRV-14 | 26 | 34 | 31 | 31 | 29 | 33 | 30 | 53 | 54 | 52 | 56 | 52 | 47 | 54 | 53 | 100 | 61 | 46 | 49 | 23 | 21 | 22 | 21 | 19 | 21 |
| HRV-2 | 25 | 33 | 28 | 25 | 30 | 32 | 26 | 51 | 55 | 48 | 51 | 53 | 50 | 52 | 49 | 61 | 100 | 46 | 46 | 22 | 21 | 22 | 24 | 20 | 22 |
| SV2 | 31 | 40 | 36 | 32 | 39 | 36 | 28 | 42 | 43 | 49 | 51 | 46 | 43 | 45 | 45 | 46 | 46 | 100 | 74 | 23 | 22 | 25 | 27 | 20 | 19 |
| PEV-8 | 31 | 37 | 37 | 29 | 34 | 35 | 30 | 44 | 46 | 48 | 48 | 49 | 45 | 46 | 45 | 49 | 46 | 74 | 100 | 24 | 22 | 25 | 26 | 18 | 21 |
| AiV | 27 | 20 | 21 | 15 | 22 | 21 | 18 | 21 | 19 | 18 | 20 | 20 | 21 | 20 | 21 | 23 | 22 | 23 | 24 | 100 | 56 | 19 | 18 | 16 | 16 |
| BKV | 21 | 21 | 21 | 17 | 22 | 21 | 22 | 23 | 23 | 19 | 17 | 20 | 20 | 23 | 23 | 21 | 21 | 22 | 22 | 56 | 100 | 16 | 18 | 16 | 16 |
| HAV | 20 | 25 | 25 | 24 | 20 | 26 | 25 | 19 | 23 | 22 | 23 | 25 | 21 | 21 | 24 | 22 | 22 | 25 | 25 | 19 | 16 | 100 | 57 | 18 | 23 |
| AEV | 19 | 25 | 25 | 24 | 24 | 28 | 22 | 21 | 22 | 22 | 22 | 23 | 22 | 21 | 25 | 21 | 24 | 27 | 26 | 18 | 18 | 57 | 100 | 18 | 23 |
| HPeV-1 | 18 | 22 | 23 | 19 | 18 | 20 | 19 | 18 | 20 | 19 | 20 | 18 | 22 | 16 | 20 | 19 | 20 | 20 | 18 | 16 | 16 | 18 | 18 | 100 | 53 |
| LV | 18 | 21 | 21 | 17 | 22 | 21 | 22 | 20 | 19 | 17 | 18 | 17 | 19 | 16 | 18 | 21 | 22 | 19 | 21 | 16 | 16 | 23 | 23 | 53 | 100 |

Fig. 38

| | Cardiovirus | | | Aphthovirus | | Erbo | Tescho | Enterovirus | | | | | | | | Rhinovirus | | Entero? | | Kobu | | Hepato | | Parecho | |
|---|---|---|---|---|---|---|---|---|---|---|---|---|---|---|---|---|---|---|---|---|---|---|---|---|---|
| | SVV | EMCV | TMEV | FMDV-O | ERAV | ERBV | PTV-1 | PV-1 | CV-A21 | CV-A16 | CV-B5 | EV-70 | SEV-A | BEV-1 | PEV-9 | HRV-14 | HRV-2 | SV2 | PEV-8 | AiV | BKV | HAV | AEV | HPeV-1 | LV |
| SVV | 100 | 42 | 39 | 32 | 34 | 43 | 31 | 32 | 30 | 25 | 29 | 26 | 30 | 25 | 29 | 28 | 28 | 35 | 30 | 26 | 28 | 19 | 17 | 17 | 19 |
| EMCV | 42 | 100 | 67 | 29 | 38 | 39 | 35 | 28 | 31 | 29 | 31 | 31 | 29 | 31 | 30 | 26 | 31 | 31 | 35 | 27 | 26 | 19 | 18 | 17 | 18 |
| TMEV | 39 | 67 | 100 | 31 | 37 | 35 | 33 | 29 | 29 | 31 | 29 | 27 | 27 | 27 | 28 | 26 | 30 | 31 | 35 | 27 | 28 | 18 | 17 | 17 | 20 |
| FMDV-O | 32 | 29 | 31 | 100 | 39 | 31 | 23 | 26 | 23 | 26 | 27 | 25 | 26 | 25 | 24 | 26 | 23 | 30 | 32 | 20 | 25 | 14 | 15 | 14 | 17 |
| ERAV | 34 | 38 | 37 | 39 | 100 | 38 | 25 | 25 | 24 | 20 | 27 | 23 | 23 | 23 | 25 | 24 | 27 | 31 | 32 | 25 | 24 | 20 | 15 | 15 | 19 |
| ERBV | 43 | 39 | 35 | 31 | 38 | 100 | 30 | 27 | 29 | 27 | 28 | 27 | 30 | 29 | 28 | 28 | 28 | 34 | 34 | 27 | 29 | 21 | 19 | 18 | 17 |
| PTV-1 | 31 | 35 | 33 | 23 | 25 | 30 | 100 | 23 | 22 | 24 | 24 | 23 | 27 | 25 | 28 | 22 | 27 | 28 | 27 | 24 | 23 | 16 | 16 | 19 | 18 |
| PV-1 | 32 | 28 | 29 | 26 | 25 | 27 | 23 | 100 | 75 | 42 | 55 | 42 | 46 | 41 | 43 | 43 | 50 | 36 | 36 | 24 | 29 | 17 | 17 | 15 | 15 |
| CV-A21 | 30 | 31 | 29 | 23 | 24 | 29 | 22 | 75 | 100 | 43 | 61 | 42 | 46 | 42 | 45 | 48 | 53 | 36 | 35 | 24 | 26 | 16 | 20 | 13 | 13 |
| CV-A16 | 25 | 29 | 31 | 26 | 20 | 27 | 24 | 42 | 43 | 100 | 48 | 51 | 50 | 55 | 54 | 40 | 41 | 40 | 38 | 24 | 26 | 13 | 14 | 13 | 14 |
| CV-B5 | 29 | 31 | 29 | 27 | 27 | 28 | 24 | 55 | 61 | 48 | 100 | 47 | 48 | 49 | 49 | 51 | 52 | 40 | 36 | 24 | 27 | 16 | 17 | 14 | 15 |
| EV-70 | 26 | 31 | 27 | 25 | 23 | 27 | 23 | 42 | 42 | 51 | 47 | 100 | 49 | 49 | 53 | 45 | 44 | 32 | 38 | 25 | 27 | 16 | 15 | 15 | 15 |
| SEV-A | 30 | 29 | 27 | 26 | 23 | 30 | 27 | 46 | 46 | 50 | 48 | 49 | 100 | 48 | 53 | 41 | 43 | 37 | 37 | 28 | 28 | 13 | 14 | 14 | 15 |
| BEV-1 | 25 | 31 | 27 | 25 | 23 | 29 | 25 | 41 | 42 | 55 | 49 | 49 | 48 | 100 | 63 | 45 | 45 | 40 | 40 | 23 | 25 | 11 | 12 | 13 | 17 |
| PEV-9 | 29 | 30 | 28 | 24 | 25 | 28 | 28 | 43 | 45 | 54 | 49 | 53 | 53 | 63 | 100 | 43 | 48 | 39 | 39 | 22 | 27 | 15 | 12 | 14 | 13 |
| HRV-14 | 28 | 26 | 26 | 26 | 24 | 28 | 22 | 43 | 48 | 40 | 51 | 45 | 41 | 45 | 43 | 100 | 52 | 33 | 33 | 26 | 27 | 16 | 17 | 14 | 19 |
| HRV-2 | 28 | 31 | 30 | 23 | 27 | 28 | 27 | 50 | 53 | 41 | 52 | 44 | 43 | 45 | 48 | 52 | 100 | 35 | 38 | 25 | 28 | 19 | 16 | 14 | 17 |
| SV2 | 35 | 31 | 31 | 30 | 31 | 34 | 28 | 36 | 36 | 40 | 40 | 32 | 37 | 40 | 39 | 33 | 35 | 100 | 63 | 25 | 29 | 18 | 16 | 16 | 21 |
| PEV-8 | 30 | 35 | 35 | 32 | 32 | 34 | 27 | 36 | 35 | 38 | 36 | 38 | 37 | 40 | 39 | 33 | 38 | 63 | 100 | 24 | 30 | 18 | 17 | 18 | 18 |
| AiV | 26 | 27 | 27 | 20 | 25 | 27 | 24 | 24 | 24 | 24 | 24 | 25 | 28 | 23 | 22 | 26 | 25 | 25 | 24 | 100 | 58 | 16 | 18 | 17 | 19 |
| BKV | 28 | 26 | 28 | 25 | 24 | 29 | 23 | 29 | 26 | 26 | 27 | 27 | 28 | 25 | 27 | 27 | 28 | 29 | 30 | 58 | 100 | 17 | 20 | 16 | 19 |
| HAV | 19 | 19 | 18 | 14 | 20 | 21 | 16 | 17 | 16 | 13 | 16 | 16 | 13 | 11 | 15 | 16 | 19 | 18 | 18 | 16 | 17 | 100 | 53 | 18 | 19 |
| AEV | 17 | 18 | 17 | 15 | 15 | 19 | 16 | 17 | 20 | 14 | 17 | 15 | 14 | 12 | 12 | 17 | 16 | 16 | 17 | 18 | 20 | 53 | 100 | 20 | 20 |
| HPeV-1 | 17 | 17 | 17 | 14 | 15 | 18 | 19 | 15 | 13 | 13 | 14 | 15 | 14 | 13 | 14 | 14 | 14 | 16 | 18 | 17 | 16 | 18 | 20 | 100 | 55 |
| LV | 19 | 18 | 20 | 17 | 19 | 17 | 18 | 15 | 13 | 14 | 15 | 15 | 15 | 17 | 13 | 19 | 17 | 21 | 18 | 19 | 19 | 19 | 20 | 55 | 100 |

Fig. 39

| | Cardiovirus | | | Aphthovirus | | Erbo | Teschо | Enterovirus | | | | | | | | Rhinovirus | | Entero? | | Kobu | | Hepato | | Parecho | |
|---|---|---|---|---|---|---|---|---|---|---|---|---|---|---|---|---|---|---|---|---|---|---|---|---|---|
| | SVV | EMCV | TMEV | FMDV-O | ERAV | ERBV | PTV-1 | PV-1 | CV-A21 | CV-A16 | CV-B5 | EV-70 | SEV-A | BEV-1 | PEV-9 | HRV-14 | HRV-2 | SV2 | PEV-8 | AiV | BKV | HAV | AEV | HPeV-1 | LV |
| SVV | 100 | 20 | 21 | 12 | 13 | 13 | 11 | 17 | 16 | 16 | 16 | 14 | 17 | 13 | 13 | 18 | 16 | 14 | 13 | 13 | 13 | 9 | 10 | 11 | 8 |
| EMCV | 20 | 100 | 48 | 11 | 15 | 16 | 15 | 16 | 16 | 14 | 14 | 15 | 18 | 17 | 17 | 17 | 15 | 16 | 16 | 14 | 13 | 12 | 12 | 11 | 11 |
| TMEV | 21 | 48 | 100 | 12 | 15 | 15 | 14 | 15 | 16 | 16 | 13 | 13 | 17 | 14 | 15 | 16 | 15 | 16 | 15 | 17 | 14 | 15 | 13 | 9 | 11 |
| FMDV-O | 12 | 11 | 12 | 100 | 23 | 13 | 9 | 11 | 13 | 12 | 11 | 11 | 11 | 10 | 11 | 10 | 13 | 9 | 12 | 12 | 8 | 11 | 10 | 11 | 10 |
| ERAV | 13 | 15 | 15 | 23 | 100 | 18 | 12 | 13 | 14 | 16 | 14 | 11 | 14 | 14 | 12 | 14 | 14 | 10 | 14 | 11 | 9 | 9 | 9 | 9 | 11 |
| ERBV | 13 | 16 | 15 | 13 | 18 | 100 | 13 | 13 | 13 | 14 | 15 | 15 | 13 | 12 | 11 | 12 | 12 | 12 | 13 | 17 | 12 | 10 | 12 | 13 | 11 |
| PTV-1 | 11 | 15 | 14 | 9 | 12 | 13 | 100 | 19 | 18 | 21 | 19 | 19 | 18 | 17 | 17 | 18 | 17 | 17 | 17 | 16 | 12 | 12 | 9 | 13 | 12 |
| PV-1 | 17 | 16 | 15 | 11 | 13 | 13 | 19 | 100 | 57 | 34 | 46 | 34 | 41 | 35 | 32 | 46 | 36 | 23 | 21 | 16 | 16 | 10 | 10 | 14 | 10 |
| CV-A21 | 16 | 16 | 16 | 13 | 14 | 13 | 18 | 57 | 100 | 36 | 43 | 37 | 39 | 33 | 35 | 42 | 40 | 24 | 24 | 15 | 13 | 10 | 14 | 14 | 11 |
| CV-A16 | 16 | 14 | 16 | 12 | 16 | 14 | 21 | 34 | 36 | 100 | 37 | 38 | 42 | 43 | 40 | 35 | 37 | 27 | 24 | 15 | 15 | 12 | 13 | 13 | 8 |
| CV-B5 | 16 | 14 | 13 | 11 | 14 | 15 | 19 | 46 | 43 | 37 | 100 | 42 | 40 | 38 | 32 | 38 | 42 | 27 | 25 | 14 | 16 | 13 | 12 | 13 | 9 |
| EV-70 | 14 | 15 | 13 | 11 | 11 | 15 | 19 | 34 | 37 | 38 | 42 | 100 | 37 | 39 | 33 | 38 | 37 | 24 | 23 | 13 | 13 | 16 | 12 | 13 | 12 |
| SEV-A | 17 | 18 | 17 | 11 | 14 | 13 | 18 | 41 | 39 | 42 | 40 | 37 | 100 | 39 | 40 | 39 | 40 | 25 | 25 | 13 | 13 | 13 | 13 | 14 | 12 |
| BEV-1 | 13 | 17 | 14 | 10 | 14 | 12 | 17 | 35 | 33 | 43 | 38 | 39 | 39 | 100 | 46 | 37 | 35 | 25 | 25 | 15 | 14 | 15 | 11 | 12 | 10 |
| PEV-9 | 13 | 17 | 15 | 11 | 12 | 11 | 17 | 32 | 35 | 40 | 32 | 33 | 40 | 46 | 100 | 36 | 34 | 23 | 22 | 13 | 13 | 12 | 11 | 12 | 9 |
| HRV-14 | 18 | 17 | 16 | 10 | 14 | 12 | 18 | 46 | 42 | 35 | 38 | 38 | 39 | 37 | 36 | 100 | 42 | 23 | 22 | 15 | 16 | 11 | 13 | 14 | 10 |
| HRV-2 | 16 | 15 | 15 | 13 | 14 | 12 | 17 | 36 | 40 | 37 | 42 | 37 | 40 | 35 | 34 | 42 | 100 | 24 | 21 | 16 | 14 | 13 | 14 | 14 | 9 |
| SV2 | 14 | 16 | 16 | 9 | 10 | 12 | 17 | 23 | 24 | 27 | 27 | 24 | 25 | 25 | 23 | 23 | 24 | 100 | 46 | 15 | 12 | 11 | 12 | 12 | 11 |
| PEV-8 | 13 | 16 | 15 | 12 | 14 | 13 | 17 | 21 | 24 | 24 | 25 | 23 | 25 | 25 | 22 | 22 | 21 | 46 | 100 | 14 | 12 | 13 | 12 | 13 | 8 |
| AiV | 13 | 14 | 17 | 12 | 11 | 17 | 16 | 16 | 15 | 15 | 14 | 13 | 13 | 15 | 13 | 15 | 16 | 15 | 14 | 100 | 28 | 11 | 13 | 11 | 10 |
| BKV | 13 | 13 | 14 | 8 | 9 | 12 | 12 | 16 | 13 | 15 | 16 | 13 | 13 | 14 | 13 | 16 | 14 | 12 | 12 | 28 | 100 | 11 | 11 | 13 | 10 |
| HAV | 9 | 12 | 15 | 11 | 9 | 10 | 12 | 10 | 10 | 12 | 13 | 16 | 13 | 15 | 12 | 11 | 13 | 11 | 13 | 11 | 11 | 100 | 43 | 15 | 14 |
| AEV | 10 | 12 | 13 | 10 | 9 | 12 | 9 | 10 | 14 | 13 | 12 | 12 | 13 | 11 | 11 | 13 | 14 | 12 | 12 | 13 | 11 | 43 | 100 | 18 | 14 |
| HPeV-1 | 11 | 11 | 9 | 11 | 9 | 13 | 13 | 14 | 14 | 13 | 13 | 13 | 14 | 12 | 12 | 14 | 14 | 12 | 13 | 11 | 13 | 15 | 18 | 100 | 43 |
| LV | 8 | 11 | 11 | 10 | 11 | 11 | 12 | 10 | 11 | 8 | 9 | 12 | 12 | 10 | 9 | 10 | 9 | 11 | 8 | 10 | 10 | 14 | 14 | 43 | 100 |

Fig. 40

| | Cardiovirus | | | Aphthovirus | | Erbo | Tescho | Enterovirus | | | | | | | | Rhinovirus | | Entero? | | Kobu | | Hepato | | Parecho | |
|---|---|---|---|---|---|---|---|---|---|---|---|---|---|---|---|---|---|---|---|---|---|---|---|---|---|
| | SVV | EMCV | TMEV | FMDV-O | ERAV | ERBV | PTV-1 | PV-1 | CV-A21 | CV-A16 | CV-B5 | EV-70 | SEV-A | BEV-1 | PEV-9 | HRV-14 | HRV-2 | SV2 | PEV-8 | AiV | BKV | HAV | AEV | HPeV-1 | LV |
| SVV | 100 | 34 | 33 | 26 | 27 | 30 | 22 | 23 | 23 | 22 | 24 | 22 | 24 | 21 | 22 | 22 | 21 | 25 | 24 | 22 | 22 | 15 | 15 | 14 | 12 |
| EMCV | 34 | 100 | 59 | 25 | 28 | 31 | 25 | 22 | 21 | 22 | 24 | 22 | 23 | 23 | 22 | 21 | 23 | 24 | 25 | 21 | 21 | 15 | 16 | 15 | 14 |
| TMEV | 33 | 59 | 100 | 27 | 29 | 30 | 24 | 23 | 22 | 24 | 21 | 21 | 22 | 22 | 22 | 22 | 22 | 24 | 26 | 24 | 24 | 15 | 15 | 14 | 14 |
| FMDV-O | 26 | 25 | 27 | 100 | 33 | 27 | 20 | 21 | 20 | 21 | 20 | 20 | 22 | 19 | 18 | 22 | 19 | 22 | 23 | 17 | 19 | 13 | 12 | 13 | 12 |
| ERAV | 27 | 28 | 29 | 33 | 100 | 32 | 23 | 21 | 20 | 17 | 23 | 19 | 20 | 20 | 20 | 21 | 21 | 25 | 25 | 22 | 20 | 16 | 15 | 13 | 15 |
| ERBV | 30 | 31 | 30 | 27 | 32 | 100 | 24 | 22 | 23 | 23 | 24 | 23 | 24 | 23 | 22 | 22 | 21 | 26 | 26 | 24 | 21 | 16 | 16 | 15 | 13 |
| PTV-1 | 22 | 25 | 24 | 20 | 23 | 24 | 100 | 19 | 20 | 20 | 19 | 20 | 22 | 19 | 22 | 19 | 19 | 20 | 20 | 20 | 20 | 15 | 13 | 13 | 14 |
| PV-1 | 23 | 22 | 23 | 21 | 21 | 22 | 19 | 100 | 66 | 39 | 50 | 40 | 44 | 40 | 39 | 44 | 43 | 29 | 30 | 18 | 22 | 14 | 14 | 11 | 11 |
| CV-A21 | 23 | 21 | 22 | 20 | 20 | 23 | 20 | 66 | 100 | 40 | 51 | 42 | 42 | 40 | 40 | 44 | 44 | 29 | 32 | 18 | 20 | 14 | 15 | 10 | 11 |
| CV-A16 | 22 | 22 | 24 | 21 | 17 | 23 | 20 | 39 | 40 | 100 | 43 | 47 | 46 | 49 | 46 | 40 | 40 | 33 | 32 | 19 | 20 | 12 | 11 | 11 | 11 |
| CV-B5 | 24 | 24 | 21 | 20 | 23 | 24 | 19 | 50 | 51 | 43 | 100 | 46 | 44 | 44 | 41 | 45 | 46 | 35 | 35 | 19 | 20 | 14 | 14 | 12 | 10 |
| EV-70 | 22 | 22 | 21 | 20 | 19 | 23 | 20 | 40 | 42 | 47 | 46 | 100 | 45 | 47 | 45 | 42 | 41 | 30 | 32 | 20 | 20 | 14 | 12 | 13 | 11 |
| SEV-A | 24 | 23 | 22 | 22 | 20 | 24 | 22 | 44 | 42 | 46 | 44 | 45 | 100 | 46 | 48 | 41 | 41 | 32 | 33 | 21 | 21 | 12 | 12 | 12 | 11 |
| BEV-1 | 21 | 23 | 22 | 19 | 20 | 23 | 19 | 40 | 40 | 49 | 44 | 47 | 46 | 100 | 56 | 42 | 42 | 33 | 33 | 19 | 20 | 11 | 11 | 11 | 13 |
| PEV-9 | 22 | 22 | 22 | 18 | 20 | 22 | 22 | 39 | 40 | 46 | 41 | 45 | 48 | 56 | 100 | 41 | 42 | 32 | 32 | 18 | 20 | 12 | 10 | 12 | 10 |
| HRV-14 | 22 | 21 | 22 | 22 | 21 | 22 | 19 | 44 | 44 | 40 | 45 | 42 | 41 | 42 | 41 | 100 | 47 | 30 | 29 | 20 | 20 | 14 | 14 | 12 | 13 |
| HRV-2 | 21 | 23 | 22 | 19 | 21 | 21 | 19 | 43 | 44 | 40 | 46 | 41 | 41 | 42 | 42 | 47 | 100 | 30 | 30 | 20 | 20 | 14 | 12 | 11 | 12 |
| SV2 | 25 | 24 | 24 | 22 | 25 | 26 | 20 | 29 | 29 | 33 | 35 | 30 | 32 | 33 | 32 | 30 | 30 | 100 | 57 | 19 | 21 | 15 | 14 | 14 | 14 |
| PEV-8 | 24 | 25 | 26 | 23 | 25 | 26 | 20 | 30 | 32 | 32 | 35 | 32 | 33 | 33 | 32 | 29 | 30 | 57 | 100 | 18 | 22 | 16 | 15 | 14 | 13 |
| AiV | 22 | 21 | 24 | 17 | 22 | 24 | 20 | 18 | 18 | 19 | 19 | 20 | 21 | 19 | 18 | 20 | 20 | 19 | 18 | 100 | 46 | 14 | 14 | 14 | 14 |
| BKV | 22 | 21 | 24 | 19 | 20 | 21 | 20 | 22 | 20 | 20 | 20 | 20 | 21 | 20 | 20 | 20 | 20 | 21 | 22 | 46 | 100 | 13 | 15 | 13 | 13 |
| HAV | 15 | 15 | 15 | 13 | 16 | 16 | 15 | 14 | 14 | 12 | 14 | 14 | 12 | 11 | 12 | 14 | 14 | 15 | 16 | 14 | 13 | 100 | 49 | 16 | 15 |
| AEV | 15 | 16 | 15 | 12 | 15 | 16 | 13 | 14 | 15 | 11 | 14 | 12 | 12 | 11 | 10 | 14 | 12 | 14 | 15 | 14 | 15 | 49 | 100 | 18 | 17 |
| HPeV-1 | 14 | 15 | 14 | 13 | 13 | 15 | 13 | 11 | 10 | 11 | 12 | 13 | 12 | 11 | 12 | 12 | 11 | 14 | 14 | 14 | 13 | 16 | 18 | 100 | 50 |
| LV | 12 | 14 | 14 | 12 | 15 | 13 | 14 | 11 | 11 | 11 | 10 | 11 | 11 | 13 | 10 | 13 | 12 | 14 | 13 | 14 | 13 | 15 | 17 | 50 | 100 |

* not including VP4 or part of VP2

Fig. 41

| | Cardiovirus | | | Aphthovirus | | Erbo | Tescho | Enterovirus | | | | | | | | Rhinovirus | | Entero? | | Kobu | | Hepato | | Parecho | |
|---|---|---|---|---|---|---|---|---|---|---|---|---|---|---|---|---|---|---|---|---|---|---|---|---|---|
| | SVV | EMCV | TMEV | FMDV-O | ERAV | ERBV | PTV-1 | PV-1 | CV-A21 | CV-A16 | CV-B5 | EV-70 | SEV-A | BEV-1 | PEV-9 | HRV-14 | HRV-2 | SV2 | PEV-8 | AiV | BKV | HAV | AEV | HPeV-1 | LV |
| SVV | 100 | 41 | 39 | 35 | 34 | 35 | 32 | 29 | 28 | 30 | 29 | 30 | 26 | 26 | 26 | 29 | 27 | 28 | 29 | 26 | 23 | 25 | 26 | 21 | 20 |
| EMCV | 41 | 100 | 60 | 37 | 36 | 35 | 30 | 27 | 27 | 29 | 28 | 28 | 27 | 27 | 27 | 25 | 27 | 26 | 25 | 23 | 24 | 27 | 25 | 20 | 21 |
| TMEV | 39 | 60 | 100 | 36 | 38 | 35 | 31 | 28 | 26 | 26 | 29 | 28 | 26 | 25 | 24 | 27 | 26 | 29 | 25 | 23 | 23 | 26 | 25 | 20 | 21 |
| FMDV-O | 35 | 37 | 36 | 100 | 47 | 38 | 30 | 31 | 28 | 32 | 30 | 32 | 30 | 31 | 27 | 31 | 28 | 31 | 31 | 27 | 28 | 25 | 24 | 20 | 21 |
| ERAV | 34 | 36 | 38 | 47 | 100 | 37 | 35 | 31 | 28 | 27 | 29 | 31 | 28 | 28 | 27 | 27 | 28 | 28 | 30 | 25 | 25 | 24 | 24 | 22 | 20 |
| ERBV | 35 | 35 | 35 | 38 | 37 | 100 | 27 | 29 | 27 | 30 | 30 | 29 | 30 | 27 | 30 | 28 | 26 | 27 | 27 | 23 | 23 | 27 | 24 | 23 | 24 |
| PTV-1 | 32 | 30 | 31 | 30 | 35 | 27 | 100 | 27 | 28 | 30 | 30 | 29 | 28 | 29 | 27 | 27 | 29 | 28 | 28 | 27 | 26 | 21 | 22 | 21 | 24 |
| PV-1 | 29 | 27 | 28 | 31 | 31 | 29 | 27 | 100 | 79 | 64 | 63 | 62 | 58 | 59 | 58 | 61 | 49 | 45 | 39 | 24 | 22 | 20 | 23 | 24 | 23 |
| CV-A21 | 28 | 27 | 26 | 28 | 28 | 27 | 28 | 79 | 100 | 58 | 59 | 56 | 55 | 56 | 56 | 56 | 43 | 41 | 38 | 24 | 21 | 19 | 22 | 23 | 21 |
| CV-A16 | 30 | 29 | 26 | 32 | 27 | 30 | 30 | 64 | 58 | 100 | 66 | 60 | 63 | 64 | 58 | 55 | 48 | 44 | 40 | 24 | 23 | 22 | 24 | 24 | 24 |
| CV-B5 | 29 | 28 | 29 | 30 | 29 | 30 | 30 | 63 | 59 | 66 | 100 | 67 | 71 | 63 | 59 | 57 | 46 | 43 | 37 | 23 | 23 | 22 | 24 | 26 | 25 |
| EV-70 | 30 | 28 | 28 | 32 | 31 | 29 | 29 | 62 | 56 | 60 | 67 | 100 | 65 | 59 | 56 | 53 | 45 | 43 | 39 | 24 | 23 | 21 | 22 | 25 | 23 |
| SEV-A | 26 | 27 | 26 | 30 | 28 | 30 | 28 | 58 | 55 | 63 | 71 | 65 | 100 | 61 | 60 | 54 | 45 | 42 | 40 | 21 | 22 | 21 | 22 | 25 | 25 |
| BEV-1 | 26 | 27 | 25 | 31 | 28 | 27 | 29 | 59 | 56 | 64 | 63 | 59 | 61 | 100 | 67 | 53 | 47 | 44 | 40 | 23 | 23 | 21 | 21 | 24 | 24 |
| PEV-9 | 26 | 27 | 24 | 27 | 27 | 30 | 27 | 58 | 56 | 58 | 59 | 56 | 60 | 67 | 100 | 52 | 42 | 41 | 38 | 22 | 21 | 22 | 22 | 23 | 21 |
| HRV-14 | 29 | 25 | 27 | 31 | 27 | 28 | 27 | 61 | 56 | 55 | 57 | 53 | 54 | 53 | 52 | 100 | 47 | 42 | 40 | 21 | 20 | 21 | 19 | 22 | 23 |
| HRV-2 | 27 | 27 | 26 | 28 | 28 | 26 | 29 | 49 | 43 | 48 | 46 | 45 | 45 | 47 | 42 | 47 | 100 | 40 | 37 | 22 | 21 | 21 | 20 | 22 | 24 |
| SV2 | 28 | 26 | 29 | 31 | 28 | 27 | 28 | 45 | 41 | 44 | 43 | 43 | 42 | 44 | 41 | 42 | 40 | 100 | 52 | 22 | 22 | 22 | 20 | 21 | 21 |
| PEV-8 | 29 | 25 | 25 | 31 | 30 | 27 | 28 | 39 | 38 | 40 | 37 | 39 | 40 | 40 | 38 | 40 | 37 | 52 | 100 | 21 | 20 | 21 | 20 | 21 | 20 |
| AiV | 26 | 23 | 23 | 27 | 25 | 23 | 27 | 24 | 24 | 24 | 23 | 24 | 21 | 23 | 22 | 21 | 22 | 22 | 21 | 100 | 69 | 24 | 22 | 22 | 22 |
| BKV | 23 | 24 | 23 | 28 | 25 | 23 | 26 | 22 | 21 | 23 | 23 | 23 | 22 | 23 | 21 | 20 | 21 | 22 | 20 | 69 | 100 | 25 | 23 | 22 | 22 |
| HAV | 25 | 27 | 26 | 25 | 24 | 27 | 21 | 20 | 19 | 22 | 22 | 21 | 21 | 21 | 22 | 21 | 21 | 22 | 21 | 24 | 25 | 100 | 41 | 20 | 21 |
| AEV | 26 | 25 | 25 | 24 | 24 | 24 | 22 | 23 | 22 | 24 | 24 | 22 | 22 | 21 | 22 | 19 | 20 | 20 | 20 | 22 | 23 | 41 | 100 | 22 | 23 |
| HPeV-1 | 21 | 20 | 20 | 20 | 22 | 23 | 21 | 24 | 23 | 24 | 26 | 25 | 25 | 24 | 23 | 22 | 22 | 21 | 21 | 22 | 22 | 20 | 22 | 100 | 50 |
| LV | 20 | 21 | 21 | 21 | 20 | 24 | 24 | 23 | 21 | 24 | 25 | 23 | 25 | 24 | 21 | 23 | 24 | 21 | 20 | 22 | 22 | 21 | 23 | 50 | 100 |

Fig. 42

| | Cardiovirus | | | Aphthovirus | | Erbo | Tescho | Enterovirus | | | | | | | | Rhinovirus | | Entero? | | Kobu | | Hepato | | Parecho | |
|---|---|---|---|---|---|---|---|---|---|---|---|---|---|---|---|---|---|---|---|---|---|---|---|---|---|
| | SVV | EMCV | TMEV | FMDV-O | ERAV | ERBV | PTV-1 | PV-1 | CV-A21 | CV-A16 | CV-B5 | EV-70 | SEV-A | BEV-1 | PEV-9 | HRV-14 | HRV-2 | SV2 | PEV-8 | AiV | BKV | HAV | AEV | HPeV-1 | LV |
| SVV | 100 | 38 | 37 | 24 | 21 | 19 | 27 | 19 | 18 | 14 | 16 | 17 | 14 | 18 | 19 | 18 | 15 | 17 | 16 | 16 | 14 | 17 | 16 | 13 | 14 |
| EMCV | 38 | 100 | 48 | 27 | 27 | 18 | 24 | 18 | 18 | 18 | 20 | 20 | 19 | 17 | 20 | 19 | 20 | 19 | 19 | 15 | 16 | 22 | 18 | 17 | 19 |
| TMEV | 37 | 48 | 100 | 20 | 28 | 20 | 25 | 19 | 20 | 20 | 18 | 20 | 16 | 19 | 18 | 15 | 18 | 17 | 17 | 16 | 17 | 17 | 16 | 16 | 19 |
| FMDV-O | 24 | 27 | 20 | 100 | 40 | 23 | 20 | 19 | 19 | 22 | 17 | 19 | 17 | 22 | 23 | 18 | 20 | 20 | 20 | 16 | 13 | 20 | 20 | 13 | 15 |
| ERAV | 21 | 27 | 28 | 40 | 100 | 25 | 22 | 21 | 20 | 20 | 20 | 21 | 22 | 20 | 18 | 19 | 20 | 21 | 22 | 19 | 20 | 22 | 22 | 15 | 17 |
| ERBV | 19 | 18 | 20 | 23 | 25 | 100 | 18 | 19 | 20 | 16 | 17 | 17 | 17 | 20 | 16 | 18 | 14 | 20 | 17 | 18 | 20 | 15 | 20 | 14 | 16 |
| PTV-1 | 27 | 24 | 25 | 20 | 22 | 18 | 100 | 20 | 19 | 20 | 19 | 20 | 19 | 20 | 20 | 22 | 21 | 18 | 21 | 16 | 17 | 20 | 15 | 13 | 14 |
| PV-1 | 19 | 18 | 19 | 19 | 21 | 19 | 20 | 100 | 95 | 55 | 60 | 60 | 56 | 57 | 60 | 46 | 44 | 43 | 47 | 21 | 17 | 18 | 18 | 18 | 18 |
| CV-A21 | 18 | 18 | 20 | 19 | 20 | 20 | 19 | 95 | 100 | 56 | 59 | 57 | 57 | 57 | 60 | 46 | 44 | 42 | 46 | 22 | 17 | 18 | 18 | 19 | 18 |
| CV-A16 | 14 | 18 | 20 | 22 | 20 | 16 | 20 | 55 | 56 | 100 | 56 | 54 | 57 | 57 | 60 | 51 | 45 | 46 | 50 | 17 | 18 | 19 | 18 | 20 | 22 |
| CV-B5 | 16 | 20 | 18 | 17 | 20 | 17 | 19 | 60 | 59 | 56 | 100 | 64 | 55 | 58 | 56 | 52 | 49 | 46 | 52 | 20 | 19 | 19 | 19 | 19 | 20 |
| EV-70 | 17 | 20 | 20 | 19 | 21 | 17 | 20 | 60 | 57 | 54 | 64 | 100 | 57 | 54 | 55 | 51 | 48 | 43 | 51 | 20 | 20 | 21 | 18 | 14 | 17 |
| SEV-A | 14 | 19 | 16 | 17 | 22 | 17 | 19 | 56 | 57 | 57 | 55 | 57 | 100 | 54 | 55 | 53 | 46 | 40 | 49 | 20 | 17 | 19 | 18 | 17 | 19 |
| BEV-1 | 18 | 17 | 19 | 22 | 20 | 20 | 20 | 57 | 57 | 57 | 58 | 54 | 54 | 100 | 63 | 47 | 47 | 41 | 44 | 16 | 16 | 20 | 20 | 19 | 16 |
| PEV-9 | 19 | 20 | 18 | 23 | 18 | 16 | 20 | 60 | 60 | 60 | 56 | 55 | 55 | 63 | 100 | 50 | 45 | 45 | 50 | 18 | 14 | 19 | 18 | 18 | 21 |
| HRV-14 | 18 | 19 | 15 | 18 | 19 | 18 | 22 | 46 | 46 | 51 | 52 | 51 | 53 | 47 | 50 | 100 | 50 | 42 | 49 | 20 | 20 | 20 | 20 | 16 | 18 |
| HRV-2 | 15 | 20 | 18 | 20 | 20 | 14 | 21 | 44 | 44 | 45 | 49 | 48 | 46 | 47 | 45 | 50 | 100 | 43 | 43 | 20 | 23 | 18 | 19 | 17 | 18 |
| SV2 | 17 | 19 | 17 | 20 | 21 | 20 | 18 | 43 | 42 | 46 | 46 | 43 | 40 | 41 | 45 | 42 | 43 | 100 | 60 | 16 | 18 | 18 | 19 | 22 | 24 |
| PEV-8 | 16 | 19 | 17 | 20 | 22 | 17 | 21 | 47 | 46 | 50 | 52 | 51 | 49 | 44 | 50 | 49 | 43 | 60 | 100 | 16 | 17 | 19 | 20 | 20 | 19 |
| AiV | 16 | 15 | 16 | 16 | 19 | 18 | 16 | 21 | 22 | 17 | 20 | 20 | 20 | 16 | 18 | 20 | 20 | 16 | 16 | 100 | 48 | 19 | 17 | 13 | 15 |
| BKV | 14 | 16 | 17 | 13 | 20 | 20 | 17 | 17 | 17 | 18 | 19 | 20 | 17 | 16 | 14 | 20 | 23 | 18 | 17 | 48 | 100 | 18 | 18 | 13 | 14 |
| HAV | 17 | 22 | 17 | 20 | 22 | 15 | 20 | 18 | 18 | 19 | 19 | 21 | 19 | 20 | 19 | 20 | 18 | 18 | 19 | 19 | 18 | 100 | 40 | 16 | 15 |
| AEV | 16 | 18 | 16 | 20 | 22 | 20 | 15 | 18 | 18 | 18 | 19 | 18 | 18 | 20 | 18 | 20 | 19 | 19 | 20 | 17 | 18 | 40 | 100 | 14 | 16 |
| HPeV-1 | 13 | 17 | 16 | 13 | 15 | 14 | 13 | 18 | 19 | 20 | 19 | 14 | 17 | 19 | 18 | 16 | 17 | 22 | 20 | 13 | 13 | 16 | 14 | 100 | 48 |
| LV | 14 | 19 | 19 | 15 | 17 | 16 | 14 | 18 | 18 | 22 | 20 | 17 | 19 | 16 | 21 | 18 | 18 | 24 | 19 | 15 | 14 | 15 | 16 | 48 | 100 |

**Fig. 43**

| | Cardiovirus | | | Aphthovirus | | Erbo | Tescho | Enterovirus | | | | | | | | Rhinovirus | | Entero? | | Kobu | | Hepato | | Parecho | |
|---|---|---|---|---|---|---|---|---|---|---|---|---|---|---|---|---|---|---|---|---|---|---|---|---|---|
| | SVV | EMCV | TMEV | FMDV-O | ERAV | ERBV | PTV-1 | PV-1 | CV-A21 | CV-A16 | CV-B5 | EV-70 | SEV-A | BEV-1 | PEV-9 | HRV-14 | HRV-2 | SV2 | PEV-8 | AiV | BKV | HAV | AEV | HPeV-1 | LV |
| SVV | 100 | 58 | 58 | 47 | 48 | 42 | 40 | 33 | 32 | 30 | 30 | 32 | 31 | 31 | 32 | 34 | 31 | 32 | 31 | 33 | 32 | 24 | 24 | 23 | 23 |
| EMCV | 58 | 100 | 65 | 43 | 46 | 46 | 38 | 31 | 30 | 29 | 30 | 29 | 31 | 30 | 31 | 32 | 32 | 31 | 31 | 35 | 33 | 24 | 25 | 21 | 24 |
| TMEV | 58 | 65 | 100 | 42 | 47 | 42 | 38 | 30 | 29 | 28 | 30 | 30 | 30 | 30 | 29 | 31 | 31 | 30 | 32 | 34 | 32 | 24 | 26 | 22 | 25 |
| FMDV-O | 47 | 43 | 42 | 100 | 50 | 37 | 39 | 31 | 31 | 30 | 30 | 31 | 32 | 28 | 31 | 32 | 31 | 31 | 31 | 35 | 35 | 22 | 24 | 21 | 23 |
| ERAV | 48 | 46 | 47 | 50 | 100 | 41 | 37 | 29 | 29 | 29 | 29 | 31 | 31 | 29 | 29 | 31 | 30 | 29 | 30 | 34 | 35 | 25 | 24 | 22 | 24 |
| ERBV | 42 | 46 | 42 | 37 | 41 | 100 | 40 | 33 | 33 | 33 | 34 | 34 | 34 | 33 | 35 | 35 | 35 | 34 | 35 | 33 | 34 | 21 | 25 | 23 | 26 |
| PTV-1 | 40 | 38 | 38 | 39 | 37 | 40 | 100 | 34 | 34 | 32 | 34 | 34 | 35 | 31 | 35 | 34 | 34 | 36 | 35 | 31 | 31 | 27 | 23 | 22 | 23 |
| PV-1 | 33 | 31 | 30 | 31 | 29 | 33 | 34 | 100 | 98 | 69 | 75 | 76 | 67 | 68 | 71 | 64 | 57 | 56 | 55 | 33 | 32 | 27 | 27 | 23 | 27 |
| CV-A21 | 32 | 30 | 29 | 31 | 29 | 33 | 34 | 98 | 100 | 68 | 75 | 75 | 66 | 68 | 71 | 64 | 57 | 56 | 55 | 33 | 32 | 27 | 26 | 23 | 27 |
| CV-A16 | 30 | 29 | 28 | 30 | 29 | 33 | 32 | 69 | 68 | 100 | 67 | 65 | 59 | 62 | 66 | 61 | 54 | 55 | 53 | 33 | 35 | 27 | 26 | 23 | 26 |
| CV-B5 | 30 | 30 | 30 | 30 | 29 | 34 | 34 | 75 | 75 | 67 | 100 | 76 | 69 | 70 | 71 | 66 | 57 | 57 | 55 | 32 | 32 | 25 | 25 | 23 | 27 |
| EV-70 | 32 | 29 | 30 | 31 | 31 | 34 | 34 | 76 | 75 | 65 | 76 | 100 | 65 | 67 | 68 | 63 | 58 | 58 | 55 | 33 | 35 | 25 | 26 | 22 | 26 |
| SEV-A | 31 | 31 | 30 | 32 | 31 | 34 | 35 | 67 | 66 | 59 | 69 | 65 | 100 | 64 | 65 | 58 | 56 | 55 | 53 | 33 | 33 | 25 | 27 | 25 | 26 |
| BEV-1 | 31 | 30 | 30 | 28 | 29 | 33 | 31 | 68 | 68 | 62 | 70 | 67 | 64 | 100 | 75 | 60 | 57 | 54 | 53 | 31 | 31 | 25 | 26 | 25 | 27 |
| PEV-9 | 32 | 31 | 29 | 31 | 29 | 35 | 35 | 71 | 71 | 66 | 71 | 68 | 65 | 75 | 100 | 64 | 58 | 58 | 54 | 32 | 33 | 29 | 27 | 23 | 28 |
| HRV-14 | 34 | 32 | 31 | 32 | 31 | 35 | 34 | 64 | 64 | 61 | 66 | 63 | 58 | 60 | 64 | 100 | 56 | 53 | 54 | 34 | 34 | 27 | 28 | 24 | 27 |
| HRV-2 | 31 | 32 | 31 | 31 | 30 | 35 | 34 | 57 | 57 | 54 | 57 | 58 | 56 | 57 | 58 | 56 | 100 | 53 | 54 | 31 | 32 | 25 | 27 | 23 | 27 |
| SV2 | 32 | 31 | 30 | 31 | 29 | 34 | 36 | 56 | 56 | 55 | 57 | 58 | 55 | 54 | 58 | 53 | 53 | 100 | 67 | 35 | 36 | 27 | 28 | 21 | 26 |
| PEV-8 | 31 | 31 | 32 | 31 | 30 | 35 | 35 | 55 | 55 | 53 | 55 | 55 | 53 | 53 | 54 | 54 | 54 | 67 | 100 | 33 | 33 | 26 | 28 | 26 | 28 |
| AiV | 33 | 35 | 34 | 35 | 34 | 33 | 31 | 33 | 33 | 33 | 32 | 33 | 33 | 31 | 32 | 34 | 31 | 35 | 33 | 100 | 75 | 25 | 27 | 23 | 23 |
| BKV | 32 | 33 | 32 | 35 | 35 | 34 | 31 | 32 | 32 | 35 | 32 | 35 | 33 | 31 | 33 | 34 | 32 | 36 | 33 | 75 | 100 | 25 | 27 | 23 | 24 |
| HAV | 24 | 24 | 24 | 22 | 25 | 21 | 27 | 27 | 27 | 27 | 25 | 25 | 25 | 25 | 29 | 27 | 25 | 27 | 26 | 25 | 25 | 100 | 40 | 24 | 23 |
| AEV | 24 | 25 | 26 | 24 | 24 | 25 | 23 | 27 | 26 | 26 | 25 | 26 | 27 | 26 | 27 | 28 | 27 | 28 | 28 | 27 | 27 | 40 | 100 | 26 | 24 |
| HPeV-1 | 23 | 21 | 22 | 21 | 22 | 23 | 22 | 23 | 23 | 23 | 23 | 22 | 25 | 25 | 23 | 24 | 23 | 21 | 26 | 23 | 23 | 24 | 26 | 100 | 50 |
| LV | 23 | 24 | 25 | 23 | 24 | 26 | 23 | 27 | 27 | 26 | 27 | 26 | 26 | 27 | 28 | 27 | 27 | 26 | 28 | 23 | 24 | 23 | 24 | 50 | 100 |

Fig. 44

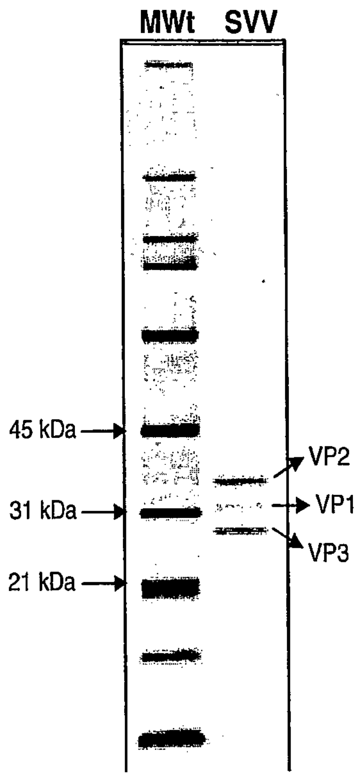


Fig. 45

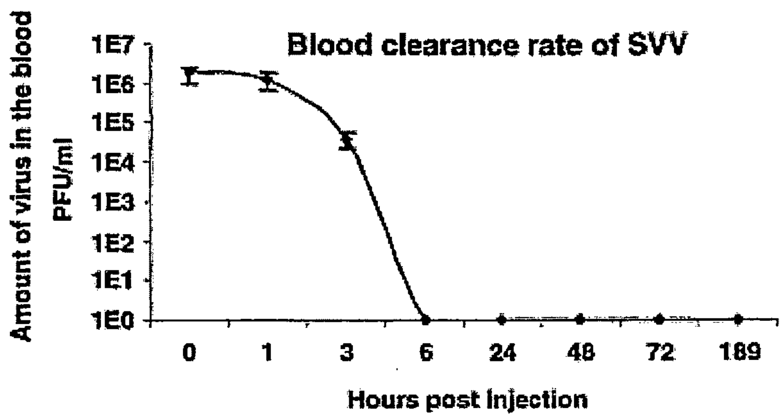


FIG. 46A

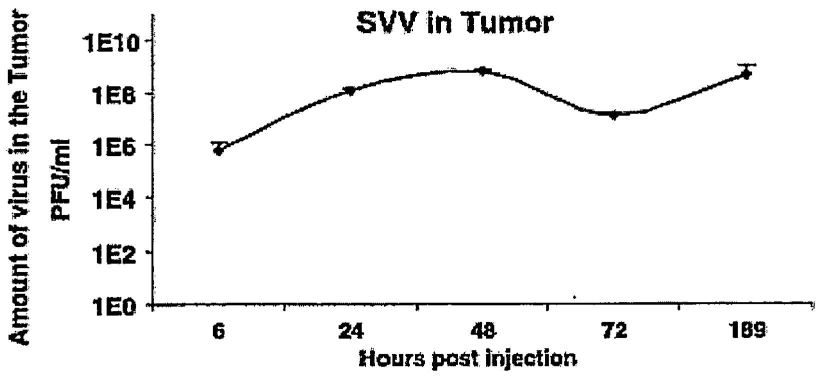


FIG. 46B

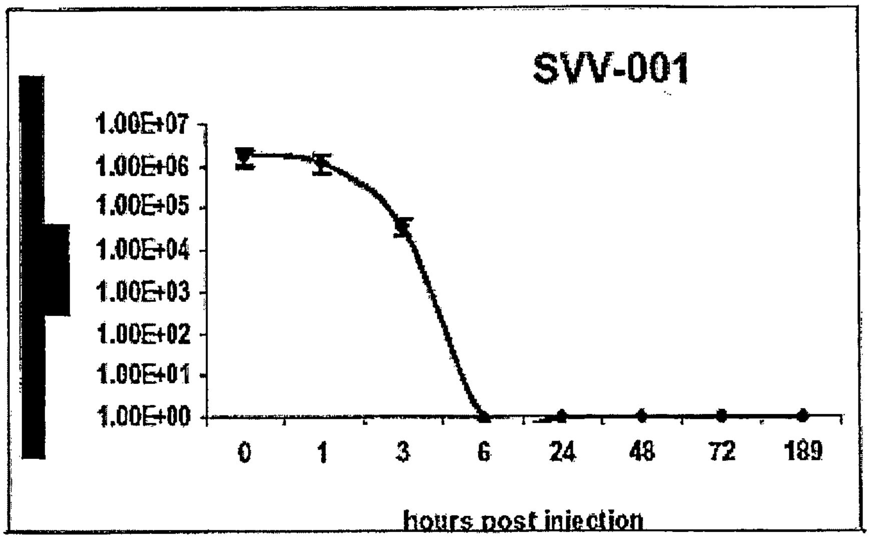


Fig. 46C

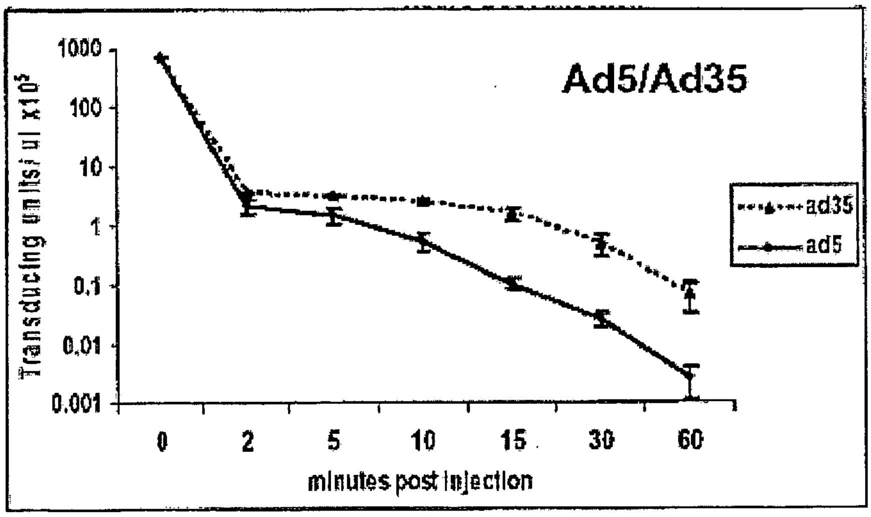


Fig. 46D

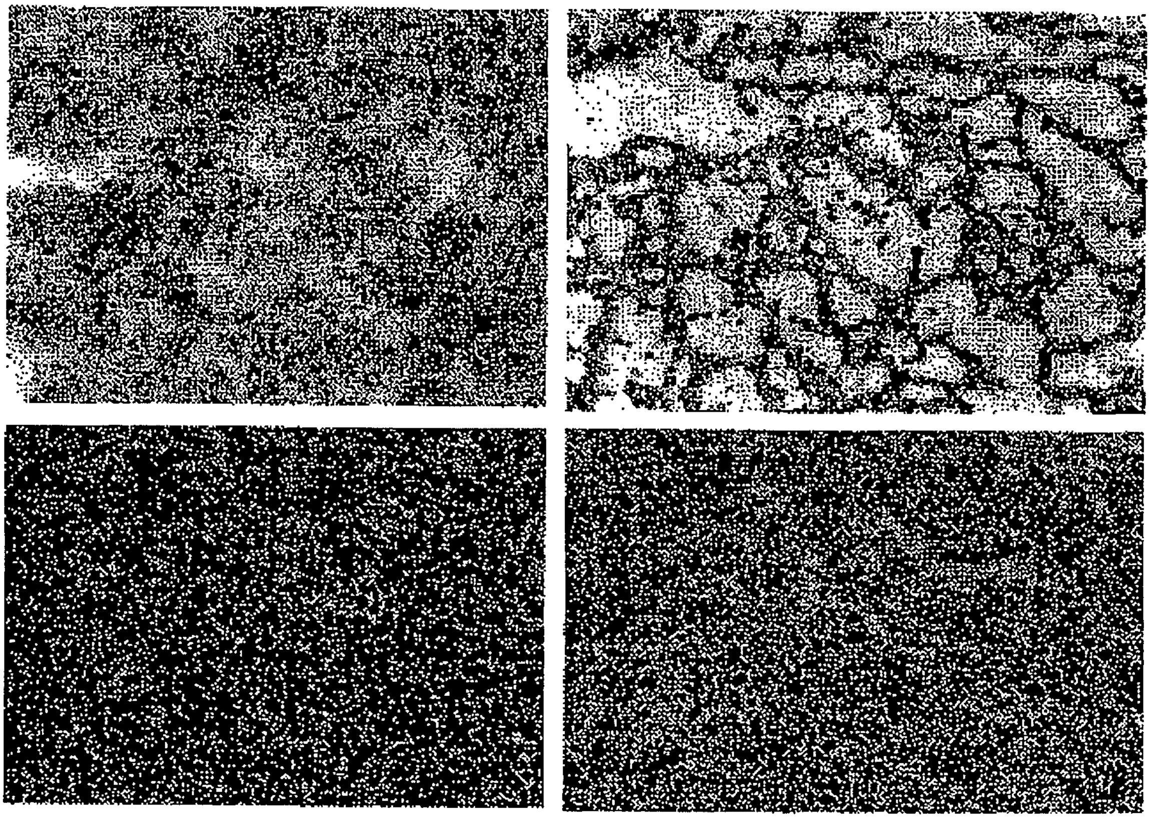

Fig. 47

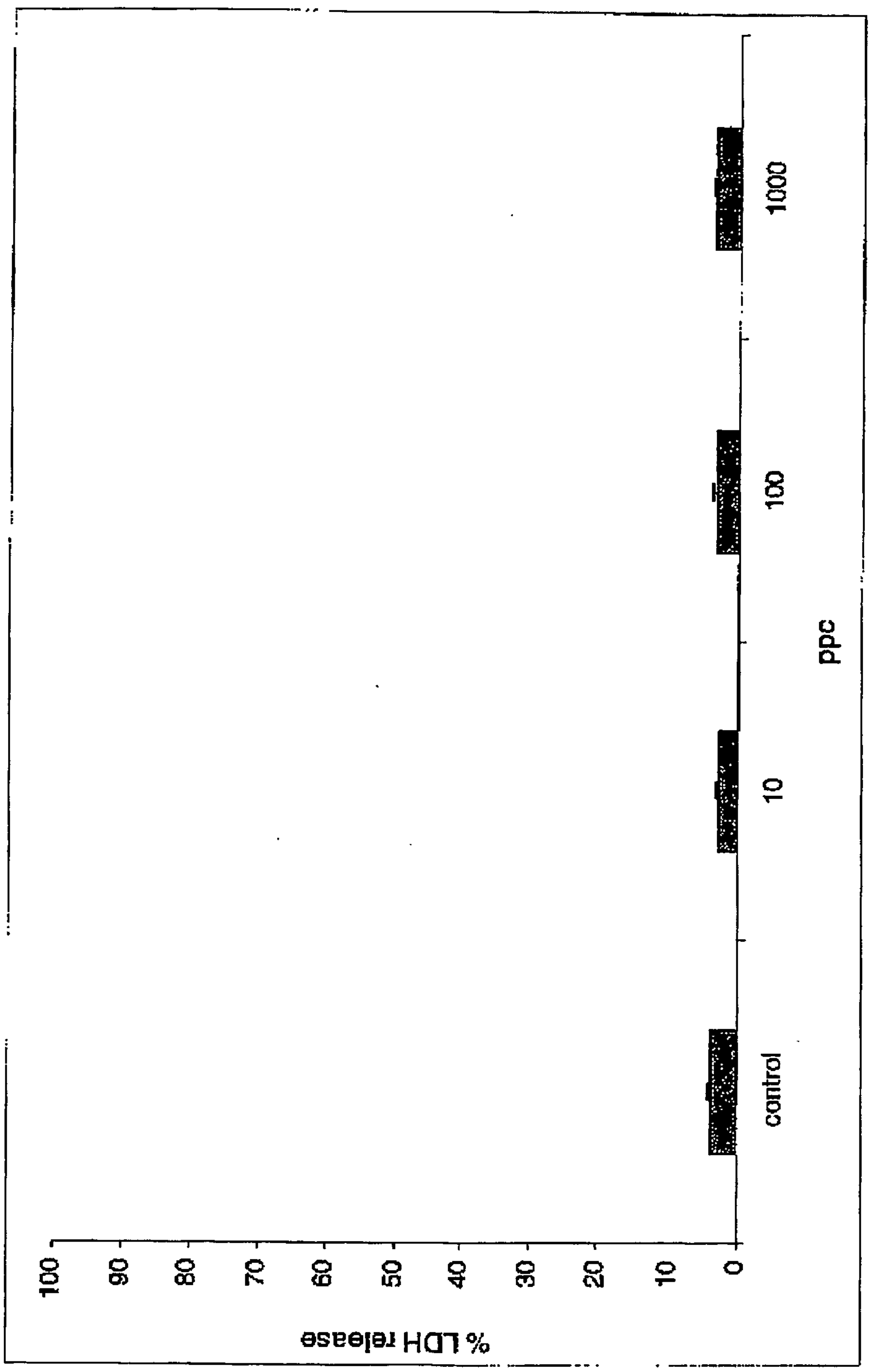
% LDH release
100
90
80
70
60
50
40
30
20
10
0
control
10
100
1000
ppc

Fig. 48

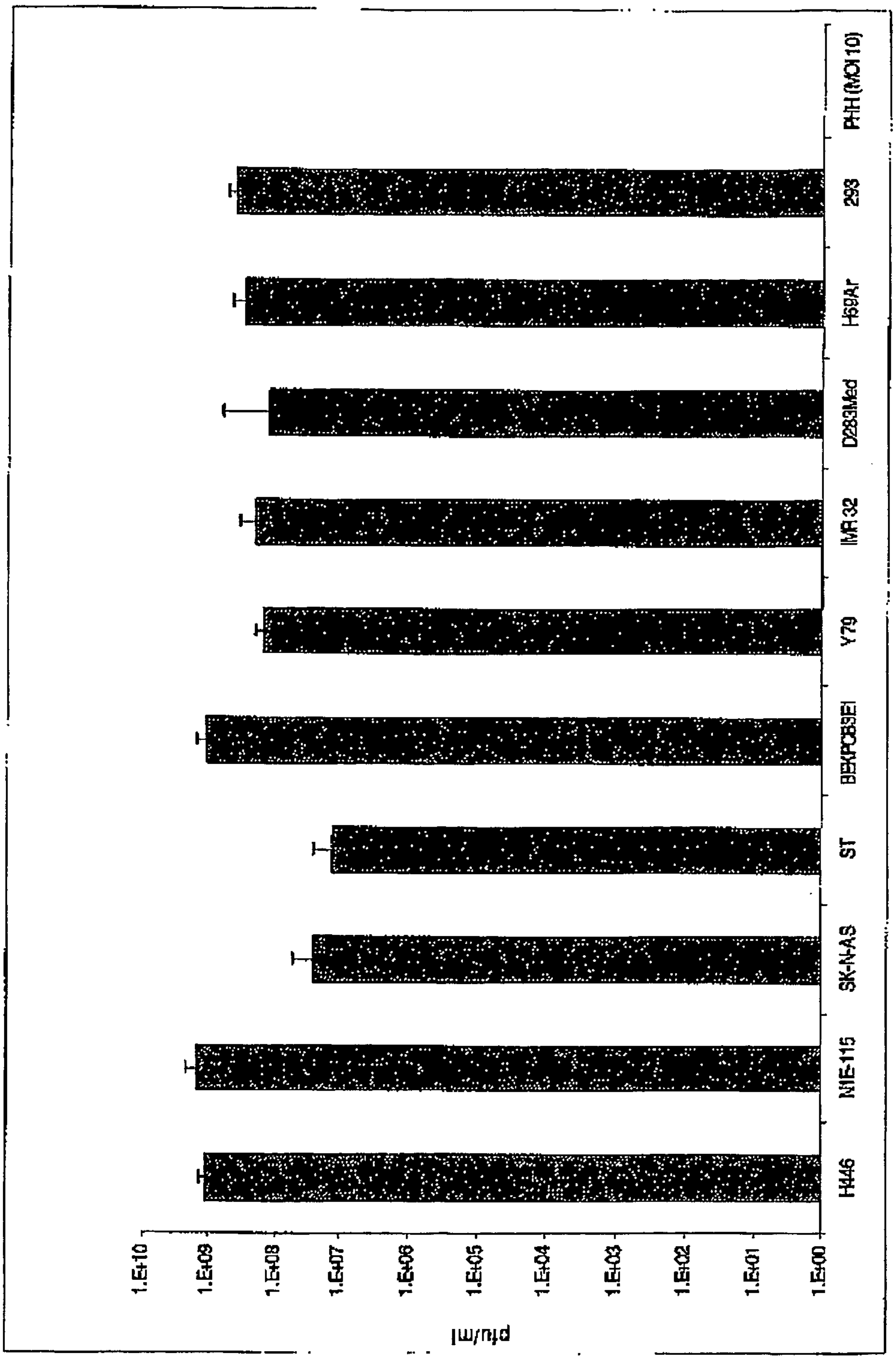
pfu/ml
1.E+10
1.E+09
1.E+08
1.E+07
1.E+06
1.E+05
1.E+04
1.E+03
1.E+02
1.E+01
1.E+00
H446
NIE-115
SK-N-AS
ST
BEKPCB3E1
Y79
IMR32
D283Med
H69Ar
293
PHH (MOI10)

Fig. 49

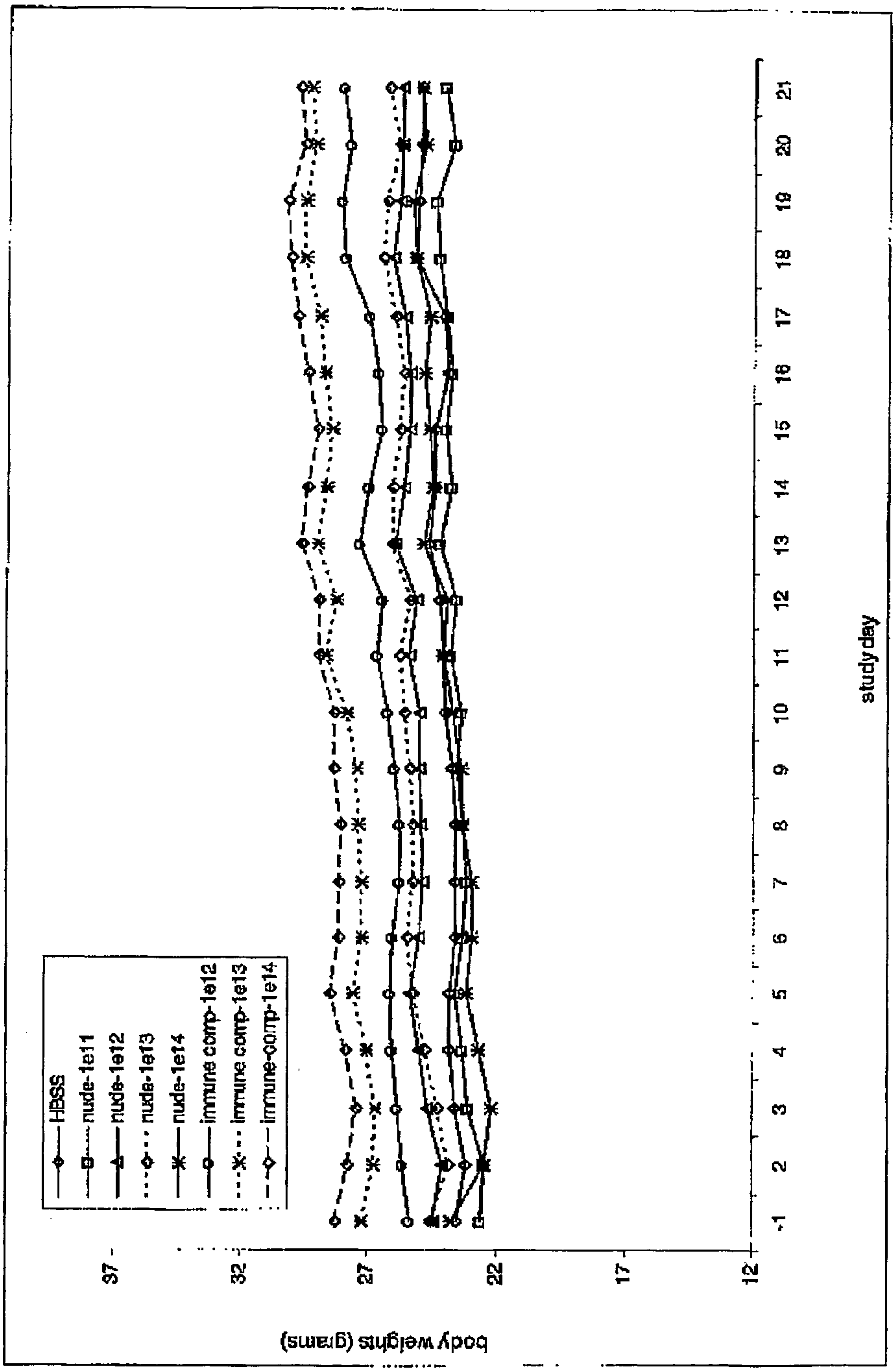
HBSS
nude-1e11
nude-1e12
nude-1e13
nude-1e14
immune comp-1e12
immune comp-1e13
immune-comp-1e14
body weights (grams)
37
32
27
22
17
12
study day
-1
2
3
4
5
6
7
8
9
10
11
12
13
14
15
16
17
18
19
20
21

Fig. 50

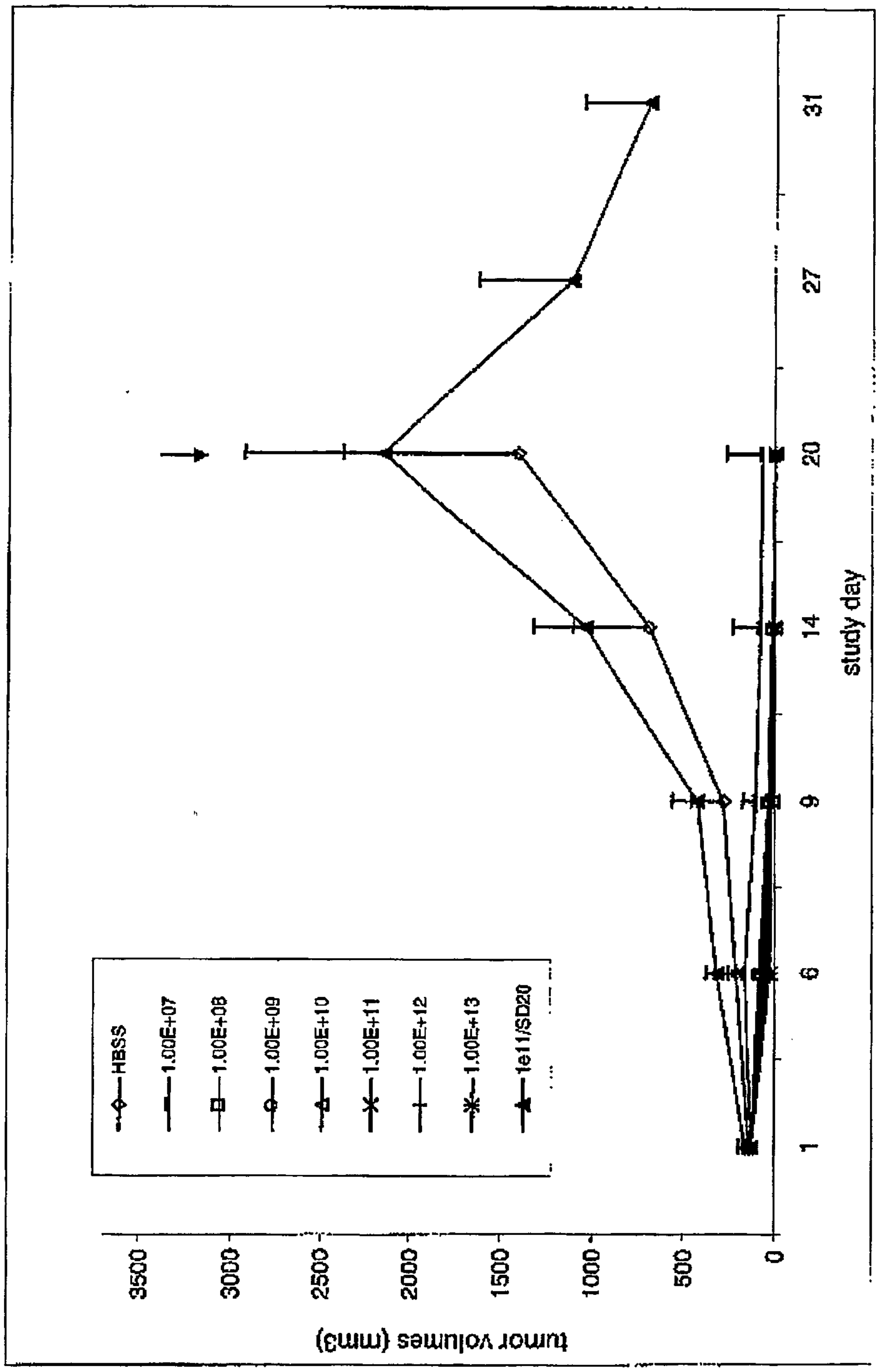
tumor volumes (mm3)
3500
3000
2500
2000
1500
1000
500
0
study day
1
6
9
14
20
27
31
HBSS
1.00E+07
1.00E+08
1.00E+09
1.00E+10
1.00E+11
1.00E+12
1.00E+13
1e11/SD20

Fig. 51

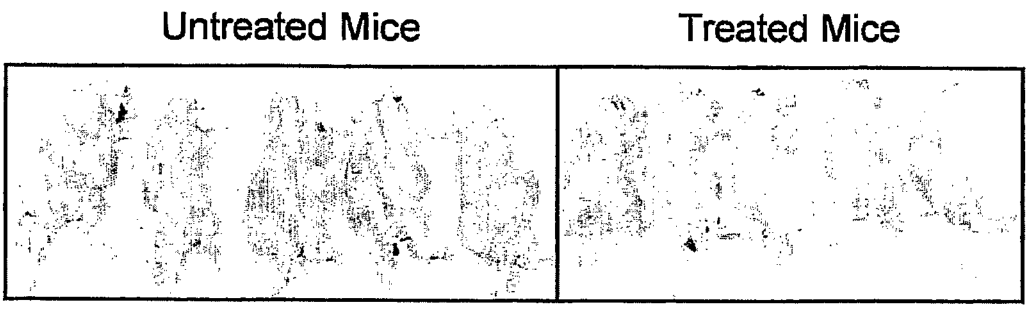


Fig. 52

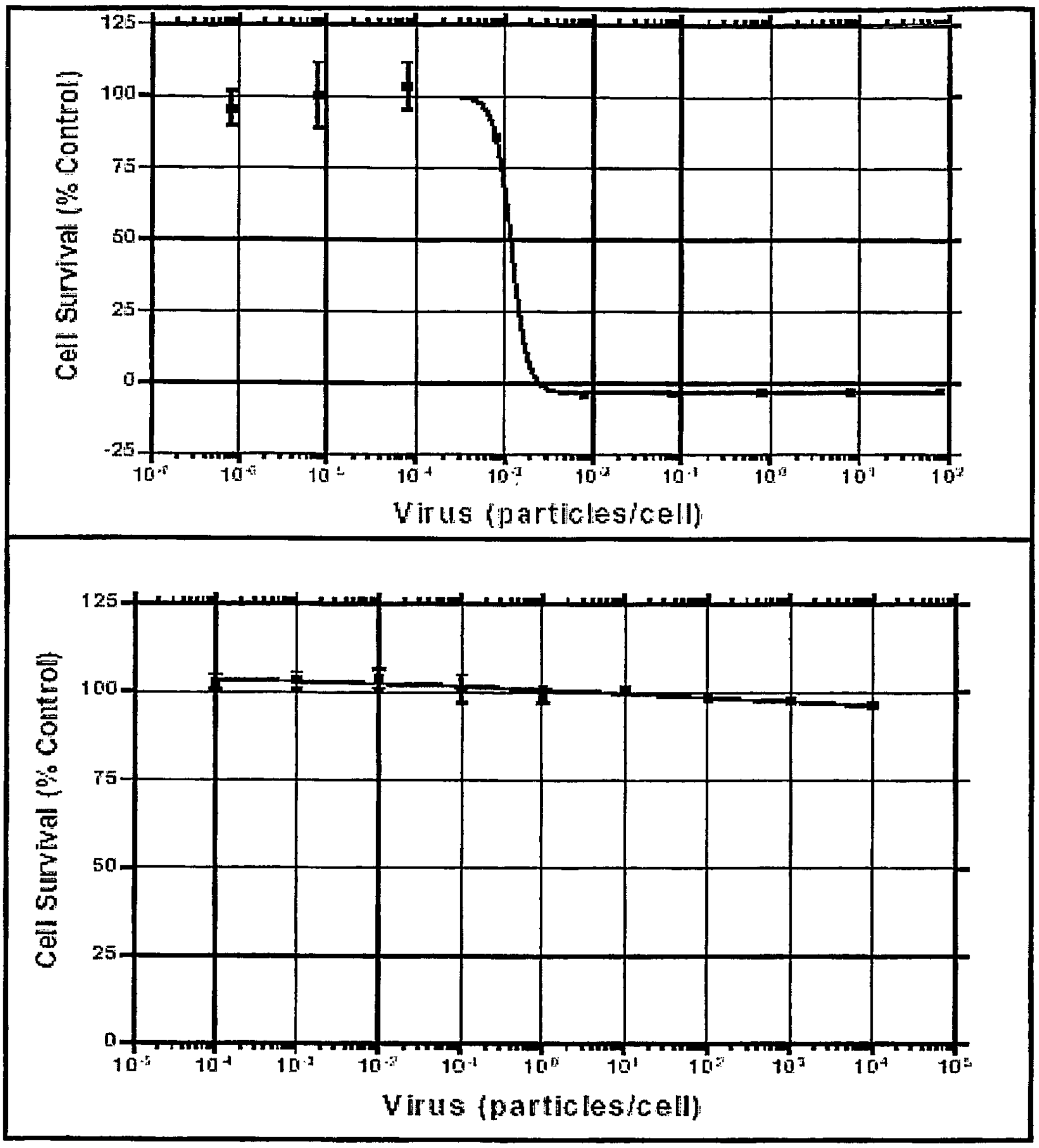
Cell Survival (% Control)
125
100
75
50
25
0
-25
Virus (particles/cell)
Cell Survival (% Control)
125
100
75
50
25
0
Virus (particles/cell)

Fig. 53

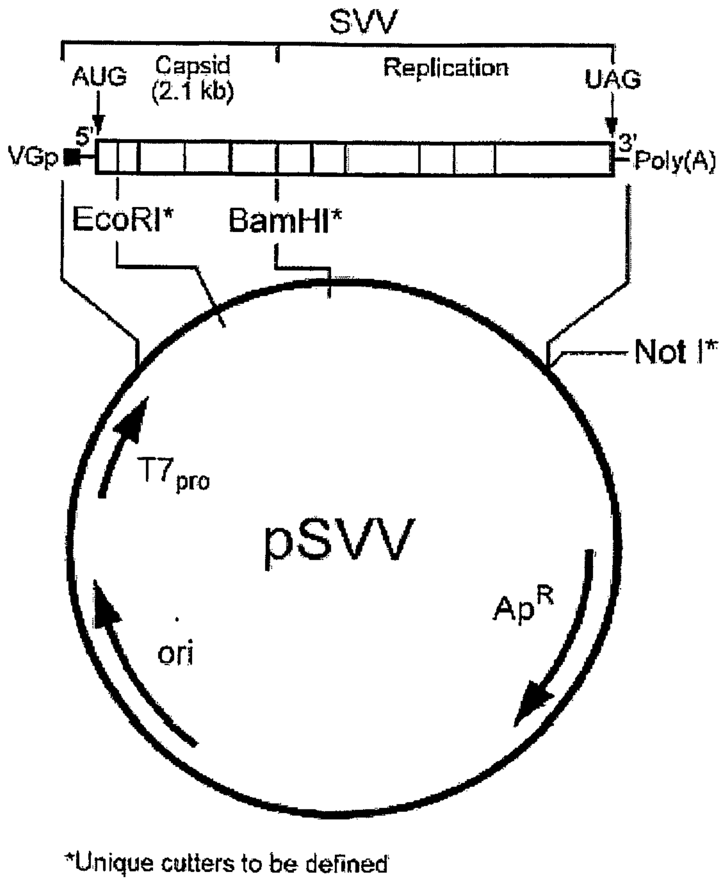
SVV
AUG
Capsid
(2.1 kb)
Replication
UAG
5'
3'
VGp
Poly(A)
EcoRI*
BamHI*
Not I*
T7pro
pSVV
ApR
ori
*Unique cutters to be defined

Fig. 54

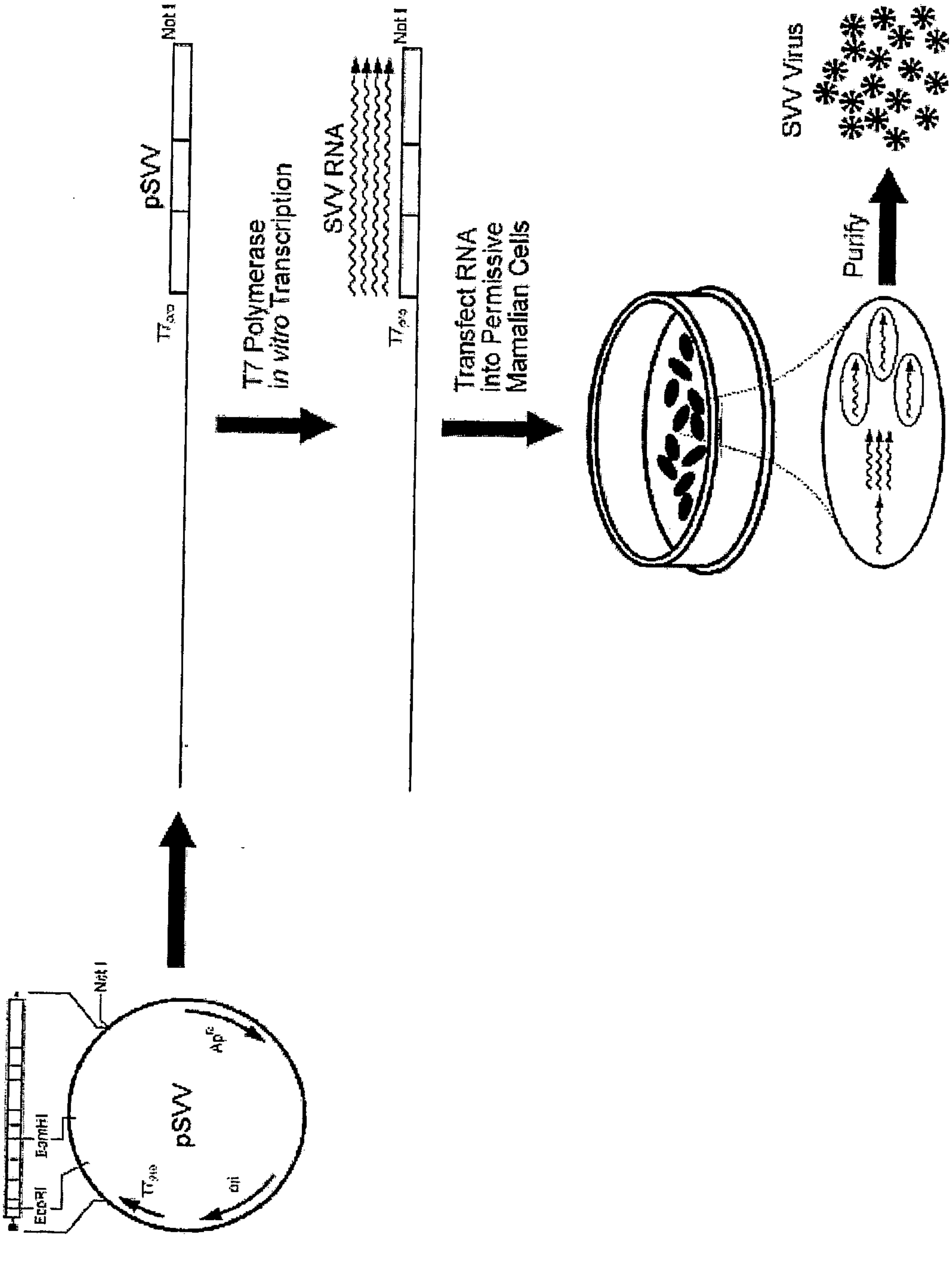
pSVV
EcoRI
BamHI
Not I
ori
pSVV
Not I
T7 Polymerase
in vitro Transcription
SVV RNA
Not I
Transfect RNA
into Permissive
Mamalian Cells
Purify
SVV Virus

Fig. 55

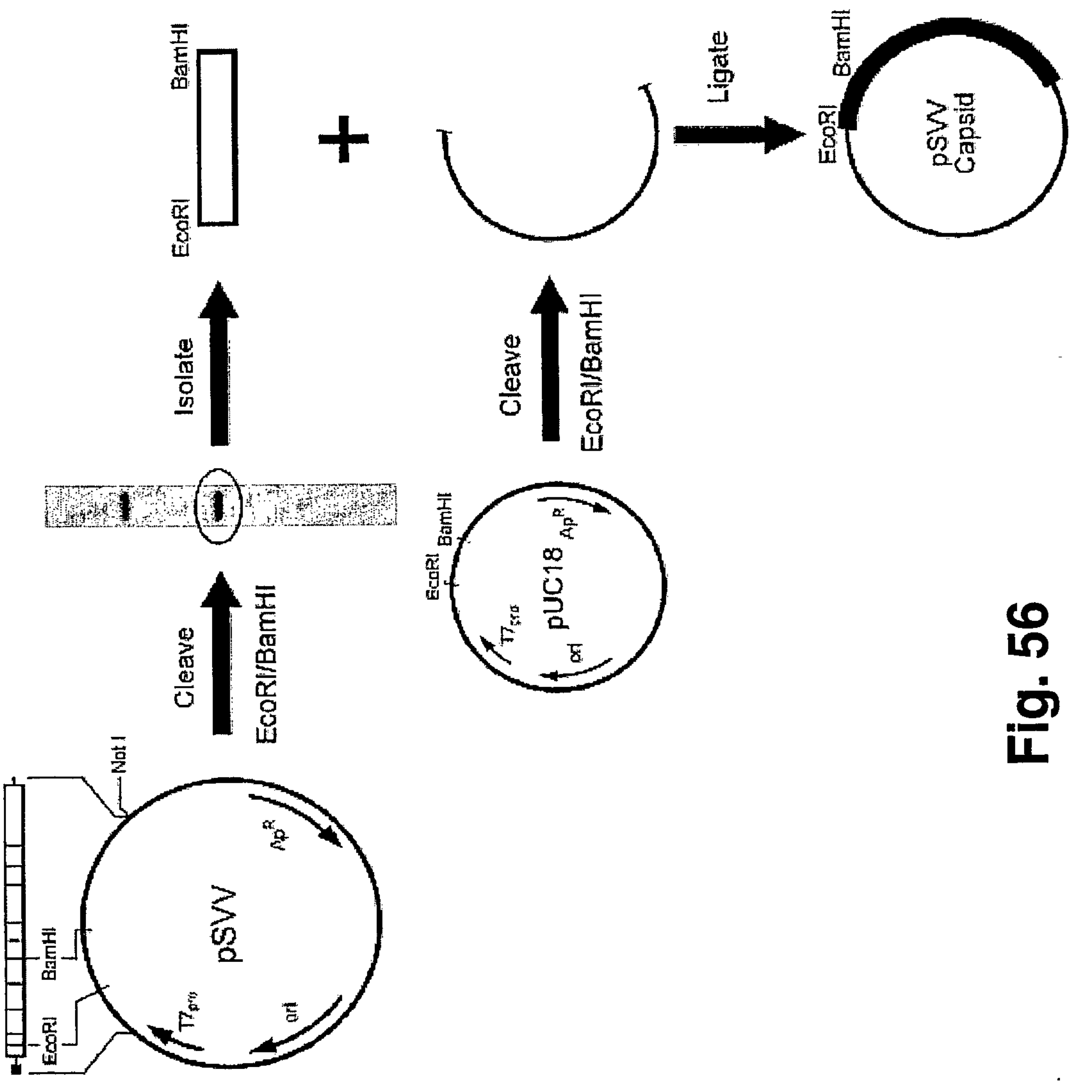
pSVV
Not I
EcoRI
BamHI
ori
Cleave
EcoRI/BamHI
Isolate
EcoRI
BamHI
pUC18
Cleave
EcoRI/BamHI
Ligate
EcoRI
BamHI
pSVV
Capsid

Fig. 56

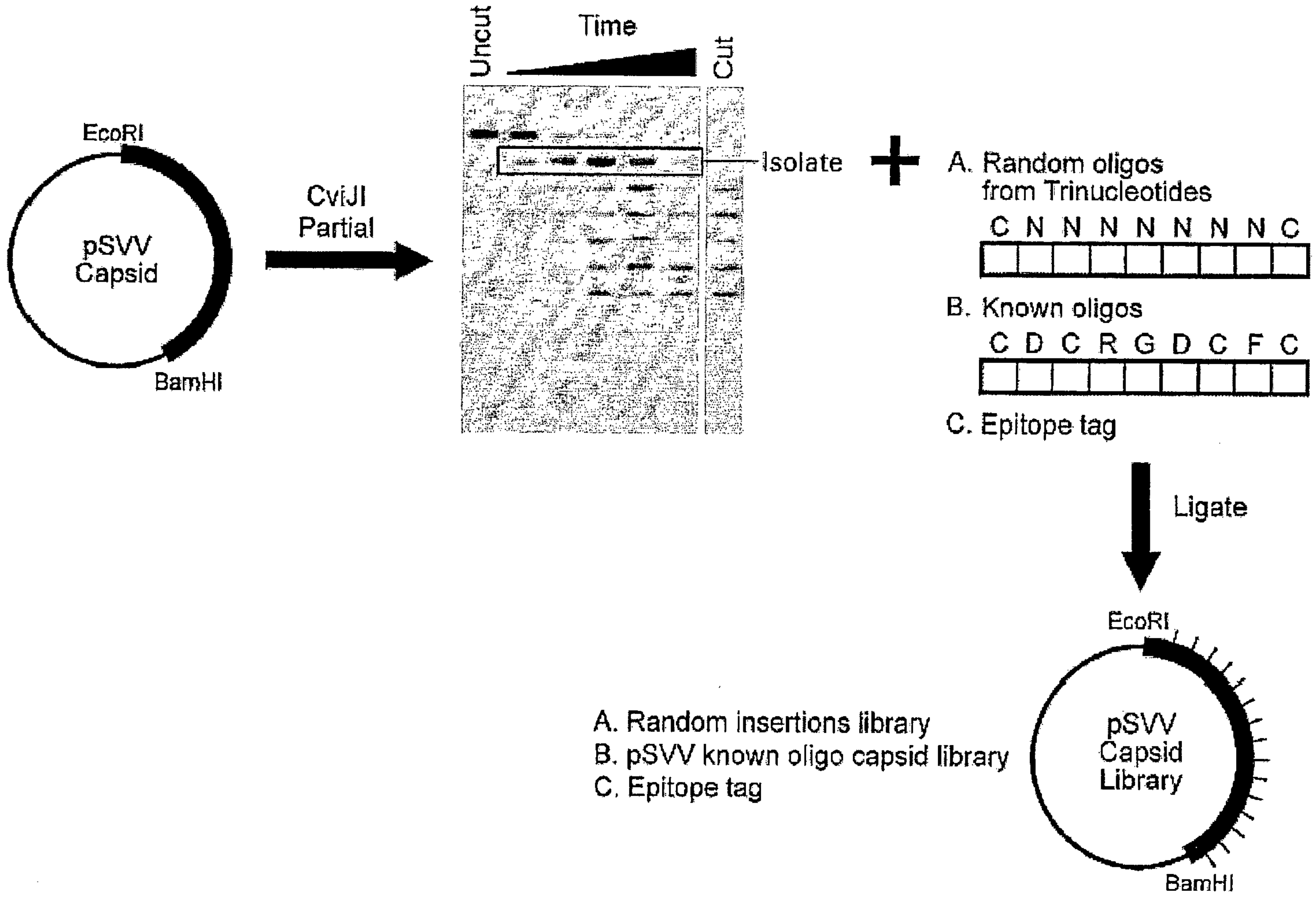
EcoRI
pSVV
Capsid
BamHI
CviJI
Partial
Uncut
Time
Cut
Isolate
A. Random oligos
from Trinucleotides
C N N N N N N N C
B. Known oligos
C D C R G D C F C
C. Epitope tag
Ligate
EcoRI
pSVV
Capsid
Library
BamHI
A. Random insertions library
B. pSVV known oligo capsid library
C. Epitope tag

Fig. 57

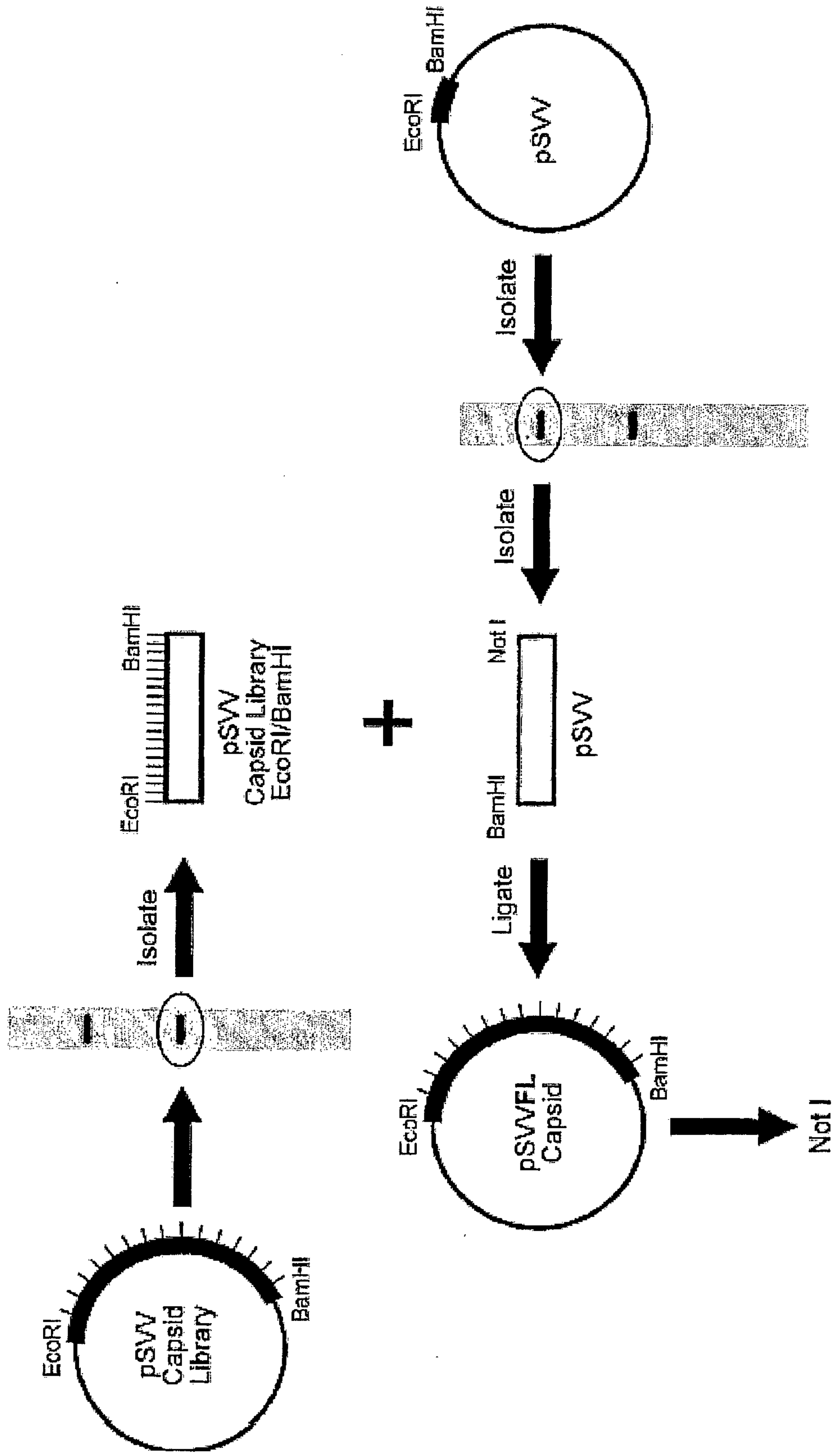
EcoRI
pSVV
Capsid
Library
BamHI
Isolate
EcoRI
BamHI
pSVV
Capsid Library
EcoRI/BamHI
EcoRI
BamHI
pSVV
Isolate
Isolate
BamHI
Not I
pSVV
Ligate
EcoRI
pSVVFL
Capsid
BamHI
Not I

Fig. 58

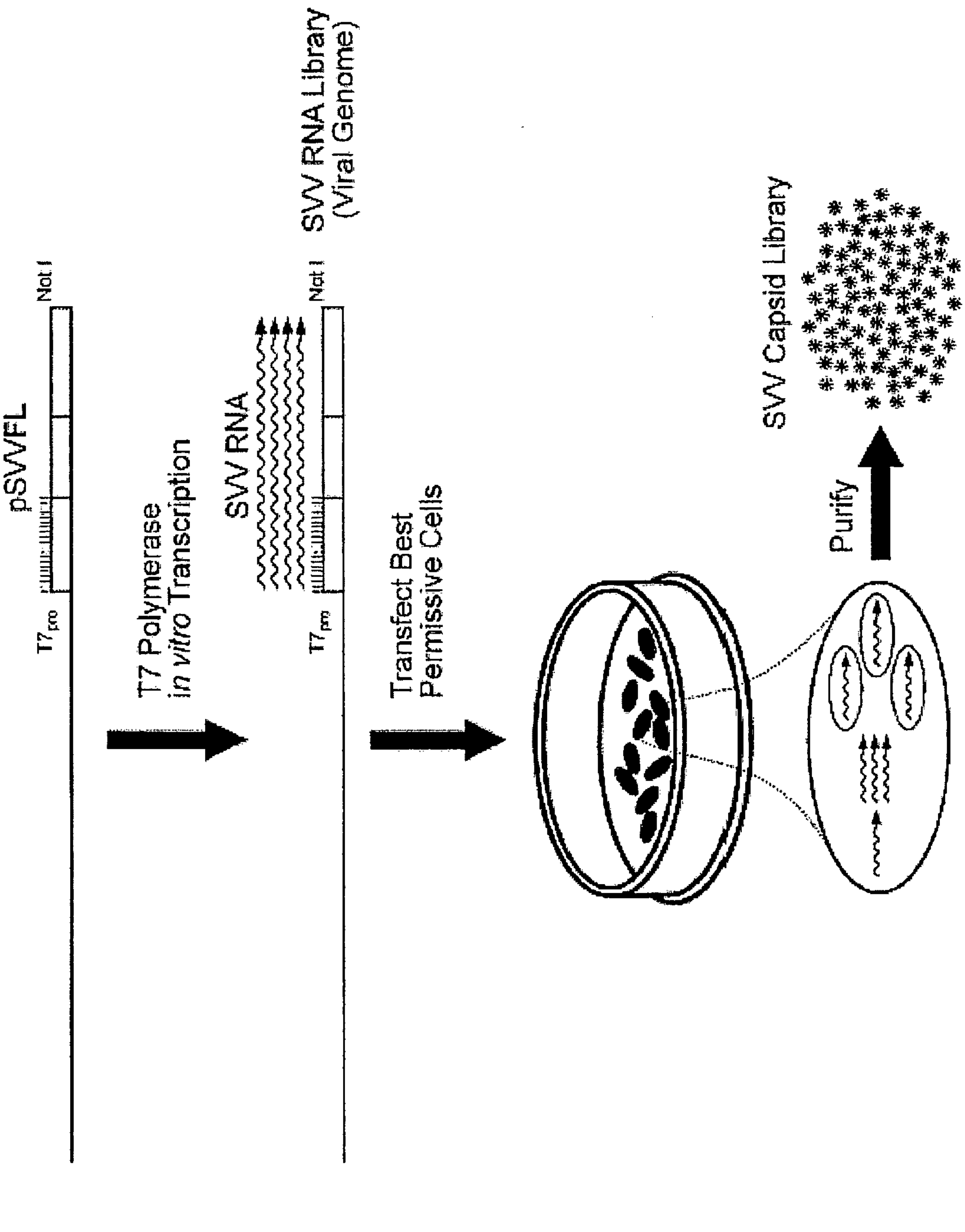
pSVVFL
T7pro
Not I
T7 Polymerase
in vitro Transcription
SVV RNA
T7pro
Not I
SVV RNA Library
(Viral Genome)
Transfect Best
Permissive Cells
Purify
SVV Capsid Library

Fig. 59

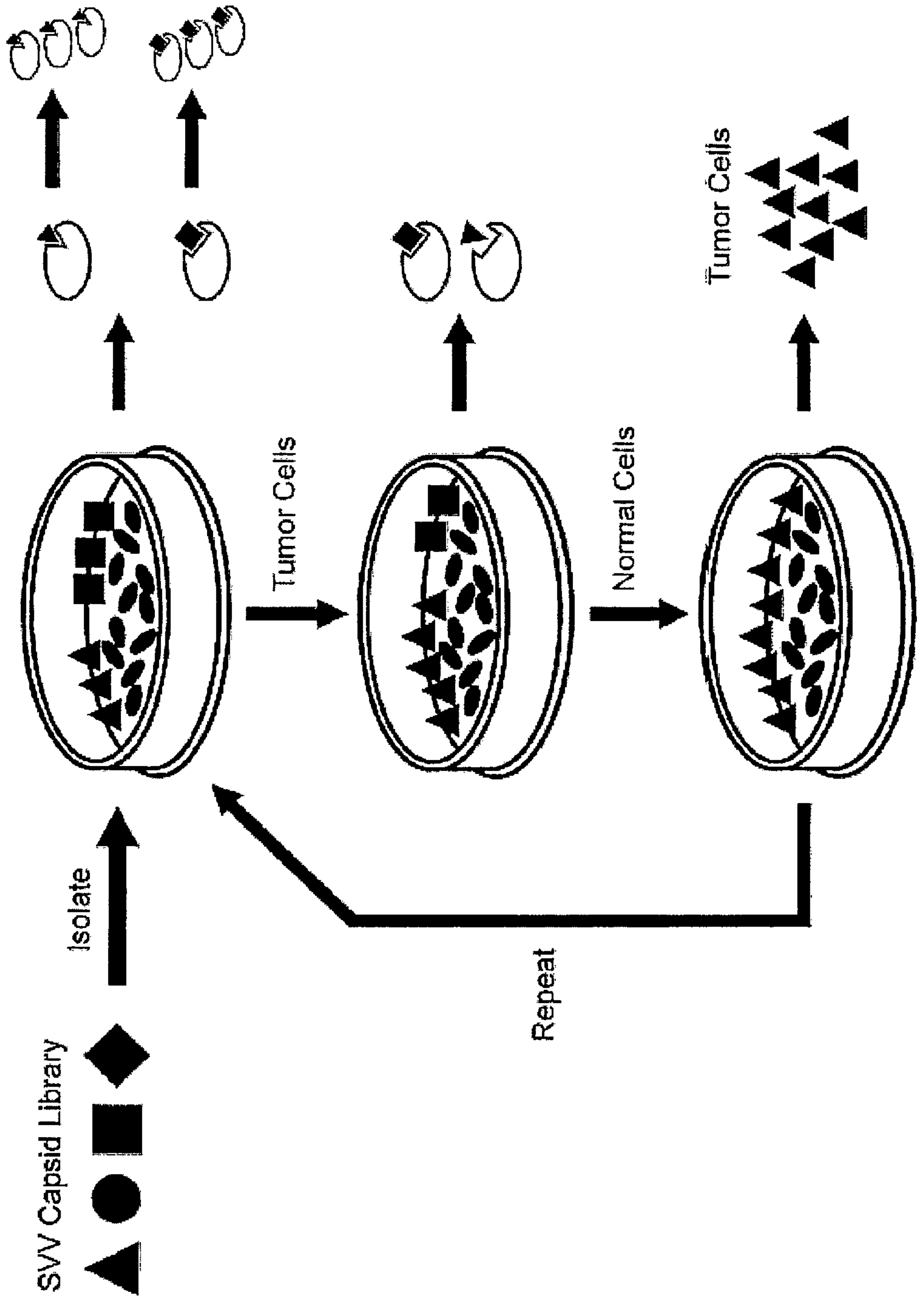
SVV Capsid Library
Isolate
Tumor Cells
Normal Cells
Tumor Cells
Repeat

Fig. 60

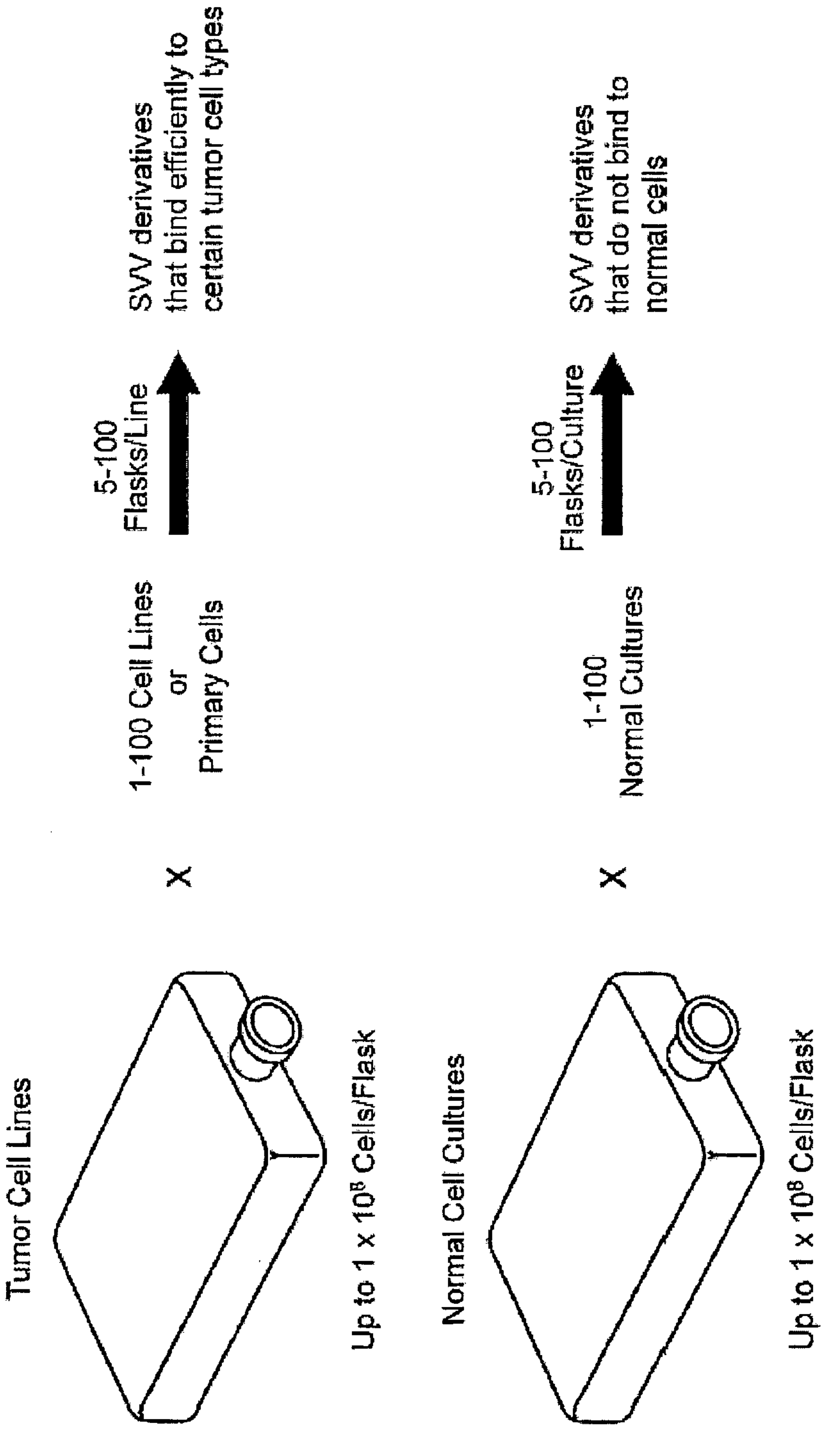

Tumor Cell Lines
Up to 1 x 10$^8$ Cells/Flask
X
1-100 Cell Lines
or
Primary Cells
5-100
Flasks/Line
SVV derivatives
that bind efficiently to
certain tumor cell types
Normal Cell Cultures
Up to 1 x 10$^8$ Cells/Flask
X
1-100
Normal Cultures
5-100
Flasks/Culture
SVV derivatives
that do not bind to
normal cells


Fig. 61

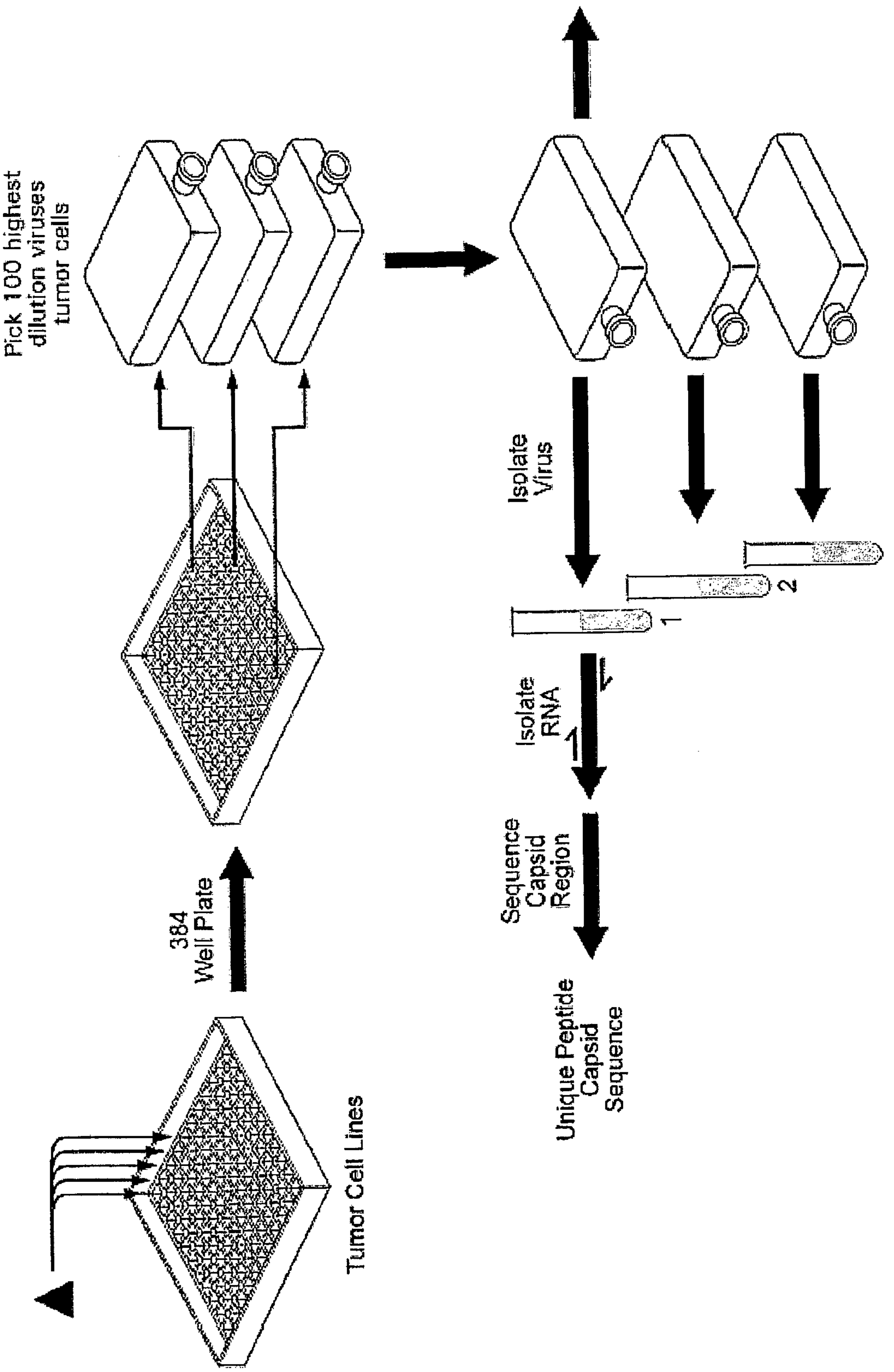
Pick 100 highest dilution viruses tumor cells
384 Well Plate
Tumor Cell Lines
Isolate Virus
1
2
Isolate RNA
Sequence Capsid Region
Unique Peptide Capsid Sequence

Fig. 62

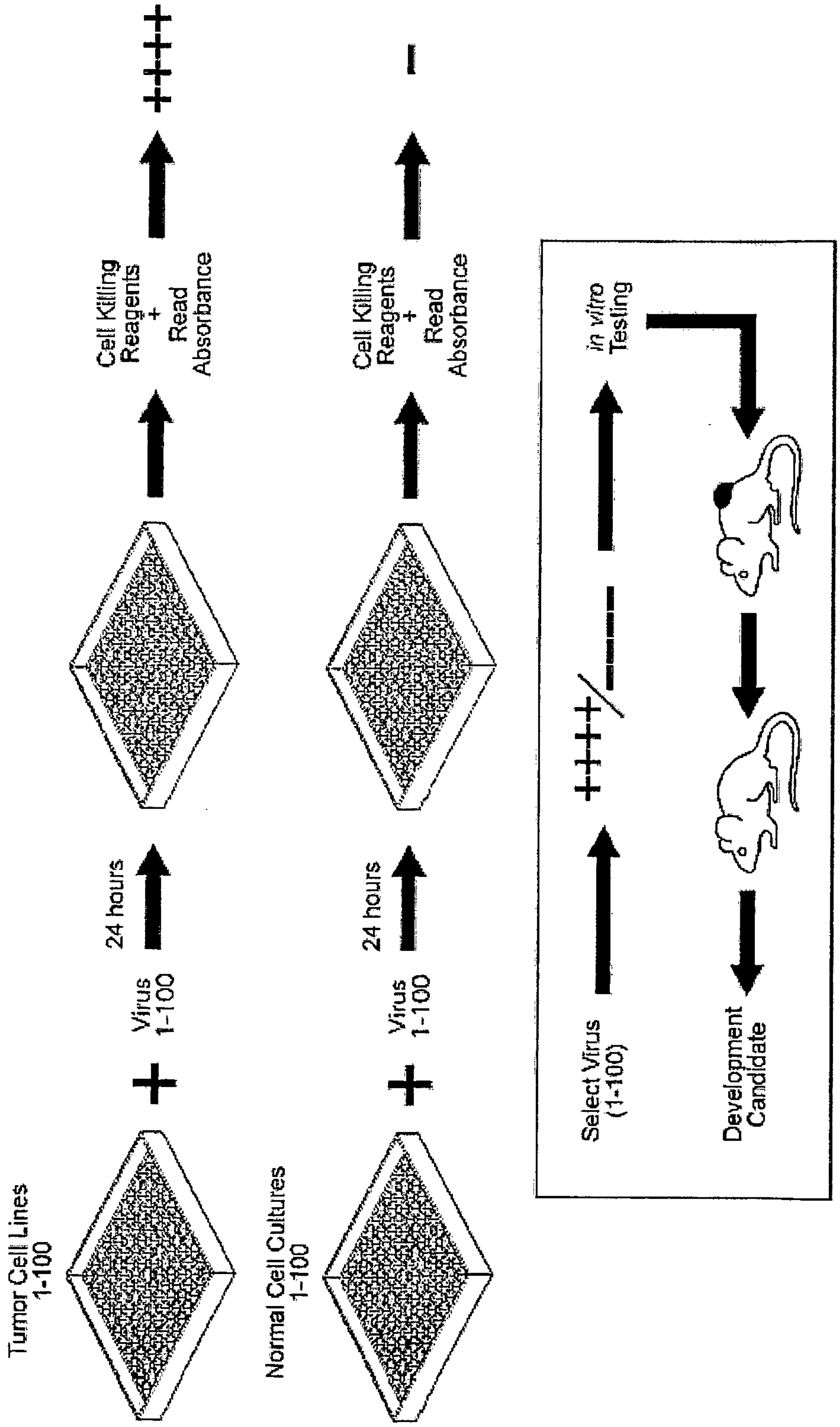

Tumor Cell Lines
1-100
+
Virus
1-100
24 hours
Cell Killing
Reagents
+
Read
Absorbance
++++
Normal Cell Cultures
1-100
+
Virus
1-100
24 hours
Cell Killing
Reagents
+
Read
Absorbance
-
Select Virus
(1-100)
++++/----
in vitro
Testing
Development
Candidate


Fig. 63

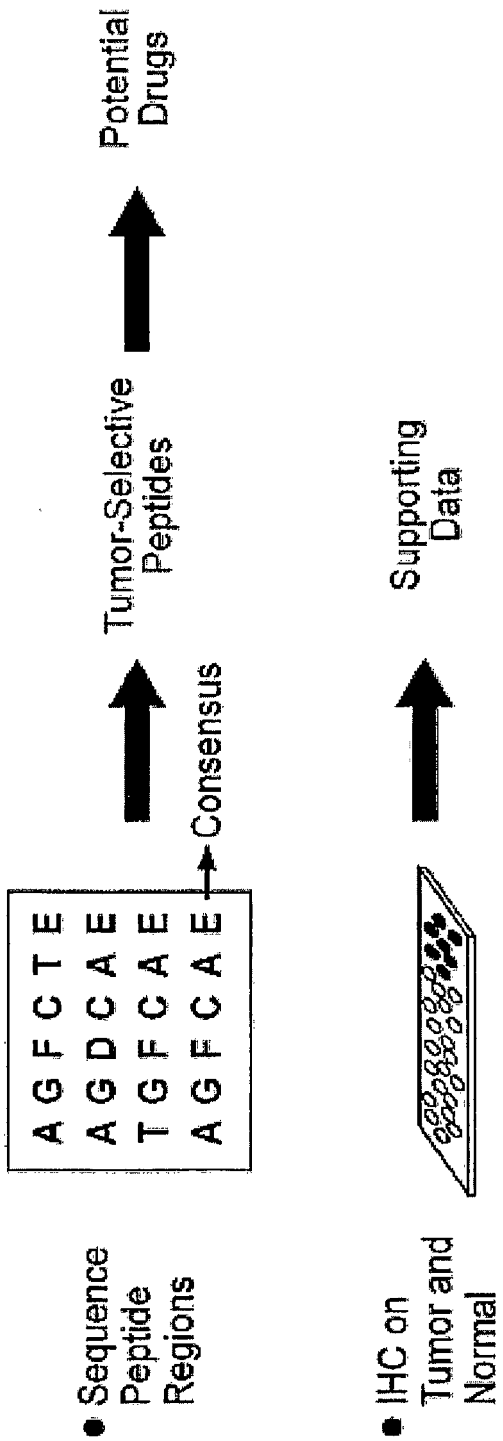
Sequence Peptide Regions
A G F C T E
A G D C A E
T G F C A E
A G F C A E
Consensus
Tumor-Selective Peptides
Potential Drugs
IHC on Tumor and Normal
Supporting Data

Fig. 64

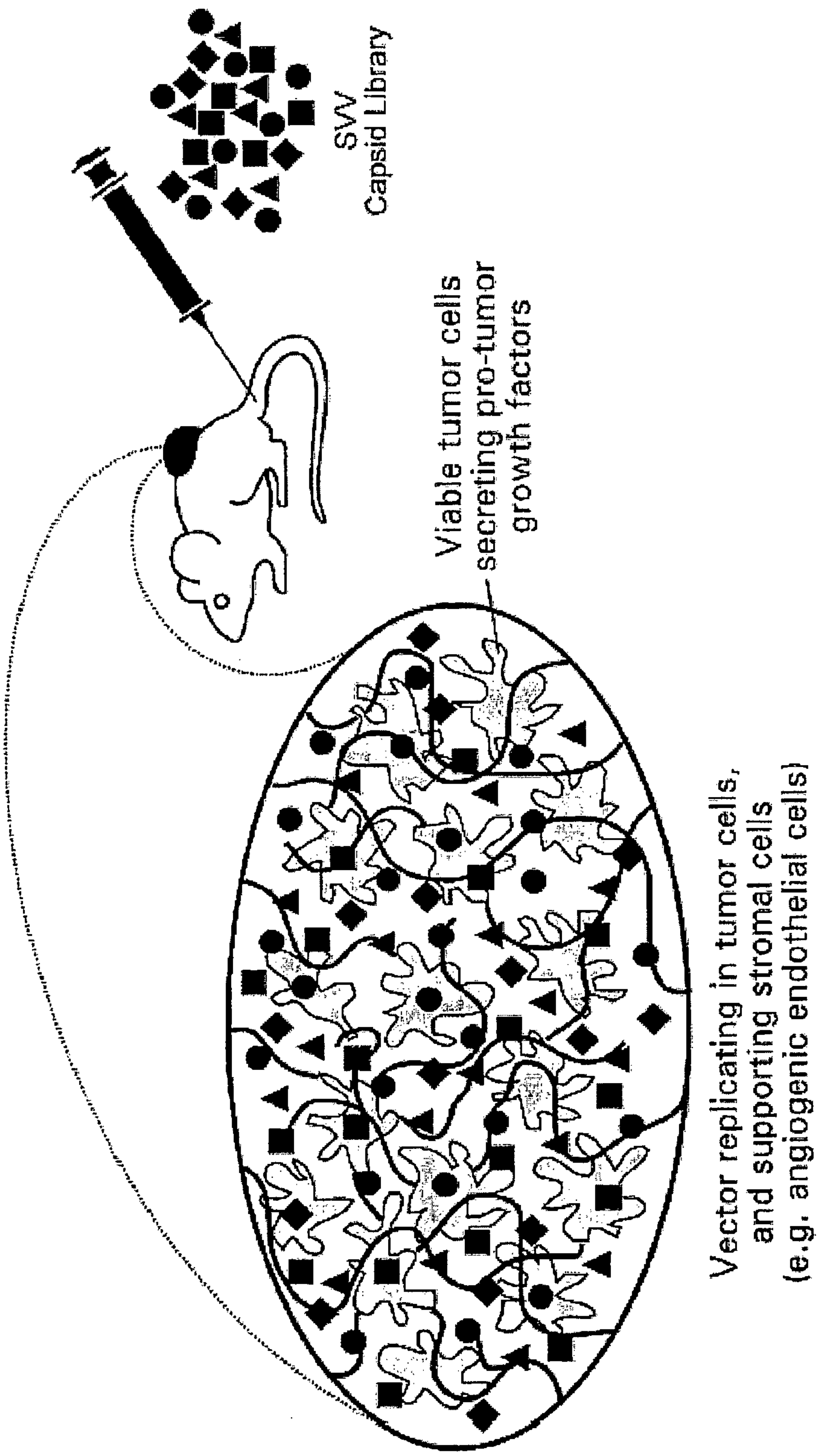
SW
Capsid Library
Viable tumor cells
secreting pro-tumor
growth factors
Vector replicating in tumor cells,
and supporting stromal cells
(e.g. angiogenic endothelial cells)

Fig. 65

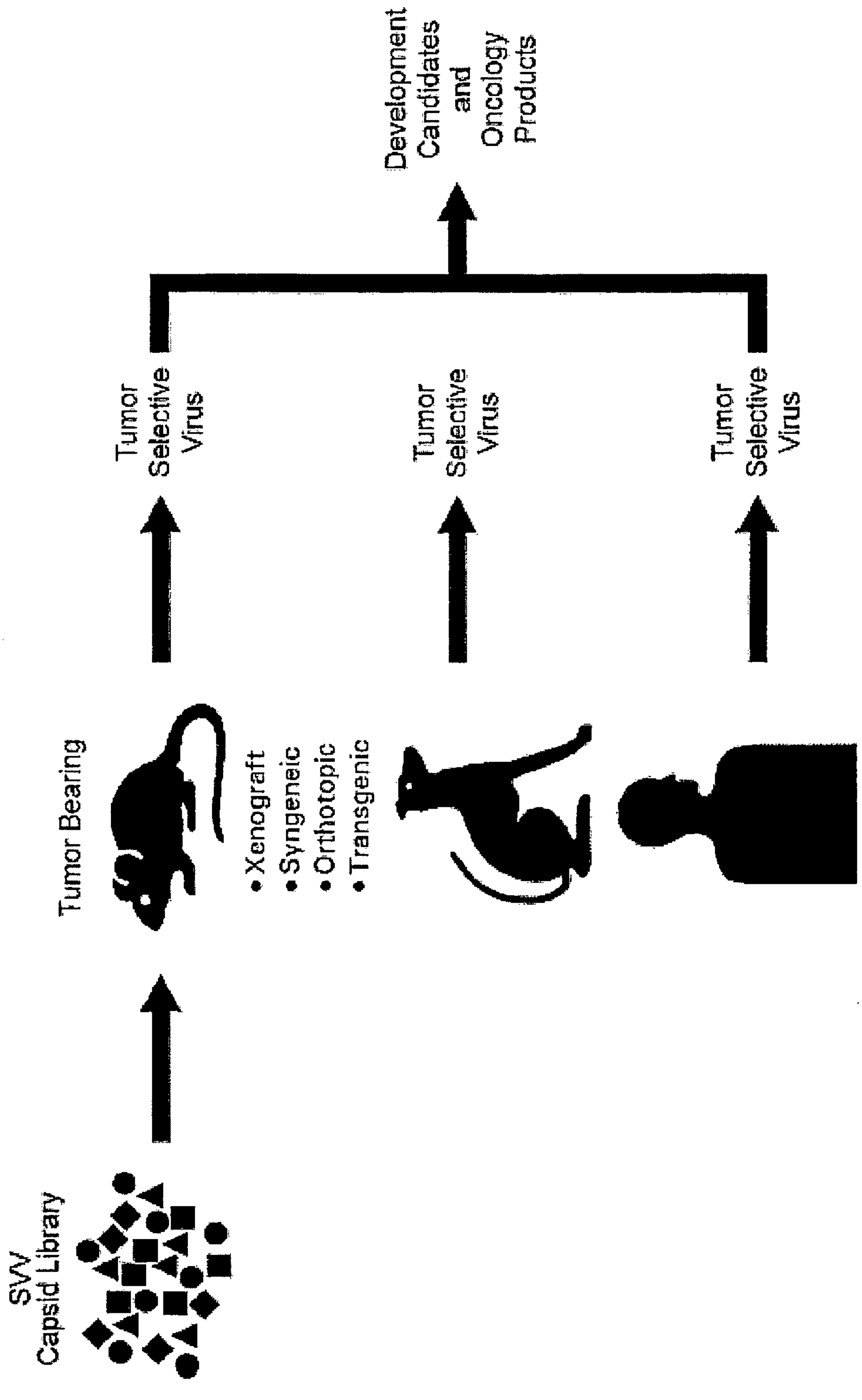
SVV
Capsid Library
Tumor Bearing
• Xenograft
• Syngeneic
• Orthotopic
• Transgenic
Tumor
Selective
Virus
Tumor
Selective
Virus
Tumor
Selective
Virus
Development
Candidates
and
Oncology
Products

Fig. 66

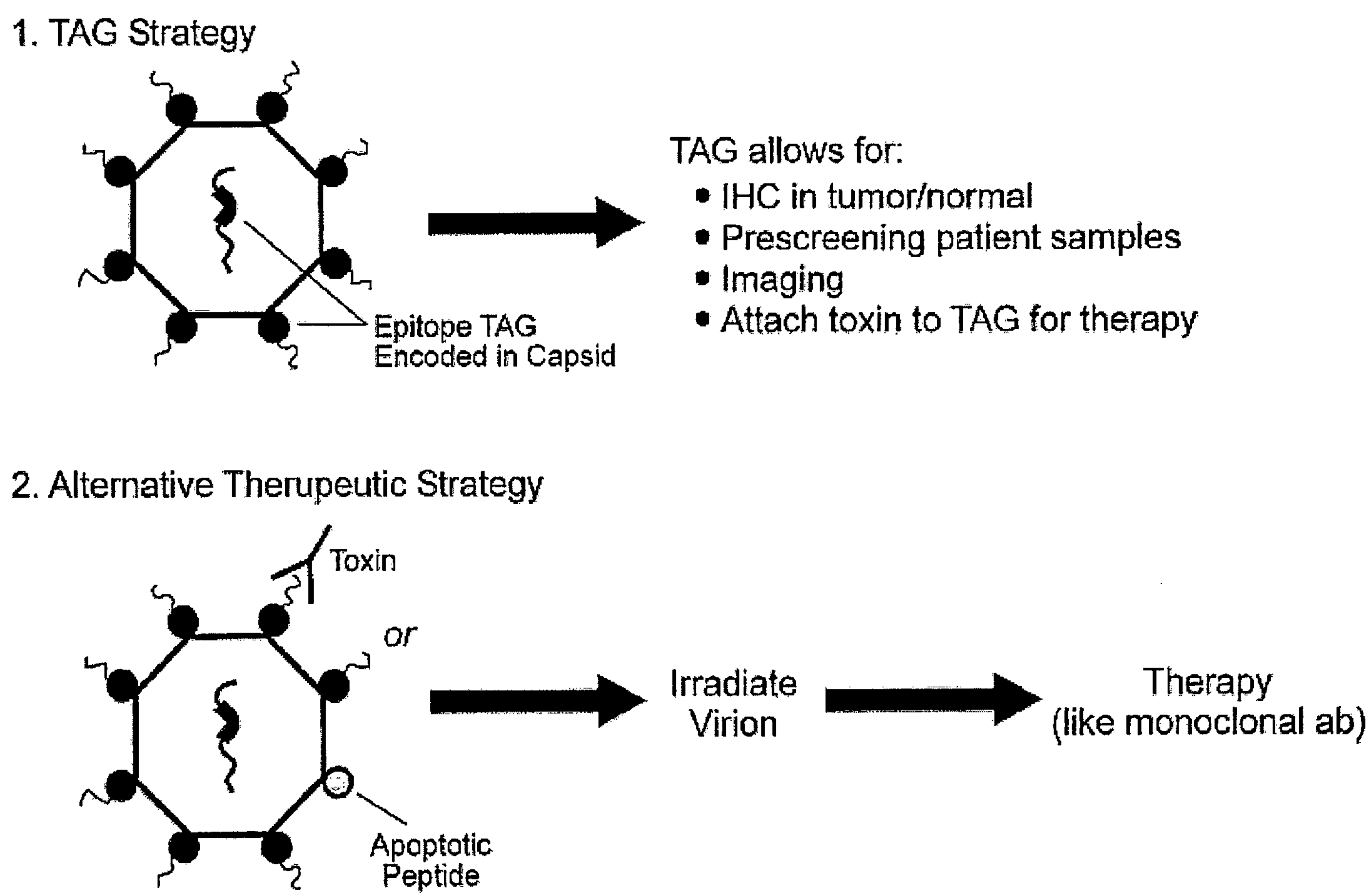
1. TAG Strategy
Epitope TAG
Encoded in Capsid
TAG allows for:
• IHC in tumor/normal
• Prescreening patient samples
• Imaging
• Attach toxin to TAG for therapy
2. Alternative Therupeutic Strategy
Toxin
or
Apoptotic
Peptide
Irradiate
Virion
Therapy
(like monoclonal ab)

Fig. 67

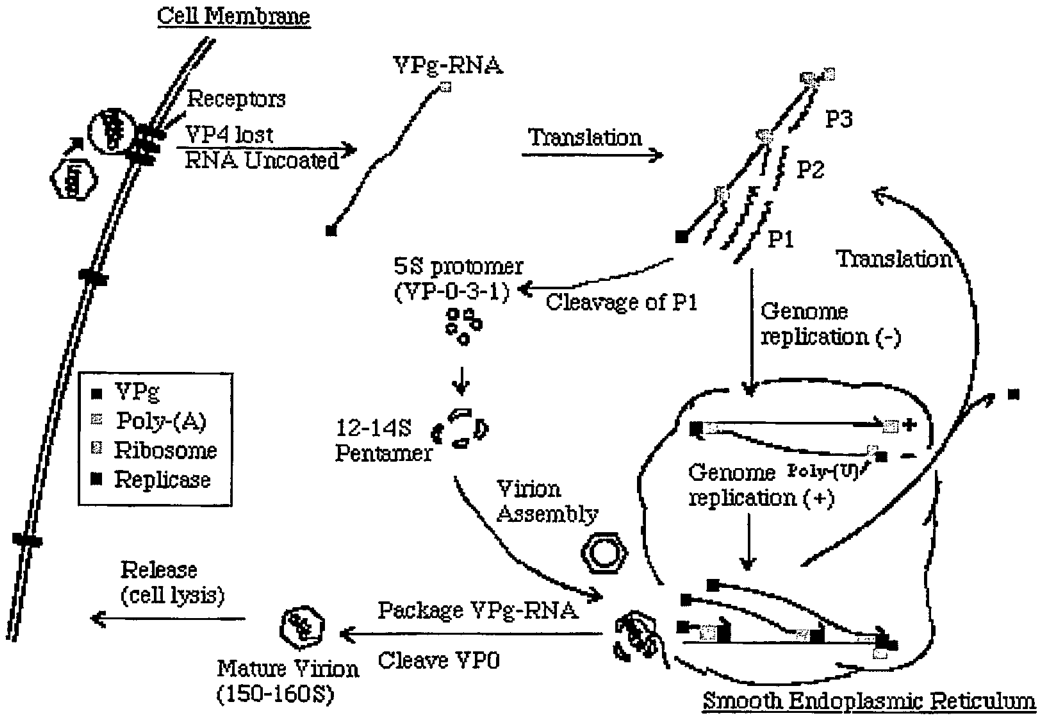
Cell Membrane
VPg-RNA
Receptors
VP4 lost
RNA Uncoated
Translation
P3
P2
P1
Translation
5S protomer
(VP-0-3-1)
Cleavage of P1
Genome
replication (-)
VPg
Poly-(A)
Ribosome
Replicase
12-14S
Pentamer
Genome
replication (+)
Poly-(U)
Virion
Assembly
Release
(cell lysis)
Package VPg-RNA
Cleave VP0
Mature Virion
(150-160S)
Smooth Endoplasmic Reticulum

**Fig. 68**

Attorney Docket No.: 287037.127-WO

Polypeptide lengths of Seneca Valley virus compared to other picornaviruses

| | SVV | EMCV | TMEV | ERBV-1 | FMDV-O | ERAV | PTV-1 | PV-1 | HRV-1B | SV2 | PEV-8 | DPV | HAV | AEV | AiV | BKV | HPeV-1 | LV |
|---|---|---|---|---|---|---|---|---|---|---|---|---|---|---|---|---|---|---|
| Polyprotein | ? | 2292 | | 2590 | 2332 | 2249 | | 2209 | 2157 | | | | 2227 | 2134 | 2432 | 2463 | 2180 | 2253 |
| P1 | ? | 834 | | 880 | 736 | 784 | | 881 | 857 | | | | 791 | 757 | 846 | 857 | 776 | 820 |
| P2 | 465 | 618 | | 616 | 488 | 467 | | 575 | 559 | | | | 631 | 590 | 636 | 634 | 598 | 606 |
| P3 | 785 | 773 | | 875 | 907 | 789 | | 753 | 741 | | | | 805 | 787 | 780 | 785 | 806 | 827 |
| Leader | ? | 67 | | 219 | 201 | 209 | | - | - | | | | - | - | 170 | 187 | - | - |
| VP0 | ? | 326 | | 327 | 303 | 310 | | 341 | 332 | | | | 245 | 242 | 370 | 367 | 289 | 259 |
| VP4 | ? | 70 | | 71 | 85 | 80 | | 69 | 69 | | | | 23 | 19 | - | - | - | - |
| VP2 | ? | 256 | | 256 | 218 | 230 | | 272 | 263 | | | | 222 | 223 | - | - | - | - |
| VP3 | 239 | 231 | | 229 | 220 | 226 | | 238 | 238 | | | | 246 | 245 | 223 | 223 | 253 | 244 |
| VP1 | 259 | 277 | | 324 | 211 | 248 | | 302 | 287 | | | | 300 | 270 | 253 | 267 | 234 | 317 |
| 2A | 14 | 143 | | 16 | 18 | 16 | | 149 | 142 | | | | 45 | 13 | 136 | 134 | 147 | 135 |
| 2B | 128 | 150 | | 283 | 154 | 136 | | 97 | 95 | | | | 251 | 251 | 165 | 165 | 122 | 138 |
| 2C | 323 | 325 | | 317 | 318 | 315 | | 329 | 322 | | | | 335 | 326 | 335 | 335 | 329 | 333 |
| 3A | 90 | 88 | | 133 | 153 | 95 | | 87 | 77 | | | | 74 | 65 | 95 | 94 | 117 | 130 |
| 3B | 22 | 20 | | 21 | 23-24† | 24 | | 22 | 21 | | | | 23 | 21 | 27 | 30 | 20 | 29 |
| 3C | 211 | 205 | | 252 | 213 | 205 | | 183 | 183 | | | | 219 | 215 | 190 | 192 | 200 | 198 |
| 3D | 462 | 460 | | 469 | 470 | 465 | | 461 | 460 | | | | 489 | 486 | 468 | 469 | 469 | 470 |

*, assuming that the 20 aa peptide ending in NPG is the C-terminus of VP1.

†, FMDV has three copies of VPg in tandem.

na, these individual polypeptides do not exist in these viruses.

Fig. 69

```
                                              10        20
                                     ....|....|....|....|..
PTV-1_Teschen-Konratice [AF231       GPGATNFSLLKQAGDVEENPGP
PTV-2_T80 [AF296087]                 GPGATNFSLLKQAGDVEENPGP
PTV-3_O2b [AF296088]                 GPGASSFSLLKQAGDVEENPGP
PTV-4_PS36 [AF296089]                GPGASNFSLLKQAGDVEENPGP
PTV-4_Vir_2500/99 [AF296113]         GPGATNFSLLKQAGDVEENPGP
PTV-5_F26 [AF296090]                 GPGAANFSLLRQAGDVEENPGP
PTV-6_PS37 [AF296091]                GPGATNFSLLKQAGDVEENPGP
PTV-7_F43 [AF296092]                 GPGATNFSLLKQAGDVEENPGP
PTV-8_UKG/173/74 [AF296093]          GPGATNFSLLKQAGDIEENPGP
PTV-9_Ger-2899/84 [AF296094]         GPGATNFSLLKQAGDVEENPGP
PTV-10_Vir 461/88_[AF296119]         GPGATNFSLLKQAGDVEENPGP
PTV-11_Dresden [AF296096]            GPGATNFSLLKRAGDVEENPGP
AY593829_FMDV-O6/UK/1/24_(iso5       ---TLNFDLLKLAGDVESNPGP
AY593828_FMDV-O5/India/62_(iso       ---LLNFDLLKLAGDVESNPGP
AY593774_FMDV-A2/Spain/43_(iso       ---LLNFDLLQLAGDVESNPGP
AY593759_FMDV-A1/Bavaria/GER/4       ---LLNFDLLKLAGDVESNPGP
AY593810_FMDV-C/UK/149/34_(iso       ---LLNFDLLKLAGDVESNPGP
AY593807_FMDV-C3/Resende/BRA/5       ---LSNFDLLKLAGDVESNPGP
AY593795_FMDV-Asia1/PAK/1/54_(       ---LLNFDLLKLAGDVESNPGP
AY593796_FMDV-Asia1/ISR/3/63_(       ---TLNFDLLKLAGDVESNPGP
AY593797_FMDV-Asia1/Kimron_(is       ---ALNFDLLKLAGDVESNPGP
AY593798_FMDV-Asia1/LEB/89_(is       ---VLNFDLLKLAGDVESNPGP
AY593839_FMDV-SAT1/RV/11/37_(i       ---MCSFDLLKLAGDVESNPGP
AY593838_FMDV-SAT1/BEC/1/48_(i       ---LCNFDLLKLAGDVESNPGP
AY593841_FMDV-SAT1/SR/2/58_(is       ---LCNFDLLMLAGDVESNPGP
AY593844_FMDV-SAT1/ISR/14/62_(       ---MANFALLKLAGDVESNPGP
AY593845_FMDV-SAT1/BOT/1/68_(i       ---LSNFDLLKLAGDVESNPGP
AY593847_FMDV-SAT2/RHO/1/48_(i       ---LFNFDLLKLAGDVESNPGP
AY593848_FMDV-SAT2/SA/106/59_(       ---LCNCDLLKLAGDVESNPGP
AY593849_FMDV-SAT2/KEN/11/60_(       ---LLNFDLLKLAGDVESNPGP
AY593850_FMDV-SAT3/SA/57/59_(i       ---LCNFDLLKLAGDVESNPGP
AY593853_FMDV-SAT3/BEC/1/65_(i       ---MCNFDLLKLAGDVESNPGP
ERAV                                 NKQCTNYALLKLAGDVESNPGP
ERBV-1                               SEGATNFSLLKLAGDVELNPGP
ERBV-2                               SQGATNFDLLKLAGDVESNPGP
EMCV-R                               NAHYAGYFADLLIHDIEINPGP
EMCV-BC                              NAHYAGYFADLLIHDIEINPGP
Mengo                                ETHYAGYFSDLLIHDVEINPGP
SVV                                  PFRSYKQMLMQSGDIEINPGP
TMEV-BeAn                            RGYHADYYRQRLIHDVEINPGP
TMEV-GD7                             RGYHADYYKQRLIHDVEMNPGP
TMEV-DA                              RAYHADYYKQRLIHDVEMNPGP
Rat TLV                              REYHAAYYKQRLMHDVEINPGP
LV_87-012                            EMDFAGGKFLNQCGDVEINPGP
LV_174F                              EMDFAGGKFLNQCGDVEINPGP
LV_145SL                             EMDYSGGKFLNQCGDVESNPGP
LV_M1146                             DMDYAGGKFLNQCGDVEINPGP
SVV                                  PFRSYKQMLMQSGDIEINPGP
Trypanosoma_brucei                   AISSIIRTKMLLSGDVEENPGP
Trypanosoma_cruzi                    AVCDAQRQKLLLSGDIEQNPGP
BoRotavirus_C_strain_Shintoku        ANSKFQIDRILISGDIELNPGP
HuRotavirus_C_strain_Bristol         ANSKFQIDKILISGDIELNPGP
PoRotavirus_C_strain_Cowden          ANAKFQIDKILISGDVELNPGP
Drosophila C virus                   KQEAARQMLLLLSGDVEINPGP
Cricket paralysis virus              RAFLRKRTQLLMSGDVESNPGP
Acute bee paralysis virus            HCGSWTDILLLLSGDVEINPGP
Cricket paralysis virus              TLTRAEIEDELIRAGIESNPGP
Perina nuda picorna-like virus       VTAQGWVPDLTVDGDVESNPGP
Ectropis obliqua picorna-like        VTAQGWAPDLTQDGDVESNPGP
Perina nuda picorna-like virus       NIIGGGQKDLTQDGDIESNPGP
Ectropis obliqua picorna-like        NIIGGGQRDLTQDGDIESNPGP
Deformed wing virus                  NLLQLSNPVQAKPEMDNPNPGP
Kakugo virus                         NLLQLSNPVQAKPEMDNPNPGP
```

Fig. 70

1 MATTMEQETC AHSLTFEECP KCSALQYRNG FYLLKYDEEW YPEELLTDGE DDVFDPELDM
61 EVVFELQGNS TSSDKNNSSS EGNEGVIINN FYSNQYQNSI DLSANAAGSD PPRTYGQFSN
121 LFSGAVNAFS NMLPLLADQN TEEMENLSDR VSQDTAGNTV TNTQSTVGRL VGYGTVHDGE
181 HPASCADTAS EKILAVERYY TFKVNDWTST QKPFEYIRIP LPHVLSGEDG GVFGAALRRH
241 YLVKTGWRVQ VQCNASQFHA GSLLVFMAPE YPTLDAFAMD NRWSKDNLPN GTRTQTNKKG
301 PFAMDHQNFW QWTLYPHQFL NLRTNTTVDL EVPYVNIAPT SSWTQHASWT LVIAVVAPLT
361 YSTGASTSLD ITASIQPVRP VFNGLRHETL SRQSPIPVTI REHAGTWYST LPDSTVPIYG
421 KTPVAPSNYM VGEYKDFLEI AQIPTFIGNK IPNAVPYIEA SNTAVKTQPL ATYQVTLSCS
481 CLANTFLAAL SRNFAQYRGS LVYTFVFTGT AMMKGKFLIA YTPPGAGKPT SRDQAMQATY
541 AIWDLGLNSS YSFTVPFISP THFRMVGTDQ VNITNADGWV TVWQLTPLTY PPGCPTSAKI
601 LTMVSAGKDF SLKMPISPAP WSPQGVENAE KGVTENTNAT ADFVAQPVYL PENQTKVAFF
661 YNRSSPIGAF TVKSGSLESG FAPFSNGTCP NSVILTPGPQ FDPAYDQLRP QRLTEIWGNG
721 NEETSKVFPL KSKQDYSFCL FSPFVYYKCD LEVTLSPHTS GNHGLLVRWC PTGTPTKPTT
781 QVLHEVSSLS EGRTPQVYSA GPGISNQISF VVPYNSPLSV LSAVWYNGHK RFDNTGSLGI
841 APNSDFGTLF FAGTKPDIKF TVYLRYKNKR VFCPRPTVFF PWPTSGDKID MTPRAGVLML
901 ESPNALDISR TYPTLHVLIQ FNHRGLEVRL FRHGHFWAET RADVILRSKT KQVSFLSNGN
961 YPSMDSRAPW NPWKNTYQAV LRAEPCRVTM DIYYKRVRPF RLPLVQKEWP VREENVFGLY
1021 RIFNAHYAGY FADLLIHDIE TNPGPFMFRP RKQVFQTQGA AVSSMAQTLL PNDLASKAMG
1081 SAFTALLDAN EDAQKAMKII KTLSSLSDAW ENVKETLNNP EFWKQLLSRC VQLIAGMTIA
1141 VMHPDPLTLL CLGTLTAAEI TSQTSLCEEI AAKFKTIFIT PPPRFPTISL FQQQSPLKQV
1201 NDIFSLAKNL DWAVKTVEKV VDWFGTWIVQ EEKEQTLDQL LQRFPEHAKR ISDLRNGMAA
1261 YVECKESFDF FEKLYNQAVK EKRTGIAAVC EKFRQKHDHA TARCEPVVIV LRGDAGQGKS
1321 LSSQVIAQAV SKTIFGRQSV YSLPPDSDFF DGYENQFAAI MDDLGQNPDG SDFTTFCQMV
1381 STTNFLPNMA SLERKGTPFT SQLVVATTNL PEFRPVTIAH YPAVERRITF DYSVSAGPVC

# Fig. 71A

1441 SKTEAGYKVL DVERAFRPTG EAPLPCFQNN CLFLEKAGLQ FRDNRTKEII SLVDVIERAV
1501 ARIERKKKVL TTVQTLVAQG PVDEVSFHSV VQQLKARQQA TDEQLEELQE AFAKVQERNS
1561 VFSDWLKISA MLCAATLALS QVVKMAKAVK QMVKPDLVRV QLDEQEQGPY NETARVKPKT
1621 LQLLDIQGPN PVMDFEKYVA KHVTAPIGFV YPTGVSTQTC LLVRGRTLVV NRHMAESDWT
1681 SIVVRGVTHA RSTVKILAIA KAGKETDVSF IRLSSGPLFR DNTSKFVKAG DVLPTGAAPV
1741 TGIMNTDIPM MYTGTFLKAG VSVPVETGQT FNHCIHYKAN TRKGWCGSAL LADLGGSKKI
1801 LGIHSAGSMG IAAASIVSQE MIRAVVNAFE PQGALERLPD GPRIHVPRKT ALRPTVARQV
1861 FQPAYAPAVL SKFDPRTEAD VDEVAFSKHT SNQESLPPVF RMVAKEYANR VFTLLGKDNG
1921 RLTVKQALEG LEGMDPMDRN TSPGLPYTAL GMRRTDVVDW ESATLIPFAA ERLRKMNEGD
1981 FSEVVYQTFL KDELRPIEKV QAAKTRIVDV PPFEHCILGR QLLGKFASKF QTQPGLELGS
2041 AIGCDPDVHW TAFGVAMQGF ERVYDVDYSN FDSTHSVAMF RLLAEEFFTP ENGFDPLTRE
2101 YLESLAISTH AFEEKRFLIT GGLPSGCAAT SMLNTIMNNI IIRAGLYLTY KNFEFDDVKV
2161 LSYGDDLLVA TNYQLDFDKV RASLAKTGYK ITPANKTSTF PLNSTLEDVV FLKRKFKKEG
2221 PLYRPVMNRE ALEAMLSYYR PGTLSEKLTS ITMLAVHSGK QEYDRLFAPF REVGVVVPSF
2281 ESVEYRWRSL FW (SEQ ID NO:23)

# Fig. 71B

1 MATTMEQETC AHSLTFEECP KCSALQYRNG FYLLKYDEEW YPEELLTDGE DDVFDPELDM
61 EVVFELQGNS TSSDKNNSSS EGNEGVIINN FYSNQYQNSI DLSANAAGSD PPRTYGQFSN
121 LFSGAVNAFS NMLPLLADQN TEEMENLSDR VSQDTAGNTV TNTQSTVGRL VGYGTVHDGE
181 HPASCADTAS EKILAVERYY TFKVNDWTST QKPFEYIRIP LPHVLSGEDG GVFGAALRRH
241 YLVKTGWRVQ VQCNASQFHA GSLLVFMAPE YPTLDTFVMD NRWSKDNLPN GARTQTNKKG
301 PFAMDHQNFW QWTLYPHQFL NLRTNTTVDL EVPYVNIAPT SSWTQHASWT LVIAVVAPLT
361 YSTGASTSLD ITASIQPVRP VFNGLRHETL SRQSPIPVTI REHAGTWYST LPDSTVPIYG
421 KTPVAPSNYM VGEYKDFLEI AQIPTFIGNK IPNAVPYIEA SNTAVKTQPL ATYQVTLSCS
481 CLANTFLAAL SRNFAQYRGS LVYTFVFTGT AMMKGKFLIA YTPPGAGKPT SRDQAMQATY
541 AIWDLGLNSS YSFTVPFISP THFRMVGTDQ VNITNADGWV TVWQLTPLTY PPGCPTSAKI
601 LTMVSAGKDF SLKMPISPAP WSPQGVENAE KGVTENADAT ADFVAQPVYL PENQTKVAFF
661 YDRSSPIGAF TVQSGSLESG FAPFSNKTCP NSVILTPGPQ FDPAYDQLRP QRLTEIWGNG
721 NEETSKVFPL KSKQDYSFCL FSPFVYYKCD LEVTLSPHTS GNHGLLVRWC PTGTPTKPTT
781 QVLHEVSSLS EGRTPQVYSA GPGISNQISF VVPYNSPLSV LPAVWYNGHK RFDNTGSLGI
841 APNSDFGTLF FAGTKPDIKF TVYLRYKNMR VFCPRPTVFF PWPTSGDKID MTPRAGVLML
901 ESPNALDISR TYPTLHVLIQ FNHRGLEVRL FRHGQFWAET RADVILRSKT KQVSFLSNGN
961 YPSMDSRAPW NPWKNTYQAV LRAEPCRVTM DIYYKRVRPF RLPLVQKEWR VREENVFGLY
1021 RIFNAHYAGY FADLLIHDIE TNPGPFMFRP RKQVFQTQGA AVSSMAQTLL PNDLASKAMG
1081 SAFTALLDAN EDARKAMKII KTLSSLSDAW ENVKETLNNP EFWKQLLSRC VQLIAGMTIA
1141 VMHPDPLTLL CLGTLTAAEI TSQTSLCEEI AAKFKTIFIT PPPRFPTISL FQQQSPLKQV
1201 NDFFSLAKNL DWAVKTVEKV VDWFGTWIVQ EEKEQTLDQL LQRFPEHAKR ISDLRNGMAA
1261 YVECKESFDF FEKLYNQAVK EKRTGIAAVC EKFRQKHDHA TARCEPVVIV LRGDAGQGKS
1321 LSSQVIAQAV SKTIFGRQSV YSLPPDSDFF DGYENQFAAI MDDLGQNPDG SDFTTFCQMV

# Fig. 72A

1381 STTNFLPNMA SLERKGTPFT SQLVVATTNL PEFRPVTIAH YPAVERRITF DYSVSAGPVC
1441 SKTEAGYKVL DVERAFRPTG EAPLPCFQNN CLFLEKAGLQ FRDNRTKEII SLVDVIERAV
1501 ARIERKKKVL TTVQTLVAQA PVDEVSFHSV VQQLKARQEA TDEQLEELQE AFAKVQERNS
1561 VFSDWLKISA MLCAATLALS QVVKMAKAVK QMVKPDLVRV QLDEQEQGPY NETARAKPKT
1621 LQLLDIQGPN PVMDFEKYVA KHVTAPIDFV YPTGVSTQTC LLVRGRTLAV NRHMAESDWT
1681 SIVVRGVTHA RSTVKILAIA KAGKETDVSF IRLSSGPLFR DNTSKFVKAG DVLPTGAAPV
1741 TGIMNTDIPM MYTGTFLKAG VSVPVETGQT FNHCIHYKAN TRKGWCGSAL LADLGGSKKI
1801 LGIHSAGSMG IAAASIVSQE MIRAVVNAFE PQGALERLPD GPRIHVPRKT ALRPTVARQV
1861 FQPAYAPAVL SKFDPRTEAD VDEVAFSKHT SNQESLPPVF RMVAKEYANR VFTLLGKDNG
1921 RLTVKQALEG LEGMDPMDRN TSPGLPYTAL GMRRTDVVDW ESATLIPFAA ERLRKMNEGD
1981 FSEVVYQTFL KDELRPIEKV QAAKTRIVDV PPFEHCILGR QLLGKFASKF QTQPGLELGS
2041 AIGCDPDVHW TAFGVAMQGF ERVYDVDYSN FDSTHSVAMF RLLAEEFFTP ENGFDPLTRE
2101 YLESLAISTH AFEEKRFLIT GGLPSGCAAT SMLNTIMNNI IIRAGLYLTY KNFEFDDVKV
2161 LSYGDDLLVA TNYQLDFDKV RASLAKTGYK ITPANKTSTF PLNSTLEDVV FLKRKFKKEG
2221 PLYRPVMNRE ALEAMLSYYR PGTLSEKLTS ITMLAVHSGK QEYDRLFAPF REVGVVVPSF
2281 ESVEYRWRSL FW (SEQ ID NO:24)

# Fig. 72B

1 MATTMEQEIC AHSLTLKGCP KCSALQYRNG FYLLKYDEEW YPEELLTDGE DDVFDPELDM
61 EVVFELQGNS TSSDKNNSSS DGNEGVIINN FYSNQYQNSI DLSANATGSD PPRTYGQFSN
121 LLSGAVNAFS NMIPLLADQN TEEMENLSDR VLQDTAGNTV TNTQSTVGRL VGYGAVHDGE
181 HPASCADTAS EKILAVERYY TFKVNDWTST QKPFEYIRIP LPHVLSGEDG GVFGAALRRH
241 YLVKTGWRVQ VQCNASQFHA GSLLVFMAPE YPTLDAFAMD NRWSKDNLPN GTKTQTNRKG
301 PFAMDHQNFW QWTLYPHQFL NLRTNTTVDL EVPYVNIAPT SSWTQHASWT LVIAVVAPLT
361 YSTGASTSLD ITASIQPVRP VFNGLRHETL SRQSPIPVTI REHAGTWYST LPDSTVPIYG
421 KTPVAPANYM VGEYKDFLEI AQIPTFIGNK IPNAVPYIEA SNTAVKTQPL ATYQVTLSCS
481 CLANTFLAAL SRNFAQYRGS LVYTFVFTGT AMMKGKFLIA YTPPGAGKPT SRDQAMQATY
541 AIWDLGLNSS YSFTVPFISP THFRMVGTDQ VNITNVDGWV TVWQLTPLTY PPGCPTSAKI
601 LTMVSAGKDF SLKMPISPAP WSPQGVENAE RGVTEDTDAT ADFVAQPVYL PENQTKVAFF
661 YDRSSPIGAF TVKSGSLESG FTPFSNQTCP NSVILTPGPQ FDPAYDQLRP QRLTEIWGNG
721 NEETSKVFPL KSKQDYSFCL FSPFVYYKCD LEVTLSPHTS GNHGLLVRWC PTGTPTKPTT
781 QVLHEVSSLS EGRTPQVYSA GPGITNQISF VVPYNSPLSV LPAVWYNGHK RFDNTGSLGI
841 APNSDFGTLF FAGTKPDIKF TVYLRYKNMR VFCPRPTVFF PWPSSGDKID MTPRAGVLML
901 ESPNALDISR TYPTLHILIQ FNHGGLEIRL FRHGMFWAEA HADVILRSRT KQISFLNNGS
961 FPSMDARAPW NPWKNTYHAV LRAEPYRVTM DVYHKRIRPF RLPLVQKEWN VREENVFGLY
1021 GIFNAHYAGY FADLLIHDIE TNPGPFMAKP KKQVFQTQGA AVSSMAQTLL PNDLASKVMG
1081 SAFTALLDAN EDAQKAMRII KTLSSLSDAW ENVKETLNNP EFWKQLLSRC VQLIAGMTIA
1141 VMHPDPLTLL CLGTLTAAEI TSQTSLCEEI VAKFKKIFTT PPPRFPTISL FQQQSPLKQV
1201 NDVFSLAKNL DWAVKTVEKV VDWFGTWVVQ EEKEQTLDQL LQRFPEHAKR ISDLRNGMSA
1261 YVECKESFDF FEKLYNQAVK EKRTGIAAVC EKFRQKHDHA TARCEPVVIV LRGDAGQGKS
1321 LSSQVIAQAV SKTIFGRQSV YSLPPDSDFF DGYENQFAAI MDDLGQNPDG SDFTTFCQMV

# Fig. 73A

1381 STTNFLPNMA SLERNGTPFT SQIVVATTNL PEFRPVTIAH YPAVERRITF DYSVSAGPVC
1441 SKTEAGYKVL DVERAFRPTG DAPLPCFQNN CLFLEKAGLQ FRDNRTKEIL SLVDVIERAV
1501 ARIERKKKVL TTVQTLVAQA PVDEVSFHSV VQQLKARQEA TDEQLEELQE AFAKTQERSS
1561 VFSDWMKISA MLCAATLALS QVVKMAKTVK QMVRPDLVRV QLDEQEQGPY NEAVRAKPKT
1621 LQLLDIQGPN PVMDFEKYVA KFVTAPIDFV YPTGVSTQTC LLVKGRTLAV NRHMAESDWS
1681 SIVVRGVTHA RSTVRILAIA KAGKETDVSF IRLSSGPLFR DNTSKFVKAD DVLPATSAPV
1741 IGIMNTDIPM MFTGTFLKAG VSVPVETGQT FNHCIHYKAN TRKGWCGSAL LADLGGKKKI
1801 LGMHSAGSMG RTAASIVSQE MICAVVSAFE PQGALERLPD GPRIHVPRKT ALRPTVARRV
1861 FQPAYAPAVL SKFDPRTEAD VDEVAFSKHT SNQESLPPVF RMVAKEYANR VFTLLGRDNG
1921 RLTVKQALEG LEGMDPMDKN TSPGLPYTAL GMRRTDVVDW ESATLIPYAA DRLKKMNEGD
1981 FSDIVYQTFL KDELRPVEKV QAAKTRIVDV PPFEHCILGR QLLGRFASKF QTQPGLELGS
2041 AIGCDPDVHW TAFGVAMQGF ERVYDVDYSN FDSTHSVAMF RLLAEEFFTP ENGFDPLVKE
2101 YLESLAISTH AFEEKRYLIT GGLPSGCAAT SMLNTIMNNI IIRAGLYLTY KNFEFDDVKV
2161 LSYGDDLLVA TNYQLNFDKV RASLAKTGYK ITPANKTSTF PLDSTLEDVV FLKRKFKKEG
2221 PLYRPVMNRE ALEAMLSYYR PGTLSEKLTS ITMLAVHSGK PEYDRLFAPF REVGVVVPSF
2281 ESVEYRWRSL FW (SEQ ID NO:25)

# Fig. 73B

1 MATTMEQEIC AHSLTFKGCP KCSALQYRNG FYLLKYDEEW YPEELLTDGE DDVFDPELDM
61 EVVFELQGNS TSSDKNNSSS DGNEGVIINN FYSNQYQNSI DLSANATGSD PPRTYGQFSN
121 LLSGAVNAFS NMIPLLADQN TEEMENLSDR VLQDTAGNTV TNTQSTVGRL VGYGAVHDGE
181 HPASCADTAS EKILAVERYY TFKVNDWTST QKPFEYIRIP LPHVLSGEDG GVFGAALRRH
241 YLVKTGWRVQ VQCNASQFHA GSLLVFMAPE YPTLDAFAMD NRWSKDNLPN GTKTQTNRKG
301 PFAMDHQNFW QWTLYPHQFL NLRTNTTVDL EVPYVNIAPT SSWTQHASWT LVIAVVAPLT
361 YSTGASTSLD ITASIQPVRP VFNGLRHETL SRQSPIPVTI REHAGTWYST LPDSTVPIYG
421 KTPVAPANYM VGEYKDFLEI AQIPTFIGNK IPNAVPYIEA SNTAVKTQPL ATYQVTLSCS
481 CLANTFLAAL SRNFAQYRGS LVYTFVFTGT AMMKGKFLIA YTPPGAGKPT SRDQAMQATY
541 AIWDLGLNSS YSFTVPFISP THFRMVGTDQ VNITNVDGWV TVWQLTPLTY PPGCPTSAKI
601 LTMVSAGKDF SLKMPISPAP WSPQGVENAE RGVTEDTDAT ADFVAQPVYL PENQTKVAFF
661 YDRSSPIGAF AVKSGSLESG FAPFSNETCP NSVILTPGPQ FDPAYDQLRP QRLTEIWGNG
721 NEETSKVFPL KSKQDYSFCL FSPFVYYKCD LEVTLSPHTS GNHGLLVRWC PTGTPAKPTT
781 QVLHEVSSLS EGRTPQVYSA GPGISNQISF VVPYNSPLSV LPAVWYNGHK RFDNTGSLGI
841 APNSDFGTLF FAGTKPDIKF TVYLRYKNMR VFCPRPTVFF PWPSSGDKID MTPRAGVLML
901 ESPNALDISR TYPTLHILIQ FNHGGLEIRL FRHGMFWAEA HADVILRSRT KQISFLNNGS
961 FPSMDARAPW NPWKNTYHAV LRAEPYRVTM DVYHKRIRPF RLPLVQKEWN VREENVFGLY
1021 GIFNAHYAGY FADLLIHDIE TNPGPFMAKP KKQVFQTQGA AVSSMAQTLL PNDLASKVMG
1081 SAFTALLDAN EDAQKAMRII KTLSSLSDAW ENVKETLNNP EFWKQLLSRC VQLIAGMTIA
1141 VMHPDPLTLL CLGTLTAAEI TSQTSLCEEI VAKFKKIFTT PPPRFPTISL FQQQSPLKQV
1201 NDVFSLAKNL DWAVKTVEKV VDWFGTWVVQ EEKEQTLDQL LQRFPEHAKR ISDLRNGMSA
1261 YVECKESFDF FEKLYNQAVK EKRTGIAAVC EKFRQKHDHA TARCEPVVIV LRGDAGQGKS
1321 LSSQVIAQAV SKTIFGRQSV YSLPPDSDFF DGYENQFAAI MDDLGQNPDG SDFTTFCQMV

# Fig. 74A

1381 STTNFLPNMA SLERNGTPFT SQIVVATTNL PEFRPVTIAH YPAVERRITF DYSVSAGPVC
1441 SKTEAGYKVL DVERAFRPTG DAPLPCFQNN CLFLEKAGLQ FRDNRTKEIL SLVDVIERAV
1501 ARIERKKKVL TTVQTLVAQA PVAEVSFHSV VQQLKARQEA TDEQLEELQE AFAKTQERSS
1561 VFSDWMKISA MLCAATLALS QVVKMAKTVK QMVRPDLVRV QLDEQEQGPY NEAVRAKPKT
1621 LQLLDIQGPN PVMDFEKYVA KFVTAPIDFV YPTGVSTQTC LLVKGRTLAV NRHMAESDWS
1681 SIVVRGVTHA RSTVRILAIA KAGKETDVSF IRLSSGPLFR DNTSKFVKAD DVLPATSAPV
1741 IGIMNTDIPM MFTGTFLKAG VSVPVETGQT FNHCIHYKAN TRKGWCGSAL LADLGGKKKI
1801 LGMHSAGSMG RTAASIVSQE MICAVVSAFE PQGALERLPD GPRIHVPRKT ALRPTVARRV
1861 FQPAYAPAVL SKFDPRTEAD VDEVAFSKHT SNQESLPPVF RMVAKEYANR VFTLLGRDNG
1921 RLTVKQALEG LEGMDPMDKN TSPGLPYTAL GMRRTDVVDW ESATLIPYAA DRLKKMNEGD
1981 FSDIVYQTFL KDELRPVEKV QAAKTRIVDV PPFEHCILGR QLLGRFASKF QTQPGLELGS
2041 AIGCDPDVHW TAFGVAMQGF ERVYDVDYSN FDSTHSVAMF RLLAEEFFTP ENGFDPLVKE
2101 YLESLAISTH AFEEKRYLIT GGLPSGCAAT SMLNTIMNNI IIRAGLYLTY KNFEFDDVKV
2161 LSYGDDLLVA TNYQLNFDKV RASLAKTGYK ITPANKTSTF PLDSTLEDVV FLKRKFKKEG
2221 PLYRPVMNRE ALEAMLSYYR PGTLSEKLTS ITMLAVHSGK PEYDRLFAPF REVGVVVPSF
2281 ESVEYRWRSL FW (SEQ ID NO:26)

# Fig. 74B

```
   1 MATTMEQEIC AHSLTFKGCP KCSALQYRNG FYLLKYDEEW YPEELLTDGE DDVFDPELDM
  61 EVVFELQGNS TSSDKNNSSS DGNEGVIINN FYSNQYQNSI DLSANATGSD PPRTYGQFSN
 121 LLSGAVNAFS NMIPLLADQN TEEMENLSDR VLQDTAGNTV TNTQSTVGRL VGYGAVHDGE
 181 HPASCADTAS EKILAVERYY TFKVNDWTST QKPFEYIRIP LPHVLSGEDG GVFGAALRRH
 241 YLVKTGWPVQ VQCNASQFHA GSLLVFMAPE YPTLDAFAMD NRWSKDNLPN GTKTQTNRKG
 301 PFAMDHQNFW QWTLYPHQFL NLRTNTTVDL EVPYVNIAPT SSWTQHASWT LVIAVVAPLT
 361 YSTGASTSLD ITASIQPVRP VFNGLRHETL SRQSPIPVTI REHAGTWYST LPDSTVPIYG
 421 KTPVAPANYM VGEYKDFLEI AQIPTFIGNK IPNAVPYIEA SNTAVKTQPL ATYQVTLSCS
 481 CLANTFLAAL SRNFAQYRGS LVYTFVFTGT AMMKGKFLIA YTPPGAGKPT SRDQAMQATY
 541 AIWDLGLNSS YSFTVPFISP THFRMVGTDQ VNITNVDGWV TVWQLTPLTY PPGCPTSAKI
 601 LTMVSAGKDF SLKMPISPAP WSPQGVENAE RGVTEDTDAT ADFVAQPVYL PENQTKVAFF
 661 YDRSSPIGAF TVKSGSLESG FAPFSNETCP NSVILTPGPQ FDPAYDQLRP QRLTEIWGNG
 721 NEETSKVFPL KSKQDYSFCL FSPFVYYKCD LEVTLSPHTS GNHGLLVRWC PTGTPAKPTT
 781 QVLHEVSSLS EGRTPQVYSA GPGVSNQISF VVPYNSPLSV LPAVWYNGHK RFDNTGSLGI
 841 APNSDFGTLF FAGTKPDIKF TVYLRYKNMR VFCPRPTVFF PWPSSGDKID MTPRAGVLML
 901 ESPNALDISR TYPTLHILIQ FNHGGLEIRL FRHVQFWAEA HADVILRSRT KQISFLNNGS
 961 FPSMDARAPW NPWKNTYHAV LRAEPYRVTM DVYHKRIRPF RLPLVQKEWN VREENVFGLY
1021 GIFNAHYAGY FADLLIHDIE TNPGPFMAKP KKQVFQTQGA AVSSMAQTLL PNDLASKVMG
1081 SAFTALLDAN EDAQKAMRII KTLSSLSDAW ENVKETLNNP EFWKQLLSRC VQLIAGMTIA
1141 VMHPDPLTLL CLGTLTAAEI TSQTSLCEEI VAKFKKIFTT PPPRFPTISL FQQQSPLKQV
1201 NDVFSLAKNL DWAVKTVEKV VDWFGTWVVQ EEKEQTLDQL LQRFPEHAKR ISDLRNGMSA
1261 YVECKESFDF FEKLYNQAVK EKRTGIAAVC EKFRQKHDHA TARCEPVVIV LRGDAGQGKS
```

# Fig. 75A

1321 LSSQVIAQAV SKTIFGRQSV YSLPPDSDFF DGYENQFAAI MDDLGQNPDG SDFTTFCQMV
1381 STTNFLPNMA SLERNGTPFT SQLVVATTNL PEFRPVTIAH YPAVERRITF DYSVSAGPVC
1441 SKTEAGYKVL DVERAFRPTG DAPLPCFQNN CLFLEKAGLQ FRDNRTKEIL SLVDVIERAV
1501 ARIERKKKVL TTVQTLVAQA PVAEVSFHSV VQQLKARQEA TDEQLEELQE AFAKTQERSS
1561 VFSDWMKISA MLCAATLALT QVVKMAKTVK QMVRPDLVRV QLDEQEQGPY NEAVRAKPKT
1621 LQLLDIQGPN PVMDFEKYVA KFVTAPIDFV YPTGVSTQTC LLVKGRTLAV NRHMAESDWS
1681 SIVVRGVTHA RSTVRILAIA KAGKETDVSF IRLSSGPLFR DNTSKFVKAD DVLPATSAPV
1741 IGIMNTDIPM MFTGTFLKAG VSVPVETGQT FNHCIHYKAN TRKGWCGSAL LADLGGKKKI
1801 LGMHSAGSMG VAAASIVSQE MICAVVSAFE PQGALERLPD GPRIHVPRKT ALRPTVARQV
1861 FQPAYAPAVL SKFDPRTEAD VDEVAFSKHT SNQESLPPVF RMVAKEYANR VFTLLGRDNG
1921 RLTVKQALEG LEGMDPMDKN TSPGLPYTAL GMRRTDVVDW ESATLIPYAA DRLKKMNEGD
1981 FSDIVYQTFL KDELRPVEKV QAAKTRIVDV PPFEHCILGR QLLGRFASKF QTQPGLELGS
2041 AIGCDPDVHW TAFGVAMQGF ERVYDVDYSN FDSTHSVAMF RLLAEEFFTP ENGFDPLVKE
2101 YLESLAISTH AFEEKRYLIT GGLPSGCAAT SMLNTIMNNI IIRAGLYLTY KNFEFDDVKV
2161 LSYGDDLLVA TNYQLNFDKV RASLAKTGYK ITPANKTSTF PLDSTLEDVV FLKRKFKKEG
2221 PLYRPVMNRE ALEAMLSYYR PGTLSEKLTS ITMLAVHSGK PEYDRLFAPF REVGVVVPSF
2281 ESVEYRWRSL FW (SEQ ID NO:27)

# Fig. 75B

1 MATTMEQEIC AHSLTFKGCP KCSALQYRNG FYLLKYDEEW YPEELLTDGE DDVFDPELDM
61 EVVFELQGNS TSSDKNNSSS DGNEGVIINN FYSNQYQNSI DLSANATGSD PPRTYGQFSN
121 LLSGAVNAFS NMIPLLADQN TEEMENLSDR VLQDTAGNTV TNTQSTVGRL VGYGAVHDGE
181 HPASCADTAS EKILAVERYY TFKVNDWTST QKPFEYIRIP LPHVLSGEDG GVFGAALRRH
241 YLVKTGWRVQ VQCNASQFHA GSLLVFMAPE YPTLDAFAMD NRWSKDNLPN GTKTQTNRKG
301 PFAMDHQNFW QWTLYPHQFL NLRTNTTVDL EVPYVNIAPT SSWTQHASWT LVIAVVAPLT
361 YSTGASTSLD ITASIQPVRP VFNGLRHETL SRQSPIPVTI REHAGTWYST LPDSTVPIYG
421 KTPVAPANYM VGEYKDFLEI AQIPTFIGNK IPNAVPYIEA SNTAVKTQPL ATYQVTLSCS
481 CLANTFLAAL SRNFAQYRGS LVYTFVFTGT AMMKGKFLIA YTPPGAGKPT SRDQAMQATY
541 AIWDLGLNSS YSFTVPFISP THFRMVGTDQ VNITNVDGWV TVWQLTPLTY PPGCPTSAKI
601 LTMVSAGKDF SLKMPISPAP WSPQGVENAE RGVTEDTDAT ADFVAQPVYL PENQTKVAFF
661 YDRSSPIGAF TVKSGSLESG FAPFSNKTCP NSVILTPGPQ FDPAYDQLRP QRLTEIWGNR
721 NEETSKVFPL KSKQDYSFCL FSPFVYYKCD LEVTLSPHTS GNHGLLVRWC PTGTPAKPTT
781 QVLHEVSSLS EGRTPQVYSA GPGISNQISF VVPYNSPLSV LPAVWYNGHK RFDNTGSLGI
841 APNSDFGTLF FAGTKPDIKF TVYLRYKNMR VFCPRPTVFF PWPSSGDKID MTPRAGVLML
901 ESPNALDISR TYPTLHILIQ FNHGGLEIRL FRHGQFWAEA HADVILRSRT KQISFLNNGS
961 FPSMDARAPW NPWKNTYHAV LRAEPYRVTM DVYHKRIRPF RLPLVQKEWN VREENVFGLY
1021 SIFNAHYAGY FADLLIHDIE TNPGPFMAKP KKQVFQTQGA AVSSMAQTLL PNDLASKVMG
1081 SAFTALLDAN EDAQKAMRII KTLSSLSDAW ENVKETLNNP EFWKQLLSRC VQLIAGMTIA
1141 VMHPDPLTLL CLGTLTAAEI TSQTSLCEEI VAKFKKIFTT PPPRFPTISL FQQQSPLKQV
1201 NDVFSLAKNL DWAVKTVEKV VDWFGTWVVQ EEKEQTLDQL LQRFPEHAKR ISDLRNGMSA
1261 YVECKESFDF FEKLYNQAVK EKRTGIAAVC EKFRQKHDHA TARCEPVVIV LRGDAGQGKS
1321 LSSQVIAQAV SKTIFGRQSV YSLPPDSDFF DGYENQFAAI MDDLGQNPDG SDFTTFCQMV

# Fig. 76A

1381 STTNFLPNMA SLERKGTPFT SQLVVATTNL PEFRPVTIAH YPAVERRITF DYSVSAGPVC

1441 SKTEAGYKVL DVERAFRPTG DAPLPCFQNN CLFLEKAGLQ FRDNRTKEIL SLVDVIERAV

1501 ARIERKKKVL TTVQTLVAQA PVDEVSFHSV VQQLKARQEA TDEQLEELQE AFAKTQERSS

1561 VFSDWMKISA MLCAATLALT QVVKMAKTVK QMVRPDLVRV QLDEQEQGPY NEAVRAKPKT

1621 LQLLDIQGPN PVMDFEKYVA KFVTAPIDFV YPTGVSTQTC LLVKGRTLAV NRHMAESDWS

1681 SIVVRGVTHA RSTVRILAIA KAGKETDVSF IRLSSGPLFR DNTSKFVKAD DVLPATSAPV

1741 IGIMNTDIPM MFTGTFLKAG VSVPVETGQT FNHCIHYKAN TRKGWCGSAL LADLGGKKKI

1801 LGMHSAGSMG RTAASIVSQE MICAVVSAFE PQGALERLPD GPRIHVPRKT ALRPTVARQV

1861 FQPAYAPAVL SKFDPRTEAD VDEVAFSKHT SNQESLPPVF RMVAKEYANR VFTLLGRDNG

1921 RLTVKQALEG LEGMDPMDKN TSPGLPYTAL GMRRTDVVDW ESATLIPYAA DRLKKMNEGD

1981 FSDIVYQTFL KDELRPVEKV QAAKTRIVDV PPFEHCILGR QLLGRFASKF QTQPGLELGS

2041 AIGCDPDVHW TAFGVAMQGF ERVYDVDYSN FDSTHSVAMF RLLAEEFFTP ENGFDPLVKE

2101 YLESLAISTH AFEEKRYLIT GGLPSGCAAT SMLNTIMNNI IIRAGLYLTY KNFEFDDVKV

2161 LSYGDDLLVA TNYQLNFDKV RASLAKTGYK ITPANKTSTF PLDSTLEDVV FLKRKFKKEG

2221 PLYRPVMNRE ALEAMLSYYR PGTLSEKLTS ITMLAVHSGK PEYDRLFAPF REVGVVVPSF

2281 ESVEYRWRSL FW (SEQ ID NO:28)

# Fig. 76B

1 MATTMEQEIC AHSMTFEECP KCSALQYRNG FYLLKYDEEW YPEESLTDGE DDVFDPDLDM
61 EVVFETQGNS TSSDKNNSSS EGNEGVIINN FYSNQYQNSI DLSANATGSD PPKTYGQFSN
121 LLSGAVNAFS NMLPLLADQN TEEMENLSDR VSQDTAGNTV TNTQSTVGRL VGYGTVHDGE
181 HPASCADTAS EKILAVERYY TFKVNDWTST QKPFEYIRIP LPHVLSGEDG GVFGATLRRH
241 YLVKTGWRVQ VQCNASQFHA GSLLVFMAPE YPTLDVFAMD NRWSKDNLPN GTRTQTNRKG
301 PFAMDHQNFW QWTLYPHQFL NLRTNTTVDL EVPYVNIAPT SSWTQHASWT LVIAVVAPLT
361 YSTGASTSLD ITASIQPVRP VFNGLRHEVL SRQSPIPVTI REHAGTWYST LPDSTVPIYG
421 KTPVAPANYM VGEYKDFLEI AQIPTFIGNK VPNAVPYIEA SNTAVKTQPL AVYQVTLSCS
481 CLANTFLAAL SRNFAQYRGS LVYTFVFTGT AMMKGKFLIA YTPPGAGKPT SRDQAMQATY
541 AIWDLGLNSS YSFTVPFISP THFRMVGTDQ ANITNVDGWV TVWQLTPLTY PPGCPTSAKI
601 LTMVSAGKDF SLKMPISPAP WSPQGVENAE KGVTENTDAT ADFVAQPVYL PENQTKVAFF
661 YDRSSPIGAF AVKSGSLESG FAPFSNKACP NSVILTPGPQ FDPAYDQLRP QRLTEIWGNG
721 NEETSEVFPL KTKQDYSFCL FSPFVYYKCD LEVTLSPHTS GAHGLLVRWC PTGTPTKPTT
781 QVLHEVSSLS EGRTPQVYSA GPGTSNQISF VVPYNSPLSV LPAVWYNGHK RFDNTGDLGI
841 APNSDFGTLF FAGTKPDIKF TVYLRYKNMR VFCPRPTVFF PWPTSGDKID MTPRAGVLML
901 ESPNPLDVSK TYPTLHILLQ FNHRGLEARI FRHGQLWAET HAEVVLRSKT KQISFLSNGS
961 YPSMDATTPL NPWKSTYQAV LRAEPHRVTM DVYHKRIRPF RLPLVQKEWR TCEENVFGLY
1021 HVFETHYAGY FSDLLIHDVE TNPGPFTFKP RQRPVFQTQG AAVSSMAQTL LPNDLASKAM
1081 GSAFTALLDA NEDAQKAMKI IKTLSSLSDA WENVKGTLNN PEFWKQLLSR CVQLIAGMTI
1141 AVMHPDPLTL LCLGVLTAAE ITSQTSLCEE IAAKFKTIFT TPPPRFPVIS LFQQQSPLKQ
1201 VNDVFSLAKN LDWAVKTVEK VVDWFGTWVA QEEREQTLDQ LLQRFPEHAK RISDLRNGMA
1261 AYVECKESFD FFEKLYNQAV KEKRTGIAAV CEKFRQKHDH ATARCEPVVI VLRGDAGQGK

# Fig. 77A

1321 SLSSQIIAQA VSKTIFGRQS VYSLPPDSDF FDGYENQFAA IMDDLGQNPD GSDFTTFCQM
1381 VSTTNLLPNM ASLERKGTPF TSQLVVATTN LPEFRPVTIA HYPAVERRIT FDYSVSAGPV
1441 CSKTEAGCKV LDVERAFRPT GDAPLPCFQN NCLFLEKAGL QFRDNRSKEI LSLVDVIERA
1501 VTRIERKKKV LTAVQTLVAQ GPVDEVSFYS VVQQLKARQE ATDEQLEELQ EAFARVQERS
1561 SVFSDWMKIS AMLCAATLAL TQVVKMAKAV KQMVRPDLVR VQLDEQEQGP YNETTRIKPK
1621 TLQLLDVQGP NPTMDFEKFV AKFVTAPIGF VYPTGVSTQT CLLVKGRTLA VNRHMAESDW
1681 TSIVVRGVSH TRSSVKIIAI AKAGKETDVS FIRLSSGPLF RDNTSKFVKA SDVLPHSSSP
1741 LIGIMNVDIP MMYTGTFLKA GVSVPVETGQ TFNHCIHYKA NTRKGWCGSA ILADLGGSKK
1801 ILGFHSAGSM GVAAASIISQ EMIDAVVQAF EPQGALERLP DGPRIHVPRK TALRPTVARQ
1861 VFQPAFAPAV LSKFDPRTDA DVDEVAFSKH TSNQETLPPV FRMVAREYAN RVFALLGRDN
1921 GRLSVKQALD GLEGMDPMDK NTSPGLPYTT LGMRRTDVVD WETATLIPFA AERLEKMNNK
1981 DFSDIVYQTF LKDELRPIEK VQAAKTRIVD VPPFEHCILG RQLLGKFASK FQTQPGLELG
2041 SAIGCDPDVH WTAFGVAMQG FERVYDVDYS NFDSTHSVAI FRLLAEEFFS EENGFDPLVK
2101 DYLESLAISK HAYEEKRYLI TGGLPSGCAA TSMLNTIMNN IIIRAGLYLT YKNFEFDDVK
2161 VLSYGDDLLV ATNYQLNFDR VRTSLAKTGY KITPANKTST FPLESTLEDV VFLKRKFKKE
2221 GPLYRPVMNR EALEAMLSYY RPGTLSEKLT SITMLAVHSG KQEYDRLFAP FREVGVIVPT
2281 FESVEYRWRS LFW (SEQ ID NO:29)

# Fig. 77B

1 MACKHGYPDV CPICTAVDVT PGFEYLLLAD GEWFPTDLLC VDLDDDVFWP SNSSNQSETM
61 EWTDLPLVRD IVMEPQGNAS SSDKSNSQSS GNEGVIINNF YSNQYQNSID LSASGGNAGD
121 APQNNGQLSN ILGGAANAFA TMAPLLLDQN TEEMENLSDR VASDKAGNSA TNTQSTVGRL
181 CGYGEAHHGE HPASCADTAT DKVLAAERYY TIDLASWTTT QEAFSHIRIP LPHVLAGEDG
241 GVFGATLRRH YLCKTGWRVQ VQCNASQFHA GSLLVFMAPE FYTGKGTKTG DMEPTDPFTM
301 DTTWRAPQGA PTGYRYDSRT GFFAMNHQNQ WQWTVYPHQI LNLRTNTTVD LEVPYVNIAP
361 TSSWTQHANW TLVVAVFSPL QYASGSSSDV QITASIQPVN PVFNGLRHET VIAQSPIAVT
421 VREHKGCFYS TNPDTTVPIY GKTISTPNDY MCGEFSDLLE LCKLPTFLGN PNSNNKRYPY
481 FSATNSVPTT SLVDYQVALS CSCMCNSMLA AVARNFNQYR GSLNFLFVFT GAAMVKGKFL
541 IAYTPPGAGK PTTRDQAMQA TYAIWDLGLN SSFVFTAPFI SPTHYRQTSY TSATIASVDG
601 WVTVWQLTPL TYPSGAPVNS DILTLVSAGD DFTLRMPISP TKWAPQGSDN AEKGKVSNDD
661 ASVDFVAEPV KLPENQTRVA FFYDRAVPIG MLRPGQNIES TFVYQENDLR LNCLLLTPLP
721 SFCPDSTSGP VKTKAPVQWR WVRSGGTTNF PLMTKQDYAF LCFSPFTYYK CDLEVTVSAL
781 GTDTVASVLR WAPTGAPADV TDQLIGYTPS LGETRNPHMW LVGAGNTQIS FVVPYNSPLS
841 VLPAAWFNGW SDFGNTKDFG VAPNADFGRL WIQGNTSASV RIRYKKMKVF CPRPTLFFPW
901 PVSTRSKINA DNPVPILELE NPAAFYRIDL FITFIDEFIT FDYKVHGRPV LTFRIPGFGL
961 TPAGRMLVCM GEKPAHGPFT SSRSLYHVIF TATCSSFSFS IYKGRYRSWK KPIHDELVDR
1021 GYTTFGEFFR AVRAYHADYY KQRLIHDVEM NPGPVQSVFQ PQGAVLTKSL APQAGIQNLL
1081 LRLLGIDGDC SEVSKAITVV TDLFAAWERA KTTLVSPEFW SKLILKTTKF IAASVLYLHN
1141 PDFTTTVCLS LMTGVDLLTN DSVFDWLKNK LSSFFRTPPP VCPNVLQPQG PLREANEGFT
1201 FAKNIEWAMK TIQSIVNWLT SWFKQEEDHP QSKLDKFLME FPDHCRNIMD MRNGRKAYCE
1261 CTASFKYFDE LYNLAVTCKR IPLASLCEKF KNRHDHSVTR PEPVVVVLRG AAGQGKSVTS
1321 QIIAQSVSKM AFGRQSVYSM PPDSEYFDGY ENQFSVIMDD LGQNPDGEDF TVFCQMVSST

# Fig. 78A

1381 NFLPNMAHLE RKGTPFTSSF IVATTNLPKF RPVTVAHYPA VDRRITFDFT VTAGPHCTTS

1441 NGMLDIEKAF DEIPGSKPQL ACFSADCPLL HKRGVMFTCN RTKAVYNLQQ VVKMVNDTIT

1501 RKTENVKKMN SLVAQSPPDW EHFENILTCL RQNNAALQDQ LDELQEAFAQ ARERSDFLSD

1561 WLKVSAIIFA GIASLSAVIK LASKFKESIW PSPVRVELSE GEQAAYAGRA RAQKQALQVL

1621 DIQGGGKVLA QAGNPVMDFE LFCAKNMVAP ITFYYPDKAE VTQSCLLLRA HLFVVNRHVA

1681 ETEWTAFKLK DVRHERDTVV TRSVNRSGAE TDLTFIKVTK GPLFKDNVNK FCSNKDDFPA

1741 RNDAVTGIMN TGLAFVYSGN FLIGNQPVNT TTGACFNHCL HYRAQTRRGW CGSAVICNVN

1801 GKKAVYGMHS AGGGGLAAAT IITRELIEAA EKSMLALEPQ GAIVDISTGS VVHVPRKTKL

1861 RRTVAHDVFQ PKFEPAVLSR YDPRTDKDVD VVAFSKHTTN MESLPPVFDI VCDEYANRVF

1921 TILGKDNGLL TVEQAVLGLP GMDPMEKDTS PGLPYTQQGL RRTDLLNFNT AKMTPQLDYA

1981 HSKLVLGVYD DVVYQSFLKD EIRPLEKIHE AKTRIVDVPP FAHCIWGRQL LGRFASKFQT

2041 KPGLELGSAI GTDPDVDWTP YAAELSGFNY VYDVDYSNFD ASHSTAMFEC LIKNFFTEQN

2101 GFDRRIAEYL RSLAVSRHAY EDRRVLIRGG LLSGCAATSM LNTIMNNVII RAALYLTYSN

2161 FEFDDIKVLS YGDDLLIGTN YQIDFNLVKE RLAPFGYKIT PANKTTTFPL TSHLQDVTFL

2221 KRRFVRFNSY LFRPQMDAVN LKAMVSYCKP GTLKEKLMSI ALLAVHSGPD IYDEIFLPFR

2281 NVGIVVPTYS SMLYRWLSLF R (SEQ ID NO:30)

# Fig. 78B

1 MACKHGYPDV CPICTAVDAT PDFEYLLMAD GEWFPTDLLC VDLDDDVFWP SDTSTQPQTM
61 EWTDVPLVCD TVMEPQGNAS SSDKSNSQSS GNEGVIINNF YSNQYQNSID LSASGGNAGD
121 APQNNGQLSS ILGGAANAFA TMAPLLMDQN TEEMENLSDR VASDKAGNSA TNTQSTVGRL
181 CGYGKSHHGE HPTSCADAAT DKVLAAERYY TIDLASWTTS QEAFSHIRIP LPHVLAGEDG
241 GVFGATLRRH YLCKTGWRVQ VQCNASQFHA GSLLVFMAPE FYTGKGTKSG TMEPSDPFTM
301 DTTWRSPQSA PTGYRYDRQA GFFAMNHQNQ WQWTVYPHQI LNLRTNTTVD LEVPYVNVAP
361 SSSWTQHANW TLVVAVLSPL QYATGSSPDV QITASLQPVN PVFNGLRHET VLAQSPIPVT
421 VREHQGCFYS TNPDTTVPIY GKTISTPSDY MCGEFSDLLE LCKLPTFLGN PSTDNKRYPY
481 FSATNSVPAT SLVDYQVALS CSCTANSMLA AVARNFNQYR GSLNFLFVFT GAAMVKGKFR
541 IAYTPPGAGK PTTRDQAMQA TYAIWDLGLN SSFNFTAPFI SPTHYRQTSY TSPTITSVDG
601 WVTVWQLTPL TYPSGTPTHS DILTLVSAGD DFTLRMPISP TKWVPQGIDN AEKGKVSNDD
661 ASVDFVAEPV KLPENQTRVA FFYDRAVPIG MLRPGQNMET TFSYQENDFR LNCLLLTPLP
721 SYCPDSSSGP VRTKAPVQWR WVRSGGANGA NFPLMTKQDY AFLCFSPFTY YKCDLEVTVS
781 AMGAGTVSSV LRWAPTGAPA DVTDQLIGYT PSLGETRNPH MWIVGSGNSQ ISFVVPYNSP
841 LSVLPAAWFN GWSDFGNTKD FGVAPTSDFG RIWIQGNSSA SVRIRYKKMK VFCPRPTLFF
901 PWPTPTTTKI NADNPVPILE LENPASLYRI DLFITFTDEL ITFDYKVHGR PVLTFRIPGF
961 GLTPAGRMLV CMGAKPAHSP FTSSKSLYHV IFTSTCNSFS FTIYKGRYRS WKKPIHDELV
1021 DRGYTTFREF FKAVRGYHAD YYKQRLIHDV EMNPGPVQSV FQPQGAVLTK SLAPQAGIQN
1081 ILLRLLGIEG DCSEVSKAIT VVTDLVAAWE KAKTTLVSPE FWSELILKTT KFIAASVLYL
1141 HNPDFTTTVC LSLMTGVDLL TNDSVFDWLK SKLSSFFRTP PPACPNVMQP QGPLREANEG
1201 FTFAKNIEWA TKTIQSIVNW LTSWFKQEED HPQSKLDKLL MEFPDHCRNI MDMRNGRKAY
1261 CECTASFKYF DDLYNLAVTC KRIPLASLCE KFKNRHDHSV TRPEPVVAVL RGAAGQGKSV
1321 TSQIIAQSVS KMAFGRQSVY SMPPDSEYFD GYENQFSVIM DDLGQNPDGE DFTVFCQMVS
1381 STNFLPNMAH LERKGTPFTS SFIVATTNLP KFRPVTVAHY PAVDRRITFD FTVTAGPHCK

# Fig. 79A

1441 TPAGMLDIEK AFDEIPGSKP QLACFSADCP LLHKRGVMFT CNRTKTVYNL QQVVKMVNDT

1501 ITRKTENVKK MNSLVAQSPP DWQHFENILT CLRQNNAALQ DQVDELQEAF TQARERSDFL

1561 SDWLKVSAII FAGIVSLSAV IKLASKFKES IWPTPVRVEL SEGEQAAYAG RARAQKQALQ

1621 VLDIQGGGKV LAQAGNPVMD FELFCAKNMV SPITFYYPDK AEVTQSCLLL RAHLFVVNRH

1681 VAETEWTAFK LRDVRHERDT VVMRSVNRSG AETDLTFVKV TKGPLFKDNV NKFCSNKDDF

1741 PARNDTVTGI MNTGLAFVYS GNFLIGNQPV NTTTGACFNH CLHYRAQTRR GWCGSAIICN

1801 VNGKKAVYGM HSAGGGGLAA ATIITRELIE AAEKSMLALE PQGAIVDIST GSVVHVPRKT

1861 KLRRTVAHDV FQPKFEPAVL SRYDPRTDKD VDVVAFSKHT TNMESLPPIF DIVCGEYANR

1921 VFTILGKDNG LLTVEQAVLG LSGMDPMEKD TSPGLPYTQQ GLRRTDLLDF NTAKMTPQLD

1981 YAHSKLVLGV YDDVVYQSFL KDEIRPLEKI HEAKTRIVDV PPFAHCIWGR QLLGRFASKF

2041 QTKPGFELGS AIGTDPDVDW TRYAAELSGF NYVYDVDYSN FDASHSTAMF ECLINNFFTE

2101 QNGFDRRIAE YLRSLAVSRH AYEDRRVLIR GGLPSGCAAT SMLNTIMNNV IIRAALYLTY

2161 SNFEFDDIKV LSYGDDLLIG TNYQIDFNLV KERLAPFGYK ITPANKTTTF PLTSHLQDVT

2221 FLKRRFVRFN SYLFRPQMDA VNLKAMVSYC KPGTLKEKLM SIALLAVHSG PDIYDEIFLP

2281 FRNVGIVVPT YDSMLYRWLS LFR (SEQ ID NO:31)

# Fig. 79B

1 MACKHGYPDV CPICTAVDAT PGFEYLLMAD GEWYPTDLLC VDLDDDVFWP SDTSNQSQTM
61 DWTDVPLIRD IVMEPQGNSS SSDKSNSQSS GNEGVIINNF YSNQYQNSID LSASGGNAGD
121 APQTNGQLSN ILGGAANAFA TMAPLLLDQN TEEMENLSDR VASDKAGNSA TNTQSTVGRL
181 CGYGKSHHGE HPASCADTAT DKVLAAERYY TIDLASWTTS QEAFSHIRIP LPHVLAGEDG
241 GVFGATLRRH YLCKTGWRVQ VQCNASQFHA GSLLVFMAPE FYTGKGTKTG TMEPSDPFTM
301 DTEWRSPQGA PTGYRYDSRT GFFATNHQNQ WQWTVYPHQI LNLRTNTTVD LEVPYVNVAP
361 SSSWTQHANW TLVVAVLSPL QYATGSSPDV QITASLQPVN PVFNGLRHET VIAQSPIPVT
421 VREHKGCFYS TNPDTTVPIY GKTISTPSDY MCGEFSDLLE LCKLPTFLGN PNTNNKRYPY
481 FSATNSVPAT SMVDYQVALS CSCMANSMLA AVARNFNQYR GSLNFLFVFT GAAMVKGKFL
541 IAYTPPGAGK PTTRDQAMQS TYAIWDLGLN SSFNFTAPFI SPTHYRQTSY TSPTITSVDG
601 WVTVWKLTPL TYPSGTPTNS DILTLVSAGD DFTLRMPISP TKWVPQGVDN AEKGKVSNDD
661 ASVDFVAEPV KLPENQTRVA FFYDRAVPIG MLRPGQNMET TFNYQENDYR LNCLLLTPLP
721 SFCPDSSSGP QKTKAPVQWR WVRSGGVNGA NFPLMTKQDY AFLCFSPFTF YKCDLEVTVS
781 ALGMTRVASV LRWAPTGAPA DVTDQLIGYT PSLGETRNPH MWLVGAGNSQ VSFVVPYNSP
841 LSVLPAAWFN GWSDFGNTKD FGVAPNADFG RLWIQGNTSA SVRIRYKKMK VFCPRPTLFF
901 PWPTPTTTKI NADNPVPILE LENPAALYRI DLFITFTDEF ITFDYKVHGR PVLTFRIPGF
961 GLTPAGRMLV CMGEQPAHGP FTSSRSLYHV IFTATCSSFS FSIYKGRYRS WKKPIHDELV
1021 DRGYTTFGEF FKAVRGYHAD YYRQRLIHDV ETNPGPVQSV FQPQGAVLTK SLAPQAGIQN
1081 LLLRLLGIDG DCSEVSKAIT VVTDLVAAWE KAKTTLVSPE FWSKLILKTT KFIAASVLYL
1141 HNPDFTTTVC LSLMTGVDLL TNDSVFDWLK QKLSSFFRTP PPACPNVMQP QGPLREANEG
1201 FTFAKNIEWA MKTIQSVVNW LTSWFKQEED HPQSKLDKLL MEFPDHCRNI MDMRNGRKAY
1261 CECTASFKYF DELYNLAVTC KRIPLASLCE KFKNRHDHSV TRPEPVVVVL RGAAGQGKSV
1321 TSQIIAQSVS KMAFGRQSVY SMPPDSEYFD GYENQFSVIM DDLGQNPDGE DFTVFCQMVS
1381 STNFLPNMAH LERKGTPFTS SFIVATTNLP KFRPVTVAHY PAVDRRITFD FTVTAGPHCK

# Fig. 80A

1441 TPAGMLDVEK AFDEIPGSKP QLACFSADCP LLHKRGVMFT CNRTQTVYNL QQVVKMVNDT

1501 ITRKTENVKK MNSLVAQSPP DWEHFENILT CLRQNNAALQ DQLDELQEAF AQARERSDFL

1561 SDWLKVSAII FAGIASLSAV IKLASKFKES IWPTPVRVEL SEGEQAAYAG RARAQKQALQ

1621 VLDIQGGGKV LAQAGNPVMD FELFCAKNIV APITFYYPDK AEVTQSCLLL RAHLFVVNRH

1681 VAETDWTAFK LKDVRHERHT VALRSVNRSG AKTDLTFIKV TKGPLFKDNV NKFCSNKDDF

1741 PARNDTVTGI MNTGLAFVYS GNFLIGNQPV NTTTGACFNH CLHYRAQTRR GWCGSAIICN

1801 VNGKKAVYGM HSAGGGGLAA ATIITKELIE AAEKSMLALE PQGAIVDIAT GSVVHVPRKT

1861 KLRRTVAHDV FQPKFEPAVL SRYDPRTDKD VDVVAFSKHT TNMESLPPIF DVVCGEYANR

1921 VFTILGKENG LLTVEQAVLG LPGMDPMEKD TSPGLPYTQQ GLRRTDLLNF ITAKMTPQLD

1981 YAHSKLVIGV YDDVVYQSFL KDEIRPIEKI HEAKTRIVDV PPFAHCIWGR QLLGRFASKF

2041 QTKPGLELGS AIGTDPDVDW TRYAVELSGF NYVYDVDYSN FDASHSTAMF ECLINNFFTE

2101 QNGFDRRIAE YLRSLAVSRH AYEDRRVLIR GGLPSGCAAT SMLNTIMNNV IIRAALYLTY

2161 SNFDFDDIKV LSYGDDLLIG TNYQIDFNLV KERLAPFGYK ITPANKTTTF PLTSHLQDVT

2221 FLKRRFVRFN SYLFRPQMDA VNLKAMVSYC KPGTLKEKLM SIALLAVHSG PDIYDEIFLP

2281 FRNVGIVVPT YSSMLYRWLS LFR (SEQ ID NO:32)

# Fig. 80B

1 MMACIHGYPS VCPICTAIDK SSDGMYLLLA DNEWFPADLL TMDLDDDVFW PNDESDVSET
61 MDWTDLPFIL DTIMEPQGNS TSSDKSNSQS SGNEGVIINN FYSNQYQNSI DLSANGGNAG
121 GAPKTEGQLG NILGNAANAF STMAPLLLDQ NTEEMENLSD RVDSDKAGNS AVNTQSSVGR
181 LCGYGMHHKG KHPASCADTA TDKVLSAERY YTIDLATWTT TLGTFSHIRI PLPHVLAGED
241 GGVFGSTLRR HYLCKCGWRI QVQCNASQFH AGSLLVFMAP EFYTGHTPVT GTTEPATPFT
301 MDSSWQTPQQ NPVGFRYDGR TGYFALNHQN YWQWMVYPHQ ILNLRTNTSV DLEVPFTNIA
361 PTSSWTQHAN WTLVVAVLTP LQYAAGSATD VQITASIQPV KPVFNGLRHE AVVPQSPIPV
421 TVREHQGTFY STNPDTTVPI YGKTIATPSD YMCGEFSDLV ELCKLPTFLG NPANTSPAGG
481 RYPYFSATNS VPATALASYQ VALSCSCMSN SMLAAVARNF NQYRGSLNFL FVFTGTAMTK
541 GKFLIAYTPP GAGKPTTREQ AMQATYAIWD LGLNSSYNFT VPFISPTHYR QTSYTSTSIT
601 SVDGWLTVWQ LTPLTYPANT PPNADILTLV SAGDDFTLRM PISPTKWIPQ GVDNAEKGKV
661 SNDDATVDFV AEPVKFPDNQ TKVSFFYDRS VPLGLLRPAQ GMEQDFAYAA NDSRANSILL
721 TPLPSYAPDS TTGPTETQAP IQWRWLRGTS DGSTTFPLMT KQDYAFLLFS PFTYYKADLE
781 VTLSAISNSN NVTVVRWAPT GAPADISRQL SGYTPSIGDT RDPHLWFVGA GNSQTSFVVP
841 YNSPLSVLPA AWFNGWSDFG NTKDFGVAPN ADFGRLWIQG NTSVAVRVRY KKMKVFCPRP
901 TLFLPWPSTT TTRIHADNPV SVMELQNPFS FYRVDLFITF TDELITFDYK VHGRPVLQYQ
961 VPGLGLTCAG RMLVCMGQMP NHAPFSTVRH LYHVVFTGSR NSFGVVIYYK RHRPWKKPLH
1021 EELHDYGFEC FSDFFKHVRE YHAAYYKQRL MHDVETNPGP PVQSVFRPQG GVLTKSQAPM
1081 SGIQNLFLRA LGIDADHGEF TRAVTMITDL CNTWEKAKNT LVSPEFWTVL IMKTVKFIAA
1141 SVLYLHNPDL TATICLSLMT GVDVLTNESI FNWLSNKLSK LFHTPPPPTS PLLQAQSPLR
1201 EANDGFNLAK NIEWAIKTVQ KIVDWLMSWF KQEEAHPQAK LDKMLADFPE HCASILAMRN
1261 GRKAYTDCAG AFKYFEDLYN LAVQCKRIPL ATLCEKFKNK HDHAVARPEP VVVVLRGNAG
1321 QGKSVTSQII AQAVSKLAFG RQSVYSIPPD SDYLDGYENQ FSVIMDDLGQ NPDGEDFKVF
1381 CQMVSSTNFL PNMAHLEKKG TPFTSNFIVA TTNLPKFRPV TVAHYPAVDR RITFDLTVEA

# Fig. 81A

1441 GPACKTPTGM LDVEKAFQEI PGEPQLDCFS SDCALLHKRG VQFICNRTKK IYNLQQIVKM

1501 VKDTIDNKVA NLKKMNTLVA QSPNNGNDME HIITCLRQNN AALQDQIDEL QEAFAQAQER

1561 QNFLSDWMKV SAIIFAGIAS LSAVCKLVGR LKNLIWPSPV HVELSEGEQA AYAGAKRGAK

1621 QALQVLDLQG GGRIIAQAGN PVMDYEVCVA KNMVAPITFY YADKAQVTQS CLLVKGRLFV

1681 VNRHVAETDW VSFELRDVRH ERDTVTMRSV NRSGMEVDLT FIKVTKGPLF KDNTKKFCSN

1741 KDDFPQKNET VTGIMNTGLP FVFNGKFIIG NHPVNTTTGA TFNHCLHYRA NTRRGWCGSA

1801 VICQVNGKKA VYGMHSAGGG GLAAATIITQ ELVEAAEQNM DRLVPQGAIM EIGTGSVVHV

1861 PRKTKLRRTV AHEIFLPKFE PAVLSRYDPR TEKDVDQVAF SKHTTNMEEL PAVFSMVAKE

1921 YANRVFTKLG KENQLLTTQQ AILGLPGMDP MEKDTSPGLP YTQQGLRRTD LVNFETGKMD

1981 HNLDYAHSKL MLGHYEDVVY QSFLKDEIRP IEKIHEAKTR IVDVPPFHHC IWGRQLLGRF

2041 ASRFQTNPGL DLGSAIGTDP DVDWTVFAHQ LAEFKYIYDV DYSNFDASHS TAIFEILIQE

2101 FFTPQNGFDP RIGEYLRSLA VSRHAYEDRR VLIRGGLPSG CAATSMINTI INNIVIRAAL

2161 YMTYANFEFD DIKVLSYGDD LLIATNYEIN FNLVKERLAP FNYKITPANK TSTFPQTSHL

2221 QDVVFLKRRF VQFNSFLFRP QMETENLKAM VSYCRPGVLK EKLMSIALLA VHSGPDVYDE

2281 IFMPFRRIGV VVPEYSTMLY RWLNLFR (SEQ ID NO:33)

# Fig. 81B

1 MACKHGYPDV CPICTAIDVT PGFEYLLLAD GEWFPTDLLC VDLDDDVFWP SDSSNQSQTM
61 EWTDIPLICD TVMEPQGNST SSDKSNSQSS GNEGVIINNF YSNQYQNSID LSANGGNAGD
121 GPKTEGQLSN ILGGAANAFA TMAPLLLDEN TEEMENLSDR VDSDKAGNSA TNTQSSVGRL
181 HGYGATHRGD HPASCADTAT DKVLAAERYY TIDLATWTTA QTTFSHIRVP LPHALAGEHG
241 GVFGATLRRH YLAKCGWRVQ VQCNASQFHA GSLLVFLAPE FYTGTGVATS GQEPNKVFLM
301 DTTWQEPQAA PTGFRYDGKN GFFTLNHQNY WQWTVYPHQI LNLRTNTSVD LEVPYVNVAP
361 TSSWTQHANW ALVVAVLTPL QYSTGAATDV AITVSLQPVN PVFNGLRHEA QVPQSPVAVT
421 VREHQGSFYS TNPDTTVPIY GKTIVTPSDY MCGEFTDLLE LCKLPTFLGN LSNDTRVPFF
481 TATNSVPTES LVEYQVTLSC SCMSNSMLAS VARNFNQYRG SLNFLFVFTG SAMTKGKFLI
541 AYTPPGAGKP TTRDQAXQST YAIWDLGLNS SYNFTVPFIS PSHYRQTSYT SPSIAAVDGW
601 LTVWQLTPLT FPANVPPSSD ILTLVSAGND FTLRMPISPT KWIPQGVDNA EKGKVSDDNA
661 SVDFVAEPIK LPENQTRVNF FYDRSSPIGL LRPNQAIESN FSYSADSNGA TNCALLTPLP
721 SYSPDRPGQS PDTSKAPIQW RWISAVTESG TVSNTFPTRT RQDYAFLLFS PFTYYKCDLE
781 VTLSSVGNGV VASLVRWAPT GAPADITTQL TTSTPSIGDT RDPHMWLVGA GNSQTSFVIP
841 YNSPLSVLPA AWFNGWSNFS NTYDFGIAPC SDFGRLWIQG NAPLAIRVRY KKMRVFCPRP
901 TLFFPWPTPT TTKVNADNPV PILDLENPAA (SEQ ID NO:34)

# Fig. 82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4036945 A, Goldenberg
- US 4331647 A


### Non-patent literature cited in the description


- **ALTSCHUL, S.F. ; GISH, W. ; MILLER, W. ; MYERS, E.W. ; LIPMAN, D.J.** Basic local alignment search tool. *J. Mol. Biol.,* 1990, vol. 215, 403-410
- **ALTSCHUL et al.** *J. Mol. Biol.,* 1990, vol. 215, 403-410
- Cardioviruses (Picornaviridae). **SCRABA, D. et al.** Encyclopedia of Virology. 1999
- **PEVEAR et al.** *J. Virol.,* 1987, vol. 61, 1507-1516
- **FALLAUX et al.** *Human Gene Therapy,* 1998, vol. 9, 1909-1917
- **ARAP, W. et al.** *Nat. Med.,* 2002, vol. 8 (2), 121-127
- **KOLONIN, M. et al.** *Curr. Opin. Chem. Biol.,* 2001, vol. 5 (3), 308-313
- **ST. CROIX, B. et al.** *Science,* 2000, vol. 289 (5482), 1997-1202
- **ARAP, W. et al.** *Science,* 1998, vol. 279 (5349), 377-380
- **ELLERBY, H.M. et al.** *Nat. Med.,* 1999, vol. 17 (8), 768-774
- **KOIVUNEN, E. et al.** *Nat. Biotechnol,* 1999, vol. 17 (8), 768-774
- **CURNIS, F. et al.** *Nat. Biotechnol.,* 2000, vol. 18 (11), 1185-1190
- **HONG. F.D. ; CLAYMAN, G.L.** *Cancer Res.,* 2000, vol. 60 (23), 6551-6556
- **TREPEL, M. et al.** *Hum. Gene Ther.,* 2000, vol. 11 (14), 1971-1981
- **HUSE et al.** *Science,* 1989, vol. 246, 1275-1281
- **WINTER ; HARRIS.** *Immunol. Today,* 1993, vol. 14, 243-246
- **WARD et al.** *Nature,* 1989, vol. 341, 544-546
- **HARLOW ; LANE.** Antibodies: A laboratory manual. Cold Spring Harbor Laboratory Press, 1988
- **HILYARD et al.** Protein Engineering: A practical approach. IRL Press, 1992
- **BORRABECK.** Antibody Engineering. Oxford University Press, 1995
- Production of Polyclonal Antisera. **GREEN et al.** Immunochemical Protocols. Humana Press, 1992, 1-5
- Production of Polyclonal Antisera in Rabbits, Rats, Mice and Hamsters. **COLIGAN et al.** Curr. Protocols Immunol. 1992
- **KOHLER ; MILSTEIN.** *Nature,* 1975, vol. 256, 495
- **BARNES et al.** Meth. Mol. Biol. Humana Press, 1992, vol. 10, 79-104
- **NISONHOFF et al.** *Arch. Biochem. Biophys.,* 1960, vol. 89, 230
- **PORTER.** *Biochem. J.,* 1959, vol. 73, 119
- **EDELMAN et al.** Meth. Enzymol. Academic Press, 1967, vol. 1, 422
- **LARRICK et al.** *Methods: A Companion to Methods in Enzymology,* 1991, vol. 2, 106
- Cardioviruses (Picornaviridae). **SCRABA D.G. ; PALMENBERG, A.C.** Encyclopedia of Virology. 1999
- **DONNELLY et al.** *J. Gen. Virol.,* 1997, vol. 78, 13-21
- **WUTZ et al.** *J. Gen. Virol.,* 1996, vol. 77, 1719-1730
- **ALLAIRE et al.** *Nature,* 1994, vol. 369, 72-76
- **BERGMANN et al.** *J. Virol.,* 1997, vol. 71, 2436-2448
- **MOSIMANN et al.** *J. Mol. Biol.,* 1997, vol. 273, 1032-1047
- **MATTHEWS et al.** *Cell,* 1994, vol. 77, 761-771
- **MATTHEWS et al.** *Proc. Natl. Acad. Sci. USA,* 1999, vol. 96, 11000-11007
- **CHOW et al.** *Nature,* 1987, vol. 327, 482-486
- **PALMENBERG.** Molecular Aspects of Picornavirus Infection and Detection. Amer. Soc. for Micro, 1989, 211-241
- **HALL.** *Nucl. Acids. Symp. Ser.,* 1999, vol. 41, 95-98
- **THOMPSON et al.** *Nucl. Acids Res.,* 1997, vol. 25, 4876-4882
- **SATIOU ; NEI.** *Mol. Biol. Evol.,* 1987, vol. 4, 406-425
- **MARVIL et al.** *J. Gen. Virol.,* 1999, vol. 80, 653-662
- **PEVEAR et al.** *J. Gen. Virol.,* 1987, vol. 61, 1507-1516

- **KIYAZAKI, K.** *Nucleic Acids Res.,* 2002, vol. 30 (24), 139
- **VIRNEKAS, B. et al.** *Nucleic Acids Res,* 1994, vol. 22 (25), 5600-5607